# EUROPEAN PATENT SPECIFICATION

(11) **EP 3 757 214 B1**
(45) Date of publication and mention of the grant of the patent: **15.06.2022**
(21) Application number: 20176637.5
(22) Date of filing: 01.04.2015
(51) Int. Cl.: C12N 15/113, C12N 15/11, A61K 31/713, A61K 48/00, A61P 25/28, C12Q 1/68, C07H 21/02, C07H 21/04

(54) **COMPOSITIONS FOR MODULATING SOD-1 EXPRESSION**
ZUSAMMENSETZUNGEN ZUR MODULIERUNG DER SOD-1-EXPRESSION
COMPOSITIONS POUR MODULER L'EXPRESSION DE SOD-1

(30) Priority: 01.04.2014 US 201461973803 P
(43) Date of publication of application: 30.12.2020
(62) Divisional of application: 15773965.7
(73) Proprietor: Biogen MA Inc., Cambridge, MA 02142 (US)
(72) Inventor: SWAYZE, Eric, E., Carlsbad, CA 92010 (US); COLE, Tracy, Carlsbad, CA 92010 (US); KORDASIEWICZ, Holly, Carlsbad, CA 2010 (US); FREIER, Susan, M., Carlsbad, CA 92010 (US); CONDON, Thomas, P., 308215 Singapore (SG); WANCEWICZ, Edward, Carlsbad, CA 92010 (US); LOCKHART, Trisha, Mukilteo, WA 98275 (US); VICKERS, Timothy, Carlsbad, CA 92010 (US); SINGH, Priyam, Carlsbad, CA 92009 (US)
(74) Representative: Carpmaels & Ransford LLP

(56) References cited:
- WO-A2-2006/066203
- WO-A2-2009/102427
- US-A1- 2009 130 195

## Description

### Sequence Listing

The present application is being filed along with a Sequence Listing in electronic format. The Sequence Listing is provided as a file entitled BIOL0240WOSEQ_ST25.pdf created March 30, 2015, which is 320 Kb in size.

### Field

Disclosed are compositions and methods for reducing expression of superoxide dismutase 1, soluble (SOD-1) mRNA and protein in an animal. Such methods are useful to treat, prevent, or ameliorate neurodegenerative diseases, including amyotrophic lateral sclerosis (ALS) by inhibiting expression of SOD-1 in an animal.

### Background

The soluble SOD-1 enzyme (also known as Cu/Zn superoxide dismutase) is one of the superoxide dismutases that provide defense against oxidative damage of biomolecules by catalyzing the dismutation of superoxide to hydrogen peroxide (H2O2) (Fridovich, Annu. Rev. Biochem., 1995, 64, 97-112). The superoxide anion (O2-) is a potentially harmful cellular by-product produced primarily by errors of oxidative phosphorylation in mitochondria (Turrens, J. Physiol. 2003, 552, 335-344)

Mutations in the SOD-1 gene are associated with a dominantly-inherited form of amyotrophic lateral sclerosis (ALS, also known as Lou Gehrig's disease) a disorder characterized by a selective degeneration of upper and lower motor neurons (Rowland, N. Engl. J. Med. 2001, 344, 1688-1700). There is a tight genetic linkage between familial ALS and missense mutations in the SOD1 gene (Rosen, Nature, 1993, 362, 59-62). The toxicity of mutant SOD1 is believed to arise from an initial misfolding (gain of function) reducing nuclear protection from the active enzyme (loss of function in the nuclei), a process that may be involved in ALS pathogenesis (Sau, Hum. Mol. Genet. 2007, 16, 1604-1618).

ALS is a devastating progressive neurodegenerative disease affecting as many as 30,000 Americans at any given time. The progressive degeneration of the motor neurons in ALS eventually leads to their death. When the motor neurons die, the ability of the brain to initiate and control muscle movement is lost. With voluntary muscle action progressively affected, patients in the later stages of the disease may become totally paralyzed.

Currently lacking are acceptable options for treating such neurodegenerative diseases. It is therefore an object herein to provide compounds and compositions for use in treating such diseases.

WO 2009/102427 relates to modified RNAi constructs with optimal biological activity, toxicity, stability and target gene specificity and uses thereof.

WO 2006/066203 relates to double stranded small interfering RNA (siRNA) molecules for modulating a SOD gene in subjects with neurological disorders.

US 2009/130195 relates to methods of treating prostate cancer using compounds comprising small siRNA molecules to inhibit PKC expression.

### Summary of the Invention

The present invention provides an antisense compound or a pharmaceutically acceptable salt thereof, according to the following formula:
mCes Aeo Ges Geo Aes Tds Ads mCds Ads Tds Tds Tds mCds Tds Ads mCeo Aes Geo mCes Te (nucleobase sequence of SEQ ID NO: 725);
wherein,
   A = an adenine,
   mC = a 5-methylcytosine,
   G = a guanine,
   T = a thymine,
   e = a 2'-O-methoxyethylribose modified sugar,
   d = a 2'-deoxyribose sugar,
   s = a phosphorothioate internucleoside linkage, and
   o = a phosphodiester internucleoside linkage;
for use in therapy, wherein said use comprises the intrathecal administration of the antisense compound or the pharmaceutically acceptable salt thereof.

The invention also provides a pharmaceutical composition comprising, a pharmaceutically acceptable diluent or carrier, and a compound of the following structure: 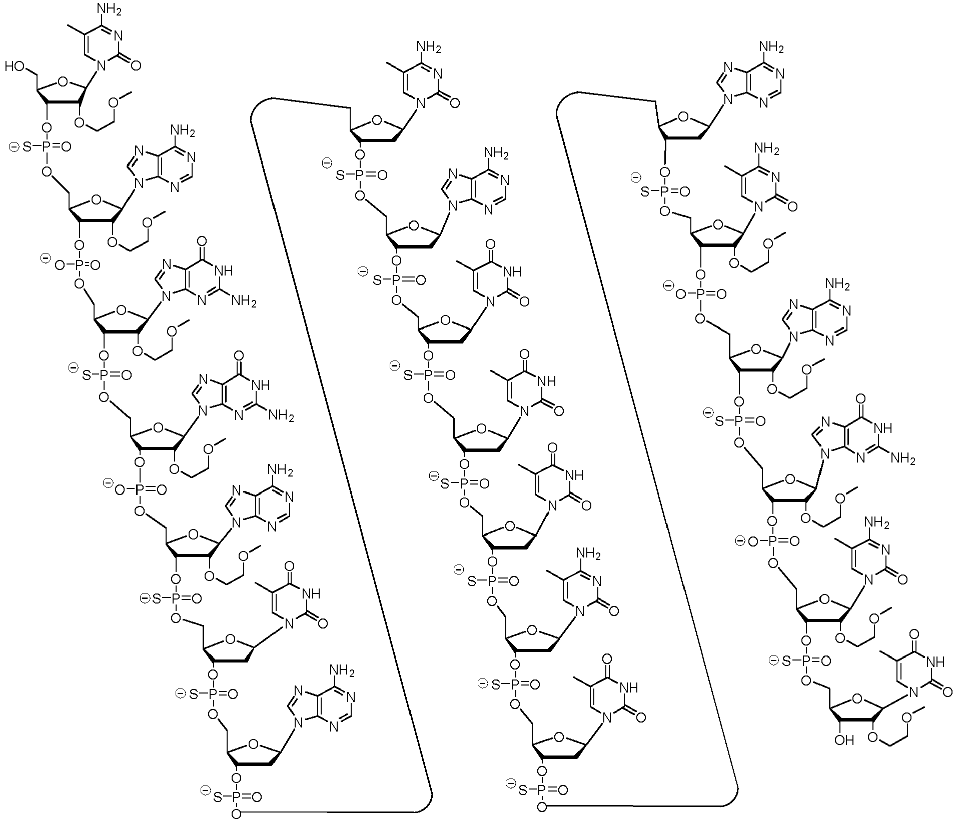 for use in therapy, wherein said use comprises the intrathecal administration of the pharmaceutical composition.

Further embodiments of the invention are defined in the appended claims.

### Summary of the Disclosure

Described herein are methods, compounds, and compositions for modulating expression of superoxide dismutase 1, soluble (SOD-1) mRNA and protein. In certain instances, compounds useful for modulating expression of SOD-1 mRNA and protein are antisense compounds. In certain instances, the antisense compounds are modified oligonucleotides.

In certain instances, modulation can occur in a cell or tissue. In certain instances, the cell or tissue is in an animal. In certain instances, the animal is a human. In certain instances, SOD-1 mRNA levels are reduced. In certain instances, SOD-1 protein levels are reduced. Such reduction can occur in a time-dependent manner or in a dose-dependent manner.

Also described are methods, compounds, and compositions useful for preventing, treating, and ameliorating diseases, disorders, and conditions. In certain instances, such SOD-1 related diseases, disorders, and conditions are neurodegenerative diseases. In certain instances, such neurodegenerative diseases, disorders, and conditions include amyotrophic lateral sclerosis (ALS).

Such diseases, disorders, and conditions can have one or more risk factors, causes, or outcomes in common. Certain risk factors and causes for development of ALS include growing older, having a personal or family history, or genetic predisposition. However, the majority of ALS cases are sporadic and no known risk factors are known. Certain symptoms and outcomes associated with development of ALS include but are not limited to: fasciculations, cramps, tight and stiff muscles (spasticity), muscle weakness affecting an arm or a leg, slurred and nasal speech, difficulty walking, difficulty chewing or swallowing (dysphagia), difficulty speaking or forming words (dysarthria), weakness or atrophy, spasticity, exaggerated reflexes (hyperreflexia), and presence of Babinski's sign. As ALS progresses, symptoms and outcomes by include weakening of other limbs, perhaps accompanied by twitching, muscle cramping, and exaggerated, faster reflexes; problems with chewing, swallowing, and breathing; drooling may occur; eventual paralysis; and death.

In certain instances, methods of treatment include administering an SOD-1 antisense compound to an individual in need thereof. In certain instances, methods of treatment include administering an SOD-1 modified oligonucleotide to an individual in need thereof.

### Detailed Description

Herein, the use of the singular includes the plural unless specifically stated otherwise. As used herein, the use of "or" means "and/or" unless stated otherwise. Additionally, as used herein, the use of "and" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise. Also, all sequences described herein are listed 5' to 3', unless otherwise stated.

The section headings used herein are for organizational purposes only and are not to be construed as limiting the subject matter described.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings:
"2'-deoxynucleoside" (also 2'-deoxyribonucleoside) means a nucleoside comprising 2'-H furanosyl sugar moiety, as found in naturally occurring deoxyribonucleosides (DNA). In certain instances, a 2'-deoxynucleoside may comprise a modified nucleobase or may comprise an RNA nucleobase (e.g., uracil).
"2'-deoxyribose sugar" means a 2'-H furanosyl sugar moiety, as found in naturally occurring deoxyribonucleic acids (DNA).
"2'-O-methoxyethyl" (also 2'-MOE and 2'-OCH₂CH₂-OCH₃ and MOE and 2'-O-methoxyethylribose) refers to an O-methoxy-ethyl modification of the 2' position of a furanose ring. A 2'-O-methoxyethylribose modified sugar is a modified sugar.
"2'-O-methoxyethylribose modified nucleoside" (also2'-MOE nucleoside) means a nucleoside comprising a 2'-MOE modified sugar moiety.
"2'-substituted nucleoside" means a nucleoside comprising a substituent at the 2'-position of the furanose ring other than H or OH. In certain instances, 2' substituted nucleosides include nucleosides with bicyclic sugar modifications.
"5-methylcytosine" means a cytosine modified with a methyl group attached to the 5 position. A 5-methylcytosine is a modified nucleobase.
"About" means within ±10% of a value. For example, if it is stated, "the compounds affected at least about 50% inhibition of SOD-1", it is implied that the SOD-1 levels are inhibited within a range of 45% and 55%. "Administered concomitantly" refers to the co-administration of two pharmaceutical agents in any manner in which the pharmacological effects of both are manifest in the patient at the same time. Concomitant administration does not require that both pharmaceutical agents be administered in a single pharmaceutical composition, in the same dosage form, or by the same route of administration. The effects of both pharmaceutical agents need not manifest themselves at the same time. The effects need only be overlapping for a period of time and need not be coextensive.
"Administering" means providing a pharmaceutical agent to an animal, and includes, but is not limited to administering by a medical professional and self-administering.
"Amelioration" refers to a lessening, slowing, stopping, or reversing of at least one indicator of the severity of a condition or disease. The severity of indicators may be determined by subjective or objective measures, which are known to those skilled in the art.
"Animal" refers to a human or non-human animal, including, but not limited to, mice, rats, rabbits, dogs, cats, pigs, and non-human primates, including, but not limited to, monkeys and chimpanzees.
"Antibody" refers to a molecule characterized by reacting specifically with an antigen in some way, where the antibody and the antigen are each defined in terms of the other. Antibody may refer to a complete antibody molecule or any fragment or region thereof, such as the heavy chain, the light chain, Fab region, and Fc region.
"Antisense activity" means any detectable or measurable activity attributable to the hybridization of an antisense compound to its target nucleic acid. In certain instances, antisense activity is a decrease in the amount or expression of a target nucleic acid or protein encoded by such target nucleic acid.
"Antisense compound" means an oligomeric compound that is capable of undergoing hybridization to a target nucleic acid through hydrogen bonding. Examples of antisense compounds include single-stranded and double-stranded compounds, such as, antisense oligonucleotides, siRNAs, shRNAs, ssRNAs, and occupancy-based compounds.
"Antisense inhibition" means reduction of target nucleic acid levels in the presence of an antisense compound complementary to a target nucleic acid compared to target nucleic acid levels or in the absence of the antisense compound.
"Antisense mechanisms" are all those mechanisms involving hybridization of a compound with a target nucleic acid, wherein the outcome or effect of the hybridization is either target degradation or target occupancy with concomitant stalling of the cellular machinery involving, for example, transcription or splicing.
"Antisense oligonucleotide" means a single-stranded oligonucleotide having a nucleobase sequence that permits hybridization to a corresponding segment of a target nucleic acid.
"Base complementarity" refers to the capacity for the precise base pairing of nucleobases of an oligonucleotide with corresponding nucleobases in a target nucleic acid (i.e., hybridization), and is mediated by Watson-Crick, Hoogsteen or reversed Hoogsteen hydrogen binding between corresponding nucleobases.
"Bicyclic sugar" means a furanose ring modified by the bridging of two atoms. A bicyclic sugar is a modified sugar.
"Bicyclic nucleic acid" or "BNA" refers to a nucleoside or nucleotide wherein the furanose portion of the nucleoside or nucleotide includes a bridge connecting two carbon atoms on the furanose ring, thereby forming a bicyclic ring system.
"Cap structure" or "terminal cap moiety" means chemical modifications, which have been incorporated at either terminus of an antisense compound.
"cEt" or "constrained ethyl" or "cEt modified sugar" means a bicyclic nucleoside having a sugar moiety comprising a bridge connecting the 4'-carbon and the 2'-carbon, wherein the bridge has the formula: 4'-CH(CH₃)-O-2'. A cEt modified sugar is a modified sugar.
"cEt modified nucleoside" means a bicyclic nucleoside having a sugar moiety comprising a bridge connecting the 4'-carbon and the 2'-carbon, wherein the bridge has the formula: 4'-CH(CH₃)-O-2'. A cEt modified sugar is a modified sugar.
"Chemically distinct region" refers to a region of an antisense compound that is in some way chemically different than another region of the same antisense compound. For example, a region having 2'-O-methoxyethyl nucleosides is chemically distinct from a region having nucleosides without 2'-O-methoxyethyl modifications.
"Chimeric antisense compound" means an antisense compound that has at least two chemically distinct regions, each position having a plurality of subunits.
"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses parallel or sequential administration.
"Complementarity" means the capacity for pairing between nucleobases of a first nucleic acid and a second nucleic acid.
"Comprise," "comprises," and "comprising" will be understood to imply the inclusion of a stated step or element or group of steps or elements but not the exclusion of any other step or element or group of steps or elements.
"Contiguous nucleobases" means nucleobases immediately adjacent to each other.
"Designing" or "designed to" refer to the process of designing an oligomeric compound that specifically hybridizes with a selected nucleic acid molecule.
"Diluent" means an ingredient in a composition that lacks pharmacological activity, but is pharmaceutically necessary or desirable. For example, in drugs that are injected, the diluent may be a liquid, e.g. saline solution.
"Dose" means a specified quantity of a pharmaceutical agent provided in a single administration, or in a specified time period. In certain instances, a dose may be administered in one, two, or more boluses, tablets, or injections. For example, in certain instances where subcutaneous administration is desired, the desired dose requires a volume not easily accommodated by a single injection, therefore, two or more injections may be used to achieve the desired dose. In certain instances, the pharmaceutical agent is administered by infusion over an extended period of time or continuously. Doses may be stated as the amount of pharmaceutical agent per hour, day, week, or month.
"Effective amount" in the context of modulating an activity or of treating or preventing a condition means the administration of that amount of pharmaceutical agent to a subject in need of such modulation, treatment, or prophylaxis, either in a single dose or as part of a series, that is effective for modulation of that effect, or for treatment or prophylaxis or improvement of that condition. The effective amount may vary among individuals depending on the health and physical condition of the individual to be treated, the taxonomic group of the individuals to be treated, the formulation of the composition, assessment of the individual's medical condition, and other relevant factors.
"Efficacy" means the ability to produce a desired effect.
"Expression" includes all the functions by which a gene's coded information is converted into structures present and operating in a cell. Such structures include, but are not limited to the products of transcription and translation.
"Fully complementary" or "100% complementary" means each nucleobase of a first nucleic acid has a complementary nucleobase in a second nucleic acid. In certain instances, a first nucleic acid is an antisense compound and a target nucleic acid is a second nucleic acid.
"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap" and the external regions may be referred to as the "wings."
"Gap-narrowed" means a chimeric antisense compound having a gap segment of 9 or fewer contiguous 2'-deoxyribonucleosides positioned between and immediately adjacent to 5' and 3' wing segments having from 1 to 6 nucleosides.
"Gap-widened" means a chimeric antisense compound having a gap segment of 12 or more contiguous 2'-deoxyribonucleosides positioned between and immediately adjacent to 5' and 3' wing segments having from 1 to 6 nucleosides.
"Hybridization" means the annealing of complementary nucleic acid molecules. In certain instances, complementary nucleic acid molecules include, but are not limited to, an antisense compound and a target nucleic acid. In certain instances, complementary nucleic acid molecules include, but are not limited to, an oligonucleotide and a nucleic acid target.
"Identifying an animal having a SOD-1 associated disease" means identifying an animal having been diagnosed with a SOD-1 associated disease or predisposed to develop a SOD-1 associated disease. Individuals predisposed to develop a SOD-1 associated disease include those having one or more risk factors for developing a SOD-1 associated disease, including, growing older, having a personal or family history, or genetic predisposition of one or more SOD-1 associated diseases. Such identification may be accomplished by any method including evaluating an individual's medical history and standard clinical tests or assessments, such as genetic testing.
"Immediately adjacent" means there are no intervening elements between the immediately adjacent elements.
"Individual" means a human or non-human animal selected for treatment or therapy.
"Inhibiting SOD-1" means reducing the level or expression of a SOD-1 mRNA and/or protein. In certain instances, SOD-1 mRNA and/or protein levels are inhibited in the presence of an antisense compound targeting SOD-1, including a modified oligonucleotide targeting SOD-1, as compared to expression of SOD-1 mRNA and/or protein levels in the absence of a SOD-1 antisense compound, such as a modified oligonucleotide.
"Inhibiting the expression or activity" refers to a reduction or blockade of the expression or activity and does not necessarily indicate a total elimination of expression or activity.
"Internucleoside linkage" refers to the chemical bond between nucleosides.
"Linked nucleosides" means adjacent nucleosides linked together by an internucleoside linkage.
"Mismatch" or "non-complementary nucleobase" refers to the case when a nucleobase of a first nucleic acid is not capable of pairing with the corresponding nucleobase of a second or target nucleic acid.
"Mixed backbone" means a pattern of internucleoside linkages including at least two different internucleoside linkages. For example, an oligonucleotide with a mixed backbone may include at least one phosphodiester linkage and at least one phosphorothioate linkage.
"Modified internucleoside linkage" refers to a substitution or any change from a naturally occurring internucleoside bond (*i.e.,* a phosphodiester internucleoside bond).
"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).

A "modified nucleoside" means a nucleoside having, independently, a modified sugar moiety and/or modified nucleobase.
"Modified nucleotide" means a nucleotide having, independently, a modified sugar moiety, modified internucleoside linkage, and/or modified nucleobase.
"Modified oligonucleotide" means an oligonucleotide comprising at least one modified internucleoside linkage, modified sugar, and/or modified nucleobase.
"Modified sugar" means substitution and/or any change from a natural sugar moiety.
"Monomer" means a single unit of an oligomer. Monomers include, but are not limited to, nucleosides and nucleotides, whether naturally occurring or modified.
"Motif" means the pattern of unmodified and modified nucleosides in an antisense compound.
"Natural sugar moiety" means a sugar moiety found in DNA (2'-H) or RNA (2'-OH).
"Naturally occurring internucleoside linkage" means a 3' to 5' phosphodiester linkage.
"Non-complementary nucleobase" refers to a pair of nucleobases that do not form hydrogen bonds with one another or otherwise support hybridization.
"Nucleic acid" refers to molecules composed of monomeric nucleotides. A nucleic acid includes, but is not limited to, ribonucleic acids (RNA), deoxyribonucleic acids (DNA), single-stranded nucleic acids, double-stranded nucleic acids, small interfering ribonucleic acids (siRNA), and microRNAs (miRNA).
"Nucleobase" means a heterocyclic moiety capable of pairing with a base of another nucleic acid.
"Nucleobase complementarity" refers to a nucleobase that is capable of base pairing with another nucleobase. For example, in DNA, adenine (A) is complementary to thymine (T). For example, in RNA, adenine (A) is complementary to uracil (U). In certain instances, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.
"Nucleobase sequence" means the order of contiguous nucleobases independent of any sugar, linkage, and/or nucleobase modification.
"Nucleoside" means a nucleobase linked to a sugar.
"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo, or tricyclo sugar mimetics, e.g., non furanose sugar units. Nucleotide mimetic includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage). Sugar surrogate overlaps with the slightly broader term nucleoside mimetic but is intended to indicate replacement of the sugar unit (furanose ring) only. The tetrahydropyranyl rings described herein are illustrative of an example of a sugar surrogate wherein the furanose sugar group has been replaced with a tetrahydropyranyl ring system. "Mimetic" refers to groups that are substituted for a sugar, a nucleobase, and/or internucleoside linkage. Generally, a mimetic is used in place of the sugar or sugar-internucleoside linkage combination, and the nucleobase is maintained for hybridization to a selected target.
"Nucleotide" means a nucleoside having a phosphate group covalently linked to the sugar portion of the nucleoside.
"Off-target effect" refers to an unwanted or deleterious biological effect associated with modulation of RNA or protein expression of a gene other than the intended target nucleic acid.
"Oligomeric compound" or "oligomer" means a polymer of linked monomeric subunits which is capable of hybridizing to at least a region of a nucleic acid molecule.
"Oligonucleotide" means a polymer of linked nucleosides each of which can be modified or unmodified, independent one from another.

According to the invention, the antisense compound or pharmaceutically acceptable salt thereof for use in therapy is administered by intrathecal administration.
"Peptide" means a molecule formed by linking at least two amino acids by amide bonds. Without limitation, as used herein, peptide refers to polypeptides and proteins.
"Pharmaceutical agent" means a substance that provides a therapeutic benefit when administered to an individual. For example, in certain instances, a modified oligonucleotide targeted to SOD-1 is a pharmaceutical agent.
"Pharmaceutical composition" means a mixture of substances suitable for administering to a subject. For example, a pharmaceutical composition may comprise a modified oligonucleotide and a sterile aqueous solution.
"Pharmaceutically acceptable derivative" encompasses pharmaceutically acceptable salts, conjugates, prodrugs or isomers of the compounds described herein.
"Pharmaceutically acceptable salts" means physiologically and pharmaceutically acceptable salts of antisense compounds, *i*.*e*., salts that retain the desired biological activity of the parent oligonucleotide and do not impart undesired toxicological effects thereto.
"Phosphorothioate linkage" means a linkage between nucleosides where the phosphodiester bond is modified by replacing one of the non-bridging oxygen atoms with a sulfur atom. A phosphorothioate linkage is a modified internucleoside linkage.
"Portion" means a defined number of contiguous (i.e., linked) nucleobases of a nucleic acid. In certain instances, a portion is a defined number of contiguous nucleobases of a target nucleic acid. In certain instances, a portion is a defined number of contiguous nucleobases of an antisense compound.
"Prevent" or "preventing" refers to delaying or forestalling the onset or development of a disease, disorder, or condition for a period of time from minutes to days, weeks to months, or indefinitely.
"Prodrug" means a therapeutic agent that is prepared in an inactive form that is converted to an active form (i.e., drug) within the body or cells thereof by the action of endogenous enzymes or other chemicals and/or conditions.
"Prophylactically effective amount" refers to an amount of a pharmaceutical agent that provides a prophylactic or preventative benefit to an animal.
"Region" is defined as a portion of the target nucleic acid having at least one identifiable structure, function, or characteristic.
"Ribonucleotide" means a nucleotide having a hydroxy at the 2' position of the sugar portion of the nucleotide. Ribonucleotides may be modified with any of a variety of substituents.
"Salts" mean a physiologically and pharmaceutically acceptable salts of antisense compounds, i.e., salts that retain the desired biological activity of the parent oligonucleotide and do not impart undesired toxicological effects thereto.
"Segments" are defined as smaller or sub-portions of regions within a target nucleic acid.
"Shortened" or "truncated" versions of oligonucleotides SOD-1ght herein have one, two or more nucleosides deleted.
"Side effects" means physiological responses attributable to a treatment other than desired effects. In certain instances, side effects include, without limitation, injection site reactions, liver function test abnormalities, renal function abnormalities, liver toxicity, renal toxicity, central nervous system abnormalities, and myopathies.
"Single-stranded oligonucleotide" means an oligonucleotide which is not hybridized to a complementary strand.
"Sites," as used herein, are defined as unique nucleobase positions within a target nucleic acid. "Slows progression" means decrease in the development of the disease.
"SOD-1" means the mammalian gene superoxide dismutase 1, soluble (SOD-1), including the human gene superoxide dismutase 1, soluble (SOD-1).
"SOD-1 associated disease" means any disease associated with any SOD-1 nucleic acid or expression product thereof. Such diseases may include a neurodegenerative disease. Such neurodegenerative diseases may include amyotrophic lateral sclerosis (ALS).
"SOD-1 mRNA" means any messenger RNA expression product of a DNA sequence encoding SOD-1.
"SOD-1 nucleic acid" means any nucleic acid encoding SOD-1. For example, in certain instances, a SOD-1 nucleic acid includes a DNA sequence encoding SOD-1, an RNA sequence transcribed from DNA encoding SOD-1 (including genomic DNA comprising introns and exons), and a mRNA sequence encoding SOD-1. "SOD-1 mRNA" means a mRNA encoding a SOD-1 protein.
"SOD-1 protein" means the polypeptide expression product of a SOD-1 nucleic acid.
"Specifically hybridizable" refers to an antisense compound having a sufficient degree of complementarity between an oligonucleotide and a target nucleic acid to induce a desired effect, while exhibiting minimal or no effects on non-target nucleic acids under conditions in which specific binding is desired, *i.e.,* under physiological conditions in the case of *in vivo* assays and therapeutic treatments.
"Stringent hybridization conditions" or "stringent conditions" refer to conditions under which an oligomeric compound will hybridize to its target sequence, but to a minimal number of other sequences.
"Subject" means a human or non-human animal selected for treatment or therapy.
"Sugar chemistry motif' means a pattern of sugar modifications including at least two different sugar modifications. For example, an oligonucleotide with a mixed backbone may include at least one 2'-O-methoxyethyl modified nucleoside, and/or one cEt modified nucleoside, and/or one 2'-deoxynucleoside.
"Target" refers to a protein, the modulation of which is desired.
"Target gene" refers to a gene encoding a target.
"Targeting" or "targeted" means the process of design and selection of an antisense compound that will specifically hybridize to a target nucleic acid and induce a desired effect.
"Target nucleic acid," "target RNA," and "target RNA transcript" and "nucleic acid target" all mean a nucleic acid capable of being targeted by antisense compounds.
"Target region" means a portion of a target nucleic acid to which one or more antisense compounds is targeted.
"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.
"Therapeutically effective amount" means an amount of a pharmaceutical agent that describes a therapeutic benefit to an individual.
"Treat" or "treating" or "treatment" refers administering a composition to effect an alteration or improvement of the disease or condition.
"Unmodified nucleobases" mean the purine bases adenine (A) and guanine (G), and the pyrimidine bases thymine (T), cytosine (C) and uracil (U).
"Unmodified nucleotide" means a nucleotide composed of naturally occuring nucleobases, sugar moieties, and internucleoside linkages. In certain instances, an unmodified nucleotide is an RNA nucleotide (*i.e.* β-D-ribonucleosides) or a DNA nucleotide (*i.e.* β-D-deoxyribonucleoside).
"Wing segment" means a plurality of nucleosides modified to impart to an oligonucleotide properties such as enhanced inhibitory activity, increased binding affinity for a target nucleic acid, or resistance to degradation by in vivo nucleases.

### Certain Instances

Certain instances herein are methods, compounds, and compositions for inhibiting SOD-1 mRNA and protein expression. Certain instances herein are methods, compounds, and composition for decreasing SOD-1 mRNA and protein levels.

Certain instances are antisense compounds targeted to a SOD-1 nucleic acid. In certain instances, the SOD-1 nucleic acid is the sequence set forth in GENBANK Accession No. NM_000454.4 (incorporated herein as SEQ ID NO: 1), GENBANK Accession No. NT_011512.10 truncated from nucleotides 18693000 to 18704000 (incorporated herein as SEQ ID NO: 2), and the complement of GENBANK Accession No. NW_001114168.1 truncated from nucleotides 2258000 to 2271000 (incorporated herein as SEQ ID NO: 3).

Certain instances are methods for the treatment, prevention, or amelioration of diseases, disorders, and conditions associated with SOD-1 in an individual in need thereof. Also contemplated are methods for the preparation of a medicament for the treatment, prevention, or amelioration of a disease, disorder, or condition associated with SOD-1. SOD-1 associated diseases, disorders, and conditions include neurodegenerative diseases. In certain instances, SOD-1 associated diseases include amyotrophic lateral sclerosis (ALS).

An exemplary instance is an antisense compound or a pharmaceutically acceptable salt thereof, according to the following formula:
mCes Aeo Ges Geo Aes Tds Ads mCds Ads Tds Tds Tds mCds Tds Ads mCeo Aes Geo mCes Te (nucleobase sequence of SEQ ID NO: 725);
wherein,
   A = an adenine,
   mC = a 5-methylcytosine,
   G = a guanine,
   T = a thymine,
   e = a 2'-O-methoxyethylribose modified sugar,
   d = a 2'-deoxyribose sugar,
   s = a phosphorothioate internucleoside linkage, and
   o = a phosphodiester internucleoside linkage;
for use in therapy, wherein said use comprises the intrathecal administration of the antisense compound or the pharmaceutically acceptable salt thereof.

Another exemplary instance is a pharmaceutical composition comprising, a pharmaceutically acceptable diluent or carrier, and a compound of the following structure: 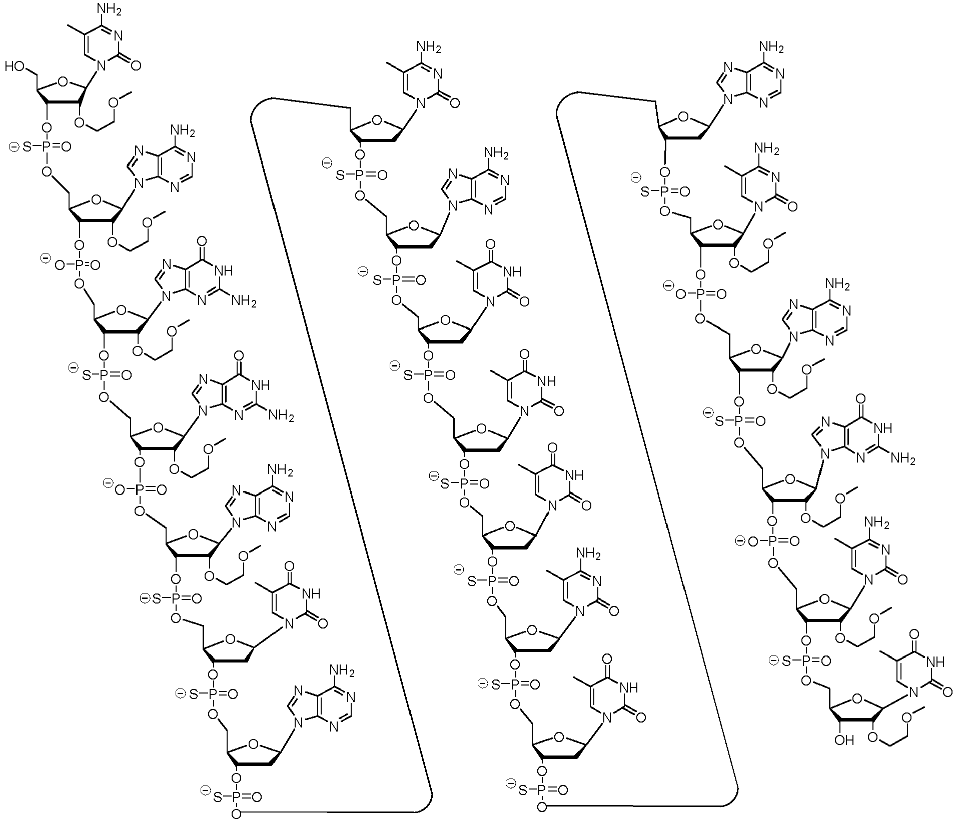 for use in therapy, wherein said use comprises the intrathecal administration of the pharmaceutical composition.

### Antisense Compounds

Oligomeric compounds include, but are not limited to, oligonucleotides, oligonucleosides, oligonucleotide analogs, oligonucleotide mimetics, antisense compounds, antisense oligonucleotides, modified oligonucleotides, and siRNAs. An oligomeric compound may be "antisense" to a target nucleic acid, meaning that is is capable of undergoing hybridization to a target nucleic acid through hydrogen bonding.

In certain instances, an antisense compound has a nucleobase sequence that, when written in the 5' to 3' direction, comprises the reverse complement of the target segment of a target nucleic acid to which it is targeted. In certain such instances, an oligonucleotide has a nucleobase sequence that, when written in the 5' to 3' direction, comprises the reverse complement of the target segment of a target nucleic acid to which it is targeted.

In certain instances, an antisense compound targeted to a SOD-1 nucleic acid is 12 to 30 subunits in length. In certain instances, an antisense compound targeted to a SOD-1 nucleic acid is 12 to 25 subunits in length. In certain instances, an antisense compound targeted to a SOD-1 nucleic acid is 12 to 22 subunits in length. In certain instances, an antisense compound targeted to a SOD-1 nucleic acid is 14 to 20 subunits in length. In certain instances, an antisense compound targeted to a SOD-1 nucleic acid is 15 to 25 subunits in length. In certain instances, an antisense compound targeted to a SOD-1 nucleic acid is 18 to 22 subunits in length. In certain instances, an antisense compound targeted to a SOD-1 nucleic acid is 19 to 21 subunits in length. In certain instances, the antisense compound is 8 to 80, 12 to 50, 13 to 30, 13 to 50, 14 to 30, 14 to 50, 15 to 30, 15 to 50, 16 to 30, 16 to 50, 17 to 30, 17 to 50, 18 to 30, 18 to 50, 19 to 30, 19 to 50, or 20 to 30 linked subunits in length.

In certain instances, an antisense compound targeted to a SOD-1 nucleic acid is 12 subunits in length. In certain instances, an antisense compound targeted to a SOD-1 nucleic acid is 13 subunits in length. In certain instances, an antisense compound targeted to a SOD-1 nucleic acid is 14 subunits in length. In certain instances, an antisense compound targeted to a SOD-1 nucleic acid is 15 subunits in length. In certain instances, an antisense compound targeted to a SOD-1 nucleic acid is 16 subunits in length. In certain instances, an antisense compound targeted to a SOD-1 nucleic acid is 17 subunits in length. In certain instances, an antisense compound targeted to a SOD-1 nucleic acid is 18 subunits in length. In certain instances, an antisense compound targeted to a SOD-1 nucleic acid is 19 subunits in length. In certain instances, an antisense compound targeted to a SOD-1 nucleic acid is 20 subunits in length. In certain instances, an antisense compound targeted to a SOD-1 nucleic acid is 21 subunits in length. In certain instances, an antisense compound targeted to a SOD-1 nucleic acid is 22 subunits in length. In certain instances, an antisense compound targeted to a SOD-1 nucleic acid is 23 subunits in length. In certain instances, an antisense compound targeted to a SOD-1 nucleic acid is 24 subunits in length. In certain instances, an antisense compound targeted to a SOD-1 nucleic acid is 25 subunits in length. In certain instances, an antisense compound targeted to a SOD-1 nucleic acid is 26 subunits in length. In certain instances, an antisense compound targeted to a SOD-1 nucleic acid is 27 subunits in length. In certain instances, an antisense compound targeted to a SOD-1 nucleic acid is 28 subunits in length. In certain instances, an antisense compound targeted to a SOD-1 nucleic acid is 29 subunits in length. In certain instances, an antisense compound targeted to a SOD-1 nucleic acid is 30 subunits in length. In certain instances, the antisense compound targeted to a SOD-1 nucleic acid is 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked subunits in length, or a range defined by any two of the above values. In certain instances the antisense compound is a modified oligonucleotide, and the linked subunits are nucleosides.

In certain instances, oligonucleotides targeted to a SOD-1 nucleic acid may be shortened or truncated. For example, a single subunit may be deleted from the 5' end (5' truncation), or alternatively from the 3' end (3' truncation). A shortened or truncated antisense compound targeted to a SOD-1 nucleic acid may have two subunits deleted from the 5' end, or alternatively may have two subunits deleted from the 3' end, of the antisense compound. Alternatively, the deleted nucleosides may be dispersed throughout the antisense compound, for example, in an antisense compound having one nucleoside deleted from the 5' end and one nucleoside deleted from the 3' end.

When a single additional subunit is present in a lengthened antisense compound, the additional subunit may be located at the 5' or 3' end of the antisense compound. When two or more additional subunits are present, the added subunits may be adjacent to each other, for example, in an antisense compound having two subunits added to the 5' end (5' addition), or alternatively to the 3' end (3' addition), of the antisense compound. Alternatively, the added subunits may be dispersed throughout the antisense compound, for example, in an antisense compound having one subunit added to the 5' end and one subunit added to the 3' end.

It is possible to increase or decrease the length of an antisense compound, such as a modified oligonucleotide, and/or introduce mismatch bases without eliminating activity. For example, in Woolf et al. (Proc. Natl. Acad. Sci. USA 89:7305-7309, 1992), a series of oligonucleotides 13-25 nucleobases in length were tested for their ability to induce cleavage of a target RNA in an oocyte injection model. Oligonucleotides 25 nucleobases in length with 8 or 11 mismatch bases near the ends of the oligonucleotides were able to direct specific cleavage of the target mRNA, albeit to a lesser extent than oligonucleotides that contained no mismatches. Similarly, target specific cleavage was achieved using 13 nucleobase oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL *in vitro* and *in vivo.* Furthermore, this oligonucleotide demonstrated potent anti-tumor activity *in vivo.*

Maher and Dolnick (Nuc. Acid. Res. 16:3341-3358,1988) tested a series of tandem 14 nucleobase oligonucleotides, and a 28 and 42 nucleobase oligonucleotides comprised of the sequence of two or three of the tandem oligonucleotides, respectively, for their ability to arrest translation of human DHFR in a rabbit reticulocyte assay. Each of the three 14 nucleobase oligonucleotides alone was able to inhibit translation, albeit at a more modest level than the 28 or 42 nucleobase oligonucleotides.

### Antisense Compound Motifs

In certain instances, antisense compounds targeted to a SOD-1 nucleic acid have chemically modified subunits arranged in patterns, or motifs, to confer to the antisense compounds properties such as enhanced inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound may optionally serve as a substrate for the cellular endonuclease RNase H, which cleaves the RNA strand of an RNA:DNA duplex.

Antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an oligonucleotide having a gapmer motif, the gap segment generally serves as the substrate for endonuclease cleavage, while the wing segments comprise modified nucleosides. In certain instances, the regions of a gapmer are differentiated by the types of sugar moieties comprising each distinct region. The types of sugar moieties that are used to differentiate the regions of a gapmer may in some instances include β-D-ribonucleosides, β-D-deoxyribonucleosides, 2'-modified nucleosides (such 2'-modified nucleosides may include 2'-MOE, and 2'-O-CH₃, among others), and bicyclic sugar modified nucleosides (such bicyclic sugar modified nucleosides may include those having a 4'-(CH₂)n-O-2' bridge, where n=1 or n=2 and 4'-CH₂-O-CH₂-2'). In certain instances, wings may include several modified sugar moieties, including, for example 2'-MOE. In certain instances, wings may include several modified and unmodified sugar moieties. In certain instances, wings may include various combinations of 2'-MOE nucleosides and 2'-deoxynucleosides.

Each distinct region may comprise uniform sugar moieties, variant, or alternating sugar moieties. The wing-gap-wing motif is frequently described as "X-Y-Z", where "X" represents the length of the 5' wing, "Y" represents the length of the gap, and "Z" represents the length of the 3' wing. "X" and "Z" may comprise uniform, variant, or alternating sugar moieties. In certain instances, "X" and "Y" may include one or more 2'-deoxynucleosides. "Y" may comprise 2'-deoxynucleosides. As used herein, a gapmer described as "X-Y-Z" has a configuration such that the gap is positioned immediately adjacent to each of the 5' wing and the 3' wing. Thus, no intervening nucleotides exist between the 5' wing and gap, or the gap and the 3' wing. Any of the antisense compounds described herein can have a gapmer motif. In certain instances, "X" and "Z" are the same; in other instances they are different.

In certain instances, gapmers described herein include, for example 20-mers having a motif of 5-10-5.

In certain instances, gapmers described herein include, for example 19-mers having a motif of 5-9-5.

In certain instances, gapmers described herein include, for example 18-mers having a motif of 5-8-5.

In certain instances, gapmers described herein include, for example 18-mers having a motif of 4-8-5.

In certain instances, gapmers described herein include, for example 18-mers having a motif of 5-8-7.

In certain instances, gapmers described herein include, for example 18-mers having a motif of 6-8-6.

In certain instances, gapmers described herein include, for example 18-mers having a motif of 6-8-5.

In certain instances, the modified oligonucleotide contains at least one 2'-O-methoxyethyl modified nucleoside , at least one cEt modified nucleoside , and at least one 2'-deoxynucleoside. In certain instances, the modified oligonucleotide has a sugar chemistry motif of any of the following:
ekddddddddekekee
kekeddddddddekek
eeeedddddddddkkee
eeeeddddddddekeke
eeeeddddddddkekee
eeeeddddddddkkeee
eeeeeddddddddkkee
eeeekddddddddkeee
eeeekdddddddkeeee
eeekddddddddkeeee
eeekkdddddddkkeee
eekkdddddddddkkee
eekkddddddddeeeee
eekkddddddddkkeee
ekekddddddddeeeee
ekekddddddddkekee
kekeddddddddeeeee, wherein
e = a 2'-O-methoxyethylribose modified sugar,
k = a cEt modified sugar,
d = a 2'-deoxyribose sugar,

### TargetNucleic Acids, Target Regions and Nucleotide Sequences

Nucleotide sequences that encode SOD-1 include, without limitation, the following: GENBANK Accession No. NM_000454.4 (incorporated herein as SEQ ID NO: 1), GENBANK Accession No. NT_011512.10 truncated from nucleotides 18693000 to 18704000 (incorporated herein as SEQ ID NO: 2), and the complement of GENBANK Accession No. NW_001114168.1 truncated from nucleotides 2258000 to 2271000 (incorporated herein as SEQ ID NO: 3).

It is understood that the sequence set forth in each SEQ ID NO in the Examples contained herein is independent of any modification to a sugar moiety, an internucleoside linkage, or a nucleobase. As such, antisense compounds defined by a SEQ ID NO may comprise, independently, one or more modifications to a sugar moiety, an internucleoside linkage, or a nucleobase. Antisense compounds described by Isis Number (Isis No) indicate a combination of nucleobase sequence and motif.

In certain instances, a target region is a structurally defined region of the target nucleic acid. For example, a target region may encompass a 3' UTR, a 5' UTR, an exon, an intron, an exon/intron junction, a coding region, a translation initiation region, translation termination region, or other defined nucleic acid region. The structurally defined regions for SOD-1 can be obtained by accession number from sequence databases such as NCBI. In certain instances, a target region may encompass the sequence from a 5' target site of one target segment within the target region to a 3' target site of another target segment within the same target region.

Targeting includes determination of at least one target segment to which an antisense compound hybridizes, such that a desired effect occurs. In certain instances, the desired effect is a reduction in mRNA target nucleic acid levels. In certain instances, the desired effect is reduction of levels of protein encoded by the target nucleic acid or a phenotypic change associated with the target nucleic acid.

A target region may contain one or more target segments. Multiple target segments within a target region may be overlapping. Alternatively, they may be non-overlapping. In certain instances, target segments within a target region are separated by no more than about 300 nucleotides. In certain emodiments, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceeding values. In certain instances, target segments within a target region are separated by no more than, or no more than about, 5 nucleotides on the target nucleic acid. In certain instances, target segments are contiguous. Contemplated are target regions defined by a range having a starting nucleic acid that is any of the 5' target sites or 3' target sites listed herein.

Suitable target segments may be found within a 5' UTR, a coding region, a 3' UTR, an intron, an exon, or an exon/intron junction. Target segments containing a start codon or a stop codon are also suitable target segments. A suitable target segment may specifcally exclude a certain structurally defined region such as the start codon or stop codon.

The determination of suitable target segments may include a comparison of the sequence of a target nucleic acid to other sequences throughout the genome. For example, the BLAST algorithm may be used to identify regions of similarity amongst different nucleic acids. This comparison can prevent the selection of antisense compound sequences that may hybridize in a non-specific manner to sequences other than a selected target nucleic acid (i.e., non-target or off-target sequences).

There may be variation in activity (e.g., as defined by percent reduction of target nucleic acid levels) of the antisense compounds within an active target region. In certain instances, reductions in SOD-1 mRNA levels are indicative of inhibition of SOD-1 expression. Reductions in levels of a SOD-1 protein are also indicative of inhibition of target mRNA expression. Phenotypic changes are indicative of inhibition of SOD-1 expression. Improvement in neurological function is indicative of inhibition of SOD-1 expression. Improved motor function is indicative of inhibition of SOD-1 expression.

### Hybridization

In some instances, hybridization occurs between an antisense compound disclosed herein and a SOD-1 nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art. In certain instances, the antisense compounds described herein are specifically hybridizable with a SOD-1 nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as a SOD-1 nucleic acid).

Non-complementary nucleobases between an antisense compound and a SOD-1 nucleic acid may be tolerated provided that the antisense compound remains able to specifically hybridize to a target nucleic acid. Moreover, an antisense compound may hybridize over one or more segments of a SOD-1 nucleic acid such that intervening or adjacent segments are not involved in the hybridization event (e.g., a loop structure, mismatch or hairpin structure).

In certain instances, the antisense compounds described herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a SOD-1 nucleic acid, a target region, target segment, or specified portion thereof. Percent complementarity of an antisense compound with a target nucleic acid can be determined using routine methods.

For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present disclosure. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain instances, the antisense compounds described herein, or specified portions thereof, are fully complementary (*i.e.,* 100% complementary) to a target nucleic acid, or specified portion thereof. For example, an antisense compound may be fully complementary to a SOD-1 nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e., linked) or non-contiguous. In one instance, a non-complementary nucleobase is located in the wing segment of a gapmer oligonucleotide.

In certain instances, antisense compounds that are, or are up to 11, 12, 13, 14, 15, 16, 17, 18, 19, or 20 nucleobases in length comprise no more than 4, no more than 3, no more than 2, or no more than 1 non-complementary nucleobase(s) relative to a target nucleic acid, such as a SOD-1 nucleic acid, or specified portion thereof.

In certain instances, antisense compounds that are, or are up to 11, 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, such as a SOD-1 nucleic acid, or specified portion thereof.

The antisense compounds described herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain instances, the antisense compounds, are complementary to at least an 8 nucleobase portion of a target segment. In certain instances, the antisense compounds are complementary to at least a 9 nucleobase portion of a target segment. In certain instances, the antisense compounds are complementary to at least a 10 nucleobase portion of a target segment. In certain instances, the antisense compounds, are complementary to at least an 11 nucleobase portion of a target segment. In certain instances, the antisense compounds, are complementary to at least a 12 nucleobase portion of a target segment. In certain instances, the antisense compounds, are complementary to at least a 13 nucleobase portion of a target segment. In certain instances, the antisense compounds, are complementary to at least a 14 nucleobase portion of a target segment. In certain instances, the antisense compounds, are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identity

The antisense compounds described herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds described herein also are contemplated. The non-identical bases may be adjacent to each other or dispersed throughout the antisense compound. Percent identity of an antisense compound is calculated according to the number of bases that have identical base pairing relative to the sequence to which it is being compared.

In certain instances, the antisense compounds, or portions thereof, are at least 70%, 75%, 80%, 85%, 90%, 95%, 96%, 97%, 98%, 99% or 100% identical to one or more of the antisense compounds or SEQ ID NOs, or a portion thereof, disclosed herein.

In certain instances, a portion of the antisense compound is compared to an equal length portion of the target nucleic acid. In certain instances, an 8, 9, 10, 11, 12, 13, 14, 15, 16, 17, 18, 19, 20, 21, 22, 23, 24, or 25 nucleobase portion is compared to an equal length portion of the target nucleic acid.

In certain instances, a portion of the oligonucleotide is compared to an equal length portion of the target nucleic acid. In certain instances, an 8, 9, 10, 11, 12, 13, 14, 15, 16, 17, 18, 19, 20, 21, 22, 23, 24, or 25 nucleobase portion is compared to an equal length portion of the target nucleic acid.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain instances, modified oligonucleotides targeted to a SOD-1 nucleic acid comprise one or more modified internucleoside linkages. In certain instances, the modified internucleoside linkages are interspersed throughout the antisense compound. In certain instances, the modified internucleoside linkages are phosphorothioate linkages. In certain instances, each internucleoside linkage of a modified oligonucleotide is a phosphorothioate internucleoside linkage.

In certain instances, the modified oligonucleotides targeted to a SOD-1 nucleic acid comprise one or more phosphodiester internucleoside linkages. In certain instances, modified oligonucleotides targeted to a SOD-1 nucleic acid comprise at least one phosphorothioate internucleoside linkage and at least one phosphodiester internucleoside linkage. In certain instances, the modified oligonucleotide has a mixed backbone motif of the following:
sossssssssoooss,
sooossssssssoss,
sooosssssssssoss,
soosssssssssooss,
sooossssssssooss,
sooosssssssssooss,
sooossssssssssooss,
sooosssssssssssooos,
soooossssssssssooss,
sooosssssssssssooss,
sososssssssssssosos, and
sooossssssssssoooss, wherein
s = a phosphorothioate internucleoside linkage, and
o = a phosphodiester internucleoside linkage.

### Modified Sugar Moieties

Antisense compounds of the invention can optionally contain one or more nucleosides wherein the sugar group has been modified. Such sugar modified nucleosides may impart enhanced nuclease stability, increased binding affinity, or some other beneficial biological property to the antisense compounds. In certain instances, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (R or S), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each R₁, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleic acids (BNAs) include without limitation nucleosides comprising a bridge between the 4' and the 2' ribosyl ring atoms. In certain instances, antisense compounds described herein include one or more BNA nucleosides wherein the bridge comprises one of the formulas: 4'-(CH₂)-O-2' (LNA); 4'-(CH₂)-S-2'; 4'-(CH₂)₂-O-2' (ENA); 4'-CH(CH₃)-O-2' and 4'-CH(CH₂OCH₃)-O-2' (and analogs thereof see U.S. Patent 7,399,845, issued on July 15, 2008); 4'-C(CH₃)(CH₃)-O-2' (and analogs thereof see PCT/US2008/068922 published as WO/2009/006478, published January 8, 2009); 4'-CH₂-N(OCH₃)-2' (and analogs thereof see PCT/US2008/064591 published as WO/2008/150729, published December 11, 2008); 4'-CH₂-ON(CH₃)-2' (see published U.S. Patent Application US2004-0171570, published September 2, 2004 ); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C(=CH₂)-2' (and analogs thereof see PCT/US2008/066154 published as WO 2008/154401, published on December 8, 2008).

Further bicyclic nucleosides have been reported in published literature (see for example: Srivastava et al., J. Am. Chem. Soc., 2007, 129(26) 8362-8379; Frieden et al., Nucleic Acids Research, 2003, 21, 6365-6372; Elayadi et al., Curr. Opinion Invens. Drugs, 2001, 2, 558-561; Braasch et al., Chem. Biol., 2001, 8, 1-7; Orum et al., Curr. OpinionMol. Ther., 2001, 3, 239-243; Wahlestedt et al., Proc. Natl. Acad. Sci. U. S. A., 2000, 97, 5633-5638; Singh et al., Chem. Commun., 1998, 4, 455-456; Koshkin et al., Tetrahedron, 1998, 54, 3607-3630; Kumar et al., Bioorg. Med. Chem. Lett., 1998, 8, 2219-2222; Singh et al., J. Org. Chem., 1998, 63, 10035-10039; U.S. Patents Nos.: 7,399,845; 7,053,207; 7,034,133; 6,794,499; 6,770,748; 6,670,461; 6,525,191; 6,268,490; U.S. Patent Publication Nos.: US2008-0039618; US2007-0287831; US2004-0171570; U.S. Patent Applications, Serial Nos.: 12/129,154; 61/099,844; 61/097,787; 61/086,231; 61/056,564; 61/026,998; 61/026,995; 60/989,574; International applications WO 2007/134181; WO 2005/021570; WO 2004/106356; WO 94/14226; and PCT International Applications Nos.: PCT/US2008/068922; PCT/US2008/066154; and PCT/US2008/064591). Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

As used herein, "monocylic nucleosides" refer to nucleosides comprising modified sugar moieties that are not bicyclic sugar moieties. In certain instances, the sugar moiety, or sugar moiety analogue, of a nucleoside may be modified or substituted at any position.

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

In certain instances, bicyclic sugar moieties of BNA nucleosides include, but are not limited to, compounds having at least one bridge between the 4' and the 2' carbon atoms of the pentofuranosyl sugar moiety including without limitation, bridges comprising 1 or from 1 to 4 linked groups independently selected from -[C(Rₐ)(R_{b})]ₙ-, -C(Rₐ)=C(R_{b})-, -C(Rₐ)=N-, -C(=NRₐ)-, - C(=O)-, -C(=S)-, -O-, -Si(Rₐ)₂-, -S(=O)ₓ-, and -N(Rₐ)-; wherein: x is 0, 1, or 2; n is 1, 2, 3, or 4; each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain instances, the bridge of a bicyclic sugar moiety is , -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain instances, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each R is, independently, H, a protecting group or C₁-C₁₂ alkyl.

In certain instances, bicyclic nucleosides are further defined by isomeric configuration. For example, a nucleoside comprising a 4'-(CH₂)-O-2' bridge, may be in the α-L configuration or in the β-D configuration. Previously, α-L-methyleneoxy (4'-CH₂-O-2') BNA's have been incorporated into antisense oligonucleotides that showed antisense activity (Frieden et al., Nucleic Acids Research, 2003, 21, 6365-6372).

In certain instances, bicyclic nucleosides include those having a 4' to 2' bridge wherein such bridges include without limitation, α-L-4'-(CH₂)-O-2', P-D-4'-CH₂-O-2', 4'-(CH₂)₂-O-2', 4'-CH₂-ON(R)-2', 4'-CH₂-N(R)-O-2', 4'-CH(CH₃)-O-2', 4'-CH₂-S-2', 4'-CH₂-N(R)-2', 4'-CH₂-CH(CH₃)-2', and 4'-(CH₂)₃-2', wherein R is H, a protecting group or C₁-C₁₂ alkyl.

In certain instance, bicyclic nucleosides have the formula: 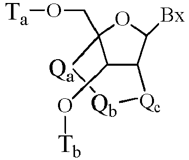 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or - N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain instances, bicyclic nucleosides have the formula: 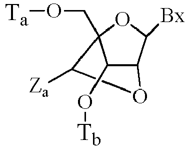 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thiol.

In one instance, each of the substituted groups, is, independently, mono or poly substituted with substituent groups independently selected from halogen, oxo, hydroxyl, OJ_{c}, NJ_{c}J_{d}, SJ_{c}, N₃, OC(=X)J_{c}, and NJₑC(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain instances, bicyclic nucleosides have the formula: 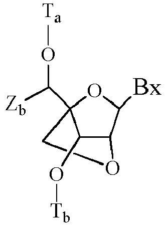 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain instances, bicyclic nucleosides have the formula: 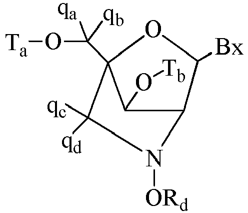 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;

In certain instances, bicyclic nucleosides have the formula: 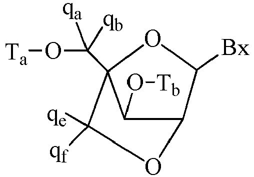 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJj, SJj, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of adenine, cytosine, guanine, 5-methyl-cytosine, thymine and uracil bicyclic nucleosides having a 4'-CH₂-O-2' bridge, along with their oligomerization, and nucleic acid recognition properties have been described (Koshkin et al., Tetrahedron, 1998, 54, 3607-3630). The synthesis of bicyclic nucleosides has also been described in WO 98/39352 and WO 99/14226.

Analogs of various bicyclic nucleosides that have 4' to 2' bridging groups such as 4'-CH₂-O-2' and 4'-CH₂-S-2', have also been prepared (Kumar et al., Bioorg. Med. Chem. Lett., 1998, 8, 2219-2222). Preparation of oligodeoxyribonucleotide duplexes comprising bicyclic nucleosides for use as substrates for nucleic acid polymerases has also been described (Wengel et al., WO 99/14226 ). Furthermore, synthesis of 2'-amino-BNA, a novel conformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain instances, bicyclic nucleosides have the formula: 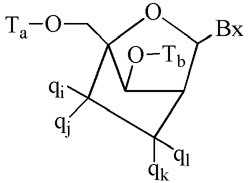 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and q₁ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJj, SJj, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJjJₖ or N(H)C(=S)NJⱼJₖ; and
qi and qⱼ or q₁ and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Frier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc. 2007, 129(26), 8362-8379).

In certain instances, bicyclic nucleosides include, but are not limited to, (A) α-L-methyleneoxy (4'-CH₂-O-2') BNA, (B) β-D-methyleneoxy (4'-CH₂-O-2') BNA, (C) ethyleneoxy (4'-(CH₂)₂-O-2') BNA, (D) aminooxy (4'-CH₂-O-N(R)-2') BNA, (E) oxyamino (4'-CH₂-N(R)-O-2') BNA, (F) methyl(methyleneoxy) (4'-CH(CH₃)-O-2') BNA (also referred to as constrained ethyl or cEt), (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, (J) propylene carbocyclic (4'-(CH₂)₃-2') BNA, and (K) vinyl BNA as depicted below. 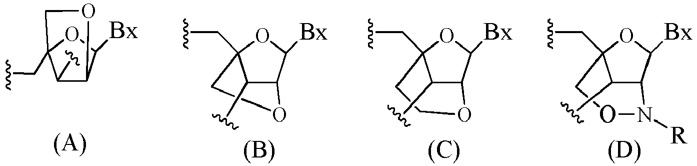 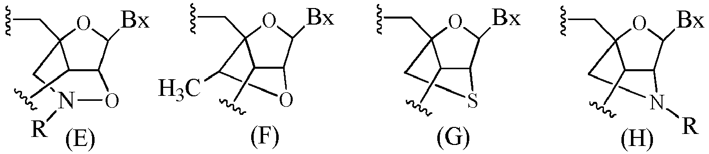 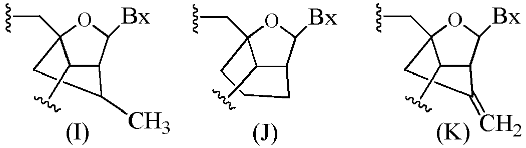 wherein Bx is the base moiety and R is, independently, H, a protecting group, C₁-C₆ alkyl or C₁-C₆ alkoxy.

As used herein, the term "modified tetrahydropyran nucleoside" or "modified THP nucleoside" means a nucleoside having a six-membered tetrahydropyran "sugar" substituted for the pentofuranosyl residue in normal nucleosides and can be referred to as a sugar surrogate. Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854) or fluoro HNA (F-HNA) having a tetrahydropyranyl ring system as illustrated below.

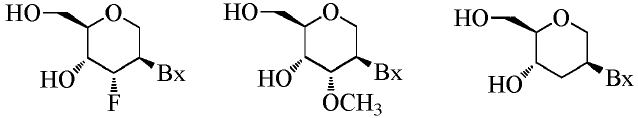

In certain instance, sugar surrogates are selected having the formula: 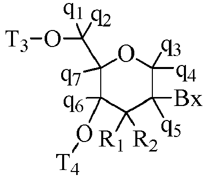 wherein:
Bx is a heterocyclic base moiety;
T₃ and T₄ are each, independently, an internucleoside linking group linking the tetrahydropyran nucleoside analog to the oligomeric compound or one of T₃ and T₄ is an internucleoside linking group linking the tetrahydropyran nucleoside analog to an oligomeric compound or oligonucleotide and the other of T₃ and T₄ is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and
one of R₁ and R₂ is hydrogen and the other is selected from halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.

In certain instances, q₁, q₂, q₃, q₄, q₅, q₆ and q₇ are each H. In certain instances, at least one of q₁, q₂, q₃, q₄, q₅, q₆ and q₇ is other than H. In certain instances, at least one of q₁, q₂, q₃, q₄, q₅, q₆ and q₇ is methyl. In certain instances, THP nucleosides are provided wherein one of R₁ and R₂ is F. In certain instances, R₁ is fluoro and R₂ is H; R₁ is methoxy and R₂ is H, and R₁ is methoxyethoxy and R₂ is H.

In certain instances, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506). As used here, the term "morpholino" means a sugar surrogate having the following formula: 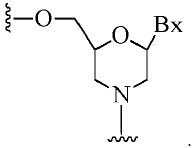 In certain instances, morpholinos may be modified, for example by adding or altering various substituent groups from the above morpholino structure. Such sugar surrogates are referred to herein as "modifed morpholinos."

Combinations of modifications are also described without limitation, such as 2'-F-5'-methyl substituted nucleosides (see PCT International Application WO 2008/101157 published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH₂-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described (*see, e.g.,* Srivastava et al., J. Am. Chem. Soc. 2007, 129(26), 8362-8379).

In certain instances, antisense compounds comprise one or more modified cyclohexenyl nucleosides, which is a nucleoside having a six-membered cyclohexenyl in place of the pentofuranosyl residue in naturally occurring nucleosides. Modified cyclohexenyl nucleosides include, but are not limited to those described in the art (see for example commonly owned, published PCT Application WO 2010/036696, published on April 10, 2010, Robeyns et al., J. Am. Chem. Soc., 2008, 130(6), 1979-1984; Horváth et al., Tetrahedron Letters, 2007, 48, 3621-3623; Nauwelaerts et al., J. Am. Chem. Soc., 2007, 129(30), 9340-9348; Gu et al.,, Nucleosides, Nucleotides & Nucleic Acids, 2005, 24(5-7), 993-998; Nauwelaerts et al., Nucleic Acids Research, 2005, 33(8), 2452-2463; Robeyns et al., Acta Crystallographica, Section F: Structural Biology and Crystallization Communications, 2005, F61(6), 585-586; Gu et a/., Tetrahedron, 2004, 60(9), 2111-2123; Gu et al., Oligonucleotides, 2003, 13(6), 479-489; Wang et al., J. Org. Chem., 2003, 68, 4499-4505; Verbeure et al., Nucleic Acids Research, 2001, 29(24), 4941-4947; Wang et al., J. Org. Chem., 2001, 66, 8478-82; Wang et al., Nucleosides, Nucleotides & Nucleic Acids, 2001, 20(4-7), 785-788; Wang et al., J. Am. Chem., 2000, 122, 8595-8602; Published PCT application, WO 06/047842; and Published PCT Application WO 01/049687). Certain modified cyclohexenyl nucleosides have Formula X. 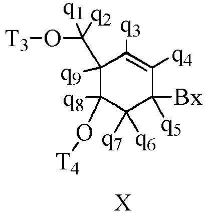 wherein independently for each of said at least one cyclohexenyl nucleoside analog of Formula X:
Bx is a heterocyclic base moiety;
T₃ and T₄ are each, independently, an internucleoside linking group linking the cyclohexenyl nucleoside analog to an antisense compound or one of T₃ and T₄ is an internucleoside linking group linking the tetrahydropyran nucleoside analog to an antisense compound and the other of T₃ and T₄ is H, a hydroxyl protecting group, a linked conjugate group, or a 5'-or 3'-terminal group; and
q₁, q₂, q₃, q₄, q₅, q₆, q₇, q₈ and q₉ are each, independently, H, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or other sugar substituent group.

Many other monocyclic, bicyclic and tricyclic ring systems are known in the art and are suitable as sugar surrogates that can be used to modify nucleosides for incorporation into oligomeric compounds as provided herein (see for example review article: Leumann, Christian J. Bioorg. & Med. Chem., 2002, 10, 841-854). Such ring systems can undergo various additional substitutions to further enhance their activity.

As used herein, "2'-modified sugar" means a furanosyl sugar modified at the 2' position. In certain instances, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain instances, 2' modifications are selected from substituents including, but not limited to: O[(CH₂)ₙO]ₘCH₃, O(CH₂)ₙNH₂, O(CH₂)ₙCH₃, O(CH₂)ₙF, O(CH₂)ₙONH₂, OCH₂C(=O)N(H)CH₃ and O(CH₂)ₙON[(CH₂)ₙCH₃]₂, where n and m are from 1 to about 10. Other 2'- substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain instances, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'- *O*-methyl, O-propyl, and O-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position of the sugar ring.

As used herein, "2'-OMe" or "2'-OCH₃", "2'-O-methyl" or "2'-methoxy" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

Methods for the preparations of modified sugars are well known to those skilled in the art. Some representative U.S. patents that teach the preparation of such modified sugars include without limitation, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,670,633; 5,700,920; 5,792,847 and 6,600,032 and International Application PCT/US2005/019219, filed June 2, 2005 and published as WO 2005/121371 on December 22, 2005.

As used herein, "oligonucleotide" refers to a compound comprising a plurality of linked nucleosides. In certain instances, one or more of the plurality of nucleosides is modified. In certain instances, an oligonucleotide comprises one or more ribonucleosides (RNA) and/or deoxyribonucleosides (DNA).

In nucleotides having modified sugar moieties, the nucleobase moieties (natural, modified or a combination thereof) are maintained for hybridization with an appropriate nucleic acid target.

In certain instances, antisense compounds comprise one or more nucleosides having modified sugar moieties. In certain instances, the modified sugar moiety is 2'-MOE. In certain instances, the 2'-MOE modified nucleosides are arranged in a gapmer motif. In certain instances, the modified sugar moiety is a bicyclic nucleoside having a (4'-CH(CH₃)-O-2') bridging group. In certain instances, the (4'-CH(CH₃)-O-2') modified nucleosides are arranged throughout the wings of a gapmer motif.

### Compositions and Methods for Formulating Pharmaceutical Compositions

Oligonucleotides may be admixed with pharmaceutically acceptable active or inert substances for the preparation of pharmaceutical compositions or formulations. Compositions and methods for the formulation of pharmaceutical compositions are dependent upon a number of criteria, including, but not limited to, route of administration, extent of disease, or dose to be administered.

An antisense compound targeted to a SOD-1 nucleic acid can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one instance, employed in the methods described herein is a pharmaceutical composition comprising an antisense compound targeted to a SOD-1 nucleic acid and a pharmaceutically acceptable diluent. In certain instances, the pharmaceutically acceptable diluent is PBS. In certain instances, the antisense compound is a modified oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an antisense compound which are cleaved by endogenous nucleases within the body, to form the active antisense compound.

### Conjugated Antisense Compounds

Antisense compounds may be covalently linked to one or more moieties or conjugates which enhance the activity, cellular distribution or cellular uptake of the resulting oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of SOD-1 nucleic acids can be tested *in vitro* in a variety of cell types. Cell types used for such analyses are available from commerical vendors (e.g. American Type Culture Collection, Manassus, VA; Zen-Bio, Inc., Research Triangle Park, NC; Clonetics Corporation, Walkersville, MD) and are cultured according to the vendor's instructions using commercially available reagents (e.g. Invitrogen Life Technologies, Carlsbad, CA). Illustrative cell types include, but are not limited to, HepG2 cells, Hep3B cells, primary hepatocytes, A431 cells, and SH-SY5Y cells.

### In vitro testing of oligonucleotides

Described herein are methods for treatment of cells with oligonucleotides, which can be modified appropriately for treatment with other antisense compounds.

Cells may be treated with oligonucleotides when the cells reach approximately 60-80% confluency in culture.

One reagent commonly used to introduce oligonucleotides into cultured cells includes the cationic lipid transfection reagent LIPOFECTIN (Invitrogen, Carlsbad, CA). Oligonucleotides may be mixed with LIPOFECTIN in OPTI-MEM 1 (Invitrogen, Carlsbad, CA) to achieve the desired final concentration of oligonucleotide and a LIPOFECTIN concentration that may range from 2 to 12 ug/mL per 100 nM oligonucleotide.

Another reagent used to introduce oligonucleotides into cultured cells includes LIPOFECTAMINE (Invitrogen, Carlsbad, CA). Oligonucleotide is mixed with LIPOFECTAMINE in OPTI-MEM 1 reduced serum medium (Invitrogen, Carlsbad, CA) to achieve the desired concentration of oligonucleotide and a LIPOFECTAMINE concentration that may range from 2 to 12 ug/mL per 100 nM oligonucleotide.

Another technique used to introduce oligonucleotides into cultured cells includes electroporation.

Cells are treated with oligonucleotides by routine methods. Cells may be harvested 16-24 hours after oligonucleotide treatment, at which time RNA or protein levels of target nucleic acids are measured by methods known in the art and described herein. In general, when treatments are performed in multiple replicates, the data are presented as the average of the replicate treatments.

The concentration of oligonucleotide used varies from cell line to cell line. Methods to determine the optimal oligonucleotide concentration for a particular cell line are well known in the art. Oligonucleotides are typically used at concentrations ranging from 1 nM to 300 nM when transfected with LIPOFECTAMINE. Oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art. RNA is prepared using methods well known in the art, for example, using the TRIZOL Reagent (Invitrogen, Carlsbad, CA) according to the manufacturer's recommended protocols.

### Analysis of inhibition of target levels or expression

Inhibition of levels or expression of a SOD-1 nucleic acid can be assayed in a variety of ways known in the art. For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels may be accomplished by quantitative real-time PCR using the ABI PRISM 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents may be obtained from Invitrogen (Carlsbad, CA). RT real-time-PCR reactions are carried out by methods well known to those skilled in the art.

Gene (or RNA) target quantities obtained by real time PCR are normalized using either the expression level of a gene whose expression is constant, such as cyclophilin A, or by quantifying total RNA using RIBOGREEN (Invitrogen, Inc. Carlsbad, CA). Cyclophilin A expression is quantified by real time PCR, by being run simultaneously with the target, multiplexing, or separately. Total RNA is quantified using RIBOGREEN RNA quantification reagent (Invetrogen, Inc. Eugene, OR). Methods of RNA quantification by RIBOGREEN are SOD-1ght in Jones, L.J., et al, (Analytical Biochemistry, 1998, 265, 368-374). A CYTOFLUOR 4000 instrument (PE Applied Biosystems) is used to measure RIBOGREEN fluorescence.

Probes and primers are designed to hybridize to a SOD-1 nucleic acid. Methods for designing real-time PCR probes and primers are well known in the art, and may include the use of software such as PRIMER EXPRESS Software (Applied Biosystems, Foster City, CA).

### Analysis of Protein Levels

Antisense inhibition of SOD-1 nucleic acids can be assessed by measuring SOD-1 protein levels. Protein levels of SOD-1 can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art. In certain instances, the compounds herein provide improved reduction in protein levels.

### In vivo testing of antisense compounds

Antisense compounds, for example, modified oligonucleotides, are tested in animals to assess their ability to inhibit expression of SOD-1 and produce phenotypic changes, such as, improved motor function. In certain instances, motor function is measured by walking initiation analysis, rotarod, grip strength, pole climb, open field performance, balance beam, hindpaw footprint testing in the animal. Testing may be performed in normal animals, or in experimental disease models. For administration to animals, oligonucleotides are formulated in a pharmaceutically acceptable diluent, such as phosphate-buffered saline. Administration includes parenteral routes of administration, such as intraperitoneal, intravenous, and subcutaneous. Oligonucleotide dosage and dosing frequency depends upon multiple factors such as, but not limited to, route of administration and animal body weight. Following a period of treatment with oligonucleotides, RNA is isolated from CNS tissue or CSF and changes in SOD-1 nucleic acid expression are measured.

### Certain Indications

In certain instances, described herein are methods, compounds, and compositions of treating an individual comprising administering one or more pharmaceutical compositions described herein. In certain instances, the individual has a neurodegenerative disease. In certain instances, the individual is at risk for developing a neurodegenerative disease, including, but not limited to, amyotrophic lateral sclerosis (ALS). In certain instances, the individual has been identified as having a SOD-1 associated disease. In certain instances, described herein are methods for prophylactically reducing SOD-1 expression in an individual. Certain instances include treating an individual in need thereof by administering to an individual a therapeutically effective amount of an antisense compound targeted to a SOD-1 nucleic acid.

In one instance, administration of a therapeutically effective amount of an antisense compound targeted to a SOD-1 nucleic acid is accompanied by monitoring of SOD-1 levels in an individual, to determine an individual's response to administration of the antisense compound. An individual's response to administration of the antisense compound may be used by a physician to determine the amount and duration of therapeutic intervention.

In certain instances, administration of an antisense compound targeted to a SOD-1 nucleic acid results in reduction of SOD-1 expression by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values. In certain instances, administration of an antisense compound targeted to a SOD-1 nucleic acid results in improved motor function in an animal. In certain instances, administration of a SOD-1 antisense compound improves motor function by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain instances, pharmaceutical compositions comprising an antisense compound targeted to SOD-1 are used for the preparation of a medicament for treating a patient suffering or susceptible to a neurodegenerative disease including amyotrophic lateral sclerosis (ALS).

### Certain Comparator Compositions

Antisense oligonucleotides targeting human SOD-1 were described in an earlier publication (see WO 2005/040180). Several oligonucleotides (ISIS 333611, ISIS 146144, ISIS 146145, ISIS 150437, ISIS 150441, ISIS 150443, ISIS 150444, ISIS 150445, ISIS 150446, ISIS 150447, ISIS 150448, ISIS 150449, ISIS 150452, ISIS 150454, ISIS 150458, ISIS 150460, ISIS 150462-150467, ISIS 150470, ISIS 150472, ISIS 150474, ISIS 150475, ISIS 150476, ISIS 150479-150483, ISIS 150488, ISIS 150489, ISIS 150490, ISIS 150491-150493, ISIS 150495-150498, ISIS 150511, ISIS 333605, ISIS 333606, ISIS 333609-333617, ISIS 333619, ISIS 333620-333636, ISIS 333638, and ISIS 333640) described therein, were used as comparator compounds throughout select screens for new antisense compounds described herein.

In certain instances, ISIS 333611, a 5-10-5 MOE gapmer, having a sequence of (from 5' to 3') CCGTCGCCCTTCAGCACGCA (incorporated herein as SEQ ID NO: 21), wherein each internucleoside linkage is a phosphorothioate linkage, each cytosine is a 5-methylcytosine, and each of nucleosides 1-5 and 16-20 (from 5' to 3') comprise a 2'-O-methoxyethyl moiety was used as a comparator compound. ISIS 333611 was selected as a comparator compound because it exhibited high levels of dose-dependent inhibition in various studies as described in WO 2005/040180. Additionally, phase 1 human clinical trials were completed using ISIS 333611. See, MILLER et al., "An antisense oligonucleotide against SOD1 delivered intrathecally for patients with SOD1 familial amyotrophic lateral sclerosis: a phase 1, randomised, first-in-man study" Lancet Neurol. (2013) 12(5): 435-442. Thus, ISIS 333611 was deemed a highly efficacious and potent compound with an acceptable safety profile (such that it was tested in human patients).

In certain instances, the compounds described herein benefit from one or more improved properties relative to the antisense compounds described in WO 2005/040180. Some of the improved properties are demonstrated in the examples described herein. In certain instances, compounds described herein are more efficacious, potent, and/or tolerable in various in vitro and in vivo studies than comparator compounds described herein, including ISIS 333611. In certain instances, ISIS 666853, ISIS 666859, ISIS 666919, ISIS 666921, ISIS 666922, ISIS 666869, ISIS 666870, and ISIS 666867 are more efficacious and/or potent in various in vitro and in vivo studies than comparator compounds described herein, including ISIS 333611. In certain instances, ISIS 666853, ISIS 666859, ISIS 666919, ISIS 666921, ISIS 666922, ISIS 666869, ISIS 666870, and ISIS 666867 are more tolerable in one or more tolerability assays in animals than comparator compounds described herein, including ISIS 333611. This is despite 333611 being sufficiently well tolerated to progress to human clinical trials.

In certain instances, certain compounds described herein are more efficacious than comparator compounds by virtue of an *in vitro* IC50 of less than 2 µM, less than 1.9 µM, less than 1.8 µM, less than 1.7µM, less than 1.6 µM, less than 1.5 µM, less than 1.4 µM, less than 1.3 µM, less than 1.2 µM, less than 1.1 µM, less than 1 µM, less than 0.9 µM, less than 0.8 µM, less than 0.7 µM, less than 0.6 µM, or less than 0.5 µM less than 0.4 µM, less than 0.3 µM, less than 0.2 µM, less than 0.1 µM, when tested in human cells, for example, in the HepG2 A431 or SH-SY5Y cell lines (For example, see Examples 6-11).

In certain instances, certain compounds described herein are more efficacious than comparator compounds by virtue of their ability to inhibit SOD-1 expression in vivo. In certain instances, the compounds inhibit SOD-1 in lumbar spinal cord and cervical spinal cord by at least 60%, at least 65%, at least 70%, at least 75%, at least 80%, at least 85%, at least 90% or at least 95% in, for example, a transgenic animal model.

In certain instances, certain compounds described herein are more tolerable than comparator compounds on the basis of reduced microglial marker levels (e.g.,IBA1), reduced astrocytic marker levels (e.g., GFAP), and/or FOB scores in rats, mice, and/or monkeys. See, for example, Examples 14, 15, 18, and 19.

### ISIS 666853

For example, as provided in Example 12 (hereinbelow), ISIS 666853 achieved 81% inhibition in lumbar spinal cord and 74% in cervical spinal cord of an SOD-1 transgenic rat model when dosed with 30 µL of 16.67 mg/ml solution of oligonucleotide diluted in PBS (500 µg final dose), whereas ISIS 333611 achieved 51% inhibition in lumbar spinal cord and 47% inhibition in cervical spinal cord.

For example, as provided in Example 14 (hereinbelow), ISIS 666853 achieved a FOB score of 0 whereas ISIS 333611 achieved a FOB score of 4 in Sprague-Dawley rats after 3 hours when treated with 3 mg of oligonucleotide. Microglial marker (IBA1) levels and astrocytic marker (GFAP) levels were also reduced in ISIS 666853 treated rats as compared to ISIS 333611 treated rats.

For example, as provided in Example 15 (hereinbelow), ISIS 666853 achieved an ED₅₀ of 81.3 and 242.6 in lumbar tissue and cervical tissue (respectively) in SOD-1 transgenic rats when treated intrathecally with 10, 30, 100, 300, or 3000 µg of oligonucleotide. ED₅₀ in lumbar and cervical tissues could not be calculated in ISIS 333611 treated transgenic rats because the highest concentration tested (3000 µg) filed to inhibit human SOD-1 mRNA greater than 55-65%.

For example, as provided in Example 16 (hereinbelow), at doses of 1 mg and 3 mg ISIS 666853 achieved 3 hour FOB scores of 0.0 and 0.5 (respectively) whereas ISIS 333611 achieved FOB scores of 3.0 and 4.9 (respectively). At doses of 1 mg and 3 mg ISIS 666853 achieved 8 week FOB scores of 0.0 and 0.0 (respectively) whereas ISIS 333611 achieved FOB scores of 0.0 and 1.2 (respectively).

For example, as provided in Example 17 (hereinbelow), ISIS 666853 achieved an ED₅₀ of 136 and 188 in lumbar tissue and cortex tissue (respectively) whereas ISIS 333611 achieved an ED₅₀ of 401 and 786 in lumbar tissue and cortex tissue (respectively) in SOD-1 transgenic mice when treated with an intracerebral ventricular bolus of 10, 30, 100, 300, or 700 µg of oligonucleotide.

For example, as provided in Example 18 (hereinbelow), ISIS 666853 achieved a FOB score of 1.25 whereas ISIS 333611 achieved a FOB score of 6.5 in C57bl6 mice after 3 hours when treated with 700 µg of oligonucleotide. Microglial marker (IBA1) levels and astrocytic marker (GFAP) levels were also reduced in ISIS 666853 treated mice as compared to ISIS 333611 treated mice.

### ISIS 666859

For example, as provided in Example 12 (hereinbelow), ISIS 666859 achieved 79% inhibition in lumbar spinal cord and 64% inhibition in cervical spinal cord of an SOD-1 transgenic rat model when dosed with 30 (µL of 16.67 mg/ml solution of oligonucleotide diluted in PBS (500 µg final dose), whereas ISIS 333611 achieved 51% inhibition in lumbar spinal cord and 47% inhibition in cervical spinal cord.

For example, as provided in Example 14 (hereinbelow), ISIS 666859 achieved a FOB score of 1 whereas ISIS 333611 achieved a FOB score of 4 in Sprague-Dawley rats after 3 hours when treated with 3 mg of oligonucleotide. Microglial marker (IBA1) levels and astrocytic marker (GFAP) levels were also reduced in ISIS 666859 treated rats as compared to ISIS 333611 treated rats.

For example, as provided in Example 15 (hereinbelow), ISIS 666859 achieved an ED₅₀ of 74.0 and 358.8 in lumbar tissue and cervical tissue (respectively) in SOD-1 transgenic rats when treated intrathecally with 10, 30, 100, 300, or 3000 µg of oligonucleotide. ED₅₀ in lumbar and cervical tissues could not be calculated in ISIS 333611 treated transgenic rats because the highest concentration tested (3000 µg) filed to inhibit human SOD-1 mRNA greater than 55-65%.

For example, as provided in Example 16 (hereinbelow), at doses of 1 mg and 3 mg ISIS 666859 achieved 3 hour FOB scores of 0.0 and 2.1 (respectively) whereas ISIS 333611 achieved FOB scores of 3.0 and 4.9 (respectively). At doses of 1 mg and 3 mg ISIS 666859 achieved 8 week FOB scores of 0.0 and 0.3 (respectively) whereas ISIS 333611 achieved FOB scores of 0.0 and 1.2 (respectively).

For example, as provided in Example 17 (hereinbelow), ISIS 666859 achieved an ED₅₀ of 106 and 206 in lumbar tissue and cortex tissue (respectively) whereas ISIS 333611 achieved an ED₅₀ of 401 and 786 in lumbar tissue and cortex tissue (respectively) in SOD-1 transgenic mice when treated with an intracerebral ventricular bolus of 10, 30, 100, 300, or 700 µg of oligonucleotide.

For example, as provided in Example 18 (hereinbelow), ISIS 666859 achieved a FOB score of 1.75 whereas ISIS 333611 achieved a FOB score of 6.5 in C57bl6 mice after 3 hours when treated with 700 µg of oligonucleotide. Microglial marker (IBA1) levels and astrocytic marker (GFAP) levels were also reduced in ISIS 666859 treated mice as compared to ISIS 333611 treated mice.

### ISIS 666919

For example, as provided in Example 12 (hereinbelow), ISIS 666919 achieved 76% inhibition in lumbar spinal cord and 68% in cervical spinal cord of an SOD-1 transgenic rat model when dosed with 30 µL of 16.67 mg/ml solution of oligonucleotide diluted in PBS (500 µg final dose), whereas ISIS 333611 achieved 51% inhibition in lumbar spinal cord and 47% inhibition in cervical spinal cord.

For example, as provided in Example 14 (hereinbelow), ISIS 666919 achieved a FOB score of 2 whereas ISIS 333611 achieved a FOB score of 4 in Sprague-Dawley rats after 3 hours when treated with 3 mg of oligonucleotide. Microglial marker (IBA1) levels and astrocytic marker (GFAP) levels were also reduced in ISIS 666919 treated rats as compared to ISIS 333611 treated rats.

For example, as provided in Example 15 (hereinbelow), ISIS 666919 achieved an ED₅₀ of 104.1 and 613.5 in lumbar tissue and cervical tissue (respectively) in SOD-1 transgenic rats when treated intrathecally with 10, 30, 100, 300, or 3000 µg of oligonucleotide. ED₅₀ in lumbar and cervical tissues could not be calculated in ISIS 333611 treated transgenic rats because the highest concentration tested (3000 µg) filed to inhibit human SOD-1 mRNA greater than 55-65%.

For example, as provided in Example 16 (hereinbelow), at doses of 1 mg and 3 mg ISIS 666919 achieved 3 hour FOB scores of 1.3 and 3.5 (respectively) whereas ISIS 333611 achieved FOB scores of 3.0 and 4.9 (respectively). At doses of 1 mg and 3 mg ISIS 666919 achieved 8 week FOB scores of 0.0 and 0.1 (respectively) whereas ISIS 333611 achieved FOB scores of 0.0 and 1.2 (respectively).

For example, as provided in Example 17 (hereinbelow), ISIS 666919 achieved an ED₅₀ of 168 in lumbar tissue whereas ISIS 333611 achieved an ED₅₀ of 401 in lumbar tissue in SOD-1 transgenic mice when treated with an intracerebral ventricular bolus of 10, 30, 100, 300, or 700 µg of oligonucleotide.

For example, as provided in Example 18 (hereinbelow), ISIS 666919 achieved a FOB score of 0.0 whereas ISIS 333611 achieved a FOB score of 6.5 in C57bl6 mice after 3 hours when treated with 700 µg of oligonucleotide. Microglial marker (IBA1) levels and astrocytic marker (GFAP) levels were also reduced in ISIS 666919 treated mice as compared to ISIS 333611 treated mice.

### ISIS 666921

For example, as provided in Example 12 (hereinbelow), ISIS 66621 achieved 71% inhibition in lumbar spinal cord and 65% in cervical spinal cord of an SOD-1 transgenic rat model when dosed with 30 µL of 16.67 mg/ml solution of oligonucleotide diluted in PBS (500 µg final dose), whereas ISIS 333611 achieved 51% inhibition in lumbar spinal cord and 47% inhibition in cervical spinal cord.

For example, as provided in Example 14 (hereinbelow), ISIS 666921 achieved a FOB score of 2 whereas ISIS 333611 achieved a FOB score of 4 in Sprague-Dawley rats after 3 hours when treated with 3 mg of oligonucleotide. Microglial marker (IBA1) levels and astrocytic marker (GFAP) levels were also reduced in ISIS 666919 treated rats as compared to ISIS 333611 treated rats.

### ISIS 666922

For example, as provided in Example 12 (hereinbelow), ISIS 666922 achieved 67% inhibition in lumbar spinal cord and 62% in cervical spinal cord of an SOD-1 transgenic rat model when dosed with 30 µL of 16.67 mg/ml solution of oligonucleotide diluted in PBS (500 µg final dose), whereas ISIS 333611 achieved 51% inhibition in lumbar spinal cord and 47% inhibition in cervical spinal cord.

For example, as provided in Example 14 (hereinbelow), ISIS 666922 achieved a FOB score of 3 whereas ISIS 333611 achieved a FOB score of 4 in Sprague-Dawley rats after 3 hours when treated with 3 mg of oligonucleotide. Microglial marker (IBA1) levels and astrocytic marker (GFAP) levels were also reduced in ISIS 666919 treated rats as compared to ISIS 333611 treated rats.

### ISIS 666869

For example, as provided in Example 12 (hereinbelow), ISIS 666869 achieved 82% inhibition in lumbar spinal cord and 81% in cervical spinal cord of an SOD-1 transgenic rat model when dosed with 30 µL of 16.67 mg/ml solution of oligonucleotide diluted in PBS (500 µg final dose), whereas ISIS 333611 achieved 51% inhibition in lumbar spinal cord and 47% inhibition in cervical spinal cord.

### ISIS 666870

For example, as provided in Example 12 (hereinbelow), ISIS 666870 achieved 76% inhibition in lumbar spinal cord and 68% in cervical spinal cord of an SOD-1 transgenic rat model when dosed with 30 µL of 16.67 mg/ml solution of oligonucleotide diluted in PBS (500 µg final dose), whereas ISIS 333611 achieved 51% inhibition in lumbar spinal cord and 47% inhibition in cervical spinal cord.

For example, as provided in Example 15 (hereinbelow), ISIS 666870 achieved an ED₅₀ of 139.4 and 1111 in lumbar tissue and cervical tissue (respectively) in SOD-1 transgenic rats when treated intrathecally with 10, 30, 100, 300, or 3000 µg of oligonucleotide. ED₅₀ in lumbar and cervical tissues could not be calculated in ISIS 333611 treated transgenic rats because the highest concentration tested (3000 µg) filed to inhibit human SOD-1 mRNA greater than 55-65%.

For example, as provided in Example 17 (hereinbelow), ISIS 666870 achieved an ED₅₀ of 148 and 409 in lumbar tissue and cortex tissue (respectively) whereas ISIS 333611 achieved an ED₅₀ of 401 and 786 in lumbar tissue and cortex tissue (respectively) in SOD-1 transgenic mice when treated with an intracerebral ventricular bolus of 10, 30, 100, 300, or 700 µg of oligonucleotide.

For example, as provided in Example 18 (hereinbelow), ISIS 666870 achieved a FOB score of 4.75 whereas ISIS 333611 achieved a FOB score of 6.5 in C57bl6 mice after 3 hours when treated with 700 µg of oligonucleotide.

### ISIS 666867

For example, as provided in Example 12 (hereinbelow), ISIS 666867 achieved 59% inhibition in lumbar spinal cord and 48% in cervical spinal cord of an SOD-1 transgenic rat model when dosed with 30 µL of 16.67 mg/ml solution of oligonucleotide diluted in PBS (500 µg final dose), whereas ISIS 333611 achieved 51% inhibition in lumbar spinal cord and 47% inhibition in cervical spinal cord.

### Certain Compositions

### 1. ISIS 666853

In certain embodimentinstances, ISIS 666853 is characterized as a 5-10-5 MOE gapmer, having a sequence of (from 5' to 3') CAGGATACATTTCTACAGCT (incorporated herein as SEQ ID NO: 725), wherein each of nucleosides 1-5 and 16-20 are 2'-O-methoxyethylribose modified nucleosides, and each of nucleosides 6-15 are 2'-deoxynucleosides, wherein the internucleoside linkages between nucleosides 2 to 3, 4 to 5, 16 to 17, and 18 to 19 are phosphodiester linkages and the internucleoside linkages between nucleosides 1 to 2, 3 to 4, 5 to 6, 6 to 7, 7 to 8, 8 to 9, 9 to 10, 10 to 11, 11 to 12, 12 to 13, 13 to 14, 14 to 15, 15 to 16, 17 to 18, and 19 to 20 are phosphorothioate linkages, and wherein each cytosine is a 5'-methylcytosine.

In certain instances, ISIS 666853 is described by the following chemical notation: mCes Aeo Ges Geo Aes Tds Ads mCds Ads Tds Tds Tds mCds Tds Ads mCeo Aes Geo mCes Te; wherein,
A = an adenine,
mC = a 5'-methylcytosine
G = a guanine,
T = a thymine,
e = a 2'-O-methoxyethylribose modified sugar,
d = a 2'-deoxyribose sugar,
s = a phosphorothioate internucleoside linkage, and
o = a phosphodiester internucleoside linkage.

In certain instances, ISIS 666853 is described by the following chemical structure:

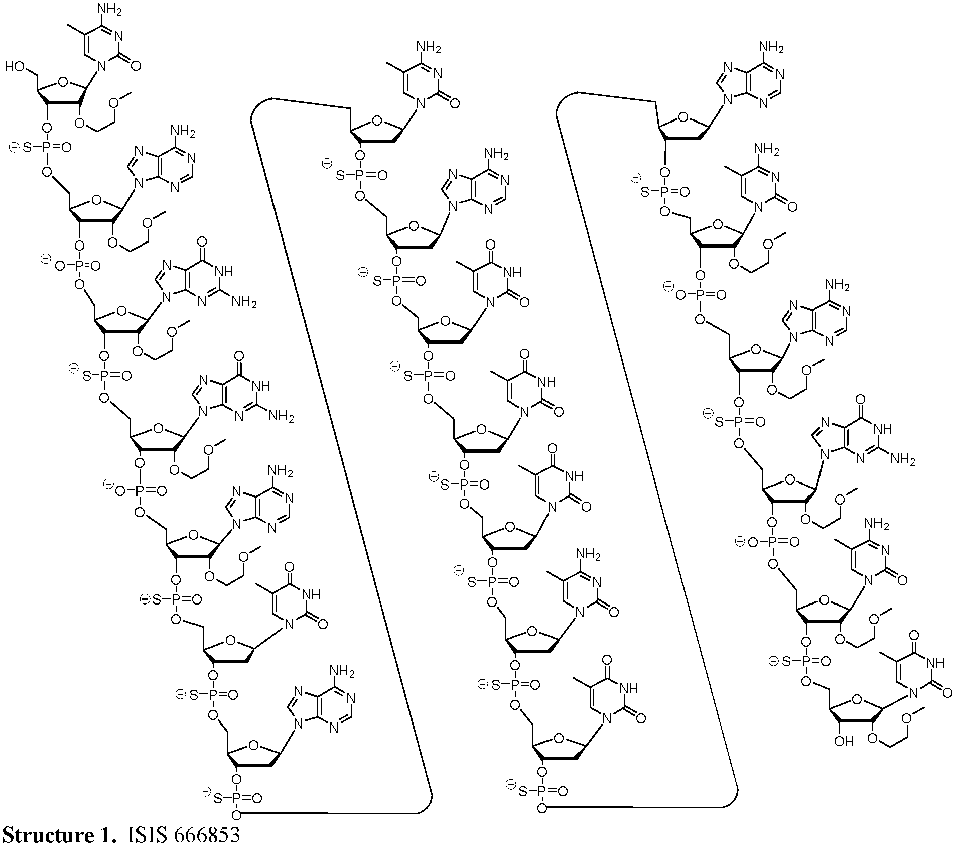

### 2. ISIS 666859

In certain instances, ISIS 666859 is characterized as a modified oligonucleotide having the nucleobase sequence (from 5' to 3') TTAATGTTTATCAGGAT (incorporated herein as SEQ ID NO: 1351), consisting of seventeen nucleosides, wherein each of nucleosides 1-4 and 15-17 are 2'-O-methoxyethylribose nucleosides, wherein each of nucleosides 13 and 14 are cEt modified nucleosides, wherein each of nucleosides 5-12 are 2'-deoxyribonucleosides, wherein the internucleoside linkages between nucleosides 2 to 3, 3 to 4, 13 to 14, 14 to 15 are phosphodiester linkages and the internucleoside linkages between nucleosides 1 to 2, 4 to 5, 5 to 6, 6 to 7, 7 to 8, 8 to 9, 9 to 10, 10 to 11, 11 to 12, 12 to 13, 15 to 16, and 16 to 17 are phosphorothioate linkages, and wherein each cytosine is a 5'-methylcytosine.

In certain instances, ISIS 666859 is described by the following chemical notation: Tes Teo Aeo Aes Tds Gds Tds Tds Tds Ads Tds mCds Ako Gko Ges Aes Te; wherein,
A = an adenine,
mC = a 5'-methylcytosine
G = a guanine,
T = a thymine,
e = a 2'-O-methoxyethylribose modified sugar,
k = a cEt modified sugar,
d = a 2'-deoxyribose sugar,
s = a phosphorothioate internucleoside linkage, and
o = a phosphodiester internucleoside linkage.

In certain instances, ISIS 666859 is described by the following chemical structure:

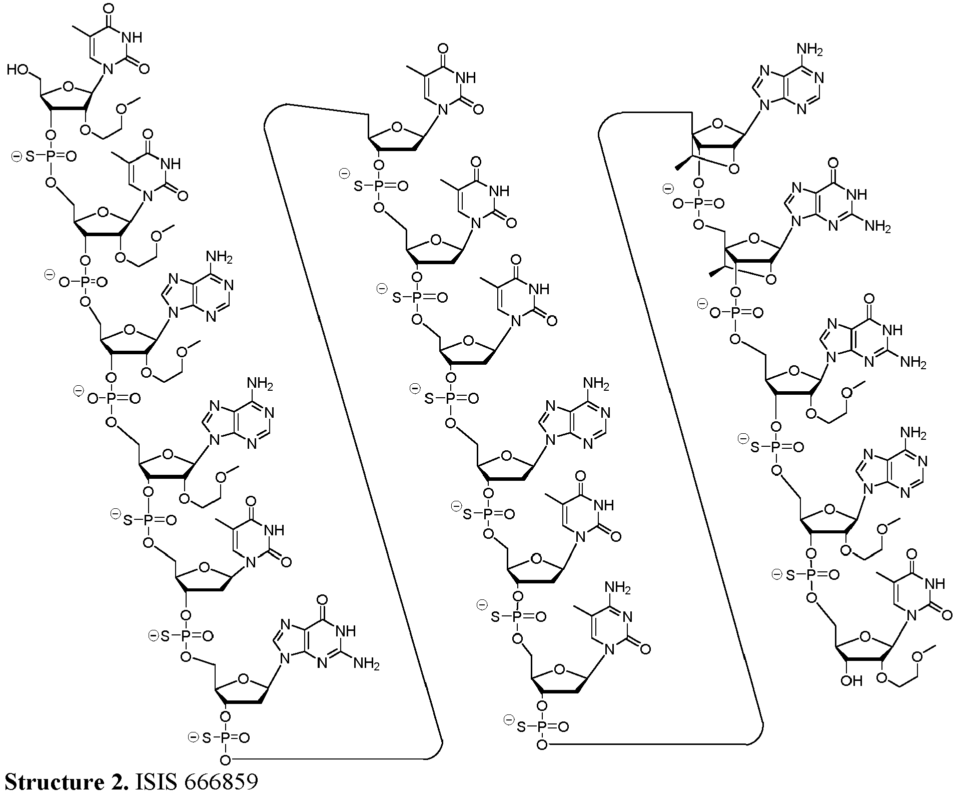

### 3. ISIS 666919

In certain instances, ISIS 666919 is characterized as a modified oligonucleotide having the nucleobase sequence (from 5' to 3') GGATACATTTCTACAGC (incorporated herein as SEQ ID NO: 1342), consisting of seventeen nucleosides, wherein each of nucleosides 1-4 and 16-17 are 2'-O-methoxyethylribose modified nucleosides, wherein each of nucleosides 14 and 15 are cEt modified nucleosides, wherein each of nucleosides 5-13 are 2'-deoxyribonucleosides, wherein the internucleoside linkages between nucleosides 2 to 3, 3 to 4, 4 to 5, and 14 to 15 are phosphodiester linkages and the internucleoside linkages between nucleosides 1 to 2, 5 to 6, 6 to 7, 7 to 8, 8 to 9, 9 to 10, 10 to 11, 11 to 12, 12 to 13, 13 to 14, 15 to 16, and 16 to 17 are phosphorothioate linkages, and wherein each cytosine is a 5'-methylcytosine.

In certain instances, ISIS 666919 is described by the following chemical notation: Ges Geo Aeo Teo Ads mCds Ads Tds Tds Tds mCds Tds Ads mCko Aks Ges mCe; wherein,
A = an adenine,
mC = a 5'-methylcytosine
G = a guanine,
T = a thymine,
e = a 2'-O-methoxyethylribose modified sugar,
k = a cEt modified sugar,
d = a 2'-deoxyribose sugar,
s = a phosphorothioate internucleoside linkage, and
o = a phosphodiester internucleoside linkage.

In certain instances, ISIS 666919 is described by the following chemical structure:

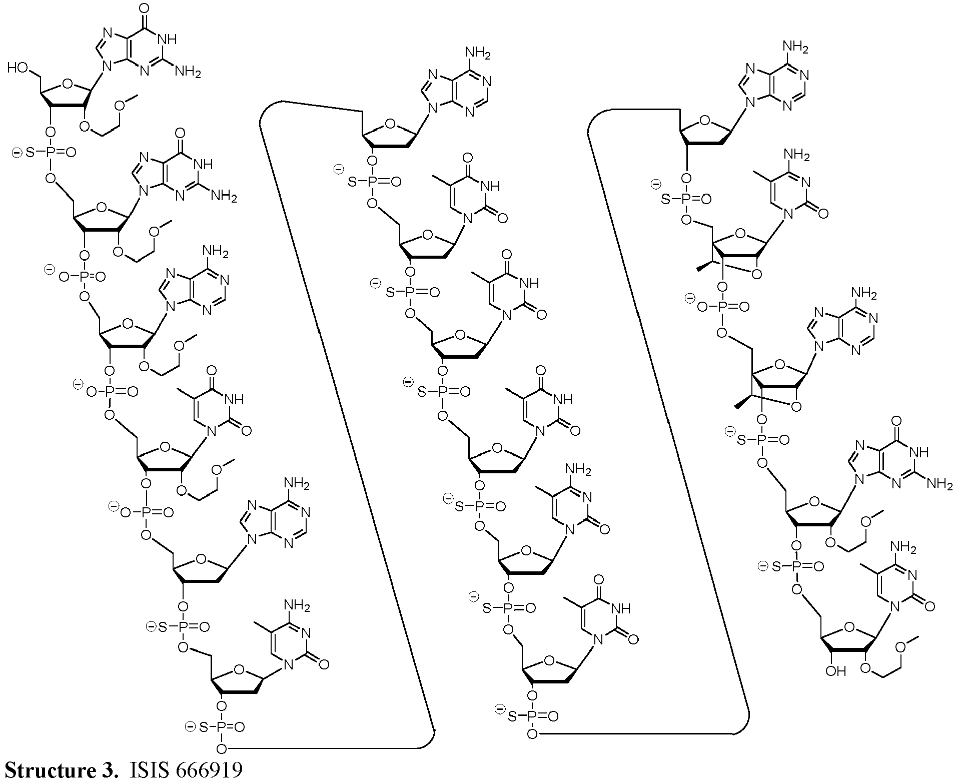

### 4. ISIS 666921

In certain instances, ISIS 666921 is characterized as a modified oligonucleotide having the nucleobase sequence (from 5' to 3') GGATACATTTCTACAGC (incorporated herein as SEQ ID NO: 1342), consisting of seventeen nucleosides, wherein each of nucleosides 1-5 and 16-17 are 2'-O-methoxyethylribose modified nucleosides, wherein each of nucleosides 14-15 are cEt modified nucleosides, wherein each of nucleosides 6-13 are 2'-deoxyribonucleosides, wherein the internucleoside linkages between nucleosides 2 to 3, 3 to 4, 4 to 5, and 14 to 15 are phosphodiester linkages and the internucleoside linkages between nucleosides 1 to 2, 5 to 6, 6 to 7, 7 to 8, 8 to 9, 9 to 10, 10 to 11, 11 to 12, 12 to 13, 13 to 14, 15 to 16, and 16 to 17 are phosphorothioate linkages, and wherein each cytosine is a 5'-methylcytosine.

In certain instances, ISIS 666921 is described by the following chemical notation: Ges Geo Aeo Teo Aes mCds Ads Tds Tds Tds mCds Tds Ads mCko Aks Ges mCe; wherein,
A = an adenine,
mC = a 5'-methylcytosine
G = a guanine,
T = a thymine,
e = a 2'-O-methoxyethylribose modified sugar,
k = a cEt modified sugar,
d = a 2'-deoxyribose sugar,
s = a phosphorothioate internucleoside linkage, and
o = a phosphodiester internucleoside linkage.

In certain instances, ISIS 666921 is described by the following chemical structure:

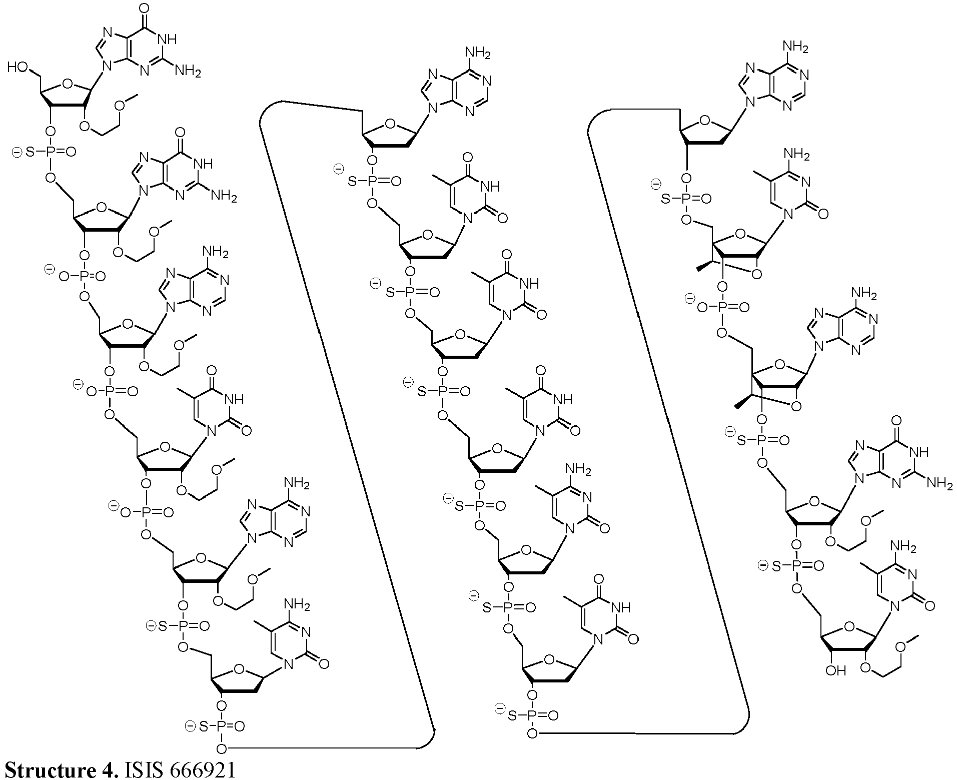

### 5. ISIS 666922

In certain instances, ISIS 666922 is characterized as a modified oligonucleotide having the nucleobase sequence (from 5' to 3') GGATACATTTCTACAGC (incorporated herein as SEQ ID NO: 1342), consisting of seventeen nucleosides, wherein each of nucleosides 1-4 and 15-17 are 2'-O-methoxyethylribose modified nucleosides, wherein each of nucleosides 5 and 14 are cEt modified nucleosides, wherein each of nucleosides 6-13 are 2'-deoxyribonucleosides, wherein the internucleoside linkages between nucleosides 2 to 3, 3 to 4, 4 to 5, and 14 to 15 are phosphodiester linkages and the internucleoside linkages between nucleosides 1 to 2, 5 to 6, 6 to 7, 7 to 8, 8 to 9, 9 to 10, 10 to 11, 11 to 12, 12 to 13, 13 to 14, 15 to 16, and 16 to 17 are phosphorothioate linkages, and wherein each cytosine is a 5'-methylcytosine.

In certain instances, ISIS 666922 is described by the following chemical notation: Ges Geo Aeo Teo Aks mCds Ads Tds Tds Tds mCds Tds Ads mCko Aes Ges mCe; wherein,
A = an adenine,
mC = a 5'-methylcytosine
G = a guanine,
T = a thymine,
e = a 2'-O-methoxyethylribose modified sugar,
k = a cEt modified sugar,
d = a 2'-deoxyribose sugar,
s = a phosphorothioate internucleoside linkage, and
o = a phosphodiester internucleoside linkage.

In certain instances, ISIS 666922 is described by the following chemical structure:

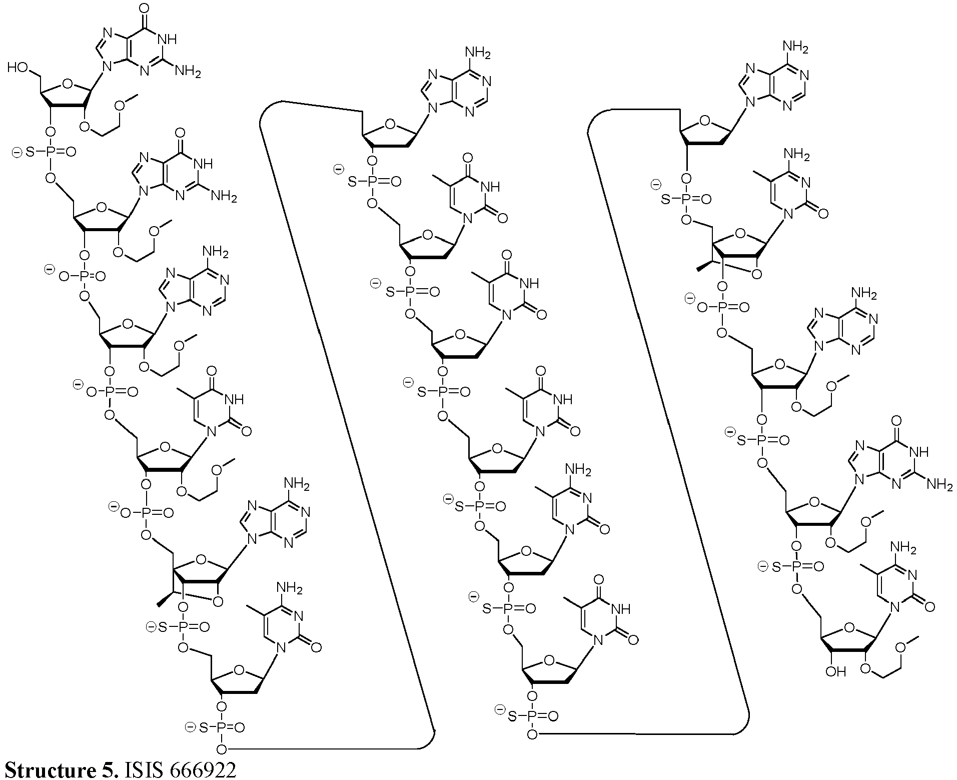

### 6. ISIS 666869

In certain instances, ISIS 666869 is characterized as a modified oligonucleotide having the nucleobase sequence (from 5' to 3') AGTGTTTAATGTTTATC (incorporated herein as SEQ ID NO: 1173), consisting of seventeen nucleosides, wherein each of nucleosides 1, 3, 14, and 16-17 are 2'-O-methoxyethylribose modified nucleosides, wherein each of nucleosides 2, 4, 13, and 15 are cEt modified nucleosides, wherein each of nucleosides 5-12 are 2'-deoxyribonucleosides, wherein the internucleoside linkages between nucleosides 2 to 3, 3 to 4, 13 to14, and 14 to 15 are phosphodiester linkages and the internucleoside linkages between nucleosides 1 to 2, 4 to 5, 5 to 6, 6 to 7, 7 to 8, 8 to 9, 9 to 10, 10 to 11, 11 to 12, 12 to 13, 15 to 16, and 16 to 17 are phosphorothioate linkages, and wherein each cytosine is a 5'-methylcytosine.

In certain instances, ISIS 666869 is described by the following chemical notation: Aes Gko Teo Gks Tds Tds Tds Ads Ads Tds Gds Tds Tko Teo Aks Tes mCe; wherein,
A = an adenine,
mC = a 5'-methylcytosine
G = a guanine,
T = a thymine,
e = a 2'-O-methoxyethylribose modified sugar,
k = a cEt modified sugar,
d = a 2'-deoxyribose sugar,
s = a phosphorothioate internucleoside linkage, and
o = a phosphodiester internucleoside linkage.

In certain instances, ISIS 666869 is described by the following chemical structure:

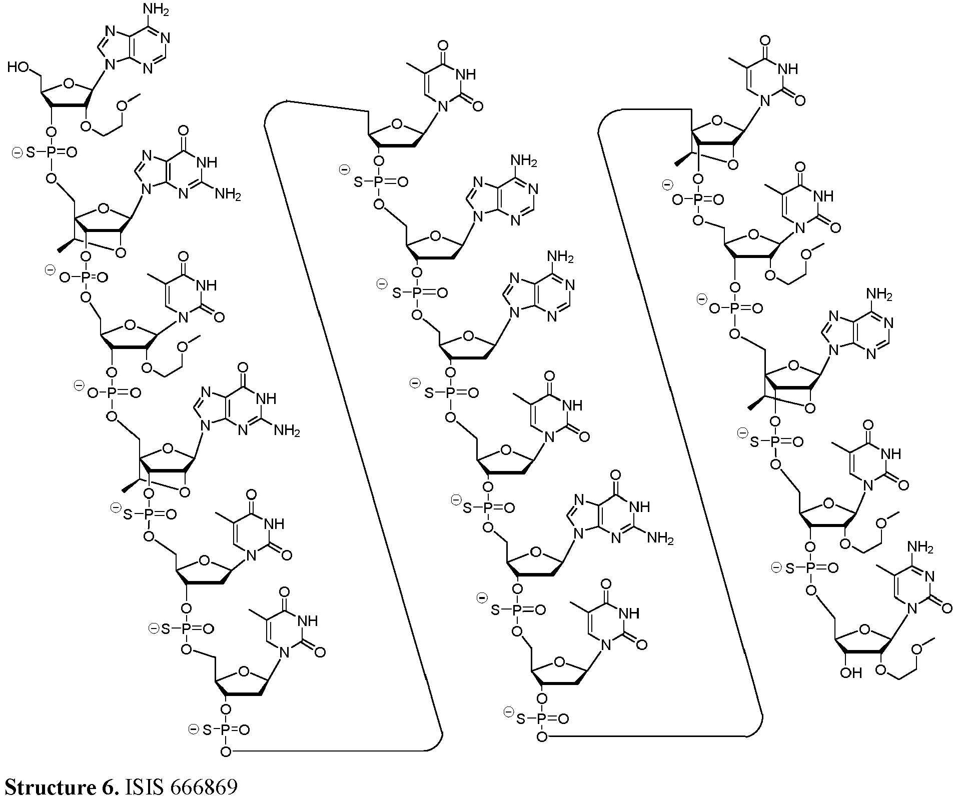

### 7. ISIS 666870

In certain instances, ISIS 666870 is characterized as a modified oligonucleotide having the nucleobase sequence (from 5' to 3') AGTGTTTAATGTTTATC (incorporated herein as SEQ ID NO: 1173), consisting of seventeen nucleosides, wherein each of nucleosides 1, 3, 13-17 are 2'-O-methoxyethylribose modified nucleosides, wherein each of nucleosides 2 and 4 are cEt modified nucleosides, wherein each of nucleosides 5-12 are 2'-deoxyribonucleosides, wherein the internucleoside linkages between nucleosides 2 to 3, 3 to 4, 13 to14, and 14 to 15 are phosphodiester linkages and the internucleoside linkages between nucleosides 1 to 2, 4 to 5, 5 to 6, 6 to 7, 7 to 8, 8 to 9, 9 to 10, 10 to 11, 11 to 12, 12 to 13, 15 to 16, and 16 to 17 are phosphorothioate linkages, and wherein each cytosine is a 5'-methylcytosine.

In certain instances, ISIS 666870 is described by the following chemical notation: Aes Gko Teo Gks Tds Tds Tds Ads Ads Tds Gds Tds Teo Teo Aes Tes mCe; wherein,
A = an adenine,
mC = a 5'-methylcytosine
G = a guanine,
T = a thymine,
e = a 2'-O-methoxyethylribose modified sugar,
k = a cEt modified sugar,
d = a 2'-deoxyribose sugar,
s = a phosphorothioate internucleoside linkage, and
o = a phosphodiester internucleoside linkage.

In certain instances, ISIS 666870 is described by the following chemical structure:

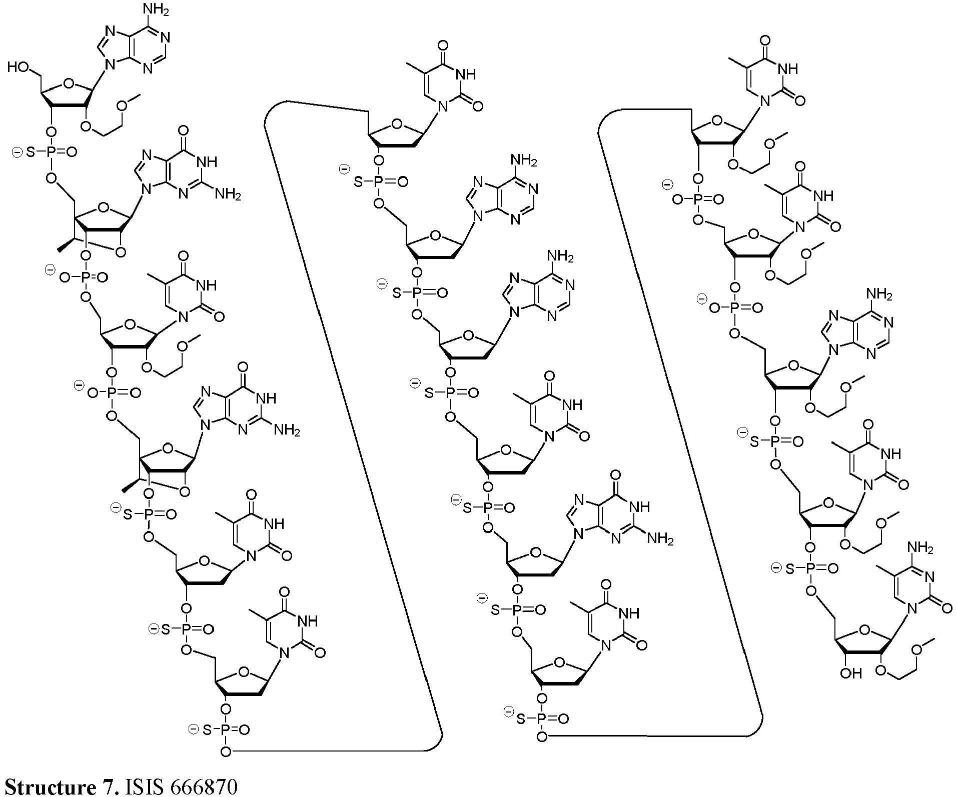

### 8. ISIS 666867

In certain instances, ISIS 666867 is characterized as a modified oligonucleotide having the nucleobase sequence (from 5' to 3') AGTGTTTAATGTTTATC (incorporated herein as SEQ ID NO: 1173), consisting of seventeen nucleosides, wherein each of nucleosides 1-2 and 13-17 are 2'-O-methoxyethylribose modified nucleosides, wherein each of nucleosides 3 and 4 are cEt modified nucleosides, wherein each of nucleosides 5-12 are 2'-deoxyribonucleosides, wherein the internucleoside linkages between nucleosides 2 to 3, 3 to 4, 13 to14, and 14 to 15 are phosphodiester linkages and the internucleoside linkages between nucleosides 1 to 2, 4 to 5, 5 to 6, 6 to 7, 7 to 8, 8 to 9, 9 to 10, 10 to 11, 11 to 12, 12 to 13, 15 to 16, and 16 to 17 are phosphorothioate linkages, and wherein each cytosine is a 5'-methylcytosine.

In certain instances, ISIS 666867 is described by the following chemical notation: Aes Geo Tko Gks Tds Tds Tds Ads Ads Tds Gds Tds Teo Teo Aes Tes mCe; wherein,
A = an adenine,
mC = a 5'-methylcytosine
G = a guanine,
T = a thymine,
e = a 2'-O-methoxyethylribose modified sugar,
k = a cEt modified sugar,
d = a 2'-deoxyribose sugar,
s = a phosphorothioate internucleoside linkage, and
o = a phosphodiester internucleoside linkage.

In certain instances, ISIS 666867 is described by the following chemical structure:

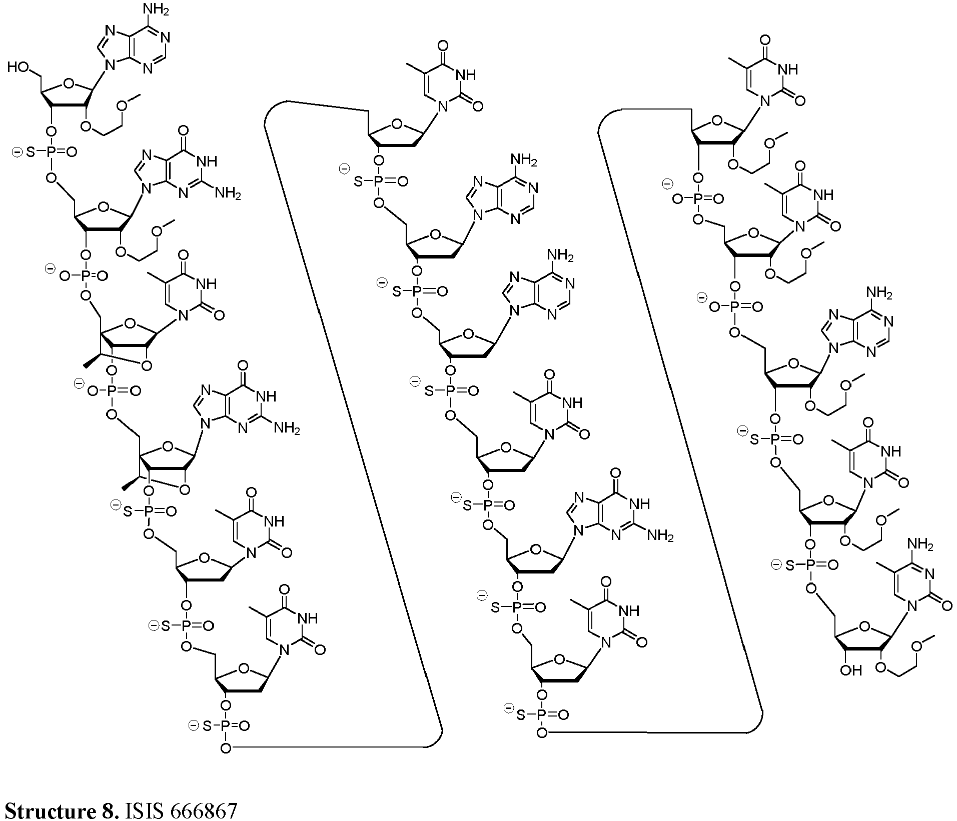

### EXAMPLES

### Non-limiting disclosure

While certain compounds, compositions, and methods described herein have been described with specificity in accordance with certain instances, the following examples serve only to illustrate the compounds described herein and are not intended to limit the same.

### Example 1: Inhibition of human superoxide dismutase 1, soluble (SOD-1) in HepG2 cells by MOE gapmers

Modified oligonucleotides were designed targeting a superoxide dismutase 1, soluble (SOD-1) nucleic acid and were tested for their effects on SOD-1 mRNA in vitro. ISIS 146144, ISIS 146145, ISIS 150437, ISIS 150441, ISIS 150443, ISIS 150444, ISIS 150445, ISIS 150446, ISIS 150447, ISIS 150448, ISIS 150449, ISIS 150452, ISIS 150454, ISIS 150458, ISIS 150460, ISIS 150462-150467, ISIS 150470, ISIS 150472, ISIS 150474, ISIS 150475, ISIS 150476, ISIS 150479-150483, ISIS 150488, ISIS 150489, ISIS 150490, ISIS 150491-150493, ISIS 150495-150498, ISIS 150511, ISIS 333605, ISIS 333606, ISIS 333609-333617, ISIS 333619, ISIS 333620-333636, ISIS 333638, and ISIS 333640, previously disclosed in WO 2005/040180, were also included in this assay. ISIS 333611, previously disclosed in WO 2005/040180, was also designated as a benchmark or comparator oligonucleotide. ISIS 333611 was recently tested in human clinical trials. See, MILLER et al., "An antisense oligonucleotide against SOD1 delivered intrathecally for patients with SOD1 familial amyotrophic lateral sclerosis: a phase 1, randomised, first-in-man study" Lancet Neurol. (2013) 12(5): 435-442.

The modified oligonucleotides were tested in a series of experiments that had similar culture conditions. The results for each experiment are presented in separate tables shown below. Cultured HepG2 cells at a density of 20,000 cells per well were transfected using electroporation with 7,000 nM modified oligonucleotide. After a treatment period of approximately 24 hours, RNA was isolated from the cells and SOD-1 mRNA levels were measured by quantitative real-time PCR.

Human primer probe set RTS3898 (forward sequence CTCTCAGGAGACCATTGCATCA, designated herein as SEQ ID NO: 11; reverse sequence TCCTGTCTTTGTACTTTCTTCATTTCC; designated herein as SEQ ID NO: 12; probe sequence CCGCACACTGGTGGTCCATGAAAA, designated herein as SEQ ID NO: 13) was used to measure mRNA levels. In cases where the oligonucleotide overlapped the amplicon of the primer probe set, an alternative primer probe set, HTS90 (forward sequence CGTGGCCTAGCGAGTTATGG, designated herein as SEQ ID NO: 14; reverse sequence GAAATTGATGATGCCCTGCA; designated herein as SEQ ID NO: 15; probe sequence ACGAAGGCCGTGTGCGTGCTGX, designated herein as SEQ ID NO: 16), was used to measure mRNA levels. SOD-1 mRNA levels were adjusted according to total RNA content, as measured by RIBOGREEN^{®}. Results are presented as percent inhibition of SOD-1, relative to untreated control cells. 'n.d.' indicates that inhibition levels were not measured using the particular primer probe set.

The newly designed modified oligonucleotides in the Tables below were designed as 5-10-5 MOE gapmers. The 5-10-5 MOE gapmers are 20 nucleosides in length, wherein the central gap segment is comprised of ten 2'-deoxyribonucleosides and is flanked by wing segments on the 5' direction and the 3' directions comprising five nucleosides each. Each nucleoside in the 5' wing segment and each nucleoside in the 3' wing segment has a 2'-MOE modification. The internucleoside linkages throughout each gapmer are phosphorothioate linkages. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000). 'n/a' indicates that the modified oligonucleotide does not target that particular gene sequence with 100% complementarity.

**Table 1**

| Percent Inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | % inhibition with RTS3898 | SEQ ID NO: 2 Start Site | SEQ ID NO: 2 Stop Site | SEQ ID NO |
| 590065 | 1 | 20 | CGCCCACTCTGGCCCCAAAC | 7 | 807 | 826 | 118 |
| 590066 | 35 | 54 | CCGCGACTACTTTATAGGCC | 5 | 841 | 860 | 119 |
| 333611 | 167 | 186 | CCGTCGCCCTTCAGCACGCA | 85 | 973 | 992 | 21 |
| 590067 | 202 | 221 | CCTTCTGCTCGAAATTGATG | 74 | 1008 | 1027 | 120 |
| 590068 | 203 | 222 | TCCTTCTGCTCGAAATTGAT | 58 | n/a | n/a | 121 |
| 590069 | 204 | 223 | TTCCTTCTGCTCGAAATTGA | 50 | n/a | n/a | 122 |
| 590070 | 205 | 224 | TTTCCTTCTGCTCGAAATTG | 47 | n/a | n/a | 123 |
| 590071 | 206 | 225 | CTTTCCTTCTGCTCGAAATT | 31 | n/a | n/a | 124 |
| 590072 | 207 | 226 | ACTTTCCTTCTGCTCGAAAT | 42 | n/a | n/a | 125 |
| 590073 | 208 | 227 | TACTTTCCTTCTGCTCGAAA | 38 | n/a | n/a | 126 |
| 590074 | 209 | 228 | TTACTTTCCTTCTGCTCGAA | 33 | n/a | n/a | 127 |
| 590075 | 210 | 229 | ATTACTTTCCTTCTGCTCGA | 39 | n/a | n/a | 128 |
| 590076 | 211 | 230 | CATTACTTTCCTTCTGCTCG | 28 | n/a | n/a | 129 |
| 590077 | 212 | 231 | CCATTACTTTCCTTCTGCTC | 58 | n/a | n/a | 130 |
| 590078 | 213 | 232 | TCCATTACTTTCCTTCTGCT | 58 | n/a | n/a | 131 |
| 590079 | 214 | 233 | GTCCATTACTTTCCTTCTGC | 69 | n/a | n/a | 132 |
| 590080 | 215 | 234 | GGTCCATTACTTTCCTTCTG | 68 | n/a | n/a | 133 |
| 590081 | 216 | 235 | TGGTCCATTACTTTCCTTCT | 61 | n/a | n/a | 134 |
| 590082 | 217 | 236 | CTGGTCCATTACTTTCCTTC | 69 | n/a | n/a | 135 |
| 590083 | 218 | 237 | ACTGGTCCATTACTTTCCTT | 54 | 4972 | 4991 | 136 |
| 150445 | 219 | 238 | CACTGGTCCATTACTTTCCT | 84 | 4973 | 4992 | 22 |
| 590084 | 220 | 239 | TCACTGGTCCATTACTTTCC | 65 | 4974 | 4993 | 137 |
| 590085 | 221 | 240 | TTCACTGGTCCATTACTTTC | 45 | 4975 | 4994 | 138 |
| 590086 | 222 | 241 | CTTCACTGGTCCATTACTTT | 43 | 4976 | 4995 | 139 |
| 590087 | 223 | 242 | CCTTCACTGGTCCATTACTT | 67 | 4977 | 4996 | 140 |
| 590088 | 224 | 243 | ACCTTCACTGGTCCATTACT | 59 | 4978 | 4997 | 141 |
| 436841 | 225 | 244 | CACCTTCACTGGTCCATTAC | 65 | 4979 | 4998 | 142 |
| 150446 | 226 | 245 | ACACCTTCACTGGTCCATTA | 83 | 4980 | 4999 | 23 |
| 393336 | 227 | 246 | CACACCTTCACTGGTCCATT | 81 | 4981 | 5000 | 143 |
| 150447 | 228 | 247 | CCACACCTTCACTGGTCCAT | 89 | 4982 | 5001 | 24 |
| 590089 | 229 | 248 | CCCACACCTTCACTGGTCCA | 82 | 4983 | 5002 | 144 |
| 590090 | 230 | 249 | CCCCACACCTTCACTGGTCC | 89 | 4984 | 5003 | 145 |
| 590091 | 231 | 250 | TCCCCACACCTTCACTGGTC | 84 | 4985 | 5004 | 146 |
| 590092 | 232 | 251 | TTCCCCACACCTTCACTGGT | 61 | 4986 | 5005 | 147 |
| 590093 | 233 | 252 | CTTCCCCACACCTTCACTGG | 60 | 4987 | 5006 | 148 |
| 590094 | 234 | 253 | GCTTCCCCACACCTTCACTG | 78 | 4988 | 5007 | 149 |
| 590095 | 235 | 254 | TGCTTCCCCACACCTTCACT | 72 | 4989 | 5008 | 150 |
| 590096 | 236 | 255 | ATGCTTCCCCACACCTTCAC | 76 | 4990 | 5009 | 151 |
| 393337 | 237 | 256 | AATGCTTCCCCACACCTTCA | 76 | 4991 | 5010 | 152 |
| 590097 | 238 | 257 | TAATGCTTCCCCACACCTTC | 68 | 4992 | 5011 | 153 |
| 590098 | 264 | 283 | TCCATGCAGGCCTTCAGTCA | 63 | 5018 | 5037 | 154 |
| 590099 | 265 | 284 | ATCCATGCAGGCCTTCAGTC | 64 | 5019 | 5038 | 155 |
| 590100 | 266 | 285 | AATCCATGCAGGCCTTCAGT | 52 | 5020 | 5039 | 156 |
| 590101 | 267 | 286 | GAATCCATGCAGGCCTTCAG | 53 | 5021 | 5040 | 157 |
| 590102 | 268 | 287 | GGAATCCATGCAGGCCTTCA | 65 | 5022 | 5041 | 158 |
| 393339 | 269 | 288 | TGGAATCCATGCAGGCCTTC | 43 | 5023 | 5042 | 159 |
| 590103 | 270 | 289 | ATGGAATCCATGCAGGCCTT | 56 | 5024 | 5043 | 160 |
| 590104 | 271 | 290 | CATGGAATCCATGCAGGCCT | 57 | 5025 | 5044 | 161 |
| 590105 | 272 | 291 | ACATGGAATCCATGCAGGCC | 52 | 5026 | 5045 | 162 |
| 590106 | 273 | 292 | AACATGGAATCCATGCAGGC | 54 | 5027 | 5046 | 163 |
| 590107 | 274 | 293 | GAACATGGAATCCATGCAGG | 51 | 5028 | 5047 | 164 |
| 590108 | 275 | 294 | TGAACATGGAATCCATGCAG | 58 | 5029 | 5048 | 165 |
| 393340 | 276 | 295 | ATGAACATGGAATCCATGCA | 62 | 5030 | 5049 | 166 |
| 590109 | 316 | 335 | GACCTGCACTGGTACAGCCT | 69 | 7632 | 7651 | 167 |
| 436847 | 317 | 336 | GGACCTGCACTGGTACAGCC | 74 | 7633 | 7652 | 168 |
| 590110 | 318 | 337 | AGGACCTGCACTGGTACAGC | 70 | 7634 | 7653 | 169 |
| 590111 | 319 | 338 | GAGGACCTGCACTGGTACAG | 74 | 7635 | 7654 | 170 |
| 590112 | 320 | 339 | TGAGGACCTGCACTGGTACA | 68 | 7636 | 7655 | 171 |
| 590113 | 321 | 340 | GTGAGGACCTGCACTGGTAC | 80 | 7637 | 7656 | 172 |
| 393343 | 322 | 341 | AGTGAGGACCTGCACTGGTA | 79 | 7638 | 7657 | 173 |
| 590114 | 323 | 342 | AAGTGAGGACCTGCACTGGT | 65 | 7639 | 7658 | 174 |
| 590115 | 324 | 343 | AAAGTGAGGACCTGCACTGG | 48 | 7640 | 7659 | 175 |
| 590116 | 325 | 344 | TAAAGTGAGGACCTGCACTG | 51 | 7641 | 7660 | 176 |
| 436848 | 326 | 345 | TTAAAGTGAGGACCTGCACT | 59 | 7642 | 7661 | 177 |
| 590117 | 327 | 346 | ATTAAAGTGAGGACCTGCAC | 43 | 7643 | 7662 | 178 |
| 590118 | 328 | 347 | GATTAAAGTGAGGACCTGCA | 43 | 7644 | 7663 | 179 |
| 590119 | 329 | 348 | GGATTAAAGTGAGGACCTGC | 67 | 7645 | 7664 | 180 |
| 590120 | 330 | 349 | AGGATTAAAGTGAGGACCTG | 63 | 7646 | 7665 | 181 |
| 436849 | 331 | 350 | GAGGATTAAAGTGAGGACCT | 64 | 7647 | 7666 | 182 |
| 393344 | 332 | 351 | AGAGGATTAAAGTGAGGACC | 59 | 7648 | 7667 | 183 |
| 590121 | 333 | 352 | TAGAGGATTAAAGTGAGGAC | 52 | 7649 | 7668 | 184 |
| 590122 | 334 | 353 | ATAGAGGATTAAAGTGAGGA | 36 | 7650 | 7669 | 185 |
| 590123 | 335 | 354 | GATAGAGGATTAAAGTGAGG | 25 | 7651 | 7670 | 186 |
| 590124 | 336 | 355 | GGATAGAGGATTAAAGTGAG | 34 | 7652 | 7671 | 187 |
| 590125 | 337 | 356 | TGGATAGAGGATTAAAGTGA | 49 | 7653 | 7672 | 188 |
| 590126 | 338 | 357 | CTGGATAGAGGATTAAAGTG | 34 | 7654 | 7673 | 189 |
| 590127 | 339 | 358 | TCTGGATAGAGGATTAAAGT | 39 | 7655 | 7674 | 190 |
| 590128 | 360 | 379 | ATCCTTTGGCCCACCGTGTT | 60 | 7676 | 7695 | 191 |

**Table 2**

| Percent inhibition of SOD-1 mRNA by 5-10-5 MOE gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | % inhibition with RTS3898 | % inhibitio n with HTS90 | SEQ ID NO: 2 Start Site | SEQ ID NO: 2 Stop Site | SEQ ID NO |
| 333611 | 167 | 186 | CCGTCGCCCTTCAGCACGCA | 76 | 95 | 973 | 992 | 21 |
| 393347 | 361 | 380 | CATCCTTTGGCCCACCGTGT | 70 | 72 | 7677 | 7696 | 192 |
| 590129 | 362 | 381 | TCATCCTTTGGCCCACCGTG | 66 | 68 | 7678 | 7697 | 193 |
| 590130 | 363 | 382 | TTCATCCTTTGGCCCACCGT | 53 | 55 | 7679 | 7698 | 194 |
| 590131 | 364 | 383 | CTTCATCCTTTGGCCCACCG | 52 | 50 | 7680 | 7699 | 195 |
| 590132 | 365 | 384 | TCTTCATCCTTTGGCCCACC | 61 | 64 | 7681 | 7700 | 196 |
| 590133 | 366 | 385 | CTCTTCATCCTTTGGCCCAC | 45 | 54 | 7682 | 7701 | 197 |
| 590134 | 367 | 386 | TCTCTTCATCCTTTGGCCCA | 44 | 34 | 7683 | 7702 | 198 |
| 590135 | 368 | 387 | CTCTCTTCATCCTTTGGCCC | 52 | 49 | 7684 | 7703 | 199 |
| 590136 | 369 | 388 | CCTCTCTTCATCCTTTGGCC | 48 | 47 | 7685 | 7704 | 200 |
| 590137 | 370 | 389 | GCCTCTCTTCATCCTTTGGC | 35 | 44 | n/a | n/a | 201 |
| 590138 | 371 | 390 | TGCCTCTCTTCATCCTTTGG | 52 | 45 | n/a | n/a | 202 |
| 590139 | 372 | 391 | ATGCCTCTCTTCATCCTTTG | 50 | 45 | n/a | n/a | 203 |
| 590140 | 373 | 392 | CATGCCTCTCTTCATCCTTT | 49 | 27 | n/a | n/a | 204 |
| 590141 | 374 | 393 | ACATGCCTCTCTTCATCCTT | 34 | 18 | n/a | n/a | 205 |
| 590142 | 375 | 394 | AACATGCCTCTCTTCATCCT | 38 | 35 | n/a | n/a | 206 |
| 333612 | 376 | 395 | CAACATGCCTCTCTTCATCC | 34 | 33 | n/a | n/a | 25 |
| 333613 | 377 | 396 | CCAACATGCCTCTCTTCATC | 46 | 55 | n/a | n/a | 26 |
| 333614 | 378 | 397 | TCCAACATGCCTCTCTTCAT | 42 | 48 | n/a | n/a | 27 |
| 333615 | 379 | 398 | CTCCAACATGCCTCTCTTCA | 42 | 15 | n/a | n/a | 28 |
| 333616 | 380 | 399 | TCTCCAACATGCCTCTCTTC | 35 | 44 | n/a | n/a | 29 |
| 333617 | 381 | 400 | GTCTCCAACATGCCTCTCTT | 42 | 47 | n/a | n/a | 30 |
| 590143 | 501 | 520 | TGCTTTTTCATGGACCACCA | n.d. | 65 | n/a | n/a | 207 |
| 590144 | 502 | 521 | CTGCTTTTTCATGGACCACC | n.d. | 70 | n/a | n/a | 208 |
| 590145 | 503 | 522 | TCTGCTTTTTCATGGACCAC | n.d. | 64 | n/a | n/a | 209 |
| 436860 | 504 | 523 | ATCTGCTTTTTCATGGACCA | n.d. | 65 | n/a | n/a | 210 |
| 590146 | 505 | 524 | CATCTGCTTTTTCATGGACC | n.d. | 68 | 9655 | 9674 | 211 |
| 590147 | 506 | 525 | TCATCTGCTTTTTCATGGAC | n.d. | 59 | 9656 | 9675 | 212 |
| 393359 | 507 | 526 | GTCATCTGCTTTTTCATGGA | n.d. | 56 | 9657 | 9676 | 213 |
| 590148 | 508 | 527 | AGTCATCTGCTTTTTCATGG | n.d. | 45 | 9658 | 9677 | 214 |
| 590149 | 509 | 528 | AAGTCATCTGCTTTTTCATG | n.d. | 23 | 9659 | 9678 | 215 |
| 590150 | 510 | 529 | CAAGTCATCTGCTTTTTCAT | n.d. | 43 | 9660 | 9679 | 216 |
| 590151 | 511 | 530 | CCAAGTCATCTGCTTTTTCA | n.d. | 72 | 9661 | 9680 | 217 |
| 489513 | 512 | 531 | CCCAAGTCATCTGCTTTTTC | n.d. | 73 | 9662 | 9681 | 218 |
| 590152 | 513 | 532 | GCCCAAGTCATCTGCTTTTT | n.d. | 74 | 9663 | 9682 | 219 |
| 436861 | 514 | 533 | TGCCCAAGTCATCTGCTTTT | n.d. | 75 | 9664 | 9683 | 220 |
| 590153 | 515 | 534 | TTGCCCAAGTCATCTGCTTT | n.d. | 47 | 9665 | 9684 | 221 |
| 393360 | 516 | 535 | TTTGCCCAAGTCATCTGCTT | n.d. | 57 | 9666 | 9685 | 222 |
| 590154 | 517 | 536 | CTTTGCCCAAGTCATCTGCT | n.d. | 79 | 9667 | 9686 | 223 |
| 590155 | 518 | 537 | CCTTTGCCCAAGTCATCTGC | n.d. | 67 | 9668 | 9687 | 224 |
| 590156 | 519 | 538 | ACCTTTGCCCAAGTCATCTG | n.d. | 57 | 9669 | 9688 | 225 |
| 333620 | 520 | 539 | CACCTTTGCCCAAGTCATCT | n.d. | 68 | 9670 | 9689 | 31 |
| 333621 | 521 | 540 | CCACCTTTGCCCAAGTCATC | n.d. | 72 | 9671 | 9690 | 32 |
| 333622 | 522 | 541 | TCCACCTTTGCCCAAGTCAT | n.d. | 77 | 9672 | 9691 | 33 |
| 333623 | 523 | 542 | TTCCACCTTTGCCCAAGTCA | n.d. | 73 | 9673 | 9692 | 34 |
| 333624 | 524 | 543 | TTTCCACCTTTGCCCAAGTC | n.d. | 77 | 9674 | 9693 | 35 |
| 333625 | 525 | 544 | ATTTCCACCTTTGCCCAAGT | n.d. | 79 | 9675 | 9694 | 36 |
| 333626 | 526 | 545 | CATTTCCACCTTTGCCCAAG | n.d. | 72 | 9676 | 9695 | 37 |
| 333627 | 527 | 546 | TCATTTCCACCTTTGCCCAA | n.d. | 55 | 9677 | 9696 | 38 |
| 333628 | 528 | 547 | TTCATTTCCACCTTTGCCCA | n.d. | 59 | 9678 | 9697 | 39 |
| 333629 | 529 | 548 | CTTCATTTCCACCTTTGCCC | n.d. | 73 | 9679 | 9698 | 40 |
| 333630 | 530 | 549 | TCTTCATTTCCACCTTTGCC | n.d. | 76 | 9680 | 9699 | 41 |
| 333631 | 531 | 550 | TTCTTCATTTCCACCTTTGC | n.d. | 62 | 9681 | 9700 | 42 |
| 333632 | 532 | 551 | TTTCTTCATTTCCACCTTTG | n.d. | 64 | 9682 | 9701 | 43 |
| 333633 | 533 | 552 | CTTTCTTCATTTCCACCTTT | n.d. | 69 | 9683 | 9702 | 44 |
| 333634 | 534 | 553 | ACTTTCTTCATTTCCACCTT | n.d. | 55 | 9684 | 9703 | 45 |
| 333635 | 535 | 554 | TACTTTCTTCATTTCCACCT | n.d. | 72 | 9685 | 9704 | 46 |
| 489517 | 582 | 601 | CCCAATTACACCACAAGCCA | 68 | 72 | 9732 | 9751 | 226 |
| 436863 | 583 | 602 | TCCCAATTACACCACAAGCC | 83 | 86 | 9733 | 9752 | 227 |
| 590157 | 584 | 603 | ATCCCAATTACACCACAAGC | 64 | 62 | 9734 | 9753 | 228 |
| 590158 | 585 | 604 | GATCCCAATTACACCACAAG | 51 | 61 | 9735 | 9754 | 229 |
| 590159 | 586 | 605 | CGATCCCAATTACACCACAA | 60 | 55 | 9736 | 9755 | 230 |
| 590160 | 587 | 606 | GCGATCCCAATTACACCACA | 59 | 63 | 9737 | 9756 | 231 |
| 150463 | 588 | 607 | GGCGATCCCAATTACACCAC | 78 | 79 | 9738 | 9757 | 47 |
| 393363 | 589 | 608 | GGGCGATCCCAATTACACCA | 65 | 65 | 9739 | 9758 | 232 |
| 590161 | 590 | 609 | TGGGCGATCCCAATTACACC | 56 | 60 | 9740 | 9759 | 233 |
| 590162 | 591 | 610 | TTGGGCGATCCCAATTACAC | 48 | 51 | 9741 | 9760 | 234 |
| 489518 | 592 | 611 | ATTGGGCGATCCCAATTACA | 51 | 59 | 9742 | 9761 | 235 |
| 436864 | 593 | 612 | TATTGGGCGATCCCAATTAC | 39 | 41 | 9743 | 9762 | 236 |
| 590163 | 594 | 613 | TTATTGGGCGATCCCAATTA | 35 | 34 | 9744 | 9763 | 237 |
| 590164 | 595 | 614 | TTTATTGGGCGATCCCAATT | 42 | 44 | 9745 | 9764 | 238 |
| 590165 | 596 | 615 | GTTTATTGGGCGATCCCAAT | 58 | 61 | 9746 | 9765 | 239 |
| 393364 | 597 | 616 | TGTTTATTGGGCGATCCCAA | 60 | 69 | 9747 | 9766 | 240 |
| 590166 | 598 | 617 | ATGTTTATTGGGCGATCCCA | 51 | 54 | 9748 | 9767 | 241 |
| 590167 | 599 | 618 | AATGTTTATTGGGCGATCCC | 48 | 45 | 9749 | 9768 | 242 |
| 590168 | 600 | 619 | GAATGTTTATTGGGCGATCC | 60 | 65 | 9750 | 9769 | 243 |
| 150464 | 601 | 620 | GGAATGTTTATTGGGCGATC | 58 | 63 | 9751 | 9770 | 48 |
| 393365 | 607 | 626 | TCCAAGGGAATGTTTATTGG | 50 | 58 | 9757 | 9776 | 244 |

**Table 3**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | % inhibition with RTS3898 | SEQ ID NO: 2 Start Site | SEQ ID NO: 2 Stop Site | SEQ ID NO |
| 333611 | 167 | 186 | CCGTCGCCCTTCAGCACGCA | 80 | 973 | 992 | 21 |
| 590169 | 608 | 627 | ATCCAAGGGAATGTTTATTG | 63 | 9758 | 9777 | 245 |
| 590170 | 609 | 628 | CATCCAAGGGAATGTTTATT | 53 | 9759 | 9778 | 246 |
| 590171 | 610 | 629 | ACATCCAAGGGAATGTTTAT | 49 | 9760 | 9779 | 247 |
| 590172 | 611 | 630 | TACATCCAAGGGAATGTTTA | 56 | 9761 | 9780 | 248 |
| 489519 | 612 | 631 | CTACATCCAAGGGAATGTTT | 60 | 9762 | 9781 | 249 |
| 590173 | 613 | 632 | ACTACATCCAAGGGAATGTT | 61 | 9763 | 9782 | 250 |
| 590174 | 614 | 633 | GACTACATCCAAGGGAATGT | 65 | 9764 | 9783 | 251 |
| 393366 | 615 | 634 | AGACTACATCCAAGGGAATG | 58 | 9765 | 9784 | 252 |
| 590175 | 616 | 635 | CAGACTACATCCAAGGGAAT | 50 | 9766 | 9785 | 253 |
| 436865 | 617 | 636 | TCAGACTACATCCAAGGGAA | 69 | 9767 | 9786 | 254 |
| 590176 | 618 | 637 | CTCAGACTACATCCAAGGGA | 78 | 9768 | 9787 | 255 |
| 150465 | 619 | 638 | CCTCAGACTACATCCAAGGG | 91 | 9769 | 9788 | 49 |
| 590177 | 620 | 639 | GCCTCAGACTACATCCAAGG | 90 | 9770 | 9789 | 256 |
| 590178 | 621 | 640 | GGCCTCAGACTACATCCAAG | 92 | 9771 | 9790 | 257 |
| 489520 | 622 | 641 | GGGCCTCAGACTACATCCAA | 88 | 9772 | 9791 | 258 |
| 590179 | 643 | 662 | CAGGATAACAGATGAGTTAA | 79 | 9793 | 9812 | 259 |
| 590180 | 644 | 663 | GCAGGATAACAGATGAGTTA | 83 | 9794 | 9813 | 260 |
| 590181 | 645 | 664 | AGCAGGATAACAGATGAGTT | 81 | 9795 | 9814 | 261 |
| 590182 | 646 | 665 | TAGCAGGATAACAGATGAGT | 68 | 9796 | 9815 | 262 |
| 590183 | 647 | 666 | CTAGCAGGATAACAGATGAG | 74 | 9797 | 9816 | 263 |
| 590184 | 648 | 667 | GCTAGCAGGATAACAGATGA | 70 | 9798 | 9817 | 264 |
| 393370 | 649 | 668 | AGCTAGCAGGATAACAGATG | 61 | 9799 | 9818 | 265 |
| 590185 | 650 | 669 | CAGCTAGCAGGATAACAGAT | 78 | 9800 | 9819 | 266 |
| 590186 | 651 | 670 | ACAGCTAGCAGGATAACAGA | 72 | 9801 | 9820 | 267 |
| 489522 | 652 | 671 | TACAGCTAGCAGGATAACAG | 78 | 9802 | 9821 | 268 |
| 590187 | 653 | 672 | CTACAGCTAGCAGGATAACA | 88 | 9803 | 9822 | 269 |
| 378879 | 654 | 673 | TCTACAGCTAGCAGGATAAC | 86 | 9804 | 9823 | 270 |
| 590188 | 655 | 674 | TTCTACAGCTAGCAGGATAA | 85 | 9805 | 9824 | 271 |
| 393371 | 656 | 675 | TTTCTACAGCTAGCAGGATA | 84 | 9806 | 9825 | 272 |
| 436868 | 657 | 676 | ATTTCTACAGCTAGCAGGAT | 81 | 9807 | 9826 | 273 |
| 590189 | 658 | 677 | CATTTCTACAGCTAGCAGGA | 87 | 9808 | 9827 | 274 |
| 590190 | 659 | 678 | ACATTTCTACAGCTAGCAGG | 92 | 9809 | 9828 | 275 |
| 590191 | 660 | 679 | TACATTTCTACAGCTAGCAG | 88 | 9810 | 9829 | 276 |
| 590192 | 661 | 680 | ATACATTTCTACAGCTAGCA | 88 | 9811 | 9830 | 277 |
| 489523 | 662 | 681 | GATACATTTCTACAGCTAGC | 93 | 9812 | 9831 | 278 |
| 590193 | 683 | 702 | ACAGTGTTTAATGTTTATCA | 74 | 9833 | 9852 | 279 |
| 590194 | 684 | 703 | TACAGTGTTTAATGTTTATC | 64 | 9834 | 9853 | 280 |
| 590195 | 685 | 704 | TTACAGTGTTTAATGTTTAT | 56 | 9835 | 9854 | 281 |
| 590196 | 686 | 705 | ATTACAGTGTTTAATGTTTA | 50 | 9836 | 9855 | 282 |
| 590197 | 687 | 706 | GATTACAGTGTTTAATGTTT | 74 | 9837 | 9856 | 283 |
| 590198 | 688 | 707 | AGATTACAGTGTTTAATGTT | 37 | 9838 | 9857 | 284 |
| 590199 | 689 | 708 | AAGATTACAGTGTTTAATGT | 58 | 9839 | 9858 | 285 |
| 393375 | 690 | 709 | TAAGATTACAGTGTTTAATG | 58 | 9840 | 9859 | 286 |
| 590200 | 691 | 710 | TTAAGATTACAGTGTTTAAT | 46 | 9841 | 9860 | 287 |
| 436876 | 772 | 791 | CAAATCTTCCAAGTGATCAT | 36 | 9922 | 9941 | 288 |
| 590201 | 773 | 792 | ACAAATCTTCCAAGTGATCA | 33 | 9923 | 9942 | 289 |
| 590202 | 774 | 793 | TACAAATCTTCCAAGTGATC | 34 | 9924 | 9943 | 290 |
| 150474 | 775 | 794 | ATACAAATCTTCCAAGTGAT | 47 | 9925 | 9944 | 50 |
| 590203 | 776 | 795 | TATACAAATCTTCCAAGTGA | 29 | 9926 | 9945 | 291 |
| 393382 | 777 | 796 | CTATACAAATCTTCCAAGTG | 41 | 9927 | 9946 | 292 |
| 436877 | 778 | 797 | ACTATACAAATCTTCCAAGT | 45 | 9928 | 9947 | 293 |
| 590204 | 779 | 798 | AACTATACAAATCTTCCAAG | 27 | 9929 | 9948 | 294 |
| 590205 | 780 | 799 | AAACTATACAAATCTTCCAA | 33 | 9930 | 9949 | 295 |
| 590206 | 781 | 800 | AAAACTATACAAATCTTCCA | 35 | 9931 | 9950 | 296 |
| 489533 | 782 | 801 | TAAAACTATACAAATCTTCC | 26 | 9932 | 9951 | 297 |
| 590207 | 783 | 802 | ATAAAACTATACAAATCTTC | 19 | 9933 | 9952 | 298 |
| 590208 | 784 | 803 | TATAAAACTATACAAATCTT | 2 | 9934 | 9953 | 299 |
| 590209 | 785 | 804 | TTATAAAACTATACAAATCT | 7 | 9935 | 9954 | 300 |
| 590210 | 786 | 805 | TTTATAAAACTATACAAATC | 0 | 9936 | 9955 | 301 |
| 590211 | 787 | 806 | TTTTATAAAACTATACAAAT | 4 | 9937 | 9956 | 302 |
| 590212 | 788 | 807 | GTTTTATAAAACTATACAAA | 5 | 9938 | 9957 | 303 |
| 590213 | 789 | 808 | AGTTTTATAAAACTATACAA | 3 | 9939 | 9958 | 304 |
| 436878 | 790 | 809 | GAGTTTTATAAAACTATACA | 7 | 9940 | 9959 | 305 |
| 150475 | 791 | 810 | TGAGTTTTATAAAACTATAC | 28 | 9941 | 9960 | 51 |
| 489536 | 812 | 831 | CATTGAAACAGACATTTTAA | 28 | 9962 | 9981 | 306 |
| 150479 | 813 | 832 | TCATTGAAACAGACATTTTA | 36 | 9963 | 9982 | 52 |
| 393385 | 814 | 833 | GTCATTGAAACAGACATTTT | 50 | 9964 | 9983 | 307 |
| 590214 | 815 | 834 | GGTCATTGAAACAGACATTT | 45 | 9965 | 9984 | 308 |
| 590215 | 816 | 835 | AGGTCATTGAAACAGACATT | 47 | 9966 | 9985 | 309 |
| 590216 | 817 | 836 | CAGGTCATTGAAACAGACAT | 39 | 9967 | 9986 | 310 |
| 590217 | 818 | 837 | ACAGGTCATTGAAACAGACA | 44 | 9968 | 9987 | 311 |
| 590218 | 819 | 838 | TACAGGTCATTGAAACAGAC | 42 | 9969 | 9988 | 312 |
| 150480 | 820 | 839 | ATACAGGTCATTGAAACAGA | 46 | 9970 | 9989 | 53 |
| 393386 | 821 | 840 | AATACAGGTCATTGAAACAG | 36 | 9971 | 9990 | 313 |
| 489537 | 822 | 841 | AAATACAGGTCATTGAAACA | 12 | 9972 | 9991 | 314 |
| 590219 | 823 | 842 | AAAATACAGGTCATTGAAAC | 16 | 9973 | 9992 | 315 |
| 590220 | 824 | 843 | CAAAATACAGGTCATTGAAA | 21 | 9974 | 9993 | 316 |

**Table 4**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | % inhibition with RTS3898 | SEQ ID NO: 2 Start Site | SEQ ID NO: 2 Stop Site | SEQ ID NO |
| 590251 | n/a | n/a | CCTTGCCTTCTGCTCGAAAT | 57 | 1013 | 1032 | 317 |
| 590252 | n/a | n/a | AATAAAGTTGACCTCTTTTT | 45 | 5479 | 5498 | 318 |
| 590253 | n/a | n/a | CTCTGATATAAAAATCTTGT | 54 | 8142 | 8161 | 319 |
| 590254 | n/a | n/a | GCCCCGCGGCGGCCTCGGTC | 38 | 1238 | 1257 | 320 |
| 590255 | n/a | n/a | GCTATCGCCATTATTACAAG | 38 | 7722 | 7741 | 321 |
| 590256 | n/a | n/a | CTCAAATGTGAAAGTTGTCC | 57 | 3414 | 3433 | 322 |
| 590257 | n/a | n/a | GTTCTATATTCAATAAATGC | 37 | 7925 | 7944 | 323 |
| 590258 | n/a | n/a | AATTAAAGTTCCCAAATACA | 0 | 7578 | 7597 | 324 |
| 590259 | n/a | n/a | GATCATTACAAAAGTTAAGA | 17 | 6150 | 6169 | 325 |
| 590260 | n/a | n/a | CCTTCTCTGCCCTTGCAGCC | 55 | 1685 | 1704 | 326 |
| 590261 | n/a | n/a | ACCCAAATAACTATGTTGTA | n.d. | 9394 | 9413 | 327 |
| 590262 | n/a | n/a | CCAGGTTTTAAACTTAACAA | n.d. | 8890 | 8909 | 328 |
| 590263 | n/a | n/a | ATCTCAGGACTAAAATAAAC | 44 | 3663 | 3682 | 329 |
| 590264 | n/a | n/a | AAATAACTATGTTGTAGACC | n.d. | 9390 | 9409 | 330 |
| 590265 | n/a | n/a | AAGAACCTTTTCCAGAAAAT | 37 | 2449 | 2468 | 331 |
| 590266 | n/a | n/a | GGAACAGAAACAAGTCTATG | 25 | 7458 | 7477 | 332 |
| 590267 | n/a | n/a | AGAAAGCTATCGCCATTATT | 27 | 7727 | 7746 | 333 |
| 590268 | n/a | n/a | TTCCCAAATACATTCTAAAA | 7 | 7570 | 7589 | 334 |
| 590269 | n/a | n/a | AACTGCTCTAGGCCTGTGTC | 53 | 4787 | 4806 | 335 |
| 590270 | n/a | n/a | AAATGGATCAAATCTGATCA | 31 | 6595 | 6614 | 336 |
| 590271 | n/a | n/a | GTAGGTGCACATCAAAATCA | 58 | 1928 | 1947 | 337 |
| 590272 | n/a | n/a | TCTGATATAAAAATCTTGTC | 28 | 8141 | 8160 | 338 |
| 590273 | n/a | n/a | ACCATATGAACTCCAGAAAG | 45 | 7741 | 7760 | 339 |
| 590274 | n/a | n/a | AACATCAAGGTAGTTCATGA | 10 | 8379 | 8398 | 340 |
| 590275 | n/a | n/a | GCAATTACAGAAATGGATCA | 42 | 6605 | 6624 | 341 |
| 590276 | n/a | n/a | TTTTAAGCATATTCCAAAGT | 45 | 6331 | 6350 | 342 |
| 590277 | n/a | n/a | TCAACCCCCAGCTCAAACAC | 26 | 6174 | 6193 | 343 |
| 590278 | n/a | n/a | AGAAAAATAACATTAATCCT | n.d. | 9541 | 9560 | 344 |
| 590279 | n/a | n/a | AAGATTTTAAACACGGAATA | 31 | 3085 | 3104 | 345 |
| 146145 | 165 | 184 | GTCGCCCTTCAGCACGCACA | 82 | 971 | 990 | 54 |
| 333611 | 167 | 186 | CCGTCGCCCTTCAGCACGCA | 81 | 973 | 992 | 21 |
| 590250 | 399 | 418 | AGCAGTCACATTGCCCAAGT | 75 | 8454 | 8473 | 346 |
| 489525 | 682 | 701 | CAGTGTTTAATGTTTATCAG | 69 | 9832 | 9851 | 347 |
| 436879 | 825 | 844 | GCAAAATACAGGTCATTGAA | 49 | 9975 | 9994 | 348 |
| 590221 | 826 | 845 | GGCAAAATACAGGTCATTGA | 54 | 9976 | 9995 | 349 |
| 590222 | 827 | 846 | TGGCAAAATACAGGTCATTG | 52 | 9977 | 9996 | 350 |
| 393387 | 828 | 847 | CTGGCAAAATACAGGTCATT | 51 | 9978 | 9997 | 351 |
| 590223 | 829 | 848 | TCTGGCAAAATACAGGTCAT | 47 | 9979 | 9998 | 352 |
| 590224 | 830 | 849 | GTCTGGCAAAATACAGGTCA | 44 | 9980 | 9999 | 353 |
| 590225 | 831 | 850 | AGTCTGGCAAAATACAGGTC | 50 | 9981 | 10000 | 354 |
| 489538 | 832 | 851 | AAGTCTGGCAAAATACAGGT | 38 | 9982 | 10001 | 355 |
| 590226 | 833 | 852 | TAAGTCTGGCAAAATACAGG | 33 | 9983 | 10002 | 356 |
| 590227 | 834 | 853 | TTAAGTCTGGCAAAATACAG | 20 | 9984 | 10003 | 357 |
| 150482 | 853 | 872 | TTTAATACCCATCTGTGATT | 29 | 10003 | 10022 | 55 |
| 590228 | 854 | 873 | GTTTAATACCCATCTGTGAT | 33 | 10004 | 10023 | 358 |
| 150483 | 855 | 874 | AGTTTAATACCCATCTGTGA | 44 | 10005 | 10024 | 56 |
| 590229 | 856 | 875 | AAGTTTAATACCCATCTGTG | 51 | 10006 | 10025 | 359 |
| 590230 | 857 | 876 | CAAGTTTAATACCCATCTGT | 42 | 10007 | 10026 | 360 |
| 590231 | 858 | 877 | ACAAGTTTAATACCCATCTG | 38 | 10008 | 10027 | 361 |
| 393389 | 859 | 878 | GACAAGTTTAATACCCATCT | 48 | 10009 | 10028 | 362 |
| 590232 | 860 | 879 | TGACAAGTTTAATACCCATC | 55 | 10010 | 10029 | 363 |
| 590233 | 861 | 880 | CTGACAAGTTTAATACCCAT | 49 | 10011 | 10030 | 364 |
| 489541 | 862 | 881 | TCTGACAAGTTTAATACCCA | 52 | 10012 | 10031 | 365 |
| 590234 | 863 | 882 | TTCTGACAAGTTTAATACCC | 39 | 10013 | 10032 | 366 |
| 590235 | 864 | 883 | ATTCTGACAAGTTTAATACC | 21 | 10014 | 10033 | 367 |
| 590236 | 865 | 884 | AATTCTGACAAGTTTAATAC | 4 | 10015 | 10034 | 368 |
| 393390 | 866 | 885 | AAATTCTGACAAGTTTAATA | 7 | 10016 | 10035 | 369 |
| 590237 | 867 | 886 | GAAATTCTGACAAGTTTAAT | 5 | 10017 | 10036 | 370 |
| 436881 | 868 | 887 | AGAAATTCTGACAAGTTTAA | 33 | 10018 | 10037 | 371 |
| 590238 | 869 | 888 | AAGAAATTCTGACAAGTTTA | 20 | 10019 | 10038 | 372 |
| 590239 | 891 | 910 | TTATTCACAGGCTTGAATGA | 23 | 10041 | 10060 | 373 |
| 489544 | 892 | 911 | TTTATTCACAGGCTTGAATG | 41 | 10042 | 10061 | 374 |
| 590240 | 893 | 912 | TTTTATTCACAGGCTTGAAT | 40 | 10043 | 10062 | 375 |
| 436884 | 894 | 913 | TTTTTATTCACAGGCTTGAA | 31 | 10044 | 10063 | 376 |
| 590241 | 895 | 914 | GTTTTTATTCACAGGCTTGA | 39 | 10045 | 10064 | 377 |
| 150488 | 896 | 915 | GGTTTTTATTCACAGGCTTG | 51 | 10046 | 10065 | 57 |
| 590242 | 897 | 916 | GGGTTTTTATTCACAGGCTT | 46 | 10047 | 10066 | 378 |
| 150489 | 898 | 917 | AGGGTTTTTATTCACAGGCT | 52 | 10048 | 10067 | 58 |
| 590243 | 899 | 918 | CAGGGTTTTTATTCACAGGC | 49 | 10049 | 10068 | 379 |
| 590244 | 900 | 919 | ACAGGGTTTTTATTCACAGG | 38 | 10050 | 10069 | 380 |
| 590245 | 901 | 920 | TACAGGGTTTTTATTCACAG | 34 | 10051 | 10070 | 381 |
| 150490 | 902 | 921 | ATACAGGGTTTTTATTCACA | 30 | 10052 | 10071 | 59 |
| 590246 | 903 | 922 | CATACAGGGTTTTTATTCAC | 34 | 10053 | 10072 | 382 |
| 150491 | 904 | 923 | CCATACAGGGTTTTTATTCA | 34 | 10054 | 10073 | 60 |
| 590247 | 905 | 924 | GCCATACAGGGTTTTTATTC | 34 | 10055 | 10074 | 383 |
| 590248 | 906 | 925 | TGCCATACAGGGTTTTTATT | 33 | 10056 | 10075 | 384 |
| 393393 | 907 | 926 | GTGCCATACAGGGTTTTTAT | 43 | 10057 | 10076 | 385 |
| 590249 | 908 | 927 | AGTGCCATACAGGGTTTTTA | 12 | 10058 | 10077 | 386 |

**Table 5**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | % inhibition with RTS3898 | SEQ ID NO: 2 Start Site | SEQ ID NO: 2 Stop Site | SEQ ID NO |
| 333611 | 167 | 186 | CCGTCGCCCTTCAGCACGCA | 86 | 973 | 992 | 21 |
| 590280 | n/a | n/a | TGGAAAAACTCAAATGTGAA | 51 | 3422 | 3441 | 387 |
| 590281 | n/a | n/a | TTTCCCTTTCTTTTCCACAC | 76 | 5738 | 5757 | 388 |
| 590282 | n/a | n/a | TCTTTCCCTTTCTTTTCCAC | 65 | 5740 | 5759 | 389 |
| 590283 | n/a | n/a | TACCTTCTCTGCCCTTGCAG | 74 | 1687 | 1706 | 390 |
| 590284 | n/a | n/a | GCAAGGGCCAAGGCTGCTGC | 75 | 6879 | 6898 | 391 |
| 590285 | n/a | n/a | AAAGCTAAATTATGAATTAA | 12 | 7592 | 7611 | 392 |
| 590286 | n/a | n/a | CTAATGAAGGCTCAGTATGA | 59 | 3193 | 3212 | 393 |
| 590287 | n/a | n/a | GGAGTCAAATGCCAAAGAAC | 60 | 2463 | 2482 | 394 |
| 590288 | n/a | n/a | TGAATTAAAGTTCCCAAATA | 5 | 7580 | 7599 | 395 |
| 590289 | n/a | n/a | ACTTGGTGCAGGCAGAATAT | 63 | 6916 | 6935 | 396 |
| 590290 | n/a | n/a | CCTCTGATATAAAAATCTTG | 67 | 8143 | 8162 | 397 |
| 590291 | n/a | n/a | AAAGTTGGAGAGAGTTTCTG | 8 | 4940 | 4959 | 398 |
| 590292 | n/a | n/a | TCTCTGCCCTTGCAGCCCAA | 80 | 1682 | 1701 | 399 |
| 590293 | n/a | n/a | TTACTTGGTGCAGGCAGAAT | 56 | 6918 | 6937 | 400 |
| 590294 | n/a | n/a | AATGGAGTCAAATGCCAAAG | 66 | 2466 | 2485 | 401 |
| 590295 | n/a | n/a | TATGAATTAAAGTTCCCAAA | 20 | 7582 | 7601 | 402 |
| 590296 | n/a | n/a | AGTTCTATATTCAATAAATG | 21 | 7926 | 7945 | 403 |
| 590297 | n/a | n/a | TACAAGTAGTATACCATATG | 33 | 7753 | 7772 | 404 |
| 590298 | n/a | n/a | TAGCCTTAGAGCTGTACAAA | 70 | 1553 | 1572 | 405 |
| 590299 | n/a | n/a | GTCCCCATTTGTCAATTCCT | 71 | 7882 | 7901 | 406 |
| 590300 | n/a | n/a | AACCTGCCTACTGGCAGAGC | 59 | 2095 | 2114 | 407 |
| 590301 | n/a | n/a | CTTGTTCCCACACTCAATGC | 56 | 4747 | 4766 | 408 |
| 590302 | n/a | n/a | ACAAGTCATGATAACCTGCA | 61 | 8952 | 8971 | 409 |
| 590303 | n/a | n/a | TGTTTTCCAAACTCAGATCT | 52 | 8796 | 8815 | 410 |
| 590304 | n/a | n/a | AGAACCTCATAATATTAGAA | 9 | 9557 | 9576 | 411 |
| 590305 | n/a | n/a | GGTTTTAAACTTAACAAAAT | 1 | 8887 | 8906 | 412 |
| 590306 | n/a | n/a | CTCTGGTGTATTTTTAGTAA | 65 | 1831 | 1850 | 413 |
| 590307 | n/a | n/a | TATCTCTGCATATCTGGAAA | 71 | 3034 | 3053 | 414 |
| 590308 | n/a | n/a | CAGCCTTTTTAACCCAAAAG | 68 | 4407 | 4426 | 415 |
| 590309 | n/a | n/a | TGGAATGCTCCACTATCCAA | 57 | 3012 | 3031 | 416 |
| 590310 | n/a | n/a | CGTTCAGAAGTTTGTCTCTG | 67 | 2126 | 2145 | 417 |
| 590311 | n/a | n/a | CTGCTCAGGGAAGGTGGAAA | 53 | 2922 | 2941 | 418 |
| 590312 | n/a | n/a | TCAAGAGAAGCTAGGAAAAC | 50 | 3154 | 3173 | 419 |
| 590313 | n/a | n/a | TCCCTTTCTTTTCCACACCT | 74 | 5736 | 5755 | 420 |
| 590314 | n/a | n/a | TTGTTCCCACACTCAATGCA | 56 | 4746 | 4765 | 421 |
| 590315 | n/a | n/a | TCACCAGCACAGCACAACAC | 58 | 5076 | 5095 | 422 |
| 590316 | n/a | n/a | CCTGGGATCATTACAAAAGT | 42 | 6155 | 6174 | 423 |
| 590317 | n/a | n/a | AGTAGTATACCATATGAACT | 35 | 7749 | 7768 | 424 |
| 590318 | n/a | n/a | TCTAATATGGTCAAATGTAA | 27 | 8779 | 8798 | 425 |
| 590319 | n/a | n/a | GGTTGGGCTCTGGTGTATTT | 64 | 1838 | 1857 | 426 |
| 590320 | n/a | n/a | TGCCCTTTACTTGGTGCAGG | 56 | 6924 | 6943 | 427 |
| 590321 | n/a | n/a | AGAGAGTTTCTGAACAAAGA | 24 | 4932 | 4951 | 428 |
| 590322 | n/a | n/a | GAATTTCAGCAATTACAGAA | 33 | 6613 | 6632 | 429 |
| 590323 | n/a | n/a | ACAAGTTAAACAAGTCATGA | 9 | 8961 | 8980 | 430 |
| 590324 | n/a | n/a | TGTGCCCTTTACTTGGTGCA | 47 | 6926 | 6945 | 431 |
| 590325 | n/a | n/a | TTAGGAGGAGGAAAAGGACC | 23 | 1719 | 1738 | 432 |
| 590326 | n/a | n/a | ACTGGCAGAGCAATTTTAAA | 25 | 2086 | 2105 | 433 |
| 590327 | n/a | n/a | AGTCAAATGCCAAAGAACCT | 58 | 2461 | 2480 | 434 |
| 590328 | n/a | n/a | AAGCATCAGATGGATTAGGG | 17 | 8411 | 8430 | 435 |
| 590329 | n/a | n/a | GTCCGCGGGACCCTCAGGAA | 54 | 1414 | 1433 | 436 |
| 590330 | n/a | n/a | CAATTACAGAAATGGATCAA | 42 | 6604 | 6623 | 437 |
| 590331 | n/a | n/a | GCTGTCAAGTAATCACTACC | 27 | 9606 | 9625 | 438 |
| 590332 | n/a | n/a | AGTGCAAAGTTGGAGAGAGT | 33 | 4945 | 4964 | 439 |
| 590333 | n/a | n/a | ACTTGCTTCCAATCCCAAAT | 78 | 6436 | 6455 | 440 |
| 590334 | n/a | n/a | AACTCAAATGTGAAAGTTGT | 51 | 3416 | 3435 | 441 |
| 590335 | n/a | n/a | TTTTAGTAAGATCTTCAAAT | 14 | 1820 | 1839 | 442 |
| 590336 | n/a | n/a | ATTTCAGCAATTACAGAAAT | 27 | 6611 | 6630 | 443 |
| 590337 | n/a | n/a | TTAAGTGTCCCCATTTGTCA | 56 | 7888 | 7907 | 444 |
| 590338 | n/a | n/a | TTAGCAACCTGCCTACTGGC | 57 | 2100 | 2119 | 445 |
| 590339 | n/a | n/a | TATTACAAGAGTTAAGCATC | 41 | 7711 | 7730 | 446 |
| 590340 | n/a | n/a | ATGTTGAATATACATGTACA | 36 | 4545 | 4564 | 447 |
| 590341 | n/a | n/a | TTTGTCTCTGACCATCTTAG | 74 | 2116 | 2135 | 448 |
| 590342 | n/a | n/a | TTTTCCACCAGTTGGTAACT | 59 | 2253 | 2272 | 449 |
| 590343 | n/a | n/a | CAACAGCTTCCCACAAGTTA | 28 | 8973 | 8992 | 450 |
| 590344 | n/a | n/a | CAAATGTGAAAGTTGTCCCT | 62 | 3412 | 3431 | 451 |
| 590345 | n/a | n/a | GCTACCTTCTCTGCCCTTGC | 73 | 1689 | 1708 | 452 |
| 590346 | n/a | n/a | TCTTAGCAGAACAGTGTTCT | 51 | 8743 | 8762 | 453 |
| 590347 | n/a | n/a | ATACATTCTAAAAAGAAACA | 41 | 7563 | 7582 | 454 |
| 590348 | n/a | n/a | GCACATATTTACAAGTAGTA | 58 | 7762 | 7781 | 455 |
| 590349 | n/a | n/a | GGGTCACCAGCACAGCACAA | 35 | 5079 | 5098 | 456 |
| 590350 | n/a | n/a | GTGCAAGGGCCAAGGCTGCT | 66 | 6881 | 6900 | 457 |
| 590351 | n/a | n/a | ACCTGGGTTCATGCATGGAT | 72 | 2902 | 2921 | 458 |
| 590352 | n/a | n/a | ATCACTATTTGAAACTAAAT | 0 | 6569 | 6588 | 459 |
| 590353 | n/a | n/a | ATACAATAAAGTTGACCTCT | 64 | 5483 | 5502 | 460 |
| 590354 | n/a | n/a | TTTTAAACTTAACAAAATGT | 10 | 8885 | 8904 | 461 |
| 590355 | n/a | n/a | CTCCCCGCGCTCCCGCCACG | 15 | 1268 | 1287 | 462 |
| 590356 | n/a | n/a | GAAGGCTCAGTATGAAGAGA | 65 | 3188 | 3207 | 463 |

**Table 6**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | % inhibition with RTS3898 | SEQ ID NO: 2 Start Site | SEQ ID NO: 2 Stop Site | SEQ ID NO |
| 333611 | 167 | 186 | CCGTCGCCCTTCAGCACGCA | 87 | 973 | 992 | 21 |
| 590357 | n/a | n/a | AGAAAACAGCTGATTTACCT | 40 | 4915 | 4934 | 464 |
| 590358 | n/a | n/a | CCACAAGTTAAACAAGTCAT | n.d. | 8963 | 8982 | 465 |
| 590359 | n/a | n/a | CAAATTTGCAAACAAGTAGC | 61 | 8331 | 8350 | 466 |
| 590360 | n/a | n/a | CCTAATTTGAACTGCAAGTA | n.d. | 8665 | 8684 | 467 |
| 590361 | n/a | n/a | AAAAAACTCATCTCCCCAGC | 70 | 6969 | 6988 | 468 |
| 590362 | n/a | n/a | AGGCTCAGTATGAAGAGATC | 67 | 3186 | 3205 | 469 |
| 590363 | n/a | n/a | TGTTATCAAGAGCACAGGGC | 58 | 3383 | 3402 | 470 |
| 590364 | n/a | n/a | CCTCAAAAGGGAGATGGTAA | 41 | 4768 | 4787 | 471 |
| 590365 | n/a | n/a | AGTATGGGTCACCAGCACAG | 71 | 5084 | 5103 | 472 |
| 590366 | n/a | n/a | TCACAATCTAGTGCAGTTAC | 70 | 5584 | 5603 | 473 |
| 590367 | n/a | n/a | CAAGTGAGAAACCCAATCCT | n.d. | 8856 | 8875 | 474 |
| 590368 | n/a | n/a | AGAAAATCTGGCCATTTTAA | n.d. | 8832 | 8851 | 475 |
| 590369 | n/a | n/a | ACAGGTAATGGTGCTCCGTG | 71 | 3716 | 3735 | 476 |
| 590370 | n/a | n/a | TGAAAGGCTTTCAGAAAACA | 44 | 8102 | 8121 | 477 |
| 590371 | n/a | n/a | CAGGCAAGTTACAGGAAGCA | 64 | 6687 | 6706 | 478 |
| 590372 | n/a | n/a | CAGCAAGCTGCTTAACTGCT | 65 | 4800 | 4819 | 479 |
| 590373 | n/a | n/a | TGTTGCAAAGACATTACCTT | n.d. | 9455 | 9474 | 480 |
| 590374 | n/a | n/a | GAAACTAAATTAGCAAGATG | 43 | 6559 | 6578 | 481 |
| 590375 | n/a | n/a | TCAAGAGCACAGGGCCAAAA | 60 | 3378 | 3397 | 482 |
| 590376 | n/a | n/a | AGGAGGAGGAAAAGGACCTC | 53 | 1717 | 1736 | 483 |
| 590377 | n/a | n/a | CCTCAGCCTTTTTAACCCAA | 73 | 4410 | 4429 | 484 |
| 590378 | n/a | n/a | CTATGTTGTAGACCACCACA | n.d. | 9384 | 9403 | 485 |
| 590379 | n/a | n/a | CTCCGTGGCTACATACAGAA | 66 | 3703 | 3722 | 486 |
| 590380 | n/a | n/a | TTTATCTGGATCTTTAGAAA | n.d. | 8642 | 8661 | 487 |
| 590381 | n/a | n/a | AAAAAAAGGAAAGTGAAAGT | n.d. | 9279 | 9298 | 488 |
| 590382 | n/a | n/a | GGTTCATGCATGGATTCTCA | 76 | 2897 | 2916 | 489 |
| 590383 | n/a | n/a | CTGCAAAGTGTCACACAAAC | 76 | 1630 | 1649 | 490 |
| 590384 | n/a | n/a | TTCAGAAGTACCAAAGGGTA | 53 | 8227 | 8246 | 491 |
| 590385 | n/a | n/a | TAAAAGCATTCCAGCATTTG | 44 | 7848 | 7867 | 492 |
| 590386 | n/a | n/a | TAGTATACCATATGAACTCC | 73 | 7747 | 7766 | 493 |
| 590387 | n/a | n/a | TGCATATCTGGAAAGCTGGA | 59 | 3028 | 3047 | 494 |
| 590388 | n/a | n/a | CTTAACTGCTCTAGGCCTGT | 54 | 4790 | 4809 | 495 |
| 590389 | n/a | n/a | AGGCACCGACCGGGCGGCAC | 21 | 1155 | 1174 | 496 |
| 590390 | n/a | n/a | TGCAAAGTTGGAGAGAGTTT | 32 | 4943 | 4962 | 497 |
| 590391 | n/a | n/a | TCCTCAAAAGGGAGATGGTA | 37 | 4769 | 4788 | 498 |
| 590392 | n/a | n/a | AGTATACCATATGAACTCCA | 76 | 7746 | 7765 | 499 |
| 590393 | n/a | n/a | TATTTGTACATGTTGAATAT | 2 | 4554 | 4573 | 500 |
| 590394 | n/a | n/a | ACCCAAAAGGTGTATGTCTC | 71 | 4396 | 4415 | 501 |
| 590395 | n/a | n/a | CTTTGGAAAAAAAGGAAAGT | n.d. | 9285 | 9304 | 502 |
| 590396 | n/a | n/a | GGGAGAAAGGCAGGCAAGTT | 20 | 6697 | 6716 | 503 |
| 590397 | n/a | n/a | TTAAGCCCAGGAAGTAAAAG | 9 | 7862 | 7881 | 504 |
| 590398 | n/a | n/a | AGACATTACCTTTAAACATT | n.d. | 9447 | 9466 | 505 |
| 590399 | n/a | n/a | GTGGCTTAAGAAATGCTCCG | 26 | 2050 | 2069 | 506 |
| 590400 | n/a | n/a | GTGAGAAGGGAACAGAAACA | 48 | 7466 | 7485 | 507 |
| 590401 | n/a | n/a | AAAAGCATCAGATGGATTAG | 21 | 8413 | 8432 | 508 |
| 590402 | n/a | n/a | TTCCACCAGTTGGTAACTTC | 78 | 2251 | 2270 | 509 |
| 590403 | n/a | n/a | TTTTTAGTAAGATCTTCAAA | 15 | 1821 | 1840 | 510 |
| 590404 | n/a | n/a | ATCTGTGTCCAAATCCCAGG | 59 | 4847 | 4866 | 511 |
| 590405 | n/a | n/a | TAAGATCTTCAAATAAGCTA | 33 | 1814 | 1833 | 512 |
| 590406 | n/a | n/a | ATCAACTCTTTCCCTTTCTT | 63 | 5746 | 5765 | 513 |
| 590407 | n/a | n/a | TGTGTCCTCAAAAGGGAGAT | 37 | 4773 | 4792 | 514 |
| 590408 | n/a | n/a | TACCTCCTCCCAACAATACC | n.d. | 9590 | 9609 | 515 |
| 590409 | n/a | n/a | TTCTGCTTTACAACTATGGC | n.d. | 9133 | 9152 | 516 |
| 590410 | n/a | n/a | GTACATGTTGAATATACATG | 35 | 4549 | 4568 | 517 |
| 590411 | n/a | n/a | TTTGTGGCTAATCTTAAGGT | 47 | 5699 | 5718 | 518 |
| 590412 | n/a | n/a | TCCTGCCTCAGCCTTTTTAA | 34 | 4415 | 4434 | 519 |
| 590413 | n/a | n/a | CGGTGTCCGCGGGACCCTCA | 59 | 1418 | 1437 | 520 |
| 590414 | n/a | n/a | GAAATGGATCAAATCTGATC | 50 | 6596 | 6615 | 521 |
| 590415 | n/a | n/a | GGTAGTTCATGAGCTAAATT | 31 | 8371 | 8390 | 522 |
| 590416 | n/a | n/a | AATGGAGTCTCGACTAGTTT | 62 | 8072 | 8091 | 523 |
| 590417 | n/a | n/a | CAAGTATGGGTCACCAGCAC | 57 | 5086 | 5105 | 524 |
| 590418 | n/a | n/a | GGTGTCCGCGGGACCCTCAG | 40 | 1417 | 1436 | 525 |
| 590419 | n/a | n/a | CGCCACGCGCAGGCCCAGCC | 37 | 1255 | 1274 | 526 |
| 590420 | n/a | n/a | TCTAGGCCTGTGTCCTCAAA | 75 | 4781 | 4800 | 527 |
| 590421 | n/a | n/a | ACTGTCCTGGGCTAATGAAG | 36 | 3204 | 3223 | 528 |
| 590422 | n/a | n/a | AAGCATCTTGTTACCTCTCT | 52 | 7698 | 7717 | 529 |
| 590423 | n/a | n/a | GCCCAGGAAGTAAAAGCATT | 38 | 7858 | 7877 | 530 |
| 590424 | n/a | n/a | GTAAGATCTTCAAATAAGCT | 46 | 1815 | 1834 | 531 |
| 590425 | n/a | n/a | AAAGGGAGATGGTAATCTTG | 48 | 4763 | 4782 | 532 |
| 590426 | n/a | n/a | GCCAAGGCTGCTGCCTTACA | 66 | 6873 | 6892 | 533 |
| 590427 | n/a | n/a | CAGACTAACTGTTCCTGTCC | 43 | 2363 | 2382 | 534 |
| 590428 | n/a | n/a | TTTGTCAATTCCTTTAAGCC | 39 | 7875 | 7894 | 535 |
| 590429 | n/a | n/a | ACTACCTCCTCCCAACAATA | n.d. | 9592 | 9611 | 536 |
| 590430 | n/a | n/a | TACCTCTCTTCATCCTTTGG | 50 | 7687 | 7706 | 537 |
| 590431 | n/a | n/a | ACTGCTCTAGGCCTGTGTCC | 59 | 4786 | 4805 | 538 |
| 590432 | n/a | n/a | CCTCCTCCCAACAATACCCA | n.d. | 9588 | 9607 | 539 |
| 590433 | n/a | n/a | GGCAGGCAAGTTACAGGAAG | 42 | 6689 | 6708 | 540 |

**Table 7**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | inhibition with RTS3898 | SEQ ID NO: 2 Start Site | SEQ ID NO: 2 Stop Site | SEQ ID NO |
| 592596 | 2 | 21 | TCGCCCACTCTGGCCCCAAA | 86 | 808 | 827 | 541 |
| 592597 | 4 | 23 | CCTCGCCCACTCTGGCCCCA | 89 | 810 | 829 | 542 |
| 592598 | 6 | 25 | CGCCTCGCCCACTCTGGCCC | 56 | 812 | 831 | 543 |
| 592599 | 8 | 27 | CGCGCCTCGCCCACTCTGGC | 68 | 814 | 833 | 544 |
| 592600 | 10 | 29 | TCCGCGCCTCGCCCACTCTG | 64 | 816 | 835 | 545 |
| 592601 | 12 | 31 | CCTCCGCGCCTCGCCCACTC | 83 | 818 | 837 | 546 |
| 592602 | 14 | 33 | GACCTCCGCGCCTCGCCCAC | 89 | 820 | 839 | 547 |
| 592603 | 16 | 35 | CAGACCTCCGCGCCTCGCCC | 88 | 822 | 841 | 548 |
| 592604 | 18 | 37 | GCCAGACCTCCGCGCCTCGC | 79 | 824 | 843 | 549 |
| 592605 | 20 | 39 | AGGCCAGACCTCCGCGCCTC | 89 | 826 | 845 | 550 |
| 592606 | 22 | 41 | ATAGGCCAGACCTCCGCGCC | 88 | 828 | 847 | 551 |
| 592607 | 24 | 43 | TTATAGGCCAGACCTCCGCG | 75 | 830 | 849 | 552 |
| 592608 | 26 | 45 | CTTTATAGGCCAGACCTCCG | 21 | 832 | 851 | 553 |
| 592609 | 28 | 47 | TACTTTATAGGCCAGACCTC | 76 | 834 | 853 | 554 |
| 592610 | 30 | 49 | ACTACTTTATAGGCCAGACC | 60 | 836 | 855 | 555 |
| 592611 | 32 | 51 | CGACTACTTTATAGGCCAGA | 0 | 838 | 857 | 556 |
| 592612 | 34 | 53 | CGCGACTACTTTATAGGCCA | 0 | 840 | 859 | 557 |
| 592613 | 36 | 55 | TCCGCGACTACTTTATAGGC | 0 | 842 | 861 | 558 |
| 592614 | 38 | 57 | TCTCCGCGACTACTTTATAG | 7 | 844 | 863 | 559 |
| 592615 | 40 | 59 | CGTCTCCGCGACTACTTTAT | 0 | 846 | 865 | 560 |
| 592616 | 42 | 61 | CCCGTCTCCGCGACTACTTT | 0 | 848 | 867 | 561 |
| 592617 | 44 | 63 | ACCCCGTCTCCGCGACTACT | 0 | 850 | 869 | 562 |
| 592618 | 46 | 65 | GCACCCCGTCTCCGCGACTA | 0 | 852 | 871 | 563 |
| 592619 | 48 | 67 | CAGCACCCCGTCTCCGCGAC | 0 | 854 | 873 | 564 |
| 592620 | 50 | 69 | ACCAGCACCCCGTCTCCGCG | 0 | 856 | 875 | 565 |
| 592621 | 52 | 71 | AAACCAGCACCCCGTCTCCG | 2 | 858 | 877 | 566 |
| 592622 | 54 | 73 | GCAAACCAGCACCCCGTCTC | 4 | 860 | 879 | 567 |
| 592623 | 56 | 75 | ACGCAAACCAGCACCCCGTC | 0 | 862 | 881 | 568 |
| 592624 | 58 | 77 | CGACGCAAACCAGCACCCCG | 0 | 864 | 883 | 569 |
| 592625 | 60 | 79 | TACGACGCAAACCAGCACCC | 0 | 866 | 885 | 570 |
| 592626 | 62 | 81 | ACTACGACGCAAACCAGCAC | 0 | 868 | 887 | 571 |
| 592627 | 64 | 83 | AGACTACGACGCAAACCAGC | 1 | 870 | 889 | 572 |
| 592628 | 66 | 85 | GGAGACTACGACGCAAACCA | 0 | 872 | 891 | 573 |
| 592629 | 68 | 87 | CAGGAGACTACGACGCAAAC | 0 | 874 | 893 | 574 |
| 592630 | 70 | 89 | TGCAGGAGACTACGACGCAA | 0 | 876 | 895 | 575 |
| 592631 | 72 | 91 | GCTGCAGGAGACTACGACGC | 1 | 878 | 897 | 576 |
| 150511 | 74 | 93 | ACGCTGCAGGAGACTACGAC | 0 | 880 | 899 | 61 |
| 592632 | 90 | 109 | GCAACGGAAACCCCAGACGC | 2 | 896 | 915 | 577 |
| 592633 | 92 | 111 | CTGCAACGGAAACCCCAGAC | 0 | 898 | 917 | 578 |
| 592634 | 94 | 113 | GACTGCAACGGAAACCCCAG | 0 | 900 | 919 | 579 |
| 345715 | 95 | 114 | GGACTGCAACGGAAACCCCA | 0 | 901 | 920 | 580 |
| 592635 | 96 | 115 | AGGACTGCAACGGAAACCCC | 1 | 902 | 921 | 581 |
| 150437 | 98 | 117 | CGAGGACTGCAACGGAAACC | 6 | 904 | 923 | 62 |
| 592636 | 100 | 119 | TCCGAGGACTGCAACGGAAA | 6 | 906 | 925 | 582 |
| 592637 | 102 | 121 | GTTCCGAGGACTGCAACGGA | 12 | 908 | 927 | 583 |
| 592638 | 104 | 123 | TGGTTCCGAGGACTGCAACG | 0 | 910 | 929 | 584 |
| 592639 | 106 | 125 | CCTGGTTCCGAGGACTGCAA | 32 | 912 | 931 | 585 |
| 592640 | 108 | 127 | GTCCTGGTTCCGAGGACTGC | 68 | 914 | 933 | 586 |
| 345717 | 110 | 129 | AGGTCCTGGTTCCGAGGACT | 65 | 916 | 935 | 587 |
| 592641 | 112 | 131 | CGAGGTCCTGGTTCCGAGGA | 84 | 918 | 937 | 588 |
| 592642 | 114 | 133 | GCCGAGGTCCTGGTTCCGAG | 86 | 920 | 939 | 589 |
| 592643 | 116 | 135 | ACGCCGAGGTCCTGGTTCCG | 78 | 922 | 941 | 590 |
| 592644 | 118 | 137 | CCACGCCGAGGTCCTGGTTC | 79 | 924 | 943 | 591 |
| 345719 | 120 | 139 | GGCCACGCCGAGGTCCTGGT | 63 | 926 | 945 | 592 |
| 150441 | 122 | 141 | TAGGCCACGCCGAGGTCCTG | 81 | 928 | 947 | 63 |
| 592645 | 124 | 143 | GCTAGGCCACGCCGAGGTCC | 63 | 930 | 949 | 593 |
| 592646 | 126 | 145 | TCGCTAGGCCACGCCGAGGT | 56 | 932 | 951 | 594 |
| 592647 | 128 | 147 | ACTCGCTAGGCCACGCCGAG | 48 | 934 | 953 | 595 |
| 345721 | 130 | 149 | TAACTCGCTAGGCCACGCCG | 63 | 936 | 955 | 596 |
| 592648 | 132 | 151 | CATAACTCGCTAGGCCACGC | 38 | 938 | 957 | 597 |
| 592649 | 134 | 153 | GCCATAACTCGCTAGGCCAC | 52 | 940 | 959 | 598 |
| 592650 | 136 | 155 | TCGCCATAACTCGCTAGGCC | 59 | 942 | 961 | 599 |
| 592651 | 138 | 157 | CGTCGCCATAACTCGCTAGG | 55 | 944 | 963 | 600 |
| 592652 | 156 | 175 | CAGCACGCACACGGCCTTCG | 56 | 962 | 981 | 601 |
| 333605 | 158 | 177 | TTCAGCACGCACACGGCCTT | 85 | 964 | 983 | 64 |
| 333606 | 160 | 179 | CCTTCAGCACGCACACGGCC | 82 | 966 | 985 | 65 |
| 146144 | 162 | 181 | GCCCTTCAGCACGCACACGG | 58 | 968 | 987 | 66 |
| 333609 | 164 | 183 | TCGCCCTTCAGCACGCACAC | 79 | 970 | 989 | 67 |
| 146145 | 165 | 184 | GTCGCCCTTCAGCACGCACA | 86 | 971 | 990 | 54 |
| 333610 | 166 | 185 | CGTCGCCCTTCAGCACGCAC | 79 | 972 | 991 | 68 |
| 333611 | 167 | 186 | CCGTCGCCCTTCAGCACGCA | 83 | 973 | 992 | 21 |
| 592653 | 168 | 187 | GCCGTCGCCCTTCAGCACGC | 79 | 974 | 993 | 602 |
| 592654 | 169 | 188 | GGCCGTCGCCCTTCAGCACG | 72 | 975 | 994 | 603 |
| 592655 | 170 | 189 | GGGCCGTCGCCCTTCAGCAC | 51 | 976 | 995 | 604 |
| 592656 | 172 | 191 | CTGGGCCGTCGCCCTTCAGC | 45 | 978 | 997 | 605 |
| 592657 | 174 | 193 | CACTGGGCCGTCGCCCTTCA | 33 | 980 | 999 | 606 |
| 592658 | 176 | 195 | TGCACTGGGCCGTCGCCCTT | 72 | 982 | 1001 | 607 |
| 592659 | 178 | 197 | CCTGCACTGGGCCGTCGCCC | 76 | 984 | 1003 | 608 |

**Table 8**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | inhibition with RTS3898 | SEQ ID NO: 2 Start Site | SEQ ID NO: 2 Stop Site | SEQ ID NO |
| 333611 | 167 | 186 | CCGTCGCCCTTCAGCACGCA | 87 | 973 | 992 | 21 |
| 150443 | 180 | 199 | GCCCTGCACTGGGCCGTCGC | 65 | 986 | 1005 | 69 |
| 592660 | 182 | 201 | ATGCCCTGCACTGGGCCGTC | 52 | 988 | 1007 | 609 |
| 592661 | 184 | 203 | TGATGCCCTGCACTGGGCCG | 30 | 990 | 1009 | 610 |
| 592662 | 186 | 205 | GATGATGCCCTGCACTGGGC | 38 | 992 | 1011 | 611 |
| 592663 | 188 | 207 | TTGATGATGCCCTGCACTGG | 36 | 994 | 1013 | 612 |
| 150444 | 190 | 209 | AATTGATGATGCCCTGCACT | 48 | 996 | 1015 | 70 |
| 592664 | 192 | 211 | GAAATTGATGATGCCCTGCA | 35 | 998 | 1017 | 15 |
| 592665 | 194 | 213 | TCGAAATTGATGATGCCCTG | 40 | 1000 | 1019 | 614 |
| 592666 | 196 | 215 | GCTCGAAATTGATGATGCCC | 68 | 1002 | 1021 | 615 |
| 592667 | 198 | 217 | CTGCTCGAAATTGATGATGC | 63 | 1004 | 1023 | 616 |
| 592668 | 200 | 219 | TTCTGCTCGAAATTGATGAT | 47 | 1006 | 1025 | 617 |
| 592669 | 239 | 258 | TTAATGCTTCCCCACACCTT | 68 | 4993 | 5012 | 618 |
| 592670 | 241 | 260 | CTTTAATGCTTCCCCACACC | 71 | 4995 | 5014 | 619 |
| 592671 | 243 | 262 | TCCTTTAATGCTTCCCCACA | 69 | 4997 | 5016 | 620 |
| 150448 | 245 | 264 | AGTCCTTTAATGCTTCCCCA | 76 | 4999 | 5018 | 71 |
| 592672 | 247 | 266 | TCAGTCCTTTAATGCTTCCC | 75 | 5001 | 5020 | 621 |
| 592673 | 249 | 268 | AGTCAGTCCTTTAATGCTTC | 58 | 5003 | 5022 | 622 |
| 592674 | 251 | 270 | TCAGTCAGTCCTTTAATGCT | 46 | 5005 | 5024 | 623 |
| 592675 | 253 | 272 | CTTCAGTCAGTCCTTTAATG | 41 | 5007 | 5026 | 624 |
| 592676 | 255 | 274 | GCCTTCAGTCAGTCCTTTAA | 62 | 5009 | 5028 | 625 |
| 150449 | 257 | 276 | AGGCCTTCAGTCAGTCCTTT | 65 | 5011 | 5030 | 72 |
| 592677 | 259 | 278 | GCAGGCCTTCAGTCAGTCCT | 69 | 5013 | 5032 | 626 |
| 592678 | 261 | 280 | ATGCAGGCCTTCAGTCAGTC | 65 | 5015 | 5034 | 627 |
| 592679 | 263 | 282 | CCATGCAGGCCTTCAGTCAG | 53 | 5017 | 5036 | 628 |
| 592680 | 277 | 296 | CATGAACATGGAATCCATGC | 63 | 5031 | 5050 | 629 |
| 592681 | 279 | 298 | CTCATGAACATGGAATCCAT | 60 | 5033 | 5052 | 630 |
| 592682 | 281 | 300 | AACTCATGAACATGGAATCC | 56 | 5035 | 5054 | 631 |
| 592683 | 284 | 303 | CCAAACTCATGAACATGGAA | 60 | 5038 | 5057 | 632 |
| 592684 | 286 | 305 | CTCCAAACTCATGAACATGG | 69 | 5040 | 5059 | 633 |
| 592685 | 288 | 307 | ATCTCCAAACTCATGAACAT | 40 | 5042 | 5061 | 634 |
| 592686 | 290 | 309 | TTATCTCCAAACTCATGAAC | 35 | 5044 | 5063 | 635 |
| 592687 | 292 | 311 | TATTATCTCCAAACTCATGA | 26 | 5046 | 5065 | 636 |
| 592688 | 294 | 313 | TGTATTATCTCCAAACTCAT | 41 | 5048 | 5067 | 637 |
| 150452 | 296 | 315 | GCTGTATTATCTCCAAACTC | 51 | 5050 | 5069 | 73 |
| 592689 | 298 | 317 | CTGCTGTATTATCTCCAAAC | 43 | 5052 | 5071 | 638 |
| 592690 | 300 | 319 | GCCTGCTGTATTATCTCCAA | 37 | n/a | n/a | 639 |
| 592691 | 302 | 321 | CAGCCTGCTGTATTATCTCC | 33 | n/a | n/a | 640 |
| 592692 | 304 | 323 | TACAGCCTGCTGTATTATCT | 27 | n/a | n/a | 641 |
| 592693 | 306 | 325 | GGTACAGCCTGCTGTATTAT | 21 | n/a | n/a | 642 |
| 592694 | 308 | 327 | CTGGTACAGCCTGCTGTATT | 23 | n/a | n/a | 643 |
| 592695 | 310 | 329 | CACTGGTACAGCCTGCTGTA | 46 | n/a | n/a | 644 |
| 592696 | 312 | 331 | TGCACTGGTACAGCCTGCTG | 40 | n/a | n/a | 645 |
| 592697 | 314 | 333 | CCTGCACTGGTACAGCCTGC | 62 | n/a | n/a | 646 |
| 592698 | 340 | 359 | TTCTGGATAGAGGATTAAAG | 41 | 7656 | 7675 | 647 |
| 592699 | 342 | 361 | TTTTCTGGATAGAGGATTAA | 29 | 7658 | 7677 | 648 |
| 592700 | 344 | 363 | TGTTTTCTGGATAGAGGATT | 51 | 7660 | 7679 | 649 |
| 592701 | 346 | 365 | CGTGTTTTCTGGATAGAGGA | 64 | 7662 | 7681 | 650 |
| 592702 | 348 | 367 | ACCGTGTTTTCTGGATAGAG | 44 | 7664 | 7683 | 651 |
| 592703 | 350 | 369 | CCACCGTGTTTTCTGGATAG | 62 | 7666 | 7685 | 652 |
| 592704 | 352 | 371 | GCCCACCGTGTTTTCTGGAT | 60 | 7668 | 7687 | 653 |
| 592705 | 354 | 373 | TGGCCCACCGTGTTTTCTGG | 62 | 7670 | 7689 | 654 |
| 592706 | 356 | 375 | TTTGGCCCACCGTGTTTTCT | 49 | 7672 | 7691 | 655 |
| 592707 | 358 | 377 | CCTTTGGCCCACCGTGTTTT | 52 | 7674 | 7693 | 656 |
| 592708 | 382 | 401 | AGTCTCCAACATGCCTCTCT | 53 | n/a | n/a | 657 |
| 592709 | 384 | 403 | CAAGTCTCCAACATGCCTCT | 39 | n/a | n/a | 658 |
| 489501 | 386 | 405 | CCCAAGTCTCCAACATGCCT | 75 | 8441 | 8460 | 659 |
| 150454 | 388 | 407 | TGCCCAAGTCTCCAACATGC | 86 | 8443 | 8462 | 74 |
| 592710 | 390 | 409 | ATTGCCCAAGTCTCCAACAT | 71 | 8445 | 8464 | 660 |
| 592711 | 392 | 411 | ACATTGCCCAAGTCTCCAAC | 64 | 8447 | 8466 | 661 |
| 592712 | 394 | 413 | TCACATTGCCCAAGTCTCCA | 59 | 8449 | 8468 | 662 |
| 489502 | 396 | 415 | AGTCACATTGCCCAAGTCTC | 70 | 8451 | 8470 | 663 |
| 592713 | 398 | 417 | GCAGTCACATTGCCCAAGTC | 70 | 8453 | 8472 | 664 |
| 592714 | 400 | 419 | CAGCAGTCACATTGCCCAAG | 84 | 8455 | 8474 | 665 |
| 592715 | 402 | 421 | GTCAGCAGTCACATTGCCCA | 83 | 8457 | 8476 | 666 |
| 592716 | 404 | 423 | TTGTCAGCAGTCACATTGCC | 59 | 8459 | 8478 | 667 |
| 489503 | 406 | 425 | CTTTGTCAGCAGTCACATTG | 47 | 8461 | 8480 | 668 |
| 592717 | 408 | 427 | ATCTTTGTCAGCAGTCACAT | 54 | 8463 | 8482 | 669 |
| 592718 | 410 | 429 | CCATCTTTGTCAGCAGTCAC | 76 | 8465 | 8484 | 670 |
| 592719 | 412 | 431 | CACCATCTTTGTCAGCAGTC | 75 | 8467 | 8486 | 671 |
| 592720 | 414 | 433 | CACACCATCTTTGTCAGCAG | 66 | 8469 | 8488 | 672 |
| 489504 | 416 | 435 | GCCACACCATCTTTGTCAGC | 60 | 8471 | 8490 | 673 |
| 592721 | 418 | 437 | CGGCCACACCATCTTTGTCA | 62 | 8473 | 8492 | 674 |
| 592722 | 420 | 439 | ATCGGCCACACCATCTTTGT | 57 | 8475 | 8494 | 675 |
| 592723 | 422 | 441 | ACATCGGCCACACCATCTTT | 54 | 8477 | 8496 | 676 |
| 150458 | 424 | 443 | ACACATCGGCCACACCATCT | 77 | 8479 | 8498 | 75 |
| 489505 | 426 | 445 | AGACACATCGGCCACACCAT | 84 | 8481 | 8500 | 677 |
| 592724 | 428 | 447 | ATAGACACATCGGCCACACC | 66 | 8483 | 8502 | 678 |

**Table 9**

| Percent inhibition of SOD-1 mRNA by 5-10-5 MOE gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | inhibition with RTS3898 | inhibitio n with HTS90 | SEQ ID NO: 2 Start Site | SEQ ID NO: 2 Stop Site | SEQ ID NO |
| 333611 | 167 | 186 | CCGTCGCCCTTCAGCACGCA | 87 | n.d. | 973 | 992 | 21 |
| 592725 | 430 | 449 | CAATAGACACATCGGCCAC A | 67 | 65 | 8485 | 8504 | 679 |
| 592726 | 432 | 451 | TTCAATAGACACATCGGCCA | 62 | 66 | 8487 | 8506 | 680 |
| 592727 | 434 | 453 | TCTTCAATAGACACATCGGC | 56 | 49 | 8489 | 8508 | 681 |
| 489506 | 436 | 455 | AATCTTCAATAGACACATCG | 25 | 28 | 8491 | 8510 | 682 |
| 592728 | 438 | 457 | AGAATCTTCAATAGACACAT | 12 | 0 | 8493 | 8512 | 683 |
| 592729 | 440 | 459 | ACAGAATCTTCAATAGACAC | 24 | 16 | 8495 | 8514 | 684 |
| 592730 | 442 | 461 | TCACAGAATCTTCAATAGAC | 34 | 24 | 8497 | 8516 | 685 |
| 592731 | 444 | 463 | GATCACAGAATCTTCAATAG | 15 | 14 | 8499 | 8518 | 686 |
| 489507 | 446 | 465 | GAGATCACAGAATCTTCAAT | 42 | 46 | 8501 | 8520 | 687 |
| 592732 | 448 | 467 | GTGAGATCACAGAATCTTCA | n.d. | 58 | 8503 | 8522 | 688 |
| 592733 | 450 | 469 | GAGTGAGATCACAGAATCTT | n.d. | 45 | 8505 | 8524 | 689 |
| 592734 | 452 | 471 | GAGAGTGAGATCACAGAAT C | n.d. | 48 | 8507 | 8526 | 690 |
| 592735 | 454 | 473 | CTGAGAGTGAGATCACAGA A | n.d. | 66 | 8509 | 8528 | 691 |
| 489508 | 456 | 475 | TCCTGAGAGTGAGATCACA G | n.d. | 60 | 8511 | 8530 | 692 |
| 333619 | 458 | 477 | TCTCCTGAGAGTGAGATCAC | n.d. | 65 | 8513 | 8532 | 76 |
| 592736 | 460 | 479 | GGTCTCCTGAGAGTGAGATC | n.d. | 40 | 8515 | 8534 | 693 |
| 592737 | 462 | 481 | ATGGTCTCCTGAGAGTGAGA | n.d. | 37 | 8517 | 8536 | 694 |
| 592738 | 464 | 483 | CAATGGTCTCCTGAGAGTGA | n.d. | 41 | 8519 | 8538 | 695 |
| 489509 | 466 | 485 | TGCAATGGTCTCCTGAGAGT | n.d. | 43 | 8521 | 8540 | 696 |
| 592739 | 468 | 487 | GATGCAATGGTCTCCTGAGA | n.d. | 16 | 8523 | 8542 | 697 |
| 592740 | 470 | 489 | ATGATGCAATGGTCTCCTGA | n.d. | 6 | 8525 | 8544 | 698 |
| 592741 | 472 | 491 | CAATGATGCAATGGTCTCCT | n.d. | 0 | 8527 | 8546 | 699 |
| 592742 | 474 | 493 | GCCAATGATGCAATGGTCTC | n.d. | 25 | 8529 | 8548 | 700 |
| 489510 | 476 | 495 | CGGCCAATGATGCAATGGTC | n.d. | 32 | 8531 | 8550 | 701 |
| 592743 | 478 | 497 | TGCGGCCAATGATGCAATG G | n.d. | 14 | 8533 | 8552 | 702 |
| 592744 | 480 | 499 | TGTGCGGCCAATGATGCAAT | n.d. | 0 | 8535 | 8554 | 703 |
| 592745 | 482 | 501 | AGTGTGCGGCCAATGATGC A | n.d. | 7 | 8537 | 8556 | 704 |
| 592746 | 484 | 503 | CCAGTGTGCGGCCAATGATG | n.d. | 25 | 8539 | 8558 | 705 |
| 489511 | 486 | 505 | CACCAGTGTGCGGCCAATG A | n.d. | 28 | 8541 | 8560 | 706 |
| 150460 | 488 | 507 | ACCACCAGTGTGCGGCCAAT | n.d. | 52 | 8543 | 8562 | 77 |
| 592747 | 490 | 509 | GGACCACCAGTGTGCGGCC A | n.d. | 44 | n/a | n/a | 707 |
| 592748 | 492 | 511 | ATGGACCACCAGTGTGCGG C | n.d. | 40 | n/a | n/a | 708 |
| 150462 | 494 | 513 | TCATGGACCACCAGTGTGCG | n.d. | 39 | n/a | n/a | 78 |
| 592749 | 496 | 515 | TTTCATGGACCACCAGTGTG | n.d. | 35 | n/a | n/a | 709 |
| 592750 | 498 | 517 | TTTTTCATGGACCACCAGTG | n.d. | 23 | n/a | n/a | 710 |
| 592751 | 500 | 519 | GCTTTTTCATGGACCACCAG | n.d. | 63 | n/a | n/a | 711 |
| 333636 | 536 | 555 | GTACTTTCTTCATTTCCACC | n.d. | 65 | 9686 | 9705 | 79 |
| 333638 | 538 | 557 | TTGTACTTTCTTCATTTCCA | n.d. | 66 | 9688 | 9707 | 80 |
| 333640 | 540 | 559 | CTTTGTACTTTCTTCATTTC | n.d. | 37 | 9690 | 9709 | 81 |
| 592752 | 543 | 562 | TGTCTTTGTACTTTCTTCAT | n.d. | 63 | 9693 | 9712 | 712 |
| 592753 | 545 | 564 | CCTGTCTTTGTACTTTCTTC | n.d. | 74 | 9695 | 9714 | 713 |
| 592754 | 547 | 566 | TTCCTGTCTTTGTACTTTCT | n.d. | 72 | 9697 | 9716 | 714 |
| 592755 | 549 | 568 | GTTTCCTGTCTTTGTACTTT | n.d. | 57 | 9699 | 9718 | 715 |
| 592756 | 568 | 587 | AAGCCAAACGACTTCCAGC G | 72 | 66 | 9718 | 9737 | 716 |
| 592757 | 570 | 589 | ACAAGCCAAACGACTTCCA G | 72 | 74 | 9720 | 9739 | 717 |
| 489516 | 572 | 591 | CCACAAGCCAAACGACTTCC | 85 | 82 | 9722 | 9741 | 718 |
| 592758 | 574 | 593 | CACCACAAGCCAAACGACT T | 72 | 73 | 9724 | 9743 | 719 |
| 592759 | 576 | 595 | TACACCACAAGCCAAACGA C | 74 | 68 | 9726 | 9745 | 720 |
| 592760 | 578 | 597 | ATTACACCACAAGCCAAAC G | 67 | 61 | 9728 | 9747 | 721 |
| 592761 | 580 | 599 | CAATTACACCACAAGCCAA A | 64 | 56 | 9730 | 9749 | 722 |
| 150466 | 640 | 659 | GATAACAGATGAGTTAAGG G | 66 | 65 | 9790 | 9809 | 82 |
| 489521 | 642 | 661 | AGGATAACAGATGAGTTAA G | 79 | 78 | 9792 | 9811 | 723 |
| 592762 | 663 | 682 | GGATACATTTCTACAGCTAG | 91 | 87 | 9813 | 9832 | 724 |
| 592763 | 665 | 684 | CAGGATACATTTCTACAGCT | 92 | 89 | 9815 | 9834 | 725 |
| 592764 | 667 | 686 | ATCAGGATACATTTCTACAG | 88 | 83 | 9817 | 9836 | 726 |
| 592765 | 669 | 688 | TTATCAGGATACATTTCTAC | 77 | 72 | 9819 | 9838 | 727 |
| 592766 | 671 | 690 | GTTTATCAGGATACATTTCT | 90 | 89 | 9821 | 9840 | 728 |
| 592767 | 673 | 692 | ATGTTTATCAGGATACATTT | 82 | 76 | 9823 | 9842 | 729 |
| 592768 | 675 | 694 | TAATGTTTATCAGGATACAT | 80 | 79 | 9825 | 9844 | 730 |
| 592769 | 677 | 696 | TTTAATGTTTATCAGGATAC | 82 | 78 | 9827 | 9846 | 731 |
| 592770 | 679 | 698 | TGTTTAATGTTTATCAGGAT | 79 | 75 | 9829 | 9848 | 732 |
| 592771 | 681 | 700 | AGTGTTTAATGTTTATCAGG | 84 | 81 | 9831 | 9850 | 733 |
| 489526 | 692 | 711 | TTTAAGATTACAGTGTTTAA | 36 | 38 | 9842 | 9861 | 734 |
| 592772 | 694 | 713 | CTTTTAAGATTACAGTGTTT | 46 | 47 | 9844 | 9863 | 735 |
| 592773 | 696 | 715 | CACTTTTAAGATTACAGTGT | 39 | 42 | 9846 | 9865 | 736 |
| 592774 | 698 | 717 | TACACTTTTAAGATTACAGT | 21 | 24 | 9848 | 9867 | 737 |
| 592775 | 700 | 719 | ATTACACTTTTAAGATTACA | 3 | 0 | 9850 | 9869 | 738 |
| 489527 | 702 | 721 | CAATTACACTTTTAAGATTA | 0 | 0 | 9852 | 9871 | 739 |
| 150467 | 704 | 723 | CACAATTACACTTTTAAGAT | 58 | 73 | 9854 | 9873 | 83 |
| 592776 | 706 | 725 | CACACAATTACACTTTTAAG | 29 | 5 | 9856 | 9875 | 740 |
| 592777 | 708 | 727 | GTCACACAATTACACTTTTA | 59 | 49 | 9858 | 9877 | 741 |
| 592778 | 710 | 729 | AAGTCACACAATTACACTTT | 40 | 34 | 9860 | 9879 | 742 |
| 489528 | 712 | 731 | AAAAGTCACACAATTACACT | 31 | 27 | 9862 | 9881 | 743 |
| 592779 | 714 | 733 | GAAAAAGTCACACAATTAC A | 21 | 7 | 9864 | 9883 | 744 |
| 592780 | 716 | 735 | CTGAAAAAGTCACACAATT A | 18 | 13 | 9866 | 9885 | 745 |
| 592781 | 718 | 737 | CTCTGAAAAAGTCACACAAT | 32 | 26 | 9868 | 9887 | 746 |
| 592782 | 720 | 739 | AACTCTGAAAAAGTCACAC A | 35 | 20 | 9870 | 9889 | 747 |

**Table 10**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | % inhibition with RTS3898 | SEQ ID NO: 2 Start Site | SEQ ID NO: 2 Stop Site | SEQ ID NO |
| 333611 | 167 | 186 | CCGTCGCCCTTCAGCACGCA | 74 | 973 | 992 | 21 |
| 489529 | 722 | 741 | GCAACTCTGAAAAAGTCACA | 41 | 9872 | 9891 | 748 |
| 592783 | 724 | 743 | AAGCAACTCTGAAAAAGTCA | 34 | 9874 | 9893 | 749 |
| 592784 | 727 | 746 | TTAAAGCAACTCTGAAAAAG | 4 | 9877 | 9896 | 750 |
| 592785 | 729 | 748 | CTTTAAAGCAACTCTGAAAA | 36 | 9879 | 9898 | 751 |
| 592786 | 731 | 750 | TACTTTAAAGCAACTCTGAA | 28 | 9881 | 9900 | 752 |
| 592787 | 733 | 752 | GGTACTTTAAAGCAACTCTG | 48 | 9883 | 9902 | 753 |
| 592788 | 735 | 754 | CAGGTACTTTAAAGCAACTC | 38 | 9885 | 9904 | 754 |
| 592789 | 737 | 756 | TACAGGTACTTTAAAGCAAC | 20 | 9887 | 9906 | 755 |
| 592790 | 739 | 758 | ACTACAGGTACTTTAAAGCA | 26 | 9889 | 9908 | 756 |
| 592791 | 741 | 760 | TCACTACAGGTACTTTAAAG | 34 | 9891 | 9910 | 757 |
| 592792 | 743 | 762 | TCTCACTACAGGTACTTTAA | 50 | 9893 | 9912 | 758 |
| 592793 | 745 | 764 | TTTCTCACTACAGGTACTTT | 36 | 9895 | 9914 | 759 |
| 592794 | 747 | 766 | AGTTTCTCACTACAGGTACT | 53 | 9897 | 9916 | 760 |
| 592795 | 749 | 768 | TCAGTTTCTCACTACAGGTA | 37 | 9899 | 9918 | 761 |
| 150470 | 751 | 770 | AATCAGTTTCTCACTACAGG | 30 | 9901 | 9920 | 84 |
| 592796 | 753 | 772 | TAAATCAGTTTCTCACTACA | 21 | 9903 | 9922 | 762 |
| 150472 | 755 | 774 | CATAAATCAGTTTCTCACTA | 37 | 9905 | 9924 | 85 |
| 592797 | 757 | 776 | ATCATAAATCAGTTTCTCAC | 35 | 9907 | 9926 | 763 |
| 592798 | 759 | 778 | TGATCATAAATCAGTTTCTC | 35 | 9909 | 9928 | 764 |
| 592799 | 761 | 780 | AGTGATCATAAATCAGTTTC | 5 | 9911 | 9930 | 765 |
| 592800 | 763 | 782 | CAAGTGATCATAAATCAGTT | 21 | 9913 | 9932 | 766 |
| 592801 | 765 | 784 | TCCAAGTGATCATAAATCAG | 41 | 9915 | 9934 | 767 |
| 592802 | 767 | 786 | CTTCCAAGTGATCATAAATC | 44 | 9917 | 9936 | 768 |
| 592803 | 769 | 788 | ATCTTCCAAGTGATCATAAA | 30 | 9919 | 9938 | 769 |
| 592804 | 771 | 790 | AAATCTTCCAAGTGATCATA | 32 | 9921 | 9940 | 770 |
| 489534 | 792 | 811 | CTGAGTTTTATAAAACTATA | 4 | 9942 | 9961 | 771 |
| 150476 | 794 | 813 | AACTGAGTTTTATAAAACTA | 9 | 9944 | 9963 | 86 |
| 592805 | 796 | 815 | TTAACTGAGTTTTATAAAAC | 14 | 9946 | 9965 | 772 |
| 592806 | 798 | 817 | TTTTAACTGAGTTTTATAAA | 3 | 9948 | 9967 | 773 |
| 592807 | 800 | 819 | CATTTTAACTGAGTTTTATA | 13 | 9950 | 9969 | 774 |
| 489535 | 802 | 821 | GACATTTTAACTGAGTTTTA | 34 | 9952 | 9971 | 775 |
| 592808 | 804 | 823 | CAGACATTTTAACTGAGTTT | 40 | 9954 | 9973 | 776 |
| 592809 | 806 | 825 | AACAGACATTTTAACTGAGT | 36 | 9956 | 9975 | 777 |
| 592810 | 808 | 827 | GAAACAGACATTTTAACTGA | 25 | 9958 | 9977 | 778 |
| 592811 | 810 | 829 | TTGAAACAGACATTTTAACT | 24 | 9960 | 9979 | 779 |
| 592812 | 835 | 854 | TTTAAGTCTGGCAAAATACA | 23 | 9985 | 10004 | 780 |
| 592813 | 837 | 856 | GATTTAAGTCTGGCAAAATA | 31 | 9987 | 10006 | 781 |
| 592814 | 839 | 858 | GTGATTTAAGTCTGGCAAAA | 41 | 9989 | 10008 | 782 |
| 592815 | 841 | 860 | CTGTGATTTAAGTCTGGCAA | 49 | 9991 | 10010 | 783 |
| 592816 | 843 | 862 | ATCTGTGATTTAAGTCTGGC | 53 | 9993 | 10012 | 784 |
| 150481 | 845 | 864 | CCATCTGTGATTTAAGTCTG | 51 | 9995 | 10014 | 87 |
| 592817 | 847 | 866 | ACCCATCTGTGATTTAAGTC | 51 | 9997 | 10016 | 785 |
| 592818 | 849 | 868 | ATACCCATCTGTGATTTAAG | 43 | 9999 | 10018 | 786 |
| 592819 | 851 | 870 | TAATACCCATCTGTGATTTA | 42 | 10001 | 10020 | 787 |
| 592820 | 870 | 889 | AAAGAAATTCTGACAAGTTT | 22 | 10020 | 10039 | 788 |
| 489542 | 872 | 891 | ACAAAGAAATTCTGACAAGT | 13 | 10022 | 10041 | 789 |
| 592821 | 874 | 893 | TGACAAAGAAATTCTGACAA | 24 | 10024 | 10043 | 790 |
| 592822 | 876 | 895 | AATGACAAAGAAATTCTGAC | 25 | 10026 | 10045 | 791 |
| 592823 | 878 | 897 | TGAATGACAAAGAAATTCTG | 6 | 10028 | 10047 | 792 |
| 592824 | 880 | 899 | CTTGAATGACAAAGAAATTC | 24 | 10030 | 10049 | 793 |
| 489543 | 882 | 901 | GGCTTGAATGACAAAGAAAT | 29 | 10032 | 10051 | 794 |
| 592825 | 884 | 903 | CAGGCTTGAATGACAAAGAA | 35 | 10034 | 10053 | 795 |
| 592826 | 886 | 905 | CACAGGCTTGAATGACAAAG | 32 | 10036 | 10055 | 796 |
| 592827 | 888 | 907 | TTCACAGGCTTGAATGACAA | 41 | 10038 | 10057 | 797 |
| 592828 | 890 | 909 | TATTCACAGGCTTGAATGAC | 30 | 10040 | 10059 | 798 |
| 150492 | 909 | 928 | AAGTGCCATACAGGGTTTTT | 32 | 10059 | 10078 | 88 |
| 592829 | 911 | 930 | ATAAGTGCCATACAGGGTTT | 0 | 10061 | 10080 | 799 |
| 150493 | 913 | 932 | TAATAAGTGCCATACAGGGT | 24 | 10063 | 10082 | 89 |
| 592830 | 915 | 934 | CATAATAAGTGCCATACAGG | 26 | 10065 | 10084 | 800 |
| 150495 | 917 | 936 | CTCATAATAAGTGCCATACA | 35 | 10067 | 10086 | 90 |
| 150496 | 919 | 938 | GCCTCATAATAAGTGCCATA | 37 | 10069 | 10088 | 91 |
| 592831 | 921 | 940 | TAGCCTCATAATAAGTGCCA | 19 | 10071 | 10090 | 801 |
| 592832 | 923 | 942 | AATAGCCTCATAATAAGTGC | 0 | 10073 | 10092 | 802 |
| 592833 | 925 | 944 | TTAATAGCCTCATAATAAGT | 19 | 10075 | 10094 | 803 |
| 150497 | 927 | 946 | TTTTAATAGCCTCATAATAA | 17 | 10077 | 10096 | 92 |
| 592834 | 929 | 948 | TCTTTTAATAGCCTCATAAT | 27 | 10079 | 10098 | 804 |
| 592835 | 931 | 950 | ATTCTTTTAATAGCCTCATA | 27 | 10081 | 10100 | 805 |
| 150498 | 933 | 952 | GGATTCTTTTAATAGCCTCA | 39 | 10083 | 10102 | 93 |
| 592836 | 935 | 954 | TTGGATTCTTTTAATAGCCT | 24 | 10085 | 10104 | 806 |
| 592837 | 937 | 956 | ATTTGGATTCTTTTAATAGC | 0 | 10087 | 10106 | 807 |
| 592838 | 939 | 958 | GAATTTGGATTCTTTTAATA | 10 | 10089 | 10108 | 808 |
| 592839 | 941 | 960 | TTGAATTTGGATTCTTTTAA | 13 | 10091 | 10110 | 809 |
| 592840 | 943 | 962 | GTTTGAATTTGGATTCTTTT | 29 | 10093 | 10112 | 810 |
| 592841 | 945 | 964 | TAGTTTGAATTTGGATTCTT | 31 | 10095 | 10114 | 811 |
| 592842 | 947 | 966 | TTTAGTTTGAATTTGGATTC | 8 | 10097 | 10116 | 812 |
| 592843 | 949 | 968 | TTTTTAGTTTGAATTTGGAT | 10 | n/a | n/a | 813 |
| 592844 | 951 | 970 | TTTTTTTAGTTTGAATTTGG | 7 | n/a | n/a | 814 |

### Example 2: Inhibition of human SOD-1 in HepG2 cells by MOE gapmers

Modified oligonucleotides were designed targeting a superoxide dismutase 1, soluble (SOD-1) nucleic acid and were tested for their effects on SOD-1 mRNA in vitro. ISIS 146143, ISIS 150438-150440, ISIS 150442, ISIS 150450, ISIS 150455-150457, ISIS 150459, ISIS 150461, ISIS 150469, ISIS 150473, ISIS 150478, ISIS 150484, ISIS 150486, ISIS 150494, ISIS 150508-150510, ISIS 333607, ISIS 333608, ISIS 333611, ISIS 333618, previously disclosed in WO 2005/040180, were also included in this assay. The modified oligonucleotides were tested in a series of experiments that had similar culture conditions. The results for each experiment are presented in separate tables shown below. Cultured HepG2 cells at a density of 20,000 cells per well were transfected using electroporation with 5,000 nM modified oligonucleotide. After a treatment period of approximately 24 hours, RNA was isolated from the cells and SOD-1 mRNA levels were measured by quantitative real-time PCR.

Human primer probe set RTS3898 was used to measure mRNA levels. In cases where the oligonucleotide overlapped the amplicon of the primer probe set, an alternative primer probe set, HTS90, was used to measure mRNA levels. SOD-1 mRNA levels were adjusted according to total RNA content, as measured by RIBOGREEN^{®}. Results are presented as percent inhibition of SOD-1, relative to untreated control cells, 'n.d.' indicates that inhibition levels were not measured using the particular primer probe set.

The newly designed modified oligonucleotides in the Tables below were designed as 5-10-5 MOE gapmers. The 5-10-5 MOE gapmers are 20 nucleosides in length, wherein the central gap segment is comprised of ten 2'-deoxyribonucleosides and is flanked by wing segments on the 5' direction and the 3' directions comprising five nucleosides each. Each nucleoside in the 5' wing segment and each nucleoside in the 3' wing segment has a 2'-MOE modification. The internucleoside linkages throughout each gapmer are phosphorothioate linkages. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT011512.10 truncated from nucleotides 18693000 to 18704000). 'n/a' indicates that the modified oligonucleotide does not target that particular gene sequence with 100% complementarity.

**Table 11**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | inhibition with RTS3898 | SEQ ID NO: 2 Start Site | SEQ ID NO: 2 Stop Site | SEQ ID NO |
| 333611 | 167 | 186 | CCGTCGCCCTTCAGCACGCA | 64 | 973 | 992 | 21 |
| 596301 | 550 | 569 | CGTTTCCTGTCTTTGTACTT | n.d. | 9700 | 9719 | 815 |
| 596302 | 569 | 588 | CAAGCCAAACGACTTCCAGC | 54 | 9719 | 9738 | 816 |
| 596303 | 571 | 590 | CACAAGCCAAACGACTTCCA | 47 | 9721 | 9740 | 817 |
| 596304 | 573 | 592 | ACCACAAGCCAAACGACTTC | 28 | 9723 | 9742 | 818 |
| 596305 | 575 | 594 | ACACCACAAGCCAAACGACT | 49 | 9725 | 9744 | 819 |
| 596306 | 577 | 596 | TTACACCACAAGCCAAACGA | 24 | 9727 | 9746 | 820 |
| 596307 | 641 | 660 | GGATAACAGATGAGTTAAGG | 48 | 9791 | 9810 | 821 |
| 596308 | 664 | 683 | AGGATACATTTCTACAGCTA | 79 | 9814 | 9833 | 822 |
| 596309 | 666 | 685 | TCAGGATACATTTCTACAGC | 70 | 9816 | 9835 | 823 |
| 596310 | 668 | 687 | TATCAGGATACATTTCTACA | 58 | 9818 | 9837 | 824 |
| 489524 | 672 | 691 | TGTTTATCAGGATACATTTC | 52 | 9822 | 9841 | 825 |
| 596311 | 674 | 693 | AATGTTTATCAGGATACATT | 54 | 9824 | 9843 | 826 |
| 596312 | 676 | 695 | TTAATGTTTATCAGGATACA | 34 | 9826 | 9845 | 827 |
| 596313 | 678 | 697 | GTTTAATGTTTATCAGGATA | 71 | 9828 | 9847 | 828 |
| 596314 | 680 | 699 | GTGTTTAATGTTTATCAGGA | 73 | 9830 | 9849 | 829 |
| 596315 | 693 | 712 | TTTTAAGATTACAGTGTTTA | 13 | 9843 | 9862 | 830 |
| 596316 | 695 | 714 | ACTTTTAAGATTACAGTGTT | 24 | 9845 | 9864 | 831 |
| 596317 | 697 | 716 | ACACTTTTAAGATTACAGTG | 15 | 9847 | 9866 | 832 |
| 596318 | 699 | 718 | TTACACTTTTAAGATTACAG | 0 | 9849 | 9868 | 833 |
| 596319 | 701 | 720 | AATTACACTTTTAAGATTAC | 1 | 9851 | 9870 | 834 |
| 596320 | 705 | 724 | ACACAATTACACTTTTAAGA | 0 | 9855 | 9874 | 835 |
| 596321 | 707 | 726 | TCACACAATTACACTTTTAA | 15 | 9857 | 9876 | 836 |
| 596322 | 711 | 730 | AAAGTCACACAATTACACTT | 15 | 9861 | 9880 | 837 |
| 596323 | 715 | 734 | TGAAAAAGTCACACAATTAC | 0 | 9865 | 9884 | 838 |
| 596324 | 717 | 736 | TCTGAAAAAGTCACACAATT | 5 | 9867 | 9886 | 839 |
| 596325 | 719 | 738 | ACTCTGAAAAAGTCACACAA | 21 | 9869 | 9888 | 840 |
| 596326 | 723 | 742 | AGCAACTCTGAAAAAGTCAC | 14 | 9873 | 9892 | 841 |
| 596327 | 730 | 749 | ACTTTAAAGCAACTCTGAAA | 0 | 9880 | 9899 | 842 |
| 489530 | 732 | 751 | GTACTTTAAAGCAACTCTGA | 22 | 9882 | 9901 | 843 |
| 596328 | 734 | 753 | AGGTACTTTAAAGCAACTCT | 36 | 9884 | 9903 | 844 |
| 596329 | 740 | 759 | CACTACAGGTACTTTAAAGC | 18 | 9890 | 9909 | 845 |
| 150469 | 742 | 761 | CTCACTACAGGTACTTTAAA | 25 | 9892 | 9911 | 94 |
| 596330 | 744 | 763 | TTCTCACTACAGGTACTTTA | 28 | 9894 | 9913 | 846 |
| 596331 | 746 | 765 | GTTTCTCACTACAGGTACTT | 30 | 9896 | 9915 | 847 |
| 596332 | 748 | 767 | CAGTTTCTCACTACAGGTAC | 25 | 9898 | 9917 | 848 |
| 596333 | 750 | 769 | ATCAGTTTCTCACTACAGGT | 22 | 9900 | 9919 | 849 |
| 489531 | 752 | 771 | AAATCAGTTTCTCACTACAG | 0 | 9902 | 9921 | 850 |
| 596334 | 756 | 775 | TCATAAATCAGTTTCTCACT | 21 | 9906 | 9925 | 851 |
| 596335 | 760 | 779 | GTGATCATAAATCAGTTTCT | 37 | 9910 | 9929 | 852 |
| 489532 | 762 | 781 | AAGTGATCATAAATCAGTTT | 8 | 9912 | 9931 | 853 |
| 596336 | 764 | 783 | CCAAGTGATCATAAATCAGT | 39 | 9914 | 9933 | 854 |
| 436935 | 766 | 785 | TTCCAAGTGATCATAAATCA | 18 | 9916 | 9935 | 855 |
| 596337 | 768 | 787 | TCTTCCAAGTGATCATAAAT | 12 | 9918 | 9937 | 856 |
| 150473 | 770 | 789 | AATCTTCCAAGTGATCATAA | 4 | 9920 | 9939 | 95 |
| 596338 | 795 | 814 | TAACTGAGTTTTATAAAACT | 0 | 9945 | 9964 | 857 |
| 596339 | 807 | 826 | AAACAGACATTTTAACTGAG | 4 | 9957 | 9976 | 858 |
| 596340 | 809 | 828 | TGAAACAGACATTTTAACTG | 0 | 9959 | 9978 | 859 |
| 150478 | 811 | 830 | ATTGAAACAGACATTTTAAC | 0 | 9961 | 9980 | 96 |
| 596341 | 836 | 855 | ATTTAAGTCTGGCAAAATAC | 16 | 9986 | 10005 | 860 |
| 596342 | 840 | 859 | TGTGATTTAAGTCTGGCAAA | 34 | 9990 | 10009 | 861 |
| 489539 | 842 | 861 | TCTGTGATTTAAGTCTGGCA | 44 | 9992 | 10011 | 862 |
| 596343 | 844 | 863 | CATCTGTGATTTAAGTCTGG | 29 | 9994 | 10013 | 863 |
| 596344 | 846 | 865 | CCCATCTGTGATTTAAGTCT | 41 | 9996 | 10015 | 864 |
| 596345 | 848 | 867 | TACCCATCTGTGATTTAAGT | 50 | 9998 | 10017 | 865 |
| 596346 | 850 | 869 | AATACCCATCTGTGATTTAA | 0 | 10000 | 10019 | 866 |
| 489540 | 852 | 871 | TTAATACCCATCTGTGATTT | 11 | 10002 | 10021 | 867 |
| 150484 | 871 | 890 | CAAAGAAATTCTGACAAGTT | 7 | 10021 | 10040 | 97 |
| 596347 | 873 | 892 | GACAAAGAAATTCTGACAAG | 8 | 10023 | 10042 | 868 |
| 596348 | 877 | 896 | GAATGACAAAGAAATTCTGA | 0 | 10027 | 10046 | 869 |
| 596349 | 883 | 902 | AGGCTTGAATGACAAAGAAA | 27 | 10033 | 10052 | 870 |
| 150486 | 885 | 904 | ACAGGCTTGAATGACAAAGA | 19 | 10035 | 10054 | 98 |
| 596350 | 910 | 929 | TAAGTGCCATACAGGGTTTT | 13 | 10060 | 10079 | 871 |
| 596351 | 914 | 933 | ATAATAAGTGCCATACAGGG | 18 | 10064 | 10083 | 872 |
| 150494 | 916 | 935 | TCATAATAAGTGCCATACAG | 0 | 10066 | 10085 | 99 |
| 596352 | 918 | 937 | CCTCATAATAAGTGCCATAC | 23 | 10068 | 10087 | 873 |
| 596353 | 920 | 939 | AGCCTCATAATAAGTGCCAT | 6 | 10070 | 10089 | 874 |
| 596354 | 922 | 941 | ATAGCCTCATAATAAGTGCC | 19 | 10072 | 10091 | 875 |
| 596355 | 928 | 947 | CTTTTAATAGCCTCATAATA | 0 | 10078 | 10097 | 876 |
| 596356 | 930 | 949 | TTCTTTTAATAGCCTCATAA | 5 | 10080 | 10099 | 877 |
| 596357 | 932 | 951 | GATTCTTTTAATAGCCTCAT | 4 | 10082 | 10101 | 878 |
| 596358 | 934 | 953 | TGGATTCTTTTAATAGCCTC | 13 | 10084 | 10103 | 879 |
| 596359 | 936 | 955 | TTTGGATTCTTTTAATAGCC | 14 | 10086 | 10105 | 880 |
| 596360 | 938 | 957 | AATTTGGATTCTTTTAATAG | 14 | 10088 | 10107 | 881 |
| 596361 | 940 | 959 | TGAATTTGGATTCTTTTAAT | 0 | 10090 | 10109 | 882 |
| 596362 | 946 | 965 | TTAGTTTGAATTTGGATTCT | 0 | 10096 | 10115 | 883 |
| 596363 | 948 | 967 | TTTTAGTTTGAATTTGGATT | 0 | n/a | n/a | 884 |
| 596364 | 950 | 969 | TTTTTTAGTTTGAATTTGGA | 0 | n/a | n/a | 885 |

**Table 12**

| Percent inhibition of SOD-1 mRNA by 5-10-5 MOE gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | inhibition with RTS3898 | inhibitio n with HTS90 | SEQ ID NO: 2 Start Site | SEQ ID NO: 2 Stop Site | SEQ ID NO |
| 333611 | 167 | 186 | CCGTCGCCCTTCAGCACGCA | 66 | n.d. | 973 | 992 | 21 |
| 596230 | 246 | 265 | CAGTCCTTTAATGCTTCCCC | 51 | 40 | 5000 | 5019 | 886 |
| 596231 | 248 | 267 | GTCAGTCCTTTAATGCTTCC | 34 | 34 | 5002 | 5021 | 887 |
| 596232 | 250 | 269 | CAGTCAGTCCTTTAATGCTT | 35 | 29 | 5004 | 5023 | 888 |
| 596233 | 252 | 271 | TTCAGTCAGTCCTTTAATGC | 24 | 21 | 5006 | 5025 | 889 |
| 596234 | 256 | 275 | GGCCTTCAGTCAGTCCTTTA | 41 | 39 | 5010 | 5029 | 890 |
| 150450 | 258 | 277 | CAGGCCTTCAGTCAGTCCTT | 56 | 51 | 5012 | 5031 | 100 |
| 596235 | 260 | 279 | TGCAGGCCTTCAGTCAGTCC | 42 | 46 | 5014 | 5033 | 891 |
| 596236 | 262 | 281 | CATGCAGGCCTTCAGTCAGT | 37 | 33 | 5016 | 5035 | 892 |
| 596237 | 278 | 297 | TCATGAACATGGAATCCATG | 24 | 19 | 5032 | 5051 | 893 |
| 596238 | 280 | 299 | ACTCATGAACATGGAATCCA | 27 | 20 | 5034 | 5053 | 894 |
| 596239 | 295 | 314 | CTGTATTATCTCCAAACTCA | 32 | 28 | 5049 | 5068 | 895 |
| 596240 | 309 | 328 | ACTGGTACAGCCTGCTGTAT | 22 | 28 | n/a | n/a | 896 |
| 596241 | 311 | 330 | GCACTGGTACAGCCTGCTGT | 31 | 24 | n/a | n/a | 897 |
| 596242 | 313 | 332 | CTGCACTGGTACAGCCTGCT | 38 | 29 | n/a | n/a | 898 |
| 596243 | 315 | 334 | ACCTGCACTGGTACAGCCTG | 46 | 48 | n/a | n/a | 899 |
| 596244 | 341 | 360 | TTTCTGGATAGAGGATTAAA | 6 | 14 | 7657 | 7676 | 900 |
| 596245 | 343 | 362 | GTTTTCTGGATAGAGGATTA | 28 | 39 | 7659 | 7678 | 901 |
| 596246 | 347 | 366 | CCGTGTTTTCTGGATAGAGG | 44 | 37 | 7663 | 7682 | 902 |
| 596247 | 349 | 368 | CACCGTGTTTTCTGGATAGA | 24 | 11 | 7665 | 7684 | 903 |
| 596248 | 351 | 370 | CCCACCGTGTTTTCTGGATA | 46 | 40 | 7667 | 7686 | 904 |
| 596249 | 353 | 372 | GGCCCACCGTGTTTTCTGGA | 46 | 41 | 7669 | 7688 | 905 |
| 596250 | 355 | 374 | TTGGCCCACCGTGTTTTCTG | 35 | 26 | 7671 | 7690 | 906 |
| 596251 | 357 | 376 | CTTTGGCCCACCGTGTTTTC | 31 | 15 | 7673 | 7692 | 907 |
| 596252 | 359 | 378 | TCCTTTGGCCCACCGTGTTT | 30 | 23 | 7675 | 7694 | 908 |
| 596253 | 383 | 402 | AAGTCTCCAACATGCCTCTC | 20 | 6 | n/a | n/a | 909 |
| 596254 | 387 | 406 | GCCCAAGTCTCCAACATGCC | 61 | 53 | 8442 | 8461 | 910 |
| 596255 | 389 | 408 | TTGCCCAAGTCTCCAACATG | 41 | 33 | 8444 | 8463 | 911 |
| 596256 | 391 | 410 | CATTGCCCAAGTCTCCAACA | 39 | 25 | 8446 | 8465 | 912 |
| 150455 | 393 | 412 | CACATTGCCCAAGTCTCCAA | 36 | 19 | 8448 | 8467 | 101 |
| 596257 | 397 | 416 | CAGTCACATTGCCCAAGTCT | 40 | 27 | 8452 | 8471 | 913 |
| 596258 | 401 | 420 | TCAGCAGTCACATTGCCCAA | 52 | 42 | 8456 | 8475 | 914 |
| 596259 | 403 | 422 | TGTCAGCAGTCACATTGCCC | 55 | 49 | 8458 | 8477 | 915 |
| 596260 | 405 | 424 | TTTGTCAGCAGTCACATTGC | 26 | 16 | 8460 | 8479 | 916 |
| 596261 | 407 | 426 | TCTTTGTCAGCAGTCACATT | 20 | 11 | 8462 | 8481 | 917 |
| 596262 | 409 | 428 | CATCTTTGTCAGCAGTCACA | 34 | 13 | 8464 | 8483 | 918 |
| 596263 | 411 | 430 | ACCATCTTTGTCAGCAGTCA | 41 | 30 | 8466 | 8485 | 919 |
| 596264 | 415 | 434 | CCACACCATCTTTGTCAGCA | 39 | 20 | 8470 | 8489 | 920 |
| 596265 | 417 | 436 | GGCCACACCATCTTTGTCAG | 23 | 5 | 8472 | 8491 | 921 |
| 150456 | 419 | 438 | TCGGCCACACCATCTTTGTC | 32 | 28 | 8474 | 8493 | 102 |
| 150457 | 421 | 440 | CATCGGCCACACCATCTTTG | 34 | 38 | 8476 | 8495 | 103 |
| 596266 | 423 | 442 | CACATCGGCCACACCATCTT | 27 | 13 | 8478 | 8497 | 922 |
| 596267 | 425 | 444 | GACACATCGGCCACACCATC | 45 | 30 | 8480 | 8499 | 923 |
| 150459 | 427 | 446 | TAGACACATCGGCCACACCA | 46 | 36 | 8482 | 8501 | 104 |
| 596268 | 429 | 448 | AATAGACACATCGGCCACAC | 30 | 25 | 8484 | 8503 | 924 |
| 596269 | 431 | 450 | TCAATAGACACATCGGCCAC | 35 | 0 | 8486 | 8505 | 925 |
| 596270 | 433 | 452 | CTTCAATAGACACATCGGCC | 39 | 16 | 8488 | 8507 | 926 |
| 596271 | 435 | 454 | ATCTTCAATAGACACATCGG | 16 | 0 | 8490 | 8509 | 927 |
| 596272 | 437 | 456 | GAATCTTCAATAGACACATC | 22 | 11 | 8492 | 8511 | 928 |
| 596273 | 439 | 458 | CAGAATCTTCAATAGACACA | 17 | 0 | 8494 | 8513 | 929 |
| 596274 | 441 | 460 | CACAGAATCTTCAATAGACA | 10 | 14 | 8496 | 8515 | 930 |
| 596275 | 443 | 462 | ATCACAGAATCTTCAATAGA | 11 | 10 | 8498 | 8517 | 931 |
| 596276 | 445 | 464 | AGATCACAGAATCTTCAATA | 14 | 29 | 8500 | 8519 | 932 |
| 596277 | 447 | 466 | TGAGATCACAGAATCTTCAA | n.d. | 30 | 8502 | 8521 | 933 |
| 596278 | 449 | 468 | AGTGAGATCACAGAATCTTC | n.d. | 30 | 8504 | 8523 | 934 |
| 596279 | 453 | 472 | TGAGAGTGAGATCACAGAAT | n.d. | 18 | 8508 | 8527 | 935 |
| 333618 | 457 | 476 | CTCCTGAGAGTGAGATCACA | n.d. | 27 | 8512 | 8531 | 105 |
| 596281 | 459 | 478 | GTCTCCTGAGAGTGAGATCA | n.d. | 23 | 8514 | 8533 | 936 |
| 596282 | 461 | 480 | TGGTCTCCTGAGAGTGAGAT | n.d. | 24 | 8516 | 8535 | 937 |
| 596283 | 463 | 482 | AATGGTCTCCTGAGAGTGAG | n.d. | 22 | 8518 | 8537 | 938 |
| 596284 | 465 | 484 | GCAATGGTCTCCTGAGAGTG | n.d. | 57 | 8520 | 8539 | 939 |
| 596285 | 467 | 486 | ATGCAATGGTCTCCTGAGAG | n.d. | 0 | 8522 | 8541 | 940 |
| 596286 | 469 | 488 | TGATGCAATGGTCTCCTGAG | n.d. | 1 | 8524 | 8543 | 941 |
| 596287 | 471 | 490 | AATGATGCAATGGTCTCCTG | n.d. | 0 | 8526 | 8545 | 942 |
| 596288 | 473 | 492 | CCAATGATGCAATGGTCTCC | n.d. | 8 | 8528 | 8547 | 943 |
| 596289 | 475 | 494 | GGCCAATGATGCAATGGTCT | n.d. | 9 | 8530 | 8549 | 944 |
| 596290 | 477 | 496 | GCGGCCAATGATGCAATGGT | n.d. | 13 | 8532 | 8551 | 945 |
| 596291 | 479 | 498 | GTGCGGCCAATGATGCAATG | n.d. | 12 | 8534 | 8553 | 946 |
| 596292 | 481 | 500 | GTGTGCGGCCAATGATGCAA | n.d. | 15 | 8536 | 8555 | 947 |
| 596293 | 483 | 502 | CAGTGTGCGGCCAATGATGC | n.d. | 0 | 8538 | 8557 | 948 |
| 596294 | 485 | 504 | ACCAGTGTGCGGCCAATGAT | n.d. | 0 | 8540 | 8559 | 949 |
| 596295 | 487 | 506 | CCACCAGTGTGCGGCCAATG | n.d. | 22 | 8542 | 8561 | 950 |
| 596296 | 489 | 508 | GACCACCAGTGTGCGGCCAA | n.d. | 16 | n/a | n/a | 951 |
| 596297 | 491 | 510 | TGGACCACCAGTGTGCGGCC | n.d. | 28 | n/a | n/a | 952 |
| 150461 | 493 | 512 | CATGGACCACCAGTGTGCGG | n.d. | 25 | n/a | n/a | 106 |
| 596298 | 495 | 514 | TTCATGGACCACCAGTGTGC | n.d. | 21 | n/a | n/a | 953 |
| 596299 | 497 | 516 | TTTTCATGGACCACCAGTGT | n.d. | 17 | n/a | n/a | 954 |
| 596300 | 499 | 518 | CTTTTTCATGGACCACCAGT | n.d. | 9 | n/a | n/a | 955 |

### Example 3: Inhibition of human SOD-1 in HepG2 cells by deoxy, MOE and cEt gapmers

Modified oligonucleotides were designed targeting a superoxide dismutase 1, soluble (SOD-1) nucleic acid and were tested for their effects on SOD-1 mRNA in vitro. ISIS 333611, which was previously described in WO 2005/040180, was included as a benchmark. ISIS 590067, ISIS 590074, ISIS 590082, ISIS 590130, ISIS 590138, and ISIS 590146, which are 5-10-5 MOE gapmers as described above in Example 1, were also included in this assay. ISIS 590512, which has a similar sequence as ISIS 333611 but with deoxy, MOE, and cEt sugar modifications, was also included in this study.

The modified oligonucleotides were tested in a series of experiments that had similar culture conditions. The results for each experiment are presented in separate tables shown below. Cultured HepG2 cells at a density of 20,000 cells per well were transfected using electroporation with 3,000 nM modified oligonucleotide. After a treatment period of approximately 24 hours, RNA was isolated from the cells and SOD-1 mRNA levels were measured by quantitative real-time PCR.

Human primer probe set RTS3898 was used to measure mRNA levels. SOD-1 mRNA levels were adjusted according to total RNA content, as measured by RIBOGREEN^{®}. Results are presented as percent inhibition of SOD-1, relative to untreated control cells. 'n.d.' indicates that inhibition levels were not measured.

The newly designed modified oligonucleotides in the Tables below were designed as deoxy, MOE, and cEt gapmers. The gapmers are 17 nucleosides in length wherein each nucleoside has a MOE sugar modification, a cEt sugar modification, or a deoxy moiety. The sugar chemistry of each oligonucleotide is denoted as in the Chemistry column, where 'k' indicates a cEt modified sugar; 'd' indicates a 2'-deoxyribose; and 'e' indicates a 2'-MOE modified sugar. The internucleoside linkages throughout each gapmer are phosphorothioate linkages. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000). 'n/a' indicates that the modified oligonucleotide does not target that particular gene sequence with 100% complementarity.

**Table 13**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | inhibition | SEQ ID NO: 2 Start Site | SEQ ID NO: 2 Stop Site | SEQ ID NO |
| 590434 | 1 | 17 | | eeekkdddddddkkeee | 17 | 807 | 823 | 956 |
| 590435 | 2 | 18 | | eeekkdddddddkkeee | 15 | 808 | 824 | 957 |
| 590436 | 3 | 19 | | eeekkdddddddkkeee | 22 | 809 | 825 | 958 |
| 590437 | 4 | 20 | | eeekkdddddddkkeee | 14 | 810 | 826 | 959 |
| 590438 | 35 | 51 | | eeekkdddddddkkeee | 12 | 841 | 857 | 960 |
| 590439 | 36 | 52 | | eeekkdddddddkkeee | 12 | 842 | 858 | 961 |
| 590440 | 37 | 53 | | eeekkdddddddkkeee | 11 | 843 | 859 | 962 |
| 590441 | 38 | 54 | | eeekkdddddddkkeee | 5 | 844 | 860 | 963 |
| 590442 | 76 | 92 | | eeekkdddddddkkeee | 0 | 882 | 898 | 964 |
| 590443 | 77 | 93 | | eeekkdddddddkkeee | 25 | 883 | 899 | 965 |
| 590444 | 167 | 183 | | eeekkdddddddkkeee | 31 | 973 | 989 | 966 |
| 590445 | 168 | 184 | | eeekkdddddddkkeee | 28 | 974 | 990 | 967 |
| 590512 | 169 | 185 | | eeekkdddddddkkeee | 8 | 975 | 991 | 968 |
| 590446 | 170 | 186 | | eeekkdddddddkkeee | 27 | 976 | 992 | 969 |
| 590447 | 171 | 187 | | eeekkdddddddkkeee | 33 | 977 | 993 | 970 |
| 590448 | 202 | 218 | | eeekkdddddddkkeee | 34 | 1008 | 1024 | 971 |
| 590449 | 203 | 219 | | eeekkdddddddkkeee | 18 | 1009 | 1025 | 972 |
| 590450 | 204 | 220 | | eeekkdddddddkkeee | 13 | 1010 | 1026 | 973 |
| 590451 | 205 | 221 | | eeekkdddddddkkeee | 16 | 1011 | 1027 | 974 |
| 590452 | 206 | 222 | | eeekkdddddddkkeee | 14 | n/a | n/a | 975 |
| 590453 | 207 | 223 | | eeekkdddddddkkeee | 13 | n/a | n/a | 976 |
| 590454 | 208 | 224 | | eeekkdddddddkkeee | 6 | n/a | n/a | 977 |
| 590455 | 209 | 225 | | eeekkdddddddkkeee | 0 | n/a | n/a | 978 |
| 590456 | 210 | 226 | | eeekkdddddddkkeee | 0 | n/a | n/a | 979 |
| 590457 | 211 | 227 | | eeekkdddddddkkeee | n.d. | n/a | n/a | 980 |
| 590458 | 212 | 228 | | eeekkdddddddkkeee | n.d. | n/a | n/a | 981 |
| 590459 | 213 | 229 | | eeekkdddddddkkeee | n.d. | n/a | n/a | 982 |
| 590461 | 214 | 230 | | eeekkdddddddkkeee | n.d. | n/a | n/a | 983 |
| 590462 | 215 | 231 | | eeekkdddddddkkeee | n.d. | n/a | n/a | 984 |
| 590463 | 216 | 232 | | eeekkdddddddkkeee | n.d. | n/a | n/a | 985 |
| 590464 | 217 | 233 | | eeekkdddddddkkeee | n.d. | n/a | n/a | 986 |
| 590465 | 218 | 234 | | eeekkdddddddkkeee | n.d. | 4972 | 4988 | 987 |
| 590466 | 219 | 235 | | eeekkdddddddkkeee | 5 | 4973 | 4989 | 988 |
| 590467 | 220 | 236 | | eeekkdddddddkkeee | 11 | 4974 | 4990 | 989 |
| 590468 | 221 | 237 | | eeekkdddddddkkeee | 14 | 4975 | 4991 | 990 |
| 590469 | 222 | 238 | | eeekkdddddddkkeee | 12 | 4976 | 4992 | 991 |
| 590470 | 223 | 239 | | eeekkdddddddkkeee | 15 | 4977 | 4993 | 992 |
| 590471 | 224 | 240 | | eeekkdddddddkkeee | 14 | 4978 | 4994 | 993 |
| 590472 | 225 | 241 | | eeekkdddddddkkeee | 11 | 4979 | 4995 | 994 |
| 590473 | 226 | 242 | | eeekkdddddddkkeee | 8 | 4980 | 4996 | 995 |
| 590474 | 227 | 243 | | eeekkdddddddkkeee | 44 | 4981 | 4997 | 996 |
| 590475 | 228 | 244 | | eeekkdddddddkkeee | 53 | 4982 | 4998 | 997 |
| 590476 | 229 | 245 | | eeekkdddddddkkeee | 20 | 4983 | 4999 | 998 |
| 590477 | 230 | 246 | | eeekkdddddddkkeee | 12 | 4984 | 5000 | 999 |
| 590478 | 231 | 247 | | eeekkdddddddkkeee | 36 | 4985 | 5001 | 1000 |
| 590479 | 232 | 248 | | eeekkdddddddkkeee | 18 | 4986 | 5002 | 1001 |
| 590480 | 233 | 249 | | eeekkdddddddkkeee | 14 | 4987 | 5003 | 1002 |
| 590481 | 235 | 251 | | eeekkdddddddkkeee | 8 | 4989 | 5005 | 1003 |
| 590482 | 236 | 252 | | eeekkdddddddkkeee | 29 | 4990 | 5006 | 1004 |
| 590483 | 237 | 253 | | eeekkdddddddkkeee | 36 | 4991 | 5007 | 1005 |
| 590484 | 238 | 254 | | eeekkdddddddkkeee | 43 | 4992 | 5008 | 1006 |
| 590485 | 239 | 255 | | eeekkdddddddkkeee | 41 | 4993 | 5009 | 1007 |
| 590486 | 240 | 256 | | eeekkdddddddkkeee | 35 | 4994 | 5010 | 1008 |
| 590487 | 241 | 257 | | eeekkdddddddkkeee | 52 | 4995 | 5011 | 1009 |
| 590488 | 264 | 280 | | eeekkdddddddkkeee | 37 | 5018 | 5034 | 1010 |
| 590489 | 265 | 281 | | eeekkdddddddkkeee | 41 | 5019 | 5035 | 1011 |
| 590490 | 266 | 282 | | eeekkdddddddkkeee | 21 | 5020 | 5036 | 1012 |
| 590491 | 267 | 283 | | eeekkdddddddkkeee | 18 | 5021 | 5037 | 1013 |
| 590492 | 268 | 284 | | eeekkdddddddkkeee | 27 | 5022 | 5038 | 1014 |
| 590493 | 269 | 285 | | eeekkdddddddkkeee | 13 | 5023 | 5039 | 1015 |
| 590494 | 270 | 286 | | eeekkdddddddkkeee | 9 | 5024 | 5040 | 1016 |
| 590495 | 271 | 287 | | eeekkdddddddkkeee | 7 | 5025 | 5041 | 1017 |
| 590496 | 272 | 288 | | eeekkdddddddkkeee | 12 | 5026 | 5042 | 1018 |
| 590497 | 273 | 289 | | eeekkdddddddkkeee | 9 | 5027 | 5043 | 1019 |
| 590498 | 274 | 290 | | eeekkdddddddkkeee | 14 | 5028 | 5044 | 1020 |
| 590499 | 275 | 291 | | eeekkdddddddkkeee | 0 | 5029 | 5045 | 1021 |
| 590500 | 276 | 292 | | eeekkdddddddkkeee | 10 | 5030 | 5046 | 1022 |
| 590501 | 277 | 293 | | eeekkdddddddkkeee | 9 | 5031 | 5047 | 1023 |
| 590502 | 278 | 294 | | eeekkdddddddkkeee | 2 | 5032 | 5048 | 1024 |
| 590503 | 279 | 295 | | eeekkdddddddkkeee | 8 | 5033 | 5049 | 1025 |
| 590504 | 316 | 332 | | eeekkdddddddkkeee | 3 | 7632 | 7648 | 1026 |
| 590505 | 317 | 333 | | eeekkdddddddkkeee | 17 | 7633 | 7649 | 1027 |
| 590506 | 318 | 334 | | eeekkdddddddkkeee | 12 | 7634 | 7650 | 1028 |
| 590507 | 319 | 335 | | eeekkdddddddkkeee | 7 | 7635 | 7651 | 1029 |
| 590508 | 320 | 336 | | eeekkdddddddkkeee | 7 | 7636 | 7652 | 1030 |
| 590509 | 321 | 337 | | eeekkdddddddkkeee | 4 | 7637 | 7653 | 1031 |
| 590510 | 322 | 338 | | eeekkdddddddkkeee | 17 | 7638 | 7654 | 1032 |
| 590511 | 323 | 339 | | eeekkdddddddkkeee | 8 | 7639 | 7655 | 1033 |

**Table 14**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 590512 | 169 | 185 | | eeekkdddddddkkeee | 45 | 975 | 991 | 968 |
| 590513 | 324 | 340 | | eeekkdddddddkkeee | 21 | 7640 | 7656 | 1034 |
| 590514 | 325 | 341 | | eeekkdddddddkkeee | 21 | 7641 | 7657 | 1035 |
| 590515 | 326 | 342 | | eeekkdddddddkkeee | 16 | 7642 | 7658 | 1036 |
| 590516 | 327 | 343 | | eeekkdddddddkkeee | 20 | 7643 | 7659 | 1037 |
| 590517 | 328 | 344 | | eeekkdddddddkkeee | 19 | 7644 | 7660 | 1038 |
| 590518 | 329 | 345 | | eeekkdddddddkkeee | 14 | 7645 | 7661 | 1039 |
| 590519 | 330 | 346 | | eeekkdddddddkkeee | 51 | 7646 | 7662 | 1040 |
| 590520 | 331 | 347 | | eeekkdddddddkkeee | 8 | 7647 | 7663 | 1041 |
| 590521 | 332 | 348 | | eeekkdddddddkkeee | 30 | 7648 | 7664 | 1042 |
| 590522 | 333 | 349 | | eeekkdddddddkkeee | 23 | 7649 | 7665 | 1043 |
| 590523 | 334 | 350 | | eeekkdddddddkkeee | 40 | 7650 | 7666 | 1044 |
| 590524 | 335 | 351 | | eeekkdddddddkkeee | 16 | 7651 | 7667 | 1045 |
| 590525 | 336 | 352 | | eeekkdddddddkkeee | 21 | 7652 | 7668 | 1046 |
| 590526 | 337 | 353 | | eeekkdddddddkkeee | 9 | 7653 | 7669 | 1047 |
| 590527 | 338 | 354 | | eeekkdddddddkkeee | 8 | 7654 | 7670 | 1048 |
| 590528 | 339 | 355 | | eeekkdddddddkkeee | 14 | 7655 | 7671 | 1049 |
| 590530 | 340 | 356 | | eeekkdddddddkkeee | 23 | 7656 | 7672 | 1050 |
| 590531 | 341 | 357 | | eeekkdddddddkkeee | 26 | 7657 | 7673 | 1051 |
| 590532 | 342 | 358 | | eeekkdddddddkkeee | 25 | 7658 | 7674 | 1052 |
| 590533 | 360 | 376 | | eeekkdddddddkkeee | 41 | 7676 | 7692 | 1053 |
| 590534 | 361 | 377 | | eeekkdddddddkkeee | 46 | 7677 | 7693 | 1054 |
| 590535 | 362 | 378 | | eeekkdddddddkkeee | 39 | 7678 | 7694 | 1055 |
| 590536 | 363 | 379 | | eeekkdddddddkkeee | n.d. | 7679 | 7695 | 1056 |
| 590537 | 364 | 380 | | eeekkdddddddkkeee | n.d. | 7680 | 7696 | 1057 |
| 590538 | 365 | 381 | | eeekkdddddddkkeee | n.d. | 7681 | 7697 | 1058 |
| 590539 | 366 | 382 | | eeekkdddddddkkeee | n.d. | 7682 | 7698 | 1059 |
| 590540 | 367 | 383 | | eeekkdddddddkkeee | n.d. | 7683 | 7699 | 1060 |
| 590541 | 368 | 384 | | eeekkdddddddkkeee | n.d. | 7684 | 7700 | 1061 |
| 590542 | 369 | 385 | | eeekkdddddddkkeee | n.d. | 7685 | 7701 | 1062 |
| 590543 | 370 | 386 | | eeekkdddddddkkeee | n.d. | 7686 | 7702 | 1063 |
| 590544 | 371 | 387 | | eeekkdddddddkkeee | 2 | 7687 | 7703 | 1064 |
| 590545 | 374 | 390 | | eeekkdddddddkkeee | 6 | n/a | n/a | 1065 |
| 590546 | 375 | 391 | | eeekkdddddddkkeee | 0 | n/a | n/a | 1066 |
| 590547 | 376 | 392 | | eeekkdddddddkkeee | 14 | n/a | n/a | 1067 |
| 590548 | 377 | 393 | | eeekkdddddddkkeee | 0 | n/a | n/a | 1068 |
| 590549 | 378 | 394 | | eeekkdddddddkkeee | 13 | n/a | n/a | 1069 |
| 590550 | 379 | 395 | | eeekkdddddddkkeee | 3 | n/a | n/a | 1070 |
| 590551 | 380 | 396 | | eeekkdddddddkkeee | 0 | n/a | n/a | 1071 |
| 590552 | 381 | 397 | | eeekkdddddddkkeee | 0 | n/a | n/a | 1072 |
| 590553 | 382 | 398 | | eeekkdddddddkkeee | 5 | n/a | n/a | 1073 |
| 590554 | 383 | 399 | | eeekkdddddddkkeee | 10 | n/a | n/a | 1074 |
| 590555 | 384 | 400 | | eeekkdddddddkkeee | 8 | n/a | n/a | 1075 |
| 590556 | 402 | 418 | | eeekkdddddddkkeee | 18 | 8457 | 8473 | 1076 |
| 590557 | 403 | 419 | | eeekkdddddddkkeee | 7 | 8458 | 8474 | 1077 |
| 590558 | 429 | 445 | | eeekkdddddddkkeee | 21 | 8484 | 8500 | 1078 |
| 590559 | 436 | 452 | | eeekkdddddddkkeee | 9 | 8491 | 8507 | 1079 |
| 590560 | 449 | 465 | | eeekkdddddddkkeee | 13 | 8504 | 8520 | 1080 |
| 590561 | 501 | 517 | | eeekkdddddddkkeee | 76 | n/a | n/a | 1081 |
| 590562 | 502 | 518 | | eeekkdddddddkkeee | 87 | n/a | n/a | 1082 |
| 590563 | 503 | 519 | | eeekkdddddddkkeee | 71 | n/a | n/a | 1083 |
| 590564 | 504 | 520 | | eeekkdddddddkkeee | 51 | n/a | n/a | 1084 |
| 590565 | 505 | 521 | | eeekkdddddddkkeee | 65 | 9655 | 9671 | 1085 |
| 590566 | 506 | 522 | | eeekkdddddddkkeee | 55 | 9656 | 9672 | 1086 |
| 590567 | 507 | 523 | | eeekkdddddddkkeee | 42 | 9657 | 9673 | 1087 |
| 590568 | 508 | 524 | | eeekkdddddddkkeee | 70 | 9658 | 9674 | 1088 |
| 590569 | 509 | 525 | | eeekkdddddddkkeee | 71 | 9659 | 9675 | 1089 |
| 590570 | 510 | 526 | | eeekkdddddddkkeee | 74 | 9660 | 9676 | 1090 |
| 590571 | 511 | 527 | | eeekkdddddddkkeee | 76 | 9661 | 9677 | 1091 |
| 590572 | 512 | 528 | | eeekkdddddddkkeee | 83 | 9662 | 9678 | 1092 |
| 590573 | 513 | 529 | | eeekkdddddddkkeee | 42 | 9663 | 9679 | 1093 |
| 590574 | 514 | 530 | | eeekkdddddddkkeee | 50 | 9664 | 9680 | 1094 |
| 590575 | 515 | 531 | | eeekkdddddddkkeee | 72 | 9665 | 9681 | 1095 |
| 590576 | 516 | 532 | | eeekkdddddddkkeee | 93 | 9666 | 9682 | 1096 |
| 590577 | 517 | 533 | | eeekkdddddddkkeee | 90 | 9667 | 9683 | 1097 |
| 590578 | 518 | 534 | | eeekkdddddddkkeee | 92 | 9668 | 9684 | 1098 |
| 590579 | 524 | 540 | | eeekkdddddddkkeee | 91 | 9674 | 9690 | 1099 |
| 590580 | 525 | 541 | | eeekkdddddddkkeee | 88 | 9675 | 9691 | 1100 |
| 590581 | 526 | 542 | | eeekkdddddddkkeee | 87 | 9676 | 9692 | 1101 |
| 590582 | 527 | 543 | | eeekkdddddddkkeee | 78 | 9677 | 9693 | 1102 |
| 590583 | 528 | 544 | | eeekkdddddddkkeee | 63 | 9678 | 9694 | 1103 |
| 590584 | 529 | 545 | | eeekkdddddddkkeee | 73 | 9679 | 9695 | 1104 |
| 590585 | 530 | 546 | | eeekkdddddddkkeee | 57 | 9680 | 9696 | 1105 |
| 590586 | 531 | 547 | | eeekkdddddddkkeee | 33 | 9681 | 9697 | 1106 |
| 590587 | 533 | 549 | | eeekkdddddddkkeee | 31 | 9683 | 9699 | 1107 |
| 590588 | 536 | 552 | | eeekkdddddddkkeee | 11 | 9686 | 9702 | 1108 |
| 590589 | 537 | 553 | | eeekkdddddddkkeee | 15 | 9687 | 9703 | 1109 |
| 590590 | 538 | 554 | | eeekkdddddddkkeee | 18 | 9688 | 9704 | 1110 |

**Table 15**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 590512 | 169 | 185 | | eeekkdddddddkkeee | 21 | 975 | 991 | 968 |
| 590591 | 582 | 598 | | eeekkdddddddkkeee | 21 | 9732 | 9748 | 1111 |
| 590592 | 583 | 599 | | eeekkdddddddkkeee | 33 | 9733 | 9749 | 1112 |
| 590593 | 584 | 600 | | eeekkdddddddkkeee | 29 | 9734 | 9750 | 1113 |
| 590594 | 585 | 601 | | eeekkdddddddkkeee | 29 | 9735 | 9751 | 1114 |
| 590595 | 588 | 604 | | eeekkdddddddkkeee | 3 | 9738 | 9754 | 1115 |
| 590596 | 589 | 605 | | eeekkdddddddkkeee | 12 | 9739 | 9755 | 1116 |
| 590597 | 590 | 606 | | eeekkdddddddkkeee | 19 | 9740 | 9756 | 1117 |
| 590598 | 591 | 607 | | eeekkdddddddkkeee | 9 | 9741 | 9757 | 1118 |
| 590599 | 592 | 608 | | eeekkdddddddkkeee | 18 | 9742 | 9758 | 1119 |
| 590600 | 593 | 609 | | eeekkdddddddkkeee | 20 | 9743 | 9759 | 1120 |
| 590601 | 594 | 610 | | eeekkdddddddkkeee | 26 | 9744 | 9760 | 1121 |
| 590602 | 595 | 611 | | eeekkdddddddkkeee | 19 | 9745 | 9761 | 1122 |
| 590603 | 596 | 612 | | eeekkdddddddkkeee | 3 | 9746 | 9762 | 1123 |
| 590604 | 597 | 613 | | eeekkdddddddkkeee | 15 | 9747 | 9763 | 1124 |
| 590605 | 598 | 614 | | eeekkdddddddkkeee | 20 | 9748 | 9764 | 1125 |
| 590606 | 599 | 615 | | eeekkdddddddkkeee | 18 | 9749 | 9765 | 1126 |
| 590607 | 600 | 616 | | eeekkdddddddkkeee | 21 | 9750 | 9766 | 1127 |
| 590608 | 601 | 617 | | eeekkdddddddkkeee | 28 | 9751 | 9767 | 1128 |
| 590609 | 602 | 618 | | eeekkdddddddkkeee | 30 | 9752 | 9768 | 1129 |
| 590610 | 603 | 619 | | eeekkdddddddkkeee | 14 | 9753 | 9769 | 1130 |
| 590611 | 604 | 620 | | eeekkdddddddkkeee | 15 | 9754 | 9770 | 1131 |
| 590612 | 607 | 623 | | eeekkdddddddkkeee | 2 | 9757 | 9773 | 1132 |
| 590613 | 608 | 624 | | eeekkdddddddkkeee | n.d. | 9758 | 9774 | 1133 |
| 590614 | 609 | 625 | | eeekkdddddddkkeee | n.d. | 9759 | 9775 | 1134 |
| 590615 | 610 | 626 | | eeekkdddddddkkeee | n.d. | 9760 | 9776 | 1135 |
| 590616 | 611 | 627 | | eeekkdddddddkkeee | n.d. | 9761 | 9777 | 1136 |
| 590617 | 612 | 628 | | eeekkdddddddkkeee | n.d. | 9762 | 9778 | 1137 |
| 590618 | 613 | 629 | | eeekkdddddddkkeee | n.d. | 9763 | 9779 | 1138 |
| 590619 | 614 | 630 | | eeekkdddddddkkeee | n.d. | 9764 | 9780 | 1139 |
| 590620 | 615 | 631 | | eeekkdddddddkkeee | n.d. | 9765 | 9781 | 1140 |
| 590621 | 616 | 632 | | eeekkdddddddkkeee | 7 | 9766 | 9782 | 1141 |
| 590622 | 617 | 633 | | eeekkdddddddkkeee | 19 | 9767 | 9783 | 1142 |
| 590623 | 618 | 634 | | eeekkdddddddkkeee | 39 | 9768 | 9784 | 1143 |
| 590624 | 619 | 635 | | eeekkdddddddkkeee | 53 | 9769 | 9785 | 1144 |
| 590625 | 620 | 636 | | eeekkdddddddkkeee | 57 | 9770 | 9786 | 1145 |
| 590626 | 621 | 637 | | eeekkdddddddkkeee | 76 | 9771 | 9787 | 1146 |
| 590627 | 622 | 638 | | eeekkdddddddkkeee | 58 | 9772 | 9788 | 1147 |
| 590628 | 623 | 639 | | eeekkdddddddkkeee | 43 | 9773 | 9789 | 1148 |
| 590629 | 624 | 640 | | eeekkdddddddkkeee | 24 | 9774 | 9790 | 1149 |
| 590630 | 625 | 641 | | eeekkdddddddkkeee | 24 | 9775 | 9791 | 1150 |
| 590631 | 643 | 659 | | eeekkdddddddkkeee | 11 | 9793 | 9809 | 1151 |
| 590632 | 644 | 660 | | eeekkdddddddkkeee | 32 | 9794 | 9810 | 1152 |
| 590633 | 645 | 661 | | eeekkdddddddkkeee | 45 | 9795 | 9811 | 1153 |
| 590634 | 646 | 662 | | eeekkdddddddkkeee | 65 | 9796 | 9812 | 1154 |
| 590635 | 647 | 663 | | eeekkdddddddkkeee | 58 | 9797 | 9813 | 1155 |
| 590636 | 648 | 664 | | eeekkdddddddkkeee | 45 | 9798 | 9814 | 1156 |
| 590637 | 649 | 665 | | eeekkdddddddkkeee | 34 | 9799 | 9815 | 1157 |
| 590638 | 650 | 666 | | eeekkdddddddkkeee | 39 | 9800 | 9816 | 1158 |
| 590639 | 651 | 667 | | eeekkdddddddkkeee | 10 | 9801 | 9817 | 1159 |
| 590640 | 652 | 668 | | eeekkdddddddkkeee | 15 | 9802 | 9818 | 1160 |
| 590641 | 653 | 669 | | eeekkdddddddkkeee | 21 | 9803 | 9819 | 1161 |
| 590642 | 654 | 670 | | eeekkdddddddkkeee | 20 | 9804 | 9820 | 1162 |
| 590643 | 655 | 671 | | eeekkdddddddkkeee | 40 | 9805 | 9821 | 1163 |
| 590644 | 656 | 672 | | eeekkdddddddkkeee | 55 | 9806 | 9822 | 1164 |
| 590645 | 657 | 673 | | eeekkdddddddkkeee | 51 | 9807 | 9823 | 1165 |
| 590646 | 658 | 674 | | eeekkdddddddkkeee | 31 | 9808 | 9824 | 1166 |
| 590647 | 659 | 675 | | eeekkdddddddkkeee | 38 | 9809 | 9825 | 1167 |
| 590648 | 660 | 676 | | eeekkdddddddkkeee | 45 | 9810 | 9826 | 1168 |
| 590649 | 661 | 677 | | eeekkdddddddkkeee | 34 | 9811 | 9827 | 1169 |
| 590650 | 664 | 680 | | eeekkdddddddkkeee | 57 | 9814 | 9830 | 1170 |
| 590651 | 665 | 681 | | eeekkdddddddkkeee | 40 | 9815 | 9831 | 1171 |
| 590652 | 683 | 699 | | eeekkdddddddkkeee | 37 | 9833 | 9849 | 1172 |
| 590653 | 684 | 700 | | eeekkdddddddkkeee | 67 | 9834 | 9850 | 1173 |
| 590654 | 685 | 701 | | eeekkdddddddkkeee | 54 | 9835 | 9851 | 1174 |
| 590655 | 686 | 702 | | eeekkdddddddkkeee | 56 | 9836 | 9852 | 1175 |
| 590656 | 687 | 703 | | eeekkdddddddkkeee | 30 | 9837 | 9853 | 1176 |
| 590657 | 688 | 704 | | eeekkdddddddkkeee | 18 | 9838 | 9854 | 1177 |
| 590658 | 689 | 705 | | eeekkdddddddkkeee | 24 | 9839 | 9855 | 1178 |
| 590659 | 690 | 706 | | eeekkdddddddkkeee | 10 | 9840 | 9856 | 1179 |
| 590660 | 691 | 707 | | eeekkdddddddkkeee | 45 | 9841 | 9857 | 1180 |
| 590661 | 692 | 708 | | eeekkdddddddkkeee | 34 | 9842 | 9858 | 1181 |
| 590662 | 693 | 709 | | eeekkdddddddkkeee | 54 | 9843 | 9859 | 1182 |
| 590663 | 694 | 710 | | eeekkdddddddkkeee | 54 | 9844 | 9860 | 1183 |
| 590664 | 772 | 788 | | eeekkdddddddkkeee | 7 | 9922 | 9938 | 1184 |
| 590665 | 773 | 789 | | eeekkdddddddkkeee | 23 | 9923 | 9939 | 1185 |
| 590666 | 774 | 790 | | eeekkdddddddkkeee | 4 | 9924 | 9940 | 1186 |
| 590667 | 775 | 791 | | eeekkdddddddkkeee | 18 | 9925 | 9941 | 1187 |

**Table 16**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 590512 | 169 | 185 | | eeekkdddddddkkeee | 16 | 975 | 991 | 968 |
| 590668 | 777 | 793 | | eeekkdddddddkkeee | 17 | 9927 | 9943 | 1188 |
| 590669 | 778 | 794 | | eeekkdddddddkkeee | 15 | 9928 | 9944 | 1189 |
| 590670 | 779 | 795 | | eeekkdddddddkkeee | 11 | 9929 | 9945 | 1190 |
| 590671 | 780 | 796 | | eeekkdddddddkkeee | 13 | 9930 | 9946 | 1191 |
| 590672 | 781 | 797 | | eeekkdddddddkkeee | 10 | 9931 | 9947 | 1192 |
| 590673 | 782 | 798 | | eeekkdddddddkkeee | 28 | 9932 | 9948 | 1193 |
| 590674 | 783 | 799 | | eeekkdddddddkkeee | 26 | 9933 | 9949 | 1194 |
| 590675 | 786 | 802 | | eeekkdddddddkkeee | 14 | 9936 | 9952 | 1195 |
| 590676 | 791 | 807 | | eeekkdddddddkkeee | 22 | 9941 | 9957 | 1196 |
| 590677 | 793 | 809 | | eeekkdddddddkkeee | 6 | 9943 | 9959 | 1197 |
| 590678 | 814 | 830 | | eeekkdddddddkkeee | 22 | 9964 | 9980 | 1198 |
| 590679 | 815 | 831 | | eeekkdddddddkkeee | 11 | 9965 | 9981 | 1199 |
| 590680 | 816 | 832 | | eeekkdddddddkkeee | 10 | 9966 | 9982 | 1200 |
| 590681 | 817 | 833 | | eeekkdddddddkkeee | 23 | 9967 | 9983 | 1201 |
| 590682 | 818 | 834 | | eeekkdddddddkkeee | 11 | 9968 | 9984 | 1202 |
| 590683 | 819 | 835 | | eeekkdddddddkkeee | 21 | 9969 | 9985 | 1203 |
| 590684 | 820 | 836 | | eeekkdddddddkkeee | 14 | 9970 | 9986 | 1204 |
| 590685 | 821 | 837 | | eeekkdddddddkkeee | 14 | 9971 | 9987 | 1205 |
| 590686 | 822 | 838 | | eeekkdddddddkkeee | 9 | 9972 | 9988 | 1206 |
| 590687 | 823 | 839 | | eeekkdddddddkkeee | 14 | 9973 | 9989 | 1207 |
| 590688 | 824 | 840 | | eeekkdddddddkkeee | 6 | 9974 | 9990 | 1208 |
| 590689 | 825 | 841 | | eeekkdddddddkkeee | 2 | 9975 | 9991 | 1209 |
| 590690 | 826 | 842 | | eeekkdddddddkkeee | n.d. | 9976 | 9992 | 1210 |
| 590691 | 827 | 843 | | eeekkdddddddkkeee | n.d. | 9977 | 9993 | 1211 |
| 590692 | 828 | 844 | | eeekkdddddddkkeee | n.d. | 9978 | 9994 | 1212 |
| 590693 | 829 | 845 | | eeekkdddddddkkeee | n.d. | 9979 | 9995 | 1213 |
| 590694 | 830 | 846 | | eeekkdddddddkkeee | n.d. | 9980 | 9996 | 1214 |
| 590695 | 831 | 847 | | eeekkdddddddkkeee | n.d. | 9981 | 9997 | 1215 |
| 590696 | 832 | 848 | | eeekkdddddddkkeee | n.d. | 9982 | 9998 | 1216 |
| 590697 | 833 | 849 | | eeekkdddddddkkeee | n.d. | 9983 | 9999 | 1217 |
| 590698 | 834 | 850 | | eeekkdddddddkkeee | 1 | 9984 | 10000 | 1218 |
| 590699 | 835 | 851 | | eeekkdddddddkkeee | 10 | 9985 | 10001 | 1219 |
| 590700 | 836 | 852 | | eeekkdddddddkkeee | 4 | 9986 | 10002 | 1220 |
| 590701 | 837 | 853 | | eeekkdddddddkkeee | 2 | 9987 | 10003 | 1221 |
| 590702 | 853 | 869 | | eeekkdddddddkkeee | 7 | 10003 | 10019 | 1222 |
| 590703 | 854 | 870 | | eeekkdddddddkkeee | 4 | 10004 | 10020 | 1223 |
| 590704 | 855 | 871 | | eeekkdddddddkkeee | 2 | 10005 | 10021 | 1224 |
| 590705 | 856 | 872 | | eeekkdddddddkkeee | 0 | 10006 | 10022 | 1225 |
| 590706 | 857 | 873 | | eeekkdddddddkkeee | 17 | 10007 | 10023 | 1226 |
| 590707 | 858 | 874 | | eeekkdddddddkkeee | 10 | 10008 | 10024 | 1227 |
| 590708 | 859 | 875 | | eeekkdddddddkkeee | 12 | 10009 | 10025 | 1228 |
| 590709 | 860 | 876 | | eeekkdddddddkkeee | 37 | 10010 | 10026 | 1229 |
| 590710 | 861 | 877 | | eeekkdddddddkkeee | 23 | 10011 | 10027 | 1230 |
| 590711 | 862 | 878 | | eeekkdddddddkkeee | 24 | 10012 | 10028 | 1231 |
| 590712 | 863 | 879 | | eeekkdddddddkkeee | 27 | 10013 | 10029 | 1232 |
| 590713 | 864 | 880 | | eeekkdddddddkkeee | 10 | 10014 | 10030 | 1233 |
| 590714 | 865 | 881 | | eeekkdddddddkkeee | 0 | 10015 | 10031 | 1234 |
| 590715 | 866 | 882 | | eeekkdddddddkkeee | 6 | 10016 | 10032 | 1235 |
| 590716 | 867 | 883 | | eeekkdddddddkkeee | 9 | 10017 | 10033 | 1236 |
| 590717 | 868 | 884 | | eeekkdddddddkkeee | 15 | 10018 | 10034 | 1237 |
| 590718 | 869 | 885 | | eeekkdddddddkkeee | 21 | 10019 | 10035 | 1238 |
| 590719 | 870 | 886 | | eeekkdddddddkkeee | 14 | 10020 | 10036 | 1239 |
| 590720 | 871 | 887 | | eeekkdddddddkkeee | 8 | 10021 | 10037 | 1240 |
| 590721 | 872 | 888 | | eeekkdddddddkkeee | 18 | 10022 | 10038 | 1241 |
| 590722 | 891 | 907 | | eeekkdddddddkkeee | 9 | 10041 | 10057 | 1242 |
| 590723 | 892 | 908 | | eeekkdddddddkkeee | 11 | 10042 | 10058 | 1243 |
| 590724 | 893 | 909 | | eeekkdddddddkkeee | 0 | 10043 | 10059 | 1244 |
| 590725 | 894 | 910 | | eeekkdddddddkkeee | 10 | 10044 | 10060 | 1245 |
| 590726 | 895 | 911 | | eeekkdddddddkkeee | 29 | 10045 | 10061 | 1246 |
| 590727 | 896 | 912 | | eeekkdddddddkkeee | 28 | 10046 | 10062 | 1247 |
| 590728 | 897 | 913 | | eeekkdddddddkkeee | 31 | 10047 | 10063 | 1248 |
| 590729 | 898 | 914 | | eeekkdddddddkkeee | 10 | 10048 | 10064 | 1249 |
| 590731 | 899 | 915 | | eeekkdddddddkkeee | 22 | 10049 | 10065 | 1250 |
| 590732 | 900 | 916 | | eeekkdddddddkkeee | 17 | 10050 | 10066 | 1251 |
| 590733 | 901 | 917 | | eeekkdddddddkkeee | 24 | 10051 | 10067 | 1252 |
| 590734 | 902 | 918 | | eeekkdddddddkkeee | 17 | 10052 | 10068 | 1253 |
| 590735 | 903 | 919 | | eeekkdddddddkkeee | 10 | 10053 | 10069 | 1254 |
| 590736 | 904 | 920 | | eeekkdddddddkkeee | 11 | 10054 | 10070 | 1255 |
| 590737 | 905 | 921 | | eeekkdddddddkkeee | 3 | 10055 | 10071 | 1256 |
| 590738 | 906 | 922 | | eeekkdddddddkkeee | 0 | 10056 | 10072 | 1257 |
| 590739 | 907 | 923 | | eeekkdddddddkkeee | 1 | 10057 | 10073 | 1258 |
| 590740 | 908 | 924 | | eeekkdddddddkkeee | 11 | 10058 | 10074 | 1259 |
| 590741 | 909 | 925 | | eeekkdddddddkkeee | 9 | 10059 | 10075 | 1260 |
| 590742 | 910 | 926 | | eeekkdddddddkkeee | 7 | 10060 | 10076 | 1261 |
| 590743 | 911 | 927 | | eeekkdddddddkkeee | 9 | 10061 | 10077 | 1262 |
| 590744 | 938 | 954 | | eeekkdddddddkkeee | 8 | 10088 | 10104 | 1263 |
| 590745 | 951 | 967 | | eeekkdddddddkkeee | 12 | n/a | n/a | 1264 |

**Table 17**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 333611 | 167 | 186 | | eeeeeddddddddddeeeee | 66 | 973 | 992 | 21 |
| 590067 | 202 | 221 | | eeeeeddddddddddeeeee | 53 | 1008 | 1027 | 120 |
| 590074 | 209 | 228 | | eeeeeddddddddddeeeee | 30 | n/a | n/a | 127 |
| 590457 | 211 | 227 | | eeekkdddddddkkeee | 14 | n/a | n/a | 980 |
| 590458 | 212 | 228 | | eeekkdddddddkkeee | 22 | n/a | n/a | 981 |
| 590459 | 213 | 229 | | eeekkdddddddkkeee | 15 | n/a | n/a | 982 |
| 590461 | 214 | 230 | | eeekkdddddddkkeee | 28 | n/a | n/a | 983 |
| 590462 | 215 | 231 | | eeekkdddddddkkeee | 37 | n/a | n/a | 984 |
| 590463 | 216 | 232 | | eeekkdddddddkkeee | 18 | n/a | n/a | 985 |
| 590082 | 217 | 236 | | eeeeeddddddddddeeeee | 50 | n/a | n/a | 135 |
| 590464 | 217 | 233 | | eeekkdddddddkkeee | 33 | n/a | n/a | 986 |
| 590465 | 218 | 234 | | eeekkdddddddkkeee | 18 | 4972 | 4988 | 987 |
| 590130 | 363 | 382 | | eeeeeddddddddddeeeee | 30 | 7679 | 7698 | 194 |
| 590536 | 363 | 379 | | eeekkdddddddkkeee | 51 | 7679 | 7695 | 1056 |
| 590537 | 364 | 380 | | eeekkdddddddkkeee | 38 | 7680 | 7696 | 1057 |
| 590538 | 365 | 381 | | eeekkdddddddkkeee | 27 | 7681 | 7697 | 1058 |
| 590539 | 366 | 382 | | eeekkdddddddkkeee | 26 | 7682 | 7698 | 1059 |
| 590540 | 367 | 383 | | eeekkdddddddkkeee | 35 | 7683 | 7699 | 1060 |
| 590541 | 368 | 384 | | eeekkdddddddkkeee | 15 | 7684 | 7700 | 1061 |
| 590542 | 369 | 385 | | eeekkdddddddkkeee | 26 | 7685 | 7701 | 1062 |
| 590543 | 370 | 386 | | eeekkdddddddkkeee | 14 | 7686 | 7702 | 1063 |
| 590138 | 371 | 390 | | eeeeeddddddddddeeeee | 32 | n/a | n/a | 202 |
| 590146 | 505 | 524 | | eeeeeddddddddddeeeee | 46 | 9655 | 9674 | 211 |
| 590613 | 608 | 624 | | eeekkdddddddkkeee | 19 | 9758 | 9774 | 1133 |
| 590614 | 609 | 625 | | eeekkdddddddkkeee | 36 | 9759 | 9775 | 1134 |
| 590615 | 610 | 626 | | eeekkdddddddkkeee | 32 | 9760 | 9776 | 1135 |
| 590616 | 611 | 627 | | eeekkdddddddkkeee | 42 | 9761 | 9777 | 1136 |
| 590617 | 612 | 628 | | eeekkdddddddkkeee | 16 | 9762 | 9778 | 1137 |
| 590618 | 613 | 629 | | eeekkdddddddkkeee | 30 | 9763 | 9779 | 1138 |
| 590619 | 614 | 630 | | eeekkdddddddkkeee | 30 | 9764 | 9780 | 1139 |
| 590620 | 615 | 631 | | eeekkdddddddkkeee | 40 | 9765 | 9781 | 1140 |
| 590690 | 826 | 842 | | eeekkdddddddkkeee | 28 | 9976 | 9992 | 1210 |
| 590691 | 827 | 843 | | eeekkdddddddkkeee | 23 | 9977 | 9993 | 1211 |
| 590692 | 828 | 844 | | eeekkdddddddkkeee | 43 | 9978 | 9994 | 1212 |
| 590693 | 829 | 845 | | eeekkdddddddkkeee | 39 | 9979 | 9995 | 1213 |
| 590694 | 830 | 846 | | eeekkdddddddkkeee | 26 | 9980 | 9996 | 1214 |
| 590695 | 831 | 847 | | eeekkdddddddkkeee | 18 | 9981 | 9997 | 1215 |
| 590696 | 832 | 848 | | eeekkdddddddkkeee | 0 | 9982 | 9998 | 1216 |
| 590697 | 833 | 849 | | eeekkdddddddkkeee | 24 | 9983 | 9999 | 1217 |

### Example 4: Inhibition of human SOD-1 in HepG2 cells by deoxy, MOE and cEt gapmers

Modified oligonucleotides were designed targeting a superoxide dismutase 1, soluble (SOD-1) nucleic acid and were tested for their effects on SOD-1 mRNA in vitro. ISIS 333611, a 5-10-5 MOE gapmer which was previously described in WO 2005/040180, was included as a benchmark.

The modified oligonucleotides were tested in a series of experiments that had similar culture conditions. The results for each experiment are presented in separate tables shown below. Cultured HepG2 cells at a density of 20,000 cells per well were transfected using electroporation with 4,000 nM modified oligonucleotide. After a treatment period of approximately 24 hours, RNA was isolated from the cells and SOD-1 mRNA levels were measured by quantitative real-time PCR.

Human primer probe set RTS3898 was used to measure mRNA levels. SOD-1 mRNA levels were adjusted according to total RNA content, as measured by RIBOGREEN^{®}. Results are presented as percent inhibition of SOD-1, relative to untreated control cells. 'n.d.' indicates that inhibition levels were not measured.

The newly designed modified oligonucleotides in the Tables below were designed as deoxy, MOE, and cEt gapmers or 5-10-5 gapmers. The 5-10-5 MOE gapmers are 20 nucleosides in length, wherein the central gap segment is comprised of ten 2'-deoxyribonucleosides and is flanked by wing segments on the 5' direction and the 3' direction comprising five nucleosides each. Each nucleoside in the 5' wing segment and each nucleoside in the 3' wing segment has a 2'-MOE modification. The deoxy, MOE and cEt oligonucleotides are 17 nucleosides in length wherein the nucleoside has a MOE sugar modification, a cEt sugar modification, or a deoxy moiety. The sugar chemistry of each oligonucleotide is denoted as in the Chemistry column, where 'k' indicates a cEt modified sugar; 'd' indicates a 2'-deoxyribose; and 'e' indicates a 2'-MOE modified sugar. The internucleoside linkages throughout each gapmer are phosphorothioate linkages. All cytosine residues throughout each gapmer are 5-methylcytosines. "Start site" indicates the 5'-most nucleoside to which the gapmer is targeted in the human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000). 'n/a' indicates that the modified oligonucleotide does not target that particular gene sequence with 100% complementarity.

**Table 18**

| Percent inhibition of SOD-1 mRNA by 5-10-5 MOE gapmers targeting SEQ ID NO: 1 and/or 2 | | | | | | | |
|---|---|---|---|---|---|---|---|
| ISIS NO | SEQ ID NO: 1 Start Site | SEQ ID NO: 1 Stop Site | Sequence | % inhibition | SEQ ID NO: 2 Start Site | SEQ ID NO: 2 Stop Site | SEQ ID NO |
| 596168 | 3 | 22 | CTCGCCCACTCTGGCCCCAA | 45 | 809 | 828 | 1265 |
| 596169 | 5 | 24 | GCCTCGCCCACTCTGGCCCC | 37 | 811 | 830 | 1266 |
| 596170 | 7 | 26 | GCGCCTCGCCCACTCTGGCC | 33 | 813 | 832 | 1267 |
| 596171 | 9 | 28 | CCGCGCCTCGCCCACTCTGG | 27 | 815 | 834 | 1268 |
| 596172 | 11 | 30 | CTCCGCGCCTCGCCCACTCT | 40 | 817 | 836 | 1269 |
| 596173 | 13 | 32 | ACCTCCGCGCCTCGCCCACT | 77 | 819 | 838 | 1270 |
| 596174 | 15 | 34 | AGACCTCCGCGCCTCGCCCA | 72 | 821 | 840 | 1271 |
| 596175 | 17 | 36 | CCAGACCTCCGCGCCTCGCC | 46 | 823 | 842 | 1272 |
| 596176 | 19 | 38 | GGCCAGACCTCCGCGCCTCG | 49 | 825 | 844 | 1273 |
| 150508 | 21 | 40 | TAGGCCAGACCTCCGCGCCT | 33 | 827 | 846 | 107 |
| 596177 | 23 | 42 | TATAGGCCAGACCTCCGCGC | 40 | 829 | 848 | 1274 |
| 596178 | 25 | 44 | TTTATAGGCCAGACCTCCGC | 69 | 831 | 850 | 1275 |
| 150509 | 27 | 46 | ACTTTATAGGCCAGACCTCC | 64 | 833 | 852 | 108 |
| 596179 | 31 | 50 | GACTACTTTATAGGCCAGAC | 74 | 837 | 856 | 1276 |
| 596180 | 33 | 52 | GCGACTACTTTATAGGCCAG | 19 | 839 | 858 | 1277 |
| 596181 | 37 | 56 | CTCCGCGACTACTTTATAGG | 27 | 843 | 862 | 1278 |
| 596182 | 39 | 58 | GTCTCCGCGACTACTTTATA | 22 | 845 | 864 | 1279 |
| 596183 | 41 | 60 | CCGTCTCCGCGACTACTTTA | 20 | 847 | 866 | 1280 |
| 596184 | 43 | 62 | CCCCGTCTCCGCGACTACTT | 16 | 849 | 868 | 1281 |
| 596185 | 45 | 64 | CACCCCGTCTCCGCGACTAC | 13 | 851 | 870 | 1282 |
| 596186 | 47 | 66 | AGCACCCCGTCTCCGCGACT | 24 | 853 | 872 | 1283 |
| 596187 | 49 | 68 | CCAGCACCCCGTCTCCGCGA | 38 | 855 | 874 | 1284 |
| 596188 | 51 | 70 | AACCAGCACCCCGTCTCCGC | 11 | 857 | 876 | 1285 |
| 596189 | 53 | 72 | CAAACCAGCACCCCGTCTCC | 13 | 859 | 878 | 1286 |
| 596190 | 55 | 74 | CGCAAACCAGCACCCCGTCT | 21 | 861 | 880 | 1287 |
| 150510 | 57 | 76 | GACGCAAACCAGCACCCCGT | 45 | 863 | 882 | 109 |
| 596191 | 59 | 78 | ACGACGCAAACCAGCACCCC | 30 | 865 | 884 | 1288 |
| 596192 | 61 | 80 | CTACGACGCAAACCAGCACC | 19 | 867 | 886 | 1289 |
| 596193 | 63 | 82 | GACTACGACGCAAACCAGCA | 40 | 869 | 888 | 1290 |
| 596194 | 65 | 84 | GAGACTACGACGCAAACCAG | 23 | 871 | 890 | 1291 |
| 596195 | 67 | 86 | AGGAGACTACGACGCAAACC | 35 | 873 | 892 | 1292 |
| 596196 | 69 | 88 | GCAGGAGACTACGACGCAAA | 33 | 875 | 894 | 1293 |
| 596197 | 71 | 90 | CTGCAGGAGACTACGACGCA | 36 | 877 | 896 | 1294 |
| 596198 | 73 | 92 | CGCTGCAGGAGACTACGACG | 23 | 879 | 898 | 1295 |
| 596199 | 91 | 110 | TGCAACGGAAACCCCAGACG | 21 | 897 | 916 | 1296 |
| 596200 | 93 | 112 | ACTGCAACGGAAACCCCAGA | 43 | 899 | 918 | 1297 |
| 596201 | 97 | 116 | GAGGACTGCAACGGAAACCC | 24 | 903 | 922 | 1298 |
| 596202 | 99 | 118 | CCGAGGACTGCAACGGAAAC | 29 | 905 | 924 | 1299 |
| 596203 | 101 | 120 | TTCCGAGGACTGCAACGGAA | 5 | 907 | 926 | 1300 |
| 150438 | 103 | 122 | GGTTCCGAGGACTGCAACGG | 35 | 909 | 928 | 110 |
| 345716 | 105 | 124 | CTGGTTCCGAGGACTGCAAC | 51 | 911 | 930 | 1301 |
| 150439 | 107 | 126 | TCCTGGTTCCGAGGACTGCA | 24 | 913 | 932 | 111 |
| 596204 | 109 | 128 | GGTCCTGGTTCCGAGGACTG | 14 | 915 | 934 | 1302 |
| 150440 | 111 | 130 | GAGGTCCTGGTTCCGAGGAC | 31 | 917 | 936 | 112 |
| 596205 | 113 | 132 | CCGAGGTCCTGGTTCCGAGG | 18 | 919 | 938 | 1303 |
| 345718 | 115 | 134 | CGCCGAGGTCCTGGTTCCGA | 24 | 921 | 940 | 1304 |
| 596206 | 117 | 136 | CACGCCGAGGTCCTGGTTCC | 23 | 923 | 942 | 1305 |
| 596207 | 119 | 138 | GCCACGCCGAGGTCCTGGTT | 38 | 925 | 944 | 1306 |
| 596208 | 123 | 142 | CTAGGCCACGCCGAGGTCCT | 39 | 929 | 948 | 1307 |
| 345720 | 125 | 144 | CGCTAGGCCACGCCGAGGTC | 52 | 931 | 950 | 1308 |
| 596209 | 127 | 146 | CTCGCTAGGCCACGCCGAGG | 46 | 933 | 952 | 1309 |
| 596210 | 129 | 148 | AACTCGCTAGGCCACGCCGA | 44 | 935 | 954 | 1310 |
| 596211 | 131 | 150 | ATAACTCGCTAGGCCACGCC | 12 | 937 | 956 | 1311 |
| 596212 | 133 | 152 | CCATAACTCGCTAGGCCACG | 22 | 939 | 958 | 1312 |
| 345722 | 135 | 154 | CGCCATAACTCGCTAGGCCA | 59 | 941 | 960 | 1313 |
| 150442 | 137 | 156 | GTCGCCATAACTCGCTAGGC | 40 | 943 | 962 | 113 |
| 146143 | 157 | 176 | TCAGCACGCACACGGCCTTC | 52 | 963 | 982 | 114 |
| 195753 | 159 | 178 | CTTCAGCACGCACACGGCCT | 57 | 965 | 984 | 115 |
| 333607 | 161 | 180 | CCCTTCAGCACGCACACGGC | 37 | 967 | 986 | 116 |
| 333608 | 163 | 182 | CGCCCTTCAGCACGCACACG | 23 | 969 | 988 | 117 |
| 333611 | 167 | 186 | CCGTCGCCCTTCAGCACGCA | 67 | 973 | 992 | 21 |
| 596213 | 171 | 190 | TGGGCCGTCGCCCTTCAGCA | 12 | 977 | 996 | 1314 |
| 596214 | 173 | 192 | ACTGGGCCGTCGCCCTTCAG | 26 | 979 | 998 | 1315 |
| 596215 | 175 | 194 | GCACTGGGCCGTCGCCCTTC | 14 | 981 | 1000 | 1316 |
| 596216 | 177 | 196 | CTGCACTGGGCCGTCGCCCT | 24 | 983 | 1002 | 1317 |
| 596217 | 181 | 200 | TGCCCTGCACTGGGCCGTCG | 38 | 987 | 1006 | 1318 |
| 596218 | 183 | 202 | GATGCCCTGCACTGGGCCGT | 15 | 989 | 1008 | 1319 |
| 596219 | 185 | 204 | ATGATGCCCTGCACTGGGCC | 20 | 991 | 1010 | 1320 |
| 596220 | 189 | 208 | ATTGATGATGCCCTGCACTG | 8 | 995 | 1014 | 1321 |
| 596221 | 191 | 210 | AAATTGATGATGCCCTGCAC | 14 | 997 | 1016 | 1322 |
| 596222 | 193 | 212 | CGAAATTGATGATGCCCTGC | 32 | 999 | 1018 | 1323 |
| 596223 | 195 | 214 | CTCGAAATTGATGATGCCCT | 31 | 1001 | 1020 | 1324 |
| 596224 | 197 | 216 | TGCTCGAAATTGATGATGCC | 20 | 1003 | 1022 | 1325 |
| 596225 | 199 | 218 | TCTGCTCGAAATTGATGATG | 14 | 1005 | 1024 | 1326 |
| 596226 | 201 | 220 | CTTCTGCTCGAAATTGATGA | 11 | 1007 | 1026 | 1327 |
| 596227 | 240 | 259 | TTTAATGCTTCCCCACACCT | 15 | 4994 | 5013 | 1328 |
| 596228 | 242 | 261 | CCTTTAATGCTTCCCCACAC | 1 | 4996 | 5015 | 1329 |
| 596229 | 244 | 263 | GTCCTTTAATGCTTCCCCAC | 9 | 4998 | 5017 | 1330 |

**Table 19**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 596530 | 164 | 180 | | eekkddddddddkkeee | 74 | 970 | 986 | 1331 |
| 596721 | 164 | 180 | | eekkdddddddddkkee | 81 | 970 | 986 | 1331 |
| 596531 | 165 | 181 | | eekkddddddddkkeee | 75 | 971 | 987 | 1332 |
| 596722 | 165 | 181 | | eekkdddddddddkkee | 60 | 971 | 987 | 1332 |
| 596532 | 166 | 182 | | eekkddddddddkkeee | 67 | 972 | 988 | 1333 |
| 596723 | 166 | 182 | | eekkdddddddddkkee | 73 | 972 | 988 | 1333 |
| 333611 | 167 | 186 | | eeeeeddddddddddeeeee | 73 | 973 | 992 | 21 |
| 596720 | 167 | 183 | | eekkddddddddkkeee | 56 | 973 | 989 | 966 |
| 596911 | 167 | 183 | | eekkdddddddddkkee | 63 | 973 | 989 | 966 |
| 596533 | 168 | 184 | | eekkddddddddkkeee | 60 | 974 | 990 | 967 |
| 596724 | 168 | 184 | | eekkdddddddddkkee | 72 | 974 | 990 | 967 |
| 596534 | 169 | 185 | | eekkddddddddkkeee | 52 | 975 | 991 | 968 |
| 596725 | 169 | 185 | | eekkdddddddddkkee | 43 | 975 | 991 | 968 |
| 596535 | 170 | 186 | | eekkddddddddkkeee | 71 | 976 | 992 | 969 |
| 596726 | 170 | 186 | | eekkdddddddddkkee | 75 | 976 | 992 | 969 |
| 596536 | 171 | 187 | | eekkddddddddkkeee | 64 | 977 | 993 | 970 |
| 596727 | 171 | 187 | | eekkdddddddddkkee | 57 | 977 | 993 | 970 |
| 596537 | 577 | 593 | | eekkddddddddkkeee | 48 | 9727 | 9743 | 1334 |
| 596728 | 577 | 593 | | eekkdddddddddkkee | 46 | 9727 | 9743 | 1334 |
| 596538 | 578 | 594 | | eekkddddddddkkeee | 27 | 9728 | 9744 | 1335 |
| 596729 | 578 | 594 | | eekkdddddddddkkee | 45 | 9728 | 9744 | 1335 |
| 596539 | 579 | 595 | | eekkddddddddkkeee | 56 | 9729 | 9745 | 1336 |
| 596730 | 579 | 595 | | eekkdddddddddkkee | 63 | 9729 | 9745 | 1336 |
| 596540 | 580 | 596 | | eekkddddddddkkeee | 60 | 9730 | 9746 | 1337 |
| 596731 | 580 | 596 | | eekkdddddddddkkee | 63 | 9730 | 9746 | 1337 |
| 596541 | 581 | 597 | | eekkddddddddkkeee | 46 | 9731 | 9747 | 1338 |
| 596732 | 581 | 597 | | eekkdddddddddkkee | 63 | 9731 | 9747 | 1338 |
| 596542 | 582 | 598 | | eekkddddddddkkeee | 62 | 9732 | 9748 | 1111 |
| 596733 | 582 | 598 | | eekkdddddddddkkee | 56 | 9732 | 9748 | 1111 |
| 596543 | 583 | 599 | | eekkddddddddkkeee | 58 | 9733 | 9749 | 1112 |
| 596734 | 583 | 599 | | eekkdddddddddkkee | 61 | 9733 | 9749 | 1112 |
| 596544 | 584 | 600 | | eekkddddddddkkeee | 66 | 9734 | 9750 | 1113 |
| 596735 | 584 | 600 | | eekkdddddddddkkee | 73 | 9734 | 9750 | 1113 |
| 596545 | 585 | 601 | | eekkddddddddkkeee | 63 | 9735 | 9751 | 1114 |
| 596736 | 585 | 601 | | eekkdddddddddkkee | 74 | 9735 | 9751 | 1114 |
| 596546 | 588 | 604 | | eekkddddddddkkeee | 41 | 9738 | 9754 | 1115 |
| 596737 | 588 | 604 | | eekkdddddddddkkee | 58 | 9738 | 9754 | 1115 |
| 596547 | 589 | 605 | | eekkddddddddkkeee | 57 | 9739 | 9755 | 1116 |
| 596738 | 589 | 605 | | eekkdddddddddkkee | 59 | 9739 | 9755 | 1116 |
| 596548 | 590 | 606 | | eekkddddddddkkeee | 31 | 9740 | 9756 | 1117 |
| 596739 | 590 | 606 | | eekkdddddddddkkee | 58 | 9740 | 9756 | 1117 |
| 596549 | 591 | 607 | | eekkddddddddkkeee | 33 | 9741 | 9757 | 1118 |
| 596740 | 591 | 607 | | eekkdddddddddkkee | 66 | 9741 | 9757 | 1118 |
| 596550 | 592 | 608 | | eekkddddddddkkeee | 30 | 9742 | 9758 | 1119 |
| 596741 | 592 | 608 | | eekkdddddddddkkee | 30 | 9742 | 9758 | 1119 |
| 596551 | 593 | 609 | | eekkddddddddkkeee | 19 | 9743 | 9759 | 1120 |
| 596742 | 593 | 609 | | eekkdddddddddkkee | 46 | 9743 | 9759 | 1120 |
| 596552 | 594 | 610 | | eekkddddddddkkeee | 14 | 9744 | 9760 | 1121 |
| 596743 | 594 | 610 | | eekkdddddddddkkee | 5 | 9744 | 9760 | 1121 |
| 596553 | 595 | 611 | | eekkddddddddkkeee | 2 | 9745 | 9761 | 1122 |
| 596744 | 595 | 611 | | eekkdddddddddkkee | 23 | 9745 | 9761 | 1122 |
| 596554 | 596 | 612 | | eekkddddddddkkeee | 19 | 9746 | 9762 | 1123 |
| 596745 | 596 | 612 | | eekkdddddddddkkee | 6 | 9746 | 9762 | 1123 |
| 596555 | 597 | 613 | | eekkddddddddkkeee | 41 | 9747 | 9763 | 1124 |
| 596746 | 597 | 613 | | eekkdddddddddkkee | 41 | 9747 | 9763 | 1124 |
| 596556 | 598 | 614 | | eekkddddddddkkeee | 34 | 9748 | 9764 | 1125 |
| 596747 | 598 | 614 | | eekkdddddddddkkee | 46 | 9748 | 9764 | 1125 |
| 596557 | 599 | 615 | | eekkddddddddkkeee | 54 | 9749 | 9765 | 1126 |
| 596748 | 599 | 615 | | eekkdddddddddkkee | 68 | 9749 | 9765 | 1126 |
| 596558 | 600 | 616 | | eekkddddddddkkeee | 50 | 9750 | 9766 | 1127 |
| 596749 | 600 | 616 | | eekkdddddddddkkee | 47 | 9750 | 9766 | 1127 |
| 596559 | 601 | 617 | | eekkddddddddkkeee | 76 | 9751 | 9767 | 1128 |
| 596750 | 601 | 617 | | eekkdddddddddkkee | 64 | 9751 | 9767 | 1128 |
| 596560 | 602 | 618 | | eekkddddddddkkeee | 61 | 9752 | 9768 | 1129 |
| 596751 | 602 | 618 | | eekkdddddddddkkee | 64 | 9752 | 9768 | 1129 |
| 596561 | 603 | 619 | | eekkddddddddkkeee | 47 | 9753 | 9769 | 1130 |
| 596752 | 603 | 619 | | eekkdddddddddkkee | 65 | 9753 | 9769 | 1130 |
| 596562 | 604 | 620 | | eekkddddddddkkeee | 37 | 9754 | 9770 | 1131 |
| 596753 | 604 | 620 | | eekkdddddddddkkee | 58 | 9754 | 9770 | 1131 |
| 596563 | 608 | 624 | | eekkddddddddkkeee | 43 | 9758 | 9774 | 1133 |
| 596754 | 608 | 624 | | eekkdddddddddkkee | 38 | 9758 | 9774 | 1133 |
| 596564 | 609 | 625 | | eekkddddddddkkeee | 57 | 9759 | 9775 | 1134 |
| 596755 | 609 | 625 | | eekkdddddddddkkee | 52 | 9759 | 9775 | 1134 |
| 596565 | 610 | 626 | | eekkddddddddkkeee | 27 | 9760 | 9776 | 1135 |
| 596756 | 610 | 626 | | eekkdddddddddkkee | 57 | 9760 | 9776 | 1135 |
| 596566 | 611 | 627 | | eekkddddddddkkeee | 35 | 9761 | 9777 | 1136 |
| 596757 | 611 | 627 | | eekkdddddddddkkee | 39 | 9761 | 9777 | 1136 |
| 596567 | 616 | 632 | | eekkddddddddkkeee | 42 | 9766 | 9782 | 1141 |
| 596758 | 616 | 632 | | eekkdddddddddkkee | 48 | 9766 | 9782 | 1141 |

**Table 20**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 333611 | 167 | 186 | | eeeeeddddddddddeeeee | 64 | 973 | 992 | 21 |
| 596720 | 167 | 183 | | eekkddddddddkkeee | 56 | 973 | 989 | 966 |
| 596911 | 167 | 183 | | eekkdddddddddkkee | 60 | 973 | 989 | 966 |
| 596568 | 617 | 633 | | eekkddddddddkkeee | 50 | 9767 | 9783 | 1142 |
| 596759 | 617 | 633 | | eekkdddddddddkkee | 57 | 9767 | 9783 | 1142 |
| 596569 | 618 | 634 | | eekkddddddddkkeee | 53 | 9768 | 9784 | 1143 |
| 596760 | 618 | 634 | | eekkdddddddddkkee | 55 | 9768 | 9784 | 1143 |
| 596570 | 619 | 635 | | eekkddddddddkkeee | 81 | 9769 | 9785 | 1144 |
| 596761 | 619 | 635 | | eekkdddddddddkkee | 78 | 9769 | 9785 | 1144 |
| 596571 | 620 | 636 | | eekkddddddddkkeee | 79 | 9770 | 9786 | 1145 |
| 596762 | 620 | 636 | | eekkdddddddddkkee | 78 | 9770 | 9786 | 1145 |
| 596572 | 621 | 637 | | eekkddddddddkkeee | 85 | 9771 | 9787 | 1146 |
| 596763 | 621 | 637 | | eekkdddddddddkkee | 76 | 9771 | 9787 | 1146 |
| 596573 | 622 | 638 | | eekkddddddddkkeee | 73 | 9772 | 9788 | 1147 |
| 596764 | 622 | 638 | | eekkdddddddddkkee | 87 | 9772 | 9788 | 1147 |
| 596574 | 623 | 639 | | eekkddddddddkkeee | 69 | 9773 | 9789 | 1148 |
| 596765 | 623 | 639 | | eekkdddddddddkkee | 82 | 9773 | 9789 | 1148 |
| 596575 | 624 | 640 | | eekkddddddddkkeee | 70 | 9774 | 9790 | 1149 |
| 596766 | 624 | 640 | | eekkdddddddddkkee | 76 | 9774 | 9790 | 1149 |
| 596576 | 625 | 641 | | eekkddddddddkkeee | 55 | 9775 | 9791 | 1150 |
| 596767 | 625 | 641 | | eekkdddddddddkkee | 58 | 9775 | 9791 | 1150 |
| 596577 | 640 | 656 | | eekkddddddddkkeee | 73 | 9790 | 9806 | 1339 |
| 596768 | 640 | 656 | | eekkdddddddddkkee | 86 | 9790 | 9806 | 1339 |
| 596578 | 641 | 657 | | eekkddddddddkkeee | 68 | 9791 | 9807 | 1340 |
| 596769 | 641 | 657 | | eekkdddddddddkkee | 80 | 9791 | 9807 | 1340 |
| 596579 | 642 | 658 | | eekkddddddddkkeee | 27 | 9792 | 9808 | 1341 |
| 596770 | 642 | 658 | | eekkdddddddddkkee | 42 | 9792 | 9808 | 1341 |
| 596580 | 643 | 659 | | eekkddddddddkkeee | 41 | 9793 | 9809 | 1151 |
| 596771 | 643 | 659 | | eekkdddddddddkkee | 28 | 9793 | 9809 | 1151 |
| 596581 | 644 | 660 | | eekkddddddddkkeee | 63 | 9794 | 9810 | 1152 |
| 596772 | 644 | 660 | | eekkdddddddddkkee | 63 | 9794 | 9810 | 1152 |
| 596582 | 645 | 661 | | eekkddddddddkkeee | 84 | 9795 | 9811 | 1153 |
| 596773 | 645 | 661 | | eekkdddddddddkkee | 86 | 9795 | 9811 | 1153 |
| 596583 | 646 | 662 | | eekkddddddddkkeee | 95 | 9796 | 9812 | 1154 |
| 596774 | 646 | 662 | | eekkdddddddddkkee | 96 | 9796 | 9812 | 1154 |
| 596584 | 647 | 663 | | eekkddddddddkkeee | 79 | 9797 | 9813 | 1155 |
| 596775 | 647 | 663 | | eekkdddddddddkkee | 86 | 9797 | 9813 | 1155 |
| 596585 | 651 | 667 | | eekkddddddddkkeee | 19 | 9801 | 9817 | 1159 |
| 596776 | 651 | 667 | | eekkdddddddddkkee | 43 | 9801 | 9817 | 1159 |
| 596586 | 652 | 668 | | eekkddddddddkkeee | 57 | 9802 | 9818 | 1160 |
| 596777 | 652 | 668 | | eekkdddddddddkkee | 54 | 9802 | 9818 | 1160 |
| 596587 | 653 | 669 | | eekkddddddddkkeee | 71 | 9803 | 9819 | 1161 |
| 596778 | 653 | 669 | | eekkdddddddddkkee | 61 | 9803 | 9819 | 1161 |
| 596588 | 654 | 670 | | eekkddddddddkkeee | 79 | 9804 | 9820 | 1162 |
| 596779 | 654 | 670 | | eekkdddddddddkkee | 83 | 9804 | 9820 | 1162 |
| 596589 | 655 | 671 | | eekkddddddddkkeee | 85 | 9805 | 9821 | 1163 |
| 596780 | 655 | 671 | | eekkdddddddddkkee | 86 | 9805 | 9821 | 1163 |
| 596590 | 656 | 672 | | eekkddddddddkkeee | 87 | 9806 | 9822 | 1164 |
| 596781 | 656 | 672 | | eekkdddddddddkkee | 91 | 9806 | 9822 | 1164 |
| 596591 | 657 | 673 | | eekkddddddddkkeee | 75 | 9807 | 9823 | 1165 |
| 596782 | 657 | 673 | | eekkdddddddddkkee | 83 | 9807 | 9823 | 1165 |
| 596592 | 658 | 674 | | eekkddddddddkkeee | 74 | 9808 | 9824 | 1166 |
| 596783 | 658 | 674 | | eekkdddddddddkkee | 79 | 9808 | 9824 | 1166 |
| 596593 | 659 | 675 | | eekkddddddddkkeee | 76 | 9809 | 9825 | 1167 |
| 596784 | 659 | 675 | | eekkdddddddddkkee | 84 | 9809 | 9825 | 1167 |
| 596594 | 660 | 676 | | eekkddddddddkkeee | 66 | 9810 | 9826 | 1168 |
| 596785 | 660 | 676 | | eekkdddddddddkkee | 75 | 9810 | 9826 | 1168 |
| 596595 | 665 | 681 | | eekkddddddddkkeee | 64 | 9815 | 9831 | 1171 |
| 596786 | 665 | 681 | | eekkdddddddddkkee | 77 | 9815 | 9831 | 1171 |
| 596596 | 666 | 682 | | eekkddddddddkkeee | 75 | 9816 | 9832 | 1342 |
| 596787 | 666 | 682 | | eekkdddddddddkkee | 84 | 9816 | 9832 | 1342 |
| 596597 | 667 | 683 | | eekkddddddddkkeee | 60 | 9817 | 9833 | 1343 |
| 596788 | 667 | 683 | | eekkdddddddddkkee | 77 | 9817 | 9833 | 1343 |
| 596598 | 668 | 684 | | eekkddddddddkkeee | 79 | 9818 | 9834 | 1344 |
| 596789 | 668 | 684 | | eekkdddddddddkkee | 85 | 9818 | 9834 | 1344 |
| 596599 | 672 | 688 | | eekkddddddddkkeee | 57 | 9822 | 9838 | 1345 |
| 596790 | 672 | 688 | | eekkdddddddddkkee | 67 | 9822 | 9838 | 1345 |
| 596600 | 674 | 690 | | eekkddddddddkkeee | 85 | 9824 | 9840 | 1346 |
| 596791 | 674 | 690 | | eekkdddddddddkkee | 88 | 9824 | 9840 | 1346 |
| 596601 | 675 | 691 | | eekkddddddddkkeee | 70 | 9825 | 9841 | 1347 |
| 596792 | 675 | 691 | | eekkdddddddddkkee | 83 | 9825 | 9841 | 1347 |
| 596602 | 676 | 692 | | eekkddddddddkkeee | 85 | 9826 | 9842 | 1348 |
| 596793 | 676 | 692 | | eekkdddddddddkkee | 81 | 9826 | 9842 | 1348 |
| 596603 | 677 | 693 | | eekkddddddddkkeee | 89 | 9827 | 9843 | 1349 |
| 596794 | 677 | 693 | | eekkdddddddddkkee | 90 | 9827 | 9843 | 1349 |
| 596604 | 678 | 694 | | eekkddddddddkkeee | 90 | 9828 | 9844 | 1350 |
| 596795 | 678 | 694 | | eekkdddddddddkkee | 85 | 9828 | 9844 | 1350 |
| 596605 | 679 | 695 | | eekkddddddddkkeee | 90 | 9829 | 9845 | 1351 |
| 596796 | 679 | 695 | | eekkdddddddddkkee | 92 | 9829 | 9845 | 1351 |

### Example 5: Inhibition of human SOD-1 in HepG2 cells by deoxy, MOE and cEt gapmers

Modified oligonucleotides were designed targeting an SOD-1 nucleic acid and were tested for their effects on SOD-1 mRNA in vitro. ISIS 333611, a 5-10-5 MOE gapmer, which was previously described in WO 2005/040180, was included as a benchmark.

The modified oligonucleotides were tested in a series of experiments that had similar culture conditions. The results for each experiment are presented in separate tables shown below. Cultured HepG2 cells at a density of 20,000 cells per well were transfected using electroporation with 5,000 nM modified oligonucleotide. After a treatment period of approximately 24 hours, RNA was isolated from the cells and SOD-1 mRNA levels were measured by quantitative real-time PCR.

Human primer probe set RTS3898 was used to measure mRNA levels. SOD-1 mRNA levels were adjusted according to total RNA content, as measured by RIBOGREEN^{®}. Results are presented as percent inhibition of SOD-1, relative to untreated control cells. 'n.d.' indicates that inhibition levels were not measured.

The newly designed modified oligonucleotides in the Tables below were designed as deoxy, MOE, and cEt gapmers. The gapmers are 17 nucleosides in length wherein each nucleoside has a MOE sugar modification, a cEt sugar modification, or a deoxy moiety. The sugar chemistry of each oligonucleotide is denoted as in the Chemistry column, where 'k' indicates a cEt modified sugar; 'd' indicates a 2'-deoxyribose; and 'e' indicates a 2'-MOE modified sugar. The internucleoside linkages throughout each gapmer are phosphorothioate linkages. All cytosine residues throughout each gapmer are 5-methylcytosines. "Start site" indicates the 5'-most nucleoside to which the gapmer is targeted in the human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000). 'n/a' indicates that the modified oligonucleotide does not target that particular gene sequence with 100% complementarity.

**Table 21**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 333611 | 167 | 186 | | eeeeeddddddddddeeeee | 71 | 973 | 992 | 21 |
| 596720 | 167 | 183 | | eekkddddddddkkeee | 53 | 973 | 989 | 966 |
| 596911 | 167 | 183 | | eekkdddddddddkkee | 61 | 973 | 989 | 966 |
| 596606 | 681 | 697 | | eekkddddddddkkeee | 87 | 9831 | 9847 | 1352 |
| 596797 | 681 | 697 | | eekkdddddddddkkee | 92 | 9831 | 9847 | 1352 |
| 596607 | 683 | 699 | | eekkddddddddkkeee | 86 | 9833 | 9849 | 1172 |
| 596798 | 683 | 699 | | eekkdddddddddkkee | 86 | 9833 | 9849 | 1172 |
| 596608 | 684 | 700 | | eekkddddddddkkeee | 88 | 9834 | 9850 | 1173 |
| 596799 | 684 | 700 | | eekkdddddddddkkee | 80 | 9834 | 9850 | 1173 |
| 596609 | 685 | 701 | | eekkddddddddkkeee | 77 | 9835 | 9851 | 1174 |
| 596800 | 685 | 701 | | eekkdddddddddkkee | 85 | 9835 | 9851 | 1174 |
| 596610 | 686 | 702 | | eekkddddddddkkeee | 83 | 9836 | 9852 | 1175 |
| 596801 | 686 | 702 | | eekkdddddddddkkee | 84 | 9836 | 9852 | 1175 |
| 596611 | 690 | 706 | | eekkddddddddkkeee | 54 | 9840 | 9856 | 1179 |
| 596802 | 690 | 706 | | eekkdddddddddkkee | 61 | 9840 | 9856 | 1179 |
| 596612 | 691 | 707 | | eekkddddddddkkeee | 68 | 9841 | 9857 | 1180 |
| 596803 | 691 | 707 | | eekkdddddddddkkee | 63 | 9841 | 9857 | 1180 |
| 596613 | 697 | 713 | | eekkddddddddkkeee | 62 | 9847 | 9863 | 1353 |
| 596804 | 697 | 713 | | eekkdddddddddkkee | 53 | 9847 | 9863 | 1353 |
| 596614 | 699 | 715 | | eekkddddddddkkeee | 37 | 9849 | 9865 | 1354 |
| 596805 | 699 | 715 | | eekkdddddddddkkee | 49 | 9849 | 9865 | 1354 |
| 596615 | 710 | 726 | | eekkddddddddkkeee | 28 | 9860 | 9876 | 1355 |
| 596806 | 710 | 726 | | eekkdddddddddkkee | 39 | 9860 | 9876 | 1355 |
| 596616 | 711 | 727 | | eekkddddddddkkeee | 28 | 9861 | 9877 | 1356 |
| 596807 | 711 | 727 | | eekkdddddddddkkee | 35 | 9861 | 9877 | 1356 |
| 596617 | 713 | 729 | | eekkddddddddkkeee | 41 | 9863 | 9879 | 1357 |
| 596808 | 713 | 729 | | eekkdddddddddkkee | 37 | 9863 | 9879 | 1357 |
| 596618 | 737 | 753 | | eekkddddddddkkeee | 35 | 9887 | 9903 | 1358 |
| 596809 | 737 | 753 | | eekkdddddddddkkee | 42 | 9887 | 9903 | 1358 |
| 596619 | 739 | 755 | | eekkddddddddkkeee | 14 | 9889 | 9905 | 1359 |
| 596810 | 739 | 755 | | eekkdddddddddkkee | 20 | 9889 | 9905 | 1359 |
| 596620 | 740 | 756 | | eekkddddddddkkeee | 23 | 9890 | 9906 | 1360 |
| 596811 | 740 | 756 | | eekkdddddddddkkee | 26 | 9890 | 9906 | 1360 |
| 596621 | 741 | 757 | | eekkddddddddkkeee | 2 | 9891 | 9907 | 1361 |
| 596812 | 741 | 757 | | eekkdddddddddkkee | 16 | 9891 | 9907 | 1361 |
| 596622 | 743 | 759 | | eekkddddddddkkeee | 27 | 9893 | 9909 | 1362 |
| 596813 | 743 | 759 | | eekkdddddddddkkee | 38 | 9893 | 9909 | 1362 |
| 596623 | 744 | 760 | | eekkddddddddkkeee | 27 | 9894 | 9910 | 1363 |
| 596814 | 744 | 760 | | eekkdddddddddkkee | 35 | 9894 | 9910 | 1363 |
| 596624 | 745 | 761 | | eekkddddddddkkeee | 40 | 9895 | 9911 | 1364 |
| 596815 | 745 | 761 | | eekkdddddddddkkee | 54 | 9895 | 9911 | 1364 |
| 596625 | 746 | 762 | | eekkddddddddkkeee | 42 | 9896 | 9912 | 1365 |
| 596816 | 746 | 762 | | eekkdddddddddkkee | 46 | 9896 | 9912 | 1365 |
| 596626 | 747 | 763 | | eekkddddddddkkeee | 26 | 9897 | 9913 | 1366 |
| 596817 | 747 | 763 | | eekkdddddddddkkee | 37 | 9897 | 9913 | 1366 |
| 596627 | 748 | 764 | | eekkddddddddkkeee | 35 | 9898 | 9914 | 1367 |
| 596818 | 748 | 764 | | eekkdddddddddkkee | 45 | 9898 | 9914 | 1367 |
| 596628 | 749 | 765 | | eekkddddddddkkeee | 25 | 9899 | 9915 | 1368 |
| 596819 | 749 | 765 | | eekkdddddddddkkee | 38 | 9899 | 9915 | 1368 |
| 596629 | 750 | 766 | | eekkddddddddkkeee | 33 | 9900 | 9916 | 1369 |
| 596820 | 750 | 766 | | eekkdddddddddkkee | 50 | 9900 | 9916 | 1369 |
| 596630 | 751 | 767 | | eekkddddddddkkeee | 38 | 9901 | 9917 | 1370 |
| 596821 | 751 | 767 | | eekkdddddddddkkee | 38 | 9901 | 9917 | 1370 |
| 596631 | 752 | 768 | | eekkddddddddkkeee | 25 | 9902 | 9918 | 1371 |
| 596822 | 752 | 768 | | eekkdddddddddkkee | 43 | 9902 | 9918 | 1371 |
| 596632 | 753 | 769 | | eekkddddddddkkeee | 31 | 9903 | 9919 | 1372 |
| 596823 | 753 | 769 | | eekkdddddddddkkee | 44 | 9903 | 9919 | 1372 |
| 596633 | 754 | 770 | | eekkddddddddkkeee | 34 | 9904 | 9920 | 1373 |
| 596824 | 754 | 770 | | eekkdddddddddkkee | 53 | 9904 | 9920 | 1373 |
| 596634 | 761 | 777 | | eekkddddddddkkeee | 34 | 9911 | 9927 | 1374 |
| 596825 | 761 | 777 | | eekkdddddddddkkee | 38 | 9911 | 9927 | 1374 |
| 596635 | 762 | 778 | | eekkddddddddkkeee | 49 | 9912 | 9928 | 1375 |
| 596826 | 762 | 778 | | eekkdddddddddkkee | 38 | 9912 | 9928 | 1375 |
| 596636 | 763 | 779 | | eekkddddddddkkeee | 33 | 9913 | 9929 | 1376 |
| 596827 | 763 | 779 | | eekkdddddddddkkee | 48 | 9913 | 9929 | 1376 |
| 596637 | 764 | 780 | | eekkddddddddkkeee | 23 | 9914 | 9930 | 1377 |
| 596828 | 764 | 780 | | eekkdddddddddkkee | 32 | 9914 | 9930 | 1377 |
| 596638 | 766 | 782 | | eekkddddddddkkeee | 47 | 9916 | 9932 | 1378 |
| 596829 | 766 | 782 | | eekkdddddddddkkee | 29 | 9916 | 9932 | 1378 |
| 596639 | 767 | 783 | | eekkddddddddkkeee | 40 | 9917 | 9933 | 1379 |
| 596830 | 767 | 783 | | eekkdddddddddkkee | 48 | 9917 | 9933 | 1379 |
| 596640 | 768 | 784 | | eekkddddddddkkeee | 42 | 9918 | 9934 | 1380 |
| 596831 | 768 | 784 | | eekkdddddddddkkee | 39 | 9918 | 9934 | 1380 |
| 596641 | 770 | 786 | | eekkddddddddkkeee | 40 | 9920 | 9936 | 1381 |
| 596832 | 770 | 786 | | eekkdddddddddkkee | 54 | 9920 | 9936 | 1381 |
| 596642 | 771 | 787 | | eekkddddddddkkeee | 33 | 9921 | 9937 | 1382 |
| 596833 | 771 | 787 | | eekkdddddddddkkee | 43 | 9921 | 9937 | 1382 |
| 596643 | 772 | 788 | | eekkddddddddkkeee | 38 | 9922 | 9938 | 1184 |
| 596834 | 772 | 788 | | eekkdddddddddkkee | 38 | 9922 | 9938 | 1184 |

**Table 22**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 333611 | 167 | 186 | | eeeeeddddddddddeeeee | 62 | 973 | 992 | 21 |
| 596720 | 167 | 183 | | eekkddddddddkkeee | 53 | 973 | 989 | 966 |
| 596911 | 167 | 183 | | eekkdddddddddkkee | 58 | 973 | 989 | 966 |
| 596644 | 773 | 789 | | eekkddddddddkkeee | 19 | 9923 | 9939 | 1185 |
| 596835 | 773 | 789 | | eekkdddddddddkkee | 38 | 9923 | 9939 | 1185 |
| 596645 | 774 | 790 | | eekkddddddddkkeee | 46 | 9924 | 9940 | 1186 |
| 596836 | 774 | 790 | | eekkdddddddddkkee | 48 | 9924 | 9940 | 1186 |
| 596646 | 782 | 798 | | eekkddddddddkkeee | 60 | 9932 | 9948 | 1193 |
| 596837 | 782 | 798 | | eekkdddddddddkkee | 63 | 9932 | 9948 | 1193 |
| 596647 | 783 | 799 | | eekkddddddddkkeee | 55 | 9933 | 9949 | 1194 |
| 596838 | 783 | 799 | | eekkdddddddddkkee | 55 | 9933 | 9949 | 1194 |
| 596648 | 806 | 822 | | eekkddddddddkkeee | 46 | 9956 | 9972 | 1383 |
| 596839 | 806 | 822 | | eekkdddddddddkkee | 53 | 9956 | 9972 | 1383 |
| 596649 | 817 | 833 | | eekkddddddddkkeee | 2 | 9967 | 9983 | 1201 |
| 596840 | 817 | 833 | | eekkdddddddddkkee | 15 | 9967 | 9983 | 1201 |
| 596650 | 819 | 835 | | eekkddddddddkkeee | 40 | 9969 | 9985 | 1203 |
| 596841 | 819 | 835 | | eekkdddddddddkkee | 44 | 9969 | 9985 | 1203 |
| 596651 | 822 | 838 | | eekkddddddddkkeee | 26 | 9972 | 9988 | 1206 |
| 596842 | 822 | 838 | | eekkdddddddddkkee | 38 | 9972 | 9988 | 1206 |
| 596652 | 823 | 839 | | eekkddddddddkkeee | 33 | 9973 | 9989 | 1207 |
| 596843 | 823 | 839 | | eekkdddddddddkkee | 22 | 9973 | 9989 | 1207 |
| 596653 | 825 | 841 | | eekkddddddddkkeee | 28 | 9975 | 9991 | 1209 |
| 596844 | 825 | 841 | | eekkdddddddddkkee | 47 | 9975 | 9991 | 1209 |
| 596654 | 827 | 843 | | eekkddddddddkkeee | 44 | 9977 | 9993 | 1211 |
| 596845 | 827 | 843 | | eekkdddddddddkkee | 56 | 9977 | 9993 | 1211 |
| 596655 | 830 | 846 | | eekkddddddddkkeee | 33 | 9980 | 9996 | 1214 |
| 596846 | 830 | 846 | | eekkdddddddddkkee | 43 | 9980 | 9996 | 1214 |
| 596656 | 831 | 847 | | eekkddddddddkkeee | 25 | 9981 | 9997 | 1215 |
| 596847 | 831 | 847 | | eekkdddddddddkkee | 53 | 9981 | 9997 | 1215 |
| 596657 | 833 | 849 | | eekkddddddddkkeee | 30 | 9983 | 9999 | 1217 |
| 596848 | 833 | 849 | | eekkdddddddddkkee | 38 | 9983 | 9999 | 1217 |
| 596658 | 836 | 852 | | eekkddddddddkkeee | 24 | 9986 | 10002 | 1220 |
| 596849 | 836 | 852 | | eekkdddddddddkkee | 46 | 9986 | 10002 | 1220 |
| 596659 | 837 | 853 | | eekkddddddddkkeee | 27 | 9987 | 10003 | 1221 |
| 596850 | 837 | 853 | | eekkdddddddddkkee | 42 | 9987 | 10003 | 1221 |
| 596660 | 840 | 856 | | eekkddddddddkkeee | 19 | 9990 | 10006 | 1384 |
| 596851 | 840 | 856 | | eekkdddddddddkkee | 35 | 9990 | 10006 | 1384 |
| 596661 | 841 | 857 | | eekkddddddddkkeee | 52 | 9991 | 10007 | 1385 |
| 596852 | 841 | 857 | | eekkdddddddddkkee | 52 | 9991 | 10007 | 1385 |
| 596662 | 842 | 858 | | eekkddddddddkkeee | 54 | 9992 | 10008 | 1386 |
| 596853 | 842 | 858 | | eekkdddddddddkkee | 69 | 9992 | 10008 | 1386 |
| 596663 | 843 | 859 | | eekkddddddddkkeee | 45 | 9993 | 10009 | 1387 |
| 596854 | 843 | 859 | | eekkdddddddddkkee | 58 | 9993 | 10009 | 1387 |
| 596664 | 844 | 860 | | eekkddddddddkkeee | n.d. | 9994 | 10010 | 1388 |
| 596855 | 844 | 860 | | eekkdddddddddkkee | 61 | 9994 | 10010 | 1388 |
| 596665 | 845 | 861 | | eekkddddddddkkeee | 49 | 9995 | 10011 | 1389 |
| 596856 | 845 | 861 | | eekkdddddddddkkee | 49 | 9995 | 10011 | 1389 |
| 596666 | 846 | 862 | | eekkddddddddkkeee | 35 | 9996 | 10012 | 1390 |
| 596857 | 846 | 862 | | eekkdddddddddkkee | 37 | 9996 | 10012 | 1390 |
| 596667 | 847 | 863 | | eekkddddddddkkeee | 42 | 9997 | 10013 | 1391 |
| 596858 | 847 | 863 | | eekkdddddddddkkee | 48 | 9997 | 10013 | 1391 |
| 596668 | 848 | 864 | | eekkddddddddkkeee | 46 | 9998 | 10014 | 1392 |
| 596859 | 848 | 864 | | eekkdddddddddkkee | 47 | 9998 | 10014 | 1392 |
| 596669 | 849 | 865 | | eekkddddddddkkeee | 49 | 9999 | 10015 | 1393 |
| 596860 | 849 | 865 | | eekkdddddddddkkee | 49 | 9999 | 10015 | 1393 |
| 596670 | 850 | 866 | | eekkddddddddkkeee | 33 | 10000 | 10016 | 1394 |
| 596861 | 850 | 866 | | eekkdddddddddkkee | 44 | 10000 | 10016 | 1394 |
| 596671 | 851 | 867 | | eekkddddddddkkeee | 29 | 10001 | 10017 | 1395 |
| 596862 | 851 | 867 | | eekkdddddddddkkee | 45 | 10001 | 10017 | 1395 |
| 596672 | 854 | 870 | | eekkddddddddkkeee | 25 | 10004 | 10020 | 1223 |
| 596863 | 854 | 870 | | eekkdddddddddkkee | 28 | 10004 | 10020 | 1223 |
| 596673 | 855 | 871 | | eekkddddddddkkeee | 28 | 10005 | 10021 | 1224 |
| 596864 | 855 | 871 | | eekkdddddddddkkee | 26 | 10005 | 10021 | 1224 |
| 596674 | 858 | 874 | | eekkddddddddkkeee | 29 | 10008 | 10024 | 1227 |
| 596865 | 858 | 874 | | eekkdddddddddkkee | 43 | 10008 | 10024 | 1227 |
| 596675 | 859 | 875 | | eekkddddddddkkeee | 54 | 10009 | 10025 | 1228 |
| 596866 | 859 | 875 | | eekkdddddddddkkee | 59 | 10009 | 10025 | 1228 |
| 596676 | 860 | 876 | | eekkddddddddkkeee | 52 | 10010 | 10026 | 1229 |
| 596867 | 860 | 876 | | eekkdddddddddkkee | 62 | 10010 | 10026 | 1229 |
| 596677 | 861 | 877 | | eekkddddddddkkeee | 58 | 10011 | 10027 | 1230 |
| 596868 | 861 | 877 | | eekkdddddddddkkee | 61 | 10011 | 10027 | 1230 |
| 596678 | 862 | 878 | | eekkddddddddkkeee | 54 | 10012 | 10028 | 1231 |
| 596869 | 862 | 878 | | eekkdddddddddkkee | 59 | 10012 | 10028 | 1231 |
| 596679 | 863 | 879 | | eekkddddddddkkeee | 43 | 10013 | 10029 | 1232 |
| 596870 | 863 | 879 | | eekkdddddddddkkee | 52 | 10013 | 10029 | 1232 |
| 596680 | 864 | 880 | | eekkddddddddkkeee | 30 | 10014 | 10030 | 1233 |
| 596871 | 864 | 880 | | eekkdddddddddkkee | 36 | 10014 | 10030 | 1233 |
| 596681 | 865 | 881 | | eekkddddddddkkeee | 33 | 10015 | 10031 | 1234 |
| 596872 | 865 | 881 | | eekkdddddddddkkee | 20 | 10015 | 10031 | 1234 |

**Table 23**

| Percent inhibition of SOD-1 mRNA by deoxy, MOE and cEt gapmers targeting SEQ ID NO: 1 and/or 2 | | | | | | | | |
|---|---|---|---|---|---|---|---|---|
| ISIS NO | SEQ ID NO: 1 Start Site | SEQ ID NO: 1 Stop Site | Sequence | Chemistry | % inhibition | SEQ ID NO: 2 Start Site | SEQ ID NO: 2 Stop Site | SEQ ID NO |
| 333611 | 167 | 186 | | eeeeeddddddddddeeeee | 68 | 973 | 992 | 21 |
| 596720 | 167 | 183 | | eekkddddddddkkeee | 64 | 973 | 989 | 966 |
| 596911 | 167 | 183 | | eekkdddddddddkkee | 71 | 973 | 989 | 966 |
| 596682 | 884 | 900 | | eekkddddddddkkeee | 24 | 10034 | 10050 | 1396 |
| 596873 | 884 | 900 | | eekkdddddddddkkee | 32 | 10034 | 10050 | 1396 |
| 596683 | 885 | 901 | | eekkddddddddkkeee | 54 | 10035 | 10051 | 1397 |
| 596874 | 885 | 901 | | eekkdddddddddkkee | 44 | 10035 | 10051 | 1397 |
| 596684 | 889 | 905 | | eekkddddddddkkeee | 34 | 10039 | 10055 | 1398 |
| 596875 | 889 | 905 | | eekkdddddddddkkee | 47 | 10039 | 10055 | 1398 |
| 596685 | 890 | 906 | | eekkddddddddkkeee | 28 | 10040 | 10056 | 1399 |
| 596876 | 890 | 906 | | eekkdddddddddkkee | 46 | 10040 | 10056 | 1399 |
| 596686 | 891 | 907 | | eekkddddddddkkeee | 20 | 10041 | 10057 | 1242 |
| 596877 | 891 | 907 | | eekkdddddddddkkee | 16 | 10041 | 10057 | 1242 |
| 596687 | 892 | 908 | | eekkddddddddkkeee | 19 | 10042 | 10058 | 1243 |
| 596878 | 892 | 908 | | eekkdddddddddkkee | 29 | 10042 | 10058 | 1243 |
| 596688 | 893 | 909 | | eekkddddddddkkeee | 24 | 10043 | 10059 | 1244 |
| 596879 | 893 | 909 | | eekkdddddddddkkee | 11 | 10043 | 10059 | 1244 |
| 596689 | 894 | 910 | | eekkddddddddkkeee | 26 | 10044 | 10060 | 1245 |
| 596880 | 894 | 910 | | eekkdddddddddkkee | 30 | 10044 | 10060 | 1245 |
| 596690 | 895 | 911 | | eekkddddddddkkeee | 44 | 10045 | 10061 | 1246 |
| 596881 | 895 | 911 | | eekkdddddddddkkee | 55 | 10045 | 10061 | 1246 |
| 596691 | 896 | 912 | | eekkddddddddkkeee | 43 | 10046 | 10062 | 1247 |
| 596882 | 896 | 912 | | eekkdddddddddkkee | 48 | 10046 | 10062 | 1247 |
| 596692 | 899 | 915 | | eekkddddddddkkeee | 38 | 10049 | 10065 | 1250 |
| 596883 | 899 | 915 | | eekkdddddddddkkee | 57 | 10049 | 10065 | 1250 |
| 596693 | 903 | 919 | | eekkddddddddkkeee | 29 | 10053 | 10069 | 1254 |
| 596884 | 903 | 919 | | eekkdddddddddkkee | 47 | 10053 | 10069 | 1254 |
| 596694 | 904 | 920 | | eekkddddddddkkeee | 13 | 10054 | 10070 | 1255 |
| 596885 | 904 | 920 | | eekkdddddddddkkee | 31 | 10054 | 10070 | 1255 |
| 596695 | 907 | 923 | | eekkddddddddkkeee | 13 | 10057 | 10073 | 1258 |
| 596886 | 907 | 923 | | eekkdddddddddkkee | 34 | 10057 | 10073 | 1258 |
| 596696 | 908 | 924 | | eekkddddddddkkeee | 13 | 10058 | 10074 | 1259 |
| 596887 | 908 | 924 | | eekkdddddddddkkee | 26 | 10058 | 10074 | 1259 |
| 596697 | 909 | 925 | | eekkddddddddkkeee | 21 | 10059 | 10075 | 1260 |
| 596888 | 909 | 925 | | eekkdddddddddkkee | 22 | 10059 | 10075 | 1260 |
| 596698 | 910 | 926 | | eekkddddddddkkeee | 20 | 10060 | 10076 | 1261 |
| 596889 | 910 | 926 | | eekkdddddddddkkee | 28 | 10060 | 10076 | 1261 |
| 596699 | 911 | 927 | | eekkddddddddkkeee | 20 | 10061 | 10077 | 1262 |
| 596890 | 911 | 927 | | eekkdddddddddkkee | 27 | 10061 | 10077 | 1262 |
| 596700 | 912 | 928 | | eekkddddddddkkeee | 15 | 10062 | 10078 | 1400 |
| 596891 | 912 | 928 | | eekkdddddddddkkee | 21 | 10062 | 10078 | 1400 |
| 596701 | 913 | 929 | | eekkddddddddkkeee | 26 | 10063 | 10079 | 1401 |
| 596892 | 913 | 929 | | eekkdddddddddkkee | 35 | 10063 | 10079 | 1401 |
| 596702 | 914 | 930 | | eekkddddddddkkeee | 36 | 10064 | 10080 | 1402 |
| 596893 | 914 | 930 | | eekkdddddddddkkee | 46 | 10064 | 10080 | 1402 |
| 596703 | 915 | 931 | | eekkddddddddkkeee | 40 | 10065 | 10081 | 1403 |
| 596894 | 915 | 931 | | eekkdddddddddkkee | 36 | 10065 | 10081 | 1403 |
| 596704 | 916 | 932 | | eekkddddddddkkeee | 22 | 10066 | 10082 | 1404 |
| 596895 | 916 | 932 | | eekkdddddddddkkee | 30 | 10066 | 10082 | 1404 |
| 596705 | 917 | 933 | | eekkddddddddkkeee | 27 | 10067 | 10083 | 1405 |
| 596896 | 917 | 933 | | eekkdddddddddkkee | 27 | 10067 | 10083 | 1405 |
| 596706 | 918 | 934 | | eekkddddddddkkeee | 32 | 10068 | 10084 | 1406 |
| 596897 | 918 | 934 | | eekkdddddddddkkee | 34 | 10068 | 10084 | 1406 |
| 596707 | 919 | 935 | | eekkddddddddkkeee | 28 | 10069 | 10085 | 1407 |
| 596898 | 919 | 935 | | eekkdddddddddkkee | 34 | 10069 | 10085 | 1407 |
| 596708 | 920 | 936 | | eekkddddddddkkeee | 30 | 10070 | 10086 | 1408 |
| 596899 | 920 | 936 | | eekkdddddddddkkee | 44 | 10070 | 10086 | 1408 |
| 596709 | 921 | 937 | | eekkddddddddkkeee | 29 | 10071 | 10087 | 1409 |
| 596900 | 921 | 937 | | eekkdddddddddkkee | 31 | 10071 | 10087 | 1409 |
| 596710 | 922 | 938 | | eekkddddddddkkeee | 41 | 10072 | 10088 | 1410 |
| 596901 | 922 | 938 | | eekkdddddddddkkee | 33 | 10072 | 10088 | 1410 |
| 596711 | 923 | 939 | | eekkddddddddkkeee | 16 | 10073 | 10089 | 1411 |
| 596902 | 923 | 939 | | eekkdddddddddkkee | 11 | 10073 | 10089 | 1411 |
| 596712 | 924 | 940 | | eekkddddddddkkeee | 11 | 10074 | 10090 | 1412 |
| 596903 | 924 | 940 | | eekkdddddddddkkee | 27 | 10074 | 10090 | 1412 |
| 596713 | 925 | 941 | | eekkddddddddkkeee | 20 | 10075 | 10091 | 1413 |
| 596904 | 925 | 941 | | eekkdddddddddkkee | 27 | 10075 | 10091 | 1413 |
| 596714 | 926 | 942 | | eekkddddddddkkeee | 20 | 10076 | 10092 | 1414 |
| 596905 | 926 | 942 | | eekkdddddddddkkee | 25 | 10076 | 10092 | 1414 |
| 596715 | 931 | 947 | | eekkddddddddkkeee | 45 | 10081 | 10097 | 1415 |
| 596906 | 931 | 947 | | eekkdddddddddkkee | 34 | 10081 | 10097 | 1415 |
| 596716 | 932 | 948 | | eekkddddddddkkeee | 52 | 10082 | 10098 | 1416 |
| 596907 | 932 | 948 | | eekkdddddddddkkee | 56 | 10082 | 10098 | 1416 |
| 596717 | 936 | 952 | | eekkddddddddkkeee | 14 | 10086 | 10102 | 1417 |
| 596908 | 936 | 952 | | eekkdddddddddkkee | 19 | 10086 | 10102 | 1417 |
| 596718 | 938 | 954 | | eekkddddddddkkeee | 23 | 10088 | 10104 | 1263 |
| 596909 | 938 | 954 | | eekkdddddddddkkee | 8 | 10088 | 10104 | 1263 |
| 596719 | 949 | 965 | | eekkddddddddkkeee | 31 | 10099 | 10115 | 1418 |
| 596910 | 949 | 965 | | eekkdddddddddkkee | 16 | 10099 | 10115 | 1418 |

### Example 6: Dose-dependent inhibition of human SOD-1 with modified oligonucleotides in HepG2 cells

Gapmers from the studies described above exhibiting significant *in vitro* inhibition of SOD-1 mRNA were selected and tested at various doses in HepG2 cells. Benchmark compound ISIS 333611 and other compounds previously disclosed in WO 2005/040180, including ISIS 146144, 146145, 150445, 150446, 150447, 150454, 150463, 150465, 333606, 333609, and 333611 were also tested.

The modified oligonucleotides were tested in a series of experiments that had similar culture conditions. The results for each experiment are presented in separate tables shown below. Cells were plated at a density of 20,000 cells per well and transfected using electroporation with 0.813 µM, 1.625 µM, 3.250 µM, 6.500 µM, and 13.000 µM concentrations of modified oligonucleotide, as specified in the Tables below. After a treatment period of approximately 16 hours, RNA was isolated from the cells and SOD-1 mRNA levels were measured by quantitative real-time PCR. Human primer probe set RTS3898 was used to measure mRNA levels. SOD-1 mRNA levels were adjusted according to total RNA content, as measured by RIBOGREEN^{®}. Results are presented as percent inhibition of SOD-1, relative to untreated control cells.

The half maximal inhibitory concentration (IC₅₀) of each oligonucleotide is also presented. SOD-1 mRNA levels were significantly reduced in a dose-dependent manner in modified oligonucleotide treated cells.

**Table 24**

| **Dose-dependent inhibition of SOD-1 mRNA** | | | | | | |
|---|---|---|---|---|---|---|
| ISIS No | 0.813 µM | 1.625 µM | 3.250 µM | 6.500 µM | 13.000 µM | IC₅₀ (µM) |
| 150445 | 7 | 21 | 44 | 56 | 82 | 4.4 |
| 150446 | 15 | 32 | 47 | 71 | 87 | 3.2 |
| 150447 | 26 | 43 | 68 | 81 | 93 | 2.0 |
| 150463 | 16 | 38 | 51 | 66 | 85 | 3.1 |
| 333611 | 18 | 39 | 57 | 66 | 79 | 3.0 |
| 393336 | 18 | 34 | 53 | 69 | 83 | 3.1 |
| 393343 | 24 | 32 | 53 | 73 | 42 | 5.1 |
| 436863 | 18 | 42 | 58 | 72 | 89 | 2.6 |
| 590089 | 28 | 59 | 70 | 82 | 90 | 1.5 |
| 590090 | 34 | 56 | 76 | 82 | 51 | 1.1 |
| 590091 | 30 | 44 | 68 | 84 | 88 | 1.9 |
| 590094 | 16 | 35 | 57 | 73 | 76 | 3.0 |
| 590113 | 34 | 35 | 57 | 73 | 84 | 2.3 |

**Table 25**

| **Dose-dependent inhibition of SOD-1 mRNA** | | | | | | |
|---|---|---|---|---|---|---|
| ISIS No | 0.813 µM | 1.625 µM | 3.250 µM | 6.500 µM | 13.000 µM | IC₅₀ (µM) |
| 150465 | 42 | 59 | 77 | 82 | 87 | 1.0 |
| 333611 | 17 | 26 | 40 | 64 | 82 | 3.8 |
| 378879 | 14 | 35 | 63 | 72 | 86 | 2.8 |
| 393371 | 28 | 42 | 57 | 74 | 80 | 2.3 |
| 489520 | 28 | 44 | 64 | 72 | 84 | 2.2 |
| 590177 | 53 | 59 | 69 | 85 | 88 | 0.7 |
| 590178 | 40 | 53 | 71 | 73 | 87 | 1.3 |
| 590180 | 18 | 42 | 51 | 64 | 73 | 3.3 |
| 590187 | 34 | 51 | 68 | 80 | 92 | 1.6 |
| 590188 | 30 | 46 | 61 | 76 | 88 | 2.0 |
| 590189 | 37 | 49 | 68 | 78 | 88 | 1.6 |
| 590190 | 38 | 58 | 77 | 84 | 89 | 1.1 |
| 590191 | 29 | 56 | 71 | 77 | 84 | 1.6 |
| 590192 | 37 | 59 | 72 | 80 | 87 | 1.2 |

**Table 26**

| **Dose-dependent inhibition of SOD-1 mRNA** | | | | | | |
|---|---|---|---|---|---|---|
| ISIS No | 0.813 µM | 1.625 µM | 3.250 µM | 6.500 µM | 13.000 µM | IC₅₀ (µM) |
| 146144 | 15 | 58 | 67 | 78 | 64 | 2.2 |
| 146145 | 31 | 53 | 67 | 81 | 90 | 1.6 |
| 333606 | 11 | 39 | 62 | 75 | 91 | 2.7 |
| 333609 | 13 | 37 | 57 | 71 | 85 | 2.9 |
| 333611 | 14 | 30 | 53 | 68 | 86 | 3.2 |
| 590250 | 8 | 19 | 47 | 64 | 84 | 3.9 |
| 590626 | 61 | 72 | 84 | 84 | 87 | 0.2 |
| 592630 | 24 | 33 | 58 | 70 | 85 | 2.7 |
| 592631 | 19 | 48 | 59 | 74 | 88 | 2.3 |
| 592645 | 20 | 31 | 53 | 74 | 89 | 2.8 |
| 592649 | 14 | 32 | 56 | 69 | 82 | 3.2 |

**Table 27**

| **Dose-dependent inhibition of SOD-1 mRNA** | | | | | | |
|---|---|---|---|---|---|---|
| ISIS No | 0.813 µM | 1.625 µM | 3.250 µM | 6.500 µM | 13.000 µM | IC₅₀ (µM) |
| 150454 | 13 | 24 | 49 | 69 | 83 | 3.5 |
| 333611 | 28 | 28 | 53 | 68 | 82 | 3.0 |
| 489505 | 13 | 24 | 38 | 56 | 81 | 4.5 |
| 489516 | 25 | 16 | 39 | 61 | 79 | 4.3 |
| 592652 | 11 | 31 | 52 | 74 | 46 | 5.1 |
| 592714 | 8 | 25 | 45 | 69 | 82 | 3.8 |
| 592715 | 18 | 35 | 50 | 70 | 83 | 3.1 |
| 592762 | 44 | 66 | 74 | 81 | 89 | 0.8 |
| 592763 | 50 | 68 | 74 | 86 | 95 | 0.7 |
| 592764 | 26 | 43 | 48 | 76 | 87 | 2.5 |
| 592766 | 36 | 53 | 66 | 77 | 89 | 1.5 |
| 592767 | 25 | 31 | 54 | 70 | 79 | 2.9 |
| 592769 | 35 | 31 | 56 | 73 | 80 | 2.5 |
| 592771 | 34 | 43 | 58 | 70 | 80 | 2.2 |

### Example 7: Dose-dependent inhibition of human SOD-1 with modified oligonucleotides in HepG2 cells

Gapmers from the studies described above exhibiting significant *in vitro* inhibition of SOD-1 mRNA were selected and tested at various doses in HepG2 cells. Benchmark compound, ISIS 333611, and ISIS 333625, both of which were previously disclosed in WO 2005/040180 were also tested.

The modified oligonucleotides were tested in a series of experiments that had similar culture conditions. The results for each experiment are presented in separate tables shown below. Cells were plated at a density of 20,000 cells per well and transfected using electroporation with 0.148 µM, 0.444 µM, 1.330 µM, 4.000 µM, and 12.000 µM concentrations of modified oligonucleotide, as specified in the Tables below. After a treatment period of approximately 16 hours, RNA was isolated from the cells and SOD-1 mRNA levels were measured by quantitative real-time PCR. Human primer probe sets RTS3898 or HTS90 was used to measure mRNA levels. SOD-1 mRNA levels were adjusted according to total RNA content, as measured by RIBOGREEN^{®}. Results are presented as percent inhibition of SOD-1, relative to untreated control cells.

The half maximal inhibitory concentration (IC₅₀) of each oligonucleotide is also presented. SOD-1 mRNA levels were significantly reduced in a dose-dependent manner in modified oligonucleotide treated cells.

**Table 28**

| **Dose response assay with primer probe set RTS3898** | | | | | | |
|---|---|---|---|---|---|---|
| ISIS No | 0.148 µM | 0.444 µM | 1.330 µM | 4.000 µM | 12.000 µM | IC50 (µM) |
| 333611 | 6 | 14 | 29 | 51 | 78 | 3.2 |
| 596911 | 8 | 12 | 23 | 54 | 74 | 3.6 |
| 596720 | 7 | 14 | 31 | 55 | 71 | 3.4 |
| 596800 | 44 | 60 | 75 | 84 | 82 | 0.2 |
| 596801 | 33 | 49 | 69 | 79 | 83 | 0.5 |
| 596610 | 16 | 44 | 65 | 78 | 84 | 0.8 |
| 596799 | 20 | 45 | 64 | 75 | 84 | 0.8 |
| 596609 | 17 | 54 | 65 | 75 | 81 | 0.7 |
| 596883 | 13 | 22 | 36 | 45 | 51 | 8.6 |
| 489523 | 16 | 40 | 62 | 78 | 90 | 0.9 |
| 590181 | 5 | 17 | 46 | 70 | 89 | 1.7 |
| 436868 | 10 | 35 | 47 | 69 | 82 | 1.4 |
| 596768 | 16 | 37 | 62 | 82 | 89 | 0.9 |
| 596775 | 36 | 50 | 66 | 83 | 89 | 0.4 |

**Table 29**

| **Dose response assay with primer probe set HTS90** | | | | | | |
|---|---|---|---|---|---|---|
| ISIS No | 0.148 µM | 0.444 µM | 1.330 µM | 4.000 µM | 12.000 µM | IC₅₀ (µM) |
| 333625 | 0 | 4 | 17 | 56 | 84 | 3.3 |
| 489532 | 54 | 70 | 78 | 86 | 96 | 0.1 |
| 590154 | 0 | 14 | 25 | 56 | 86 | 2.7 |
| 596173 | 0 | 5 | 25 | 63 | 92 | 2.4 |
| 596174 | 7 | 12 | 37 | 46 | 84 | 2.8 |
| 596178 | 2 | 16 | 40 | 68 | 82 | 2.1 |
| 596179 | 0 | 17 | 41 | 64 | 80 | 2.3 |
| 596308 | 0 | 5 | 22 | 56 | 80 | 3.3 |
| 596572 | 18 | 35 | 62 | 83 | 90 | 0.9 |
| 596589 | 10 | 45 | 61 | 77 | 91 | 0.9 |
| 596600 | 41 | 56 | 71 | 85 | 92 | 0.3 |
| 596602 | 14 | 51 | 74 | 86 | 95 | 0.6 |
| 596789 | 22 | 55 | 69 | 86 | 91 | 0.5 |
| 596795 | 16 | 43 | 64 | 82 | 93 | 0.8 |

### Example 8: Dose-dependent inhibition of human SOD-1 with modified oligonucleotides in HepG2 cells

Gapmers from the studies described above exhibiting significant *in vitro* inhibition of SOD-1 mRNA were selected and tested at various doses in HepG2 cells. Benchmark compound, ISIS 333611, and additional compounds including, ISIS 146143, 150442, 195753, 333607, and 333608, were previously disclosed in WO 2005/040180 were also tested.

The modified oligonucleotides were tested in a series of experiments that had similar culture conditions. The results for each experiment are presented in separate tables shown below. Cells were plated at a density of 20,000 cells per well and transfected using electroporation with 0.1875 µM, 0.7500 µM, 3.0000 µM, and 12.0000 µM concentrations of modified oligonucleotide, as specified in the Tables below. After a treatment period of approximately 16 hours, RNA was isolated from the cells and SOD-1 mRNA levels were measured by quantitative real-time PCR. Human primer probe sets RTS3898 was used to measure mRNA levels. SOD-1 mRNA levels were adjusted according to total RNA content, as measured by RIBOGREEN^{®}. Results are presented as percent inhibition of SOD-1, relative to untreated control cells.

The half maximal inhibitory concentration (IC₅₀) of each oligonucleotide is also presented. SOD-1 mRNA levels were significantly reduced in a dose-dependent manner in modified oligonucleotide treated cells.

**Table 30**

| **Dose-dependent inhibition of SOD-1 mRNA** | | | | | |
|---|---|---|---|---|---|
| ISIS No | 0.1875 µM | 0.75 µM | 3.00 µM | 12.00 µM | IC₅₀ (µM) |
| 333611 | 0 | 27 | 60 | 91 | 2 |
| 596572 | 38 | 65 | 87 | 96 | 0.3 |
| 596583 | 62 | 89 | 95 | 95 | <0.1 |
| 596590 | 40 | 79 | 89 | 94 | 0.2 |
| 596602 | 40 | 75 | 92 | 98 | 0.2 |
| 596603 | 51 | 79 | 92 | 96 | 0.1 |
| 596604 | 48 | 78 | 91 | 95 | 0.1 |
| 596605 | 50 | 86 | 90 | 97 | 0.1 |
| 596764 | 11 | 67 | 89 | 94 | 0.7 |
| 596768 | 27 | 49 | 82 | 92 | 0.7 |
| 596773 | 32 | 62 | 89 | 98 | 0.4 |
| 596774 | 56 | 89 | 93 | 96 | <0.1 |
| 596775 | 31 | 75 | 90 | 97 | 0.3 |
| 596780 | 24 | 71 | 85 | 98 | 0.5 |
| 596781 | 30 | 80 | 93 | 97 | 0.3 |
| 596791 | 38 | 74 | 89 | 95 | 0.3 |
| 596794 | 43 | 75 | 91 | 97 | 0.2 |
| 596795 | 28 | 66 | 91 | 98 | 0.4 |
| 596796 | 43 | 78 | 93 | 98 | 0.2 |

**Table 31**

| **Dose-dependent inhibition of SOD-1 mRNA** | | | | | |
|---|---|---|---|---|---|
| ISIS No | 0.1875 µM | 0.75 µM | 3.00 µM | 12.00 µM | IC₅₀ (µM) |
| 333611 | 0 | 15 | 68 | 95 | 2.1 |
| 596589 | 35 | 70 | 90 | 97 | 0.3 |
| 596789 | 38 | 71 | 89 | 97 | 0.3 |
| 596600 | 41 | 73 | 89 | 95 | 0.2 |
| 596582 | 30 | 71 | 87 | 95 | 0.4 |
| 596784 | 26 | 68 | 89 | 95 | 0.4 |
| 596787 | 44 | 67 | 89 | 94 | 0.2 |
| 596779 | 29 | 71 | 89 | 97 | 0.4 |
| 596792 | 37 | 63 | 83 | 95 | 0.4 |
| 596782 | 27 | 61 | 85 | 96 | 0.5 |
| 596765 | 34 | 59 | 87 | 95 | 0.4 |
| 596793 | 27 | 65 | 88 | 96 | 0.5 |
| 596570 | 25 | 60 | 84 | 91 | 0.6 |
| 596769 | 21 | 64 | 85 | 96 | 0.6 |
| 596783 | 10 | 57 | 84 | 94 | 0.9 |
| 596584 | 26 | 67 | 84 | 93 | 0.5 |
| 596571 | 37 | 71 | 81 | 92 | 0.3 |
| 596598 | 30 | 62 | 81 | 94 | 0.5 |
| 596588 | 11 | 64 | 87 | 95 | 0.7 |

**Table 32**

| **Dose-dependent inhibition of SOD-1 mRNA** | | | | | |
|---|---|---|---|---|---|
| ISIS No | 0.1875 µM | 0.75 µM | 3.00 µM | 12.00 µM | IC₅₀ (µM) |
| 146143 | 6 | 12 | 51 | 88 | 2.5 |
| 150442 | 10 | 21 | 39 | 90 | 2.5 |
| 195753 | 13 | 23 | 48 | 77 | 2.8 |
| 333607 | 17 | 26 | 59 | 83 | 1.9 |
| 333608 | 0 | 2 | 28 | 92 | 3.7 |
| 333611 | 0 | 13 | 52 | 91 | 2.4 |
| 596573 | 26 | 51 | 77 | 91 | 0.8 |
| 596577 | 32 | 55 | 78 | 93 | 0.6 |
| 596591 | 23 | 51 | 74 | 91 | 0.8 |
| 596592 | 18 | 48 | 66 | 86 | 1.1 |
| 596593 | 16 | 58 | 78 | 87 | 0.8 |
| 596596 | 4 | 49 | 72 | 87 | 1.3 |
| 596761 | 28 | 55 | 74 | 91 | 0.7 |
| 596762 | 0 | 47 | 75 | 90 | 1.3 |
| 596763 | 0 | 40 | 78 | 92 | 1.5 |
| 596766 | 4 | 50 | 68 | 86 | 1.3 |
| 596785 | 10 | 47 | 77 | 91 | 1.1 |
| 596786 | 0 | 45 | 75 | 97 | 1.3 |
| 596788 | 9 | 52 | 81 | 95 | 1 |

**Table 33**

| **Dose-dependent inhibition of SOD-1 mRNA** | | | | | |
|---|---|---|---|---|---|
| ISIS No | 0.1875 µM | 0.75 µM | 3.00 µM | 12.00 µM | IC₅₀ (µM) |
| 333611 | 3 | 22 | 60 | 92 | 2 |
| 596302 | 9 | 27 | 59 | 89 | 1.9 |
| 596308 | 29 | 47 | 82 | 96 | 0.7 |
| 596309 | 13 | 42 | 75 | 92 | 1.1 |
| 596310 | 13 | 16 | 48 | 81 | 2.8 |
| 596313 | 15 | 37 | 70 | 88 | 1.3 |
| 596314 | 18 | 45 | 74 | 92 | 1 |
| 596606 | 55 | 78 | 87 | 93 | 0.1 |
| 596607 | 44 | 71 | 83 | 84 | 0.2 |
| 596608 | 46 | 74 | 84 | 81 | 0.1 |
| 596609 | 30 | 61 | 79 | 87 | 0.5 |
| 596610 | 39 | 69 | 82 | 86 | 0.3 |
| 596612 | 16 | 50 | 62 | 77 | 1.4 |
| 596797 | 67 | 84 | 94 | 96 | <0.1 |
| 596798 | 42 | 68 | 86 | 89 | 0.2 |
| 596799 | 35 | 66 | 83 | 87 | 0.4 |
| 596800 | 45 | 73 | 84 | 87 | 0.2 |
| 596801 | 40 | 67 | 86 | 88 | 0.3 |
| 596803 | 28 | 41 | 63 | 71 | 1.4 |

**Table 34**

| Dose-dependent inhibition of SOD-1 mRNA | | | | | |
|---|---|---|---|---|---|
| ISIS No | 0.1875 µM | 0.75 µM | 3.00 µM | 12.00 µM | IC50 (µM) |
| 333611 | 0 | 30 | 56 | 69 | 3.1 |
| 590475 | 19 | 39 | 69 | 88 | 1.2 |
| 590625 | 19 | 51 | 74 | 85 | 1.0 |
| 590626 | 42 | 72 | 88 | 90 | 0.2 |
| 590627 | 16 | 42 | 70 | 84 | 1.0 |
| 590634 | 45 | 72 | 86 | 92 | 0.2 |
| 590635 | 39 | 60 | 80 | 90 | 0.4 |
| 590644 | 44 | 62 | 80 | 86 | 0.3 |
| 590650 | 34 | 56 | 82 | 93 | 0.5 |
| 590653 | 52 | 78 | 86 | 85 | 0.1 |
| 590655 | 35 | 71 | 79 | 82 | 0.3 |
| 596530 | 25 | 22 | 72 | 79 | 2.0 |
| 596531 | 8 | 38 | 74 | 96 | 1.2 |
| 596559 | 15 | 36 | 79 | 95 | 1.1 |
| 596721 | 14 | 47 | 82 | 97 | 0.9 |
| 596723 | 12 | 47 | 79 | 94 | 1.0 |
| 596726 | 24 | 42 | 80 | 94 | 0.9 |
| 596735 | 7 | 32 | 77 | 96 | 1.3 |
| 596736 | 25 | 52 | 82 | 97 | 0.7 |

### Example 9: Dose-dependent inhibition of human SOD-1 in HepG2 cells by gapmers with mixed backbone chemistry

Additional gapmers were designed based on the sequences of the oligonucleotides disclosed in studies described above. The oligonucleotides were designed as 5-10-5 MOE, 5-8-5 MOE, and deoxy, MOE and cEt oligonucleotides. The 5-10-5 MOE gapmers are 20 nucleosides in length, wherein the central gap segment is comprised of ten 2'-deoxyribonucleosides and is flanked by wing segments on the 5' direction and the 3' direction comprising five nucleosides each. The 5-8-5 MOE gapmers are 18 nucleosides in length, wherein the central gap segment is comprised of eight 2'-deoxyribonucleosides and is flanked by wing segments on the 5' direction and the 3' direction comprising five nucleosides each. Each nucleoside in the 5' wing segment and each nucleoside in the 3' wing segment has a 2'-MOE modification. The deoxy, MOE and cEt oligonucleotides are 16 or 17 nucleosides in length wherein each nucleoside has a MOE sugar modification, a cEt sugar modification, or a deoxy moiety. The sugar chemistry of each oligonucleotide is denoted as in the Chemistry column, where 'k' indicates a cEt modified sugar; 'd' indicates a 2'-deoxyribose; and 'e' indicates a 2'-MOE modified sugar. The internucleoside linkages throughout each gapmer are either phosphodiester or phosphorothioate linkages. The internucleoside linkages of each oligonucleotide are denoted in the Backbone Chemistry column, where 'o' indicates a phosphodiester linkage and 's' denotes a phosphorothioate linkage. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000).

**Table 35**

| **Modified oligonucleotides targeting human SOD-1 with mixed backbone chemistry** | | | | | | | | |
|---|---|---|---|---|---|---|---|---|
| ISIS NO | SEQ ID NO: 1 Start Site | SEQ ID NO: 1 Stop Site | Sequence | Sugar Modifications | Backbone Chemistry | SEQ ID NO: 2 Start Site | SEQ ID NO: 2 Stop Site | SEQ ID NO |
| 611458 | 226 | 245 | | eeeeeddddddddddeeeee | sooosssssssssssooos | 4980 | 4999 | 23 |
| 654335 | 233 | 248 | | ekddddddddekekee | sossssssssoooss | 4987 | 5002 | 1420 |
| 611474 | 234 | 253 | | eeeeeddddddddddeeeee | sooosssssssssssooos | 4988 | 5007 | 149 |
| 654301 | 234 | 251 | | eeeeeddddddddeeeee | sooosssssssssooss | 4988 | 5005 | 1421 |
| 654336 | 234 | 249 | | ekddddddddekekee | sossssssssoooss | 4988 | 5003 | 1422 |
| 654302 | 235 | 252 | | eeeeeddddddddeeeee | sooosssssssssooss | 4989 | 5006 | 1423 |
| 654319 | 235 | 250 | | kekeddddddddekek | sooossssssssoss | 4989 | 5004 | 1424 |
| 654337 | 235 | 250 | | ekddddddddekekee | sossssssssoooss | 4989 | 5004 | 1424 |
| 654303 | 236 | 253 | | eeeeeddddddddeeeee | sooosssssssssooss | 4990 | 5007 | 1425 |
| 654320 | 236 | 251 | | kekeddddddddekek | sooossssssssoss | 4990 | 5005 | 1426 |
| 654321 | 237 | 252 | | kekeddddddddekek | sooossssssssoss | 4991 | 5006 | 1427 |
| 611475 | 321 | 340 | | eeeeeddddddddddeeeee | sooosssssssssssooos | 7637 | 7656 | 172 |
| 611460 | 588 | 607 | | eeeeeddddddddddeeeee | sooosssssssssssooos | 9738 | 9757 | 47 |
| 654304 | 663 | 680 | | eeeeeddddddddeeeee | sooosssssssssooss | 9813 | 9830 | 1429 |
| 654305 | 664 | 681 | | eeeeeddddddddeeeee | sooosssssssssooss | 9814 | 9831 | 1430 |
| 654340 | 664 | 679 | | ekddddddddekekee | sossssssssoooss | 9814 | 9829 | 1431 |
| 611492 | 665 | 684 | | eeeeeddddddddddeeeee | sooosssssssssssooos | 9815 | 9834 | 725 |
| 654306 | 665 | 682 | | eeeeeddddddddeeeee | sooosssssssssooss | 9815 | 9832 | 1432 |
| 654323 | 665 | 680 | | kekeddddddddekek | sooossssssssoss | 9815 | 9830 | 1433 |
| 654341 | 665 | 680 | | ekddddddddekekee | sossssssssoooss | 9815 | 9830 | 1433 |
| 611500 | 666 | 685 | | eeeeeddddddddddeeeee | sooosssssssssssooos | 9816 | 9835 | 823 |
| 612941 | 666 | 682 | | eekkdddddddddkkee | sooosssssssssoss | 9816 | 9832 | 1342 |
| 654307 | 666 | 683 | | eeeeeddddddddeeeee | sooosssssssssooss | 9816 | 9833 | 1434 |
| 654324 | 666 | 681 | | kekeddddddddekek | sooossssssssoss | 9816 | 9831 | 1435 |
| 654342 | 666 | 681 | | ekddddddddekekee | sossssssssoooss | 9816 | 9831 | 1435 |
| 654308 | 667 | 684 | | eeeeeddddddddeeeee | sooosssssssssooss | 9817 | 9834 | 1436 |
| 612925 | 676 | 692 | | eekkddddddddkkeee | soosssssssssooss | 9826 | 9842 | 1348 |
| 612944 | 676 | 692 | | eekkdddddddddkkee | sooosssssssssoss | 9826 | 9842 | 1348 |
| 654343 | 677 | 692 | | ekddddddddekekee | sossssssssoooss | 9827 | 9842 | 1437 |
| 612927 | 678 | 694 | | eekkddddddddkkeee | soosssssssssooss | 9828 | 9844 | 1350 |
| 654309 | 678 | 695 | | eeeeeddddddddeeeee | sooosssssssssooss | 9828 | 9845 | 1438 |
| 612928 | 679 | 695 | | eekkddddddddkkeee | soosssssssssooss | 9829 | 9845 | 1351 |
| 654310 | 679 | 696 | | eeeeeddddddddeeeee | sooosssssssssooss | 9829 | 9846 | 1439 |
| 654311 | 680 | 697 | | eeeeeddddddddeeeee | sooosssssssssooss | 9830 | 9847 | 1440 |
| 654327 | 680 | 695 | | kekeddddddddekek | sooossssssssoss | 9830 | 9845 | 1441 |
| 654346 | 680 | 695 | | ekddddddddekekee | sossssssssoooss | 9830 | 9845 | 1441 |
| 611497 | 681 | 700 | | eeeeeddddddddddeeeee | sooosssssssssssooos | 9831 | 9850 | 733 |
| 612948 | 681 | 697 | | eekkdddddddddkkee | sooosssssssssoss | 9831 | 9847 | 1352 |
| 654312 | 681 | 698 | | eeeeeddddddddeeeee | sooosssssssssooss | 9831 | 9848 | 1442 |
| 654328 | 681 | 696 | | kekeddddddddekek | sooossssssssoss | 9831 | 9846 | 1443 |
| 654347 | 681 | 696 | | ekddddddddekekee | sossssssssoooss | 9831 | 9846 | 1443 |
| 654313 | 682 | 699 | | eeeeeddddddddeeeee | sooosssssssssooss | 9832 | 9849 | 1444 |
| 654329 | 682 | 697 | | kekeddddddddekek | sooossssssssoss | 9832 | 9847 | 1445 |
| 654348 | 682 | 697 | | ekddddddddekekee | sossssssssoooss | 9832 | 9847 | 1445 |
| 612949 | 683 | 699 | | eekkdddddddddkkee | sooosssssssssoss | 9833 | 9849 | 1172 |
| 654314 | 683 | 700 | | eeeeeddddddddeeeee | sooosssssssssooss | 9833 | 9850 | 1446 |
| 654330 | 683 | 698 | | kekeddddddddekek | sooossssssssoss | 9833 | 9848 | 1447 |
| 612931 | 684 | 700 | | eekkddddddddkkeee | soosssssssssooss | 9834 | 9850 | 1173 |
| 654315 | 684 | 701 | | eeeeeddddddddeeeee | sooosssssssssooss | 9834 | 9851 | 1448 |
| 654331 | 684 | 699 | | kekeddddddddekek | sooossssssssoss | 9834 | 9849 | 1449 |
| 654350 | 684 | 699 | | ekddddddddekekee | sossssssssoooss | 9834 | 9849 | 1449 |
| 654316 | 685 | 702 | | eeeeeddddddddeeeee | sooosssssssssooss | 9835 | 9852 | 1450 |
| 612918 | 686 | 702 | | eeekkdddddddkkeee | soosssssssssooss | 9836 | 9852 | 1175 |
| 612932 | 686 | 702 | | eekkddddddddkkeee | soosssssssssooss | 9836 | 9852 | 1175 |
| 654317 | 686 | 703 | | eeeeeddddddddeeeee | sooosssssssssooss | 9836 | 9853 | 1451 |
| 654333 | 686 | 701 | | kekeddddddddekek | sooossssssssoss | 9836 | 9851 | 1452 |
| 654352 | 686 | 701 | | ekddddddddekekee | sossssssssoooss | 9836 | 9851 | 1452 |
| 654318 | 687 | 704 | | eeeeeddddddddeeeee | sooosssssssssooss | 9837 | 9854 | 1453 |
| 654334 | 687 | 702 | | kekeddddddddekek | sooossssssssoss | 9837 | 9852 | 1454 |

The newly designed oligonucleotides were tested at various doses in HepG2 cells. The modified oligonucleotides were tested in a series of experiments that had similar culture conditions. The results for each experiment are presented in separate tables shown below. Cells were plated at a density of 20,000 cells per well and transfected using electroporation with 0.222 µM, 0.667 µM, 2.000 µM, and 6.000 µM concentrations of modified oligonucleotide, as specified in the Tables below. After a treatment period of approximately 16 hours, RNA was isolated from the cells and SOD-1 mRNA levels were measured by quantitative real-time PCR. Human primer probe sets RTS3898 was used to measure mRNA levels. SOD-1 mRNA levels were adjusted according to total RNA content, as measured by RIBOGREEN^{®}. Results are presented as percent inhibition of SOD-1, relative to untreated control cells.

The half maximal inhibitory concentration (IC₅₀) of each oligonucleotide is also presented. SOD-1 mRNA levels were significantly reduced in a dose-dependent manner in modified oligonucleotide treated cells.

**Table 36**

| **Dose response assay** | | | | | |
|---|---|---|---|---|---|
| ISIS No | 0.222 µM | 0.667 µM | 2.000 µM | 6.000 µM | IC₅₀ µM |
| 333611 | 0 | 28 | 53 | 77 | 1.9 |
| 611458 | 0 | 21 | 50 | 79 | 2.0 |
| 611460 | 24 | 34 | 55 | 79 | 1.3 |
| 611474 | 14 | 32 | 55 | 79 | 1.5 |
| 611475 | 0 | 16 | 35 | 70 | 3.0 |
| 611492 | 38 | 70 | 88 | 95 | 0.3 |
| 611497 | 28 | 55 | 80 | 89 | 0.6 |
| 611500 | 25 | 50 | 73 | 92 | 0.7 |
| 612918 | 51 | 70 | 74 | 80 | <0.2 |
| 612925 | 53 | 73 | 90 | 89 | <0.2 |
| 612927 | 64 | 89 | 92 | 94 | <0.2 |
| 612928 | 67 | 90 | 94 | 97 | <0.2 |
| 612931 | 68 | 76 | 84 | 86 | <0.2 |
| 612932 | 61 | 73 | 88 | 91 | <0.2 |
| 612941 | 62 | 78 | 91 | 95 | <0.2 |
| 612944 | 47 | 71 | 82 | 92 | 0.2 |
| 612948 | 76 | 90 | 93 | 94 | <0.2 |
| 612949 | 58 | 68 | 83 | 96 | <0.2 |
| 654301 | 7 | 4 | 17 | 42 | >6.0 |

**Table 37**

| **Dose response assay** | | | | | |
|---|---|---|---|---|---|
| ISIS No | 0.222 µM | 0.667 µM | 2.000 µM | 6.000 µM | IC₅₀ µM |
| 333611 | 14 | 20 | 35 | 69 | 3.0 |
| 611458 | 11 | 27 | 36 | 68 | 2.9 |
| 654302 | 0 | 8 | 38 | 48 | 6.2 |
| 654303 | 8 | 29 | 46 | 76 | 1.9 |
| 654304 | 7 | 28 | 54 | 79 | 1.7 |
| 654305 | 28 | 59 | 73 | 85 | 0.6 |
| 654306 | 38 | 62 | 82 | 94 | 0.4 |
| 654307 | 9 | 43 | 65 | 86 | 1.1 |
| 654308 | 14 | 31 | 54 | 84 | 1.4 |
| 654309 | 0 | 17 | 47 | 72 | 2.4 |
| 654310 | 10 | 24 | 28 | 53 | 6.6 |
| 654311 | 45 | 73 | 78 | 87 | 0.2 |
| 654312 | 14 | 39 | 59 | 77 | 1.3 |
| 654313 | 20 | 43 | 56 | 81 | 1.2 |
| 654314 | 33 | 58 | 74 | 86 | 0.5 |
| 654315 | 21 | 47 | 64 | 84 | 0.9 |
| 654316 | 19 | 30 | 46 | 70 | 2.0 |
| 654317 | 13 | 46 | 57 | 70 | 1.4 |
| 654318 | 17 | 42 | 54 | 76 | 1.4 |

**Table 38**

| **Dose response assay** | | | | | |
|---|---|---|---|---|---|
| ISIS No | 0.222 µM | 0.667 µM | 2.000 µM | 6.000 µM | IC₅₀ µM |
| 333611 | 14 | 19 | 50 | 73 | 2.1 |
| 611458 | 9 | 22 | 39 | 72 | 2.5 |
| 654319 | 19 | 9 | 31 | 61 | 5.1 |
| 654320 | 6 | 16 | 20 | 59 | 5.9 |
| 654321 | 8 | 14 | 51 | 76 | 2.1 |
| 654323 | 55 | 73 | 89 | 95 | <0.2 |
| 654324 | 54 | 78 | 89 | 96 | <0.2 |
| 654327 | 53 | 82 | 91 | 96 | <0.2 |
| 654328 | 73 | 90 | 93 | 97 | <0.2 |
| 654329 | 58 | 78 | 86 | 94 | <0.2 |
| 654330 | 42 | 54 | 69 | 86 | 0.4 |
| 654331 | 53 | 78 | 82 | 90 | <0.2 |
| 654333 | 50 | 67 | 81 | 86 | 0.2 |
| 654334 | 55 | 68 | 78 | 88 | <0.2 |
| 654335 | 15 | 31 | 58 | 81 | 1.4 |
| 654336 | 21 | 36 | 60 | 75 | 1.3 |
| 654337 | 16 | 34 | 58 | 80 | 1.4 |
| 654340 | 36 | 69 | 83 | 95 | 0.4 |
| 654341 | 43 | 58 | 79 | 91 | 0.3 |

**Table 39**

| **Dose response assay** | | | | | |
|---|---|---|---|---|---|
| ISIS No | 0.222 µM | 0.667 µM | 2.00 µM | 6.00 µM | IC₅₀ µM |
| 333611 | 0 | 6 | 38 | 64 | 3.6 |
| 611458 | 3 | 14 | 36 | 63 | 3.6 |
| 654342 | 40 | 60 | 80 | 93 | 0.4 |
| 654343 | 64 | 81 | 90 | 94 | <0.2 |
| 654346 | 52 | 73 | 84 | 93 | <0.2 |
| 654347 | 21 | 38 | 58 | 81 | 1.2 |
| 654348 | 44 | 63 | 82 | 94 | 0.3 |
| 654350 | 40 | 63 | 76 | 86 | 0.3 |
| 654352 | 54 | 79 | 84 | 88 | <0.2 |

### Example 10: Dose-dependent inhibition of human SOD-1 by gapmers with mixed backbone chemistry

Additional gapmers were designed based on the sequences of the oligonucleotides disclosed in studies described above. The oligonucleotides were designed as deoxy, MOE and cEt oligonucleotides. The deoxy, MOE and cEt oligonucleotides are 16 or 17 nucleosides in length wherein each nucleoside has a MOE sugar modification, a cEt sugar modification, or a deoxy moiety. The sugar chemistry of each oligonucleotide is denoted as in the Chemistry column, where 'k' indicates a cEt modified sugar; 'd' indicates a 2'-deoxyribose; and 'e' indicates a 2'-MOE modified sugar. The internucleoside linkages throughout each gapmer are either phosphodiester or phosphorothioate linkages. The internucleoside linkages of each oligonucleotide are denoted in the Backbone Chemistry column, where 'o' indicates a phosphodiester linkage and 's' indicates a phosphorothioate linkage. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000).

**Table 40**

| **Modified oligonucleotides targeting human SOD-1 with mixed backbone chemistry** | | | | | | | | |
|---|---|---|---|---|---|---|---|---|
| ISIS NO | SEQ ID NO: 1 Start Site | SEQ ID NO: 1 Stop Site | Sequence | Sugar Modifications | Backbone Chemistry | SEQ ID NO: 2 Start Site | SEQ ID NO: 2 Stop Site | SEQ ID NO |
| 612916 | 664 | 680 | | eeekkdddddddkkeee | soosssssssssooss | 9814 | 9830 | 1170 |
| 612947 | 679 | 695 | | eekkdddddddddkkee | sooosssssssssoss | 9829 | 9845 | 1351 |
| 654322 | 664 | 679 | | kekeddddddddekek | sooossssssssoss | 9814 | 9829 | 1431 |
| 654325 | 667 | 682 | | kekeddddddddekek | sooossssssssoss | 9817 | 9832 | 1455 |
| 654326 | 679 | 694 | | kekeddddddddekek | sooossssssssoss | 9829 | 9844 | 1456 |
| 654332 | 685 | 700 | | kekeddddddddekek | sooossssssssoss | 9835 | 9850 | 1457 |
| 654338 | 662 | 677 | | ekddddddddekekee | sossssssssoooss | 9812 | 9827 | 1458 |
| 654339 | 663 | 678 | | ekddddddddekekee | sossssssssoooss | 9813 | 9828 | 1459 |
| 654344 | 678 | 693 | | ekddddddddekekee | sossssssssoooss | 9828 | 9843 | 1460 |
| 654345 | 679 | 694 | | ekddddddddekekee | sossssssssoooss | 9829 | 9844 | 1456 |
| 654349 | 683 | 698 | | ekddddddddekekee | sossssssssoooss | 9833 | 9848 | 1447 |
| 654351 | 685 | 700 | | ekddddddddekekee | sossssssssoooss | 9835 | 9850 | 1457 |

The newly designed oligonucleotides were tested at various doses in A431 cells. The modified oligonucleotides were tested in a series of experiments that had similar culture conditions. The results for each experiment are presented in separate tables shown below. Cells were plated at a density of 5,000 cells per well and modified oligonucleotides were added to the media at 0.12 µM, 0.60 µM, 3.00 µM, and 15.00 µM concentrations of modified oligonucleotide for free uptake by the cells, as specified in the Tables below. After a treatment period of approximately 16 hours, RNA was isolated from the cells and SOD-1 mRNA levels were measured by quantitative real-time PCR. Human primer probe sets RTS3898 was used to measure mRNA levels. SOD-1 mRNA levels were adjusted according to total RNA content, as measured by RIBOGREEN^{®}. Results are presented as percent inhibition of SOD-1, relative to untreated control cells.

**Table 41**

| **Dose response assay** | | | | |
|---|---|---|---|---|
| ISIS No | 0.12 µM | 0.60 µM | 3.00 µM | 15.00 µM |
| 333611 | 0 | 7 | 17 | 31 |
| 611458 | 5 | 4 | 4 | 10 |
| 612916 | 4 | 13 | 26 | 37 |
| 612918 | 12 | 26 | 45 | 47 |
| 612944 | 1 | 4 | 21 | 29 |
| 612947 | 12 | 48 | 54 | 72 |
| 654305 | 7 | 15 | 39 | 52 |
| 654306 | 0 | 25 | 29 | 50 |
| 654313 | 1 | 15 | 36 | 50 |
| 654314 | 2 | 35 | 52 | 67 |
| 654321 | 0 | 8 | 8 | 18 |
| 654322 | 0 | 13 | 36 | 59 |
| 654329 | 7 | 7 | 41 | 66 |
| 654330 | 6 | 14 | 15 | 32 |
| 654337 | 0 | 0 | 7 | 21 |
| 654338 | 3 | 3 | 2 | 11 |
| 654345 | 1 | 9 | 22 | 46 |
| 654346 | 0 | 7 | 21 | 46 |

**Table 42**

| **Dose response assay** | | | | |
|---|---|---|---|---|
| ISIS No | 0.12 µM | 0.60 µM | 3.00 µM | 15.00 µM |
| 333611 | 0 | 0 | 0 | 2 |
| 611460 | 0 | 0 | 0 | 30 |
| 611474 | 0 | 0 | 11 | 0 |
| 612925 | 2 | 4 | 12 | 52 |
| 612927 | 0 | 38 | 54 | 68 |
| 612948 | 25 | 69 | 89 | 95 |
| 612949 | 22 | 57 | 73 | 84 |
| 654307 | 42 | 23 | 26 | 45 |
| 654308 | 2 | 31 | 9 | 18 |
| 654315 | 0 | 8 | 39 | 52 |
| 654316 | 0 | 18 | 26 | 45 |
| 654323 | 15 | 14 | 16 | 52 |
| 654324 | 12 | 22 | 21 | 34 |
| 654331 | 7 | 35 | 66 | 78 |
| 654332 | 2 | 31 | 47 | 61 |
| 654339 | 1 | 27 | 32 | 47 |
| 654340 | 37 | 0 | 22 | 12 |
| 654347 | 20 | 5 | 12 | 33 |
| 654348 | 2 | 19 | 33 | 62 |

**Table 43**

| **Dose response assay** | | | | |
|---|---|---|---|---|
| ISIS No | 0.12 µM | 0.60 µM | 3.00 µM | 15.00 µM |
| 333611 | 0 | 0 | 0 | 1 |
| 611475 | 0 | 17 | 0 | 16 |
| 611492 | 13 | 24 | 41 | 62 |
| 612928 | 12 | 36 | 49 | 72 |
| 612931 | 31 | 68 | 83 | 86 |
| 654301 | 2 | 0 | 0 | 9 |
| 654302 | 0 | 8 | 3 | 0 |
| 654309 | 18 | 7 | 11 | 9 |
| 654310 | 13 | 19 | 22 | 7 |
| 654317 | 3 | 0 | 1 | 20 |
| 654318 | 4 | 0 | 33 | 17 |
| 654325 | 0 | 0 | 0 | 14 |
| 654326 | 0 | 15 | 17 | 48 |
| 654333 | 19 | 18 | 36 | 55 |
| 654334 | 0 | 0 | 0 | 6 |
| 654341 | 0 | 9 | 0 | 25 |
| 654342 | 0 | 0 | 0 | 18 |
| 654349 | 0 | 13 | 31 | 49 |
| 654350 | 10 | 32 | 66 | 79 |

**Table 44**

| **Dose response assay** | | | | |
|---|---|---|---|---|
| ISIS No | 0.12 µM | 0.60 µM | 3.00 µM | 15.00 µM |
| 333611 | 5 | 0 | 7 | 3 |
| 611497 | 16 | 49 | 60 | 75 |
| 611500 | 9 | 8 | 21 | 49 |
| 612932 | 17 | 8 | 26 | 37 |
| 612941 | 4 | 12 | 36 | 51 |
| 654303 | 0 | 1 | 0 | 5 |
| 654304 | 9 | 10 | 27 | 43 |
| 654311 | 15 | 51 | 68 | 84 |
| 654312 | 6 | 26 | 29 | 33 |
| 654319 | 3 | 44 | 2 | 8 |
| 654320 | 4 | 12 | 5 | 12 |
| 654327 | 3 | 45 | 65 | 81 |
| 654328 | 15 | 44 | 73 | 85 |
| 654335 | 2 | 0 | 0 | 12 |
| 654336 | 0 | 0 | 0 | 0 |
| 654343 | 0 | 7 | 26 | 59 |
| 654344 | 10 | 30 | 51 | 72 |
| 654351 | 10 | 22 | 48 | 77 |
| 654352 | 8 | 26 | 57 | 76 |

### Example 11: Dose-dependent inhibition of human SOD-1 by gapmers with mixed backbone chemistry

Additional gapmers were designed based on the sequences of the oligonucleotides disclosed in studies described above. The oligonucleotides were designed as 5-10-5 MOE gapmers, 4-8-5 MOE gapmers, 5-8-5 MOE gapmers, 5-8-7 MOE gapmers, 6-8-6 MOE gapmers, 6-9-5 MOE gapmers, or deoxy, MOE and cEt oligonucleotides.

The 5-10-5 MOE gapmers are 20 nucleosides in length, wherein the central gap segment is comprised of ten 2'-deoxynucleosides and is flanked by wing segments on the 5' direction and the 3' directions comprising five nucleosides each. The 4-8-5 MOE gapmers are 17 nucleosides in length, wherein the central gap segment is comprised of eight 2'-deoxyribonucleosides and is flanked by wing segments on the 5' direction and the 3' directions comprising four and five nucleosides respectively. The 5-8-5 MOE gapmers are 18 nucleosides in length, wherein the central gap segment is comprised of eight 2'-deoxynucleosides and is flanked by wing segments on the 5' direction and the 3' directions comprising five nucleosides each. The 5-8-7 MOE gapmers are 20 nucleosides in length, wherein the central gap segment is comprised of eight 2'-deoxynucleosides and is flanked by wing segments on the 5' direction and the 3' directions comprising five and seven nucleosides respectively. The 6-8-6 MOE gapmers are 20 nucleosides in length, wherein the central gap segment is comprised of eight 2'-deoxynucleosides and is flanked by wing segments on the 5' direction and the 3' directions comprising six nucleosides each. The 6-9-5 MOE gapmers are 20 nucleosides in length, wherein the central gap segment is comprised of nine 2'-deoxynucleosides and is flanked by wing segments on the 5' direction and the 3' directions comprising six and five nucleosides respectively. Each nucleoside in the 5' wing segment and each nucleoside in the 3' wing segment has a 2'-MOE modification.

The deoxy, MOE and cEt oligonucleotides are 17 nucleosides in length wherein each nucleoside has a MOE sugar modification, a cEt sugar modification, or a deoxy moiety. The sugar chemistry of each oligonucleotide is denoted as in the Chemistry column, where 'k' indicates a cEt modified sugar; 'd' indicates a 2'-deoxyribose; and 'e' indicates a 2'-MOE modified sugar.

The internucleoside linkages throughout each gapmer are either phosphodiester or phosphorothioate linkages. The internucleoside linkages of each oligonucleotide are denoted in the Backbone Chemistry column, where 'o' indicates a phosphodiester linkage and 's' indicates a phosphorothioate linkage. All cytosine residues throughout each gapmer are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Tables below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000).

**Table 45**

| **Modified oligonucleotides targeting human SOD-1 with mixed backbone chemistry** | | | | | | | | | |
|---|---|---|---|---|---|---|---|---|---|
| ISIS NO | SEQ ID NO: 1 Start Site | SEQ ID NO: 1 Stop Site | Sequence | Motif | Sugar Modifications | Backbone Chemistry | SEQ ID NO: 2 Start Site | SEQ ID NO: 2 Stop Site | SEQ ID NO |
| 666846 | 665 | 684 | | 5-10-5 MOE | eeeeeddddddddddeeeee | soooossssssssssooss | 9815 | 9834 | 725 |
| 666849 | 665 | 684 | | 5-10-5 MOE | eeeeeddddddddddeeeee | sooosssssssssssooss | 9815 | 9834 | 725 |
| 666853 | 665 | 684 | | 5-10-5 MOE | eeeeeddddddddddeeeee | sososssssssssssosos | 9815 | 9834 | 725 |
| 666859 | 679 | 695 | | Deoxy , MOE and cEt | eeeeddddddddkkeee | soosssssssssooss | 9829 | 9845 | 1351 |
| 666861 | 679 | 695 | | Deoxy , MOE and cEt | ekekddddddddeeeee | soosssssssssooss | 9829 | 9845 | 1351 |
| 666867 | 684 | 700 | | Deoxy , MOE and cEt | eekkddddddddeeeee | soosssssssssooss | 9834 | 9850 | 1173 |
| 666869 | 684 | 700 | | Deoxy , MOE and cEt | ekekddddddddkekee | soosssssssssooss | 9834 | 9850 | 1173 |
| 666870 | 684 | 700 | | Deoxy , MOE and cEt | ekekddddddddeeeee | soosssssssssooss | 9834 | 9850 | 1173 |
| 666919 | 666 | 682 | | Deoxy , MOE and cEt | eeeedddddddddkkee | sooosssssssssoss | 9816 | 9832 | 1342 |
| 666921 | 666 | 682 | | Deoxy , MOE and cEt | eeeeeddddddddkkee | sooosssssssssoss | 9816 | 9832 | 1342 |
| 666922 | 666 | 682 | | Deoxy , MOE and cEt | eeeekddddddddkeee | sooosssssssssoss | 9816 | 9832 | 1342 |
| 684059 | 676 | 692 | | Deoxy , MOE and cEt | eeekddddddddkeeee | soosssssssssooss | 9826 | 9842 | 1348 |
| 684064 | 676 | 692 | | Deoxy , MOE and cEt | eeeeddddddddkekee | soosssssssssooss | 9826 | 9842 | 1348 |
| 684068 | 676 | 692 | | 4-8-5 MOE | eeeeddddddddeeeee | soosssssssssooss | 9826 | 9842 | 1348 |
| 684087 | 590 | 607 | | 5-8-5 MOE | eeeeeddddddddeeeee | sooosssssssssooss | 9740 | 9757 | 613 |
| 684088 | 167 | 184 | | 5-8-5 MOE | eeeeeddddddddeeeee | sooosssssssssooss | 973 | 990 | 1419 |
| 684095 | 167 | 186 | | 5-10-5 MOE | eeeeeddddddddddeeeee | soooossssssssssooss | 973 | 992 | 21 |
| 684097 | 167 | 186 | | 5-8-7 MOE | eeeeeddddddddeeeeeee | sooossssssssssoooss | 973 | 992 | 21 |
| 684101 | 588 | 607 | | 6-8-6 MOE | eeeeeeddddddddeeeeee | sooossssssssssoooss | 9738 | 9757 | 47 |
| 684102 | 588 | 607 | | 5-8-7 MOE | eeeeeddddddddeeeeeee | sooossssssssssoooss | 9738 | 9757 | 47 |
| 684104 | 588 | 607 | | 6-9-5 MOE | eeeeeedddddddddeeeee | soooossssssssssooss | 9738 | 9757 | 47 |

The newly designed oligonucleotides were tested at various doses in A431 cells. The modified oligonucleotides were tested in a series of experiments that had similar culture conditions. The results for each experiment are presented in separate tables shown below. Cells were plated at a density of 5,000 cells per well and modified oligonucleotides were added to the media at 0.062 µM, 0.185 µM, 0.556 µM, 1.667 µM, 5.000 µM, and 15.000 µM concentrations of modified oligonucleotide for free uptake by the cells, as specified in the Tables below. After a treatment period of approximately 16 hours, RNA was isolated from the cells and SOD-1 mRNA levels were measured by quantitative real-time PCR. Human primer probe sets RTS3898 was used to measure mRNA levels. SOD-1 mRNA levels were adjusted according to total RNA content, as measured by RIBOGREEN^{®}. Results are presented as percent inhibition of SOD-1, relative to untreated control cells.

**Table 46**

| **Dose response assay** | | | | | | | |
|---|---|---|---|---|---|---|---|
| ISIS No | 0.062 µM | 0.185 µM | 0.556 µM | 1.667 µM | 5.000 µM | 15.000 µM | IC₅₀ (µM) |
| 666846 | 18 | 28 | 45 | 70 | 69 | 81 | 0.8 |
| 666919 | 0 | 1 | 13 | 28 | 42 | 55 | 11.0 |
| 666849 | 33 | 29 | 52 | 62 | 74 | 82 | 0.6 |
| 666921 | 2 | 4 | 15 | 19 | 37 | 44 | >15 |
| 666853 | 20 | 29 | 49 | 69 | 76 | 83 | 0.7 |
| 666922 | 8 | 7 | 30 | 33 | 66 | 59 | 4.1 |
| 666859 | 26 | 30 | 58 | 64 | 68 | 78 | 0.6 |
| 666861 | 6 | 21 | 44 | 76 | 68 | 77 | 1.1 |
| 666867 | 16 | 43 | 65 | 68 | 79 | 83 | 0.5 |
| 666869 | 52 | 68 | 79 | 88 | 89 | 91 | <0.06 |
| 666870 | 24 | 37 | 57 | 77 | 81 | 86 | 0.4 |

**Table 47**

| **Dose response assay** | | | | | | | |
|---|---|---|---|---|---|---|---|
| ISIS No | 0.062 µM | 0.185 µM | 0.556 µM | 1.667 µM | 5.000 µM | 15.000 µM | IC₅₀ (µM) |
| 684059 | 7 | 18 | 38 | 53 | 68 | 79 | 1.5 |
| 684102 | 0 | 9 | 0 | 0 | 4 | 0 | >15 |
| 684064 | 12 | 19 | 29 | 38 | 51 | 61 | 5.0 |
| 684104 | 0 | 0 | 0 | 0 | 0 | 4 | >15 |
| 684068 | 0 | 4 | 10 | 33 | 50 | 56 | 8.0 |
| 684087 | 3 | 1 | 29 | 0 | 0 | 27 | >15 |
| 684088 | 10 | 11 | 11 | 3 | 4 | 18 | >15 |
| 684095 | 12 | 13 | 14 | 4 | 7 | 18 | >15 |
| 684097 | 8 | 4 | 5 | 4 | 3 | 9 | >15 |
| 684101 | 7 | 0 | 0 | 23 | 6 | 14 | >15 |

The newly designed oligonucleotides were also tested at various doses in SH-SY5Y cells. The modified oligonucleotides were tested in a series of experiments that had similar culture conditions. The results for each experiment are presented in separate tables shown below. Cells were plated at a density of 20,000 cells per well and modified oligonucleotides transfected using electroporation at 0.062 µM, 0.185 µM, 0.556 µM, 1.667 µM, 5.000 µM, and 15.000 µM concentrations of modified oligonucleotide, as specified in the Tables below. After a treatment period of approximately 16 hours, RNA was isolated from the cells and SOD-1 mRNA levels were measured by quantitative real-time PCR. Human primer probe set RTS3898 was used to measure mRNA levels. SOD-1 mRNA levels were adjusted according to total RNA content, as measured by RIBOGREEN^{®}. Results are presented as percent inhibition of SOD-1, relative to untreated control cells.

**Table 48**

| **Dose response assay** | | | | | | | |
|---|---|---|---|---|---|---|---|
| ISIS No | 0.062 µM | 0.185 µM | 0.556 µM | 1.667 µM | 5.000 µM | 15.000 µM | IC₅₀ (µM) |
| 666846 | 0 | 17 | 49 | 62 | 83 | 91 | 0.9 |
| 666919 | 10 | 3 | 23 | 35 | 77 | 78 | 2.2 |
| 666849 | 10 | 10 | 33 | 61 | 81 | 92 | 1.1 |
| 666921 | 0 | 0 | 12 | 30 | 56 | 68 | 4.8 |
| 666853 | 0 | 17 | 39 | 59 | 85 | 82 | 1.3 |
| 666922 | 9 | 0 | 12 | 33 | 65 | 76 | 3.2 |
| 666859 | 11 | 44 | 53 | 75 | 77 | 93 | 0.5 |
| 666861 | 0 | 0 | 34 | 61 | 81 | 90 | 1.4 |
| 666867 | 33 | 10 | 43 | 61 | 81 | 77 | 0.9 |
| 666869 | 38 | 49 | 61 | 83 | 81 | 84 | 0.2 |
| 666870 | 3 | 6 | 48 | 69 | 77 | 87 | 1.1 |

**Table 49**

| **Dose response assay** | | | | | | | |
|---|---|---|---|---|---|---|---|
| ISIS No | 0.062 µM | 0.185 µM | 0.556 µM | 1.667 µM | 5.000 µM | 15.000 µM | IC₅₀ (µM) |
| 684059 | 4 | 30 | 51 | 68 | 88 | 92 | 0.7 |
| 684102 | 5 | 2 | 16 | 25 | 36 | 61 | 12.0 |
| 684064 | 15 | 27 | 52 | 63 | 79 | 92 | 0.7 |
| 684104 | 0 | 3 | 20 | 38 | 61 | 84 | 2.6 |
| 684068 | 0 | 4 | 32 | 37 | 61 | 83 | 2.3 |
| 684087 | 0 | 3 | 21 | 31 | 47 | 66 | 5.8 |
| 684088 | 13 | 4 | 5 | 40 | 52 | 77 | 3.9 |
| 684095 | 16 | 5 | 19 | 36 | 68 | 80 | 2.4 |
| 684097 | 11 | 15 | 9 | 30 | 59 | 76 | 3.6 |
| 684101 | 0 | 0 | 8 | 23 | 49 | 66 | 6.6 |

### Example 12: Inhibition of human SOD-1 in a transgenic rat model

Gapmers from the studies described above, including benchmark compound ISIS 333611, which was previously disclosed in WO 2005/040180, were tested in an SOD-1 transgenic rat model (Taconic, Cat# 2148-F and 2148-M). These hemizygous rats express mutant human SOD-1 in the spinal cord.

Additional gapmers were designed based on the sequences of the oligonucleotides disclosed in studies described above. The oligonucleotides were designed as 5-9-5 MOE gapmers, 5-10-5 MOE gapmers or deoxy, MOE and cEt oligonucleotides. The 5-9-5 MOE gapmers are 19 nucleosides in length, wherein the central gap segment is comprised of nine 2'-deoxyribonucleosides and is flanked by wing segments on the 5' direction and the 3' directions comprising five nucleosides each. The 5-10-5 MOE gapmers are 20 nucleosides in length, wherein the central gap segment is comprised often 2'-deoxyribonucleosides and is flanked by wing segments on the 5' direction and the 3' directions comprising five nucleosides each. Each nucleoside in the 5' wing segment and each nucleoside in the 3' wing segment has a 2'-MOE modification. The deoxy, MOE and cEt oligonucleotides are 17 nucleosides in length wherein each nucleoside has a MOE sugar modification, a cEt sugar modification, or a deoxy moiety The sugar chemistry of each oligonucleotide is denoted as in the Chemistry column, where 'k' indicates a cEt modified sugar; 'd' indicates a 2'-deoxyribose; and 'e' indicates a 2'-MOE modified sugar. The internucleoside linkages throughout each gapmer are either phosphodiester or phosphorothioate linkages. The internucleoside linkages of each oligonucleotide is denoted in the Backbone Chemistry column, where 'o' indicates a phosphodiester linkage and 's' indicates a phosphorothioate linkage. All cytosine residues throughout each oligonucleotide are 5-methylcytosines. "Start site" indicates the 5'-most nucleoside to which the gapmer is targeted in the human gene sequence. "Stop site" indicates the 3'-most nucleoside to which the gapmer is targeted human gene sequence. Each gapmer listed in the Table below is targeted to either the human SOD-1 mRNA, designated herein as SEQ ID NO: 1 (GENBANK Accession No. NM_000454.4) or the human SOD-1 genomic sequence, designated herein as SEQ ID NO: 2 (GENBANK Accession No. NT_011512.10 truncated from nucleotides 18693000 to 18704000).

**Table 50**

| **Modified oligonucleotides targeting human SOD-1 with mixed backbone chemistry** | | | | | | | | | |
|---|---|---|---|---|---|---|---|---|---|
| ISIS NO | SEQ ID NO: 1 Start Site | SEQ ID NO: 1 Stop Site | Sequence | Motif | Sugar Modifications | Backbone Chemistry | SEQ ID NO: 2 Start Site | SEQ ID NO: 2 Stop Site | SEQ ID NO |
| 383872 | 167 | 186 | | 5-10-5 MOE | eeeeeddddddddddeeeee | sooosssssssssssooos | 973 | 992 | 21 |
| 611457 | 165 | 184 | | 5-10-5 MOE | eeeeeddddddddddeeeee | sooosssssssssssooos | 971 | 990 | 54 |
| 611464 | 164 | 183 | | 5-10-5 MOE | eeeeeddddddddddeeeee | sooosssssssssssooos | 970 | 989 | 67 |
| 611467 | 656 | 675 | | 5-10-5 MOE | eeeeeddddddddddeeeee | sooosssssssssssooos | 9806 | 9825 | 272 |
| 611468 | 583 | 602 | | 5-10-5 MOE | eeeeeddddddddddeeeee | sooosssssssssssooos | 9733 | 9752 | 227 |
| 611472 | 230 | 249 | | 5-10-5 MOE | eeeeeddddddddddeeeee | sooosssssssssssooos | 4984 | 5003 | 145 |
| 611473 | 231 | 250 | | 5-10-5 MOE | eeeeeddddddddddeeeee | sooosssssssssssooos | 4985 | 5004 | 146 |
| 611478 | 644 | 663 | | 5-10-5 MOE | eeeeeddddddddddeeeee | sooosssssssssssooos | 9794 | 9813 | 260 |
| 611479 | 645 | 664 | | 5-10-5 MOE | eeeeeddddddddddeeeee | sooosssssssssssooos | 9795 | 9814 | 261 |
| 611481 | 655 | 674 | | 5-10-5 MOE | eeeeeddddddddddeeeee | sooosssssssssssooos | 9805 | 9824 | 271 |
| 611484 | 660 | 679 | | 5-10-5 MOE | eeeeeddddddddddeeeee | sooosssssssssssooos | 9810 | 9829 | 276 |
| 611485 | 661 | 680 | | 5-10-5 MOE | eeeeeddddddddddeeeee | sooosssssssssssooos | 9811 | 9830 | 277 |
| 611488 | 124 | 143 | | 5-10-5 MOE | eeeeeddddddddddeeeee | sooosssssssssssooos | 930 | 949 | 593 |
| 611490 | 402 | 421 | | 5-10-5 MOE | eeeeeddddddddddeeeee | sooosssssssssssooos | 8457 | 8476 | 666 |
| 611494 | 671 | 690 | | 5-10-5 MOE | eeeeeddddddddddeeeee | sooosssssssssssooos | 9821 | 9840 | 728 |
| 611495 | 673 | 692 | | 5-10-5 MOE | eeeeeddddddddddeeeee | sooosssssssssssooos | 9823 | 9842 | 729 |
| 611498 | 569 | 588 | | 5-10-5 MOE | eeeeeddddddddddeeeee | sooosssssssssssooos | 9719 | 9738 | 816 |
| 611499 | 664 | 683 | | 5-10-5 MOE | eeeeeddddddddddeeeee | sooosssssssssssooos | 9814 | 9833 | 822 |
| 612912 | 621 | 637 | | Deoxy, MOE, and cEt | eeekkdddddddkkeee | soosssssssssooss | 9771 | 9787 | 1146 |
| 612915 | 656 | 672 | | Deoxy, MOE, and cEt | eeekkdddddddkkeee | soosssssssssooss | 9806 | 9822 | 1164 |
| 612917 | 684 | 700 | | Deoxy, MOE, and cEt | eeekkdddddddkkeee | soosssssssssooss | 9834 | 9850 | 1173 |
| 612919 | 621 | 637 | | Deoxy, MOE, and cEt | eekkddddddddkkeee | soosssssssssooss | 9771 | 9787 | 1146 |
| 612923 | 656 | 672 | | Deoxy, MOE, and cEt | eekkddddddddkkeee | soosssssssssooss | 9806 | 9822 | 1164 |
| 612924 | 674 | 690 | | Deoxy, MOE, and cEt | eekkddddddddkkeee | soosssssssssooss | 9824 | 9840 | 1346 |
| 612934 | 170 | 186 | | Deoxy, MOE, and cEt | eekkdddddddddkkee | sooosssssssssoss | 976 | 992 | 969 |
| 612935 | 585 | 601 | | Deoxy, MOE, and cEt | eekkdddddddddkkee | sooosssssssssoss | 9735 | 9751 | 1114 |
| 612940 | 659 | 675 | | Deoxy, MOE, and cEt | eekkdddddddddkkee | sooosssssssssoss | 9809 | 9825 | 1167 |
| 612942 | 668 | 684 | | Deoxy, MOE, and cEt | eekkdddddddddkkee | sooosssssssssoss | 9818 | 9834 | 1344 |
| 612943 | 674 | 690 | | Deoxy, MOE, and cEt | eekkdddddddddkkee | sooosssssssssoss | 9824 | 9840 | 1346 |
| 666854 | 665 | 681 | | 5-9-5 MOE | eeeeedddddddddeeeee | sooossssssssssooss | 9815 | 9831 | 1428 |
| 666855 | 666 | 682 | | 5-9-5 MOE | eeeeedddddddddeeeee | sooossssssssssooss | 9816 | 9832 | 1461 |
| 666857 | 679 | 695 | | Deoxy, MOE, and cEt | eeekddddddddkeeee | soosssssssssooss | 9829 | 9845 | 1351 |
| 666858 | 679 | 695 | | Deoxy, MOE, and cEt | eekkddddddddeeeee | soosssssssssooss | 9829 | 9845 | 1351 |
| 666864 | 679 | 695 | | Deoxy, MOE, and cEt | kekeddddddddeeeee | soosssssssssooss | 9829 | 9845 | 1351 |
| 666865 | 679 | 695 | | Deoxy, MOE, and cEt | eeeeddddddddekeke | soosssssssssooss | 9829 | 9845 | 1351 |
| 666866 | 684 | 700 | | Deoxy, MOE, and cEt | eeekddddddddkeeee | soosssssssssooss | 9834 | 9850 | 1173 |
| 666908 | 686 | 702 | | Deoxy, MOE, and cEt | eeeekdddddddkeeee | sooossssssssooss | 9836 | 9852 | 1175 |
| 666923 | 666 | 682 | | Deoxy, MOE, and cEt | eeekddddddddkeeee | sooossssssssooss | 9816 | 9832 | 1342 |

The modified oligonucleotides were tested in a series of experiments that had similar conditions. The results for each experiment are presented in separate tables shown below. Rats were injected intrathecally with 30µL of a 16.67mg/ml solution of modified oligonucleotide diluted in PBS (500 µg final dose). A control group of rats was injected intrathecally with PBS. Inhibition levels of SOD-1 in the lumbar spinal cord, thoracic spinal cord and cervical spinal cord were assessed. The data is presented below and indicate that several modified oligonucleotides inhibited human SOD-1 levels in this model.

**Table 51**

| **Percent inhibition of human SOD-1 in the spinal cord regions of transgenic rats** | | | | | |
|---|---|---|---|---|---|
| ISIS No | Chemistry | Lumbar | Thoracic | Cervical | SEQ ID NO |
| 333611 | 5-10-5 MOE with phosphorothioate backbone chemistry | 51 | 51 | 47 | 21 |
| 383872 | 5-10-5 MOE with mixed backbone chemistry | 29 | 36 | 26 | 21 |
| 611460 | 5-10-5 MOE with mixed backbone chemistry | 55 | 53 | 25 | 1428 |
| 611464 | 5-10-5 MOE with mixed backbone chemistry | 52 | 54 | 26 | 67 |
| 611468 | 5-10-5 MOE with mixed backbone chemistry | 46 | 44 | 19 | 227 |
| 611481 | 5-10-5 MOE with mixed backbone chemistry | 39 | 44 | 33 | 271 |

**Table 52**

| **Percent inhibition of human SOD-1 in the spinal cord regions of transgenic rats** | | | | | |
|---|---|---|---|---|---|
| ISIS No | Chemistry | Lumbar | Thoracic | Cervical | SEQ ID NO |
| 611473 | 5-10-5 MOE with mixed backbone chemistry | 47 | 42 | 5 | 146 |
| 611474 | 5-10-5 MOE with mixed backbone chemistry | 75 | 65 | 65 | 149 |
| 611479 | 5-10-5 MOE with mixed backbone chemistry | 24 | 13 | 20 | 261 |
| 611484 | 5-10-5 MOE with mixed backbone chemistry | 51 | 31 | 41 | 276 |
| 611485 | 5-10-5 MOE with mixed backbone chemistry | 52 | 40 | 35 | 277 |
| 611492 | 5-10-5 MOE with mixed backbone chemistry | 57 | 44 | 43 | 725 |

**Table 53**

| **Percent inhibition of human SOD-1 in the spinal cord regions of transgenic rats** | | | | | |
|---|---|---|---|---|---|
| ISIS No | Chemistry | Lumbar | Thoracic | Cervical | SEQ ID NO |
| 611472 | 5-10-5 MOE with mixed backbone chemistry | 0 | 19 | 15 | 145 |
| 611478 | 5-10-5 MOE with mixed backbone chemistry | 16 | 33 | 24 | 260 |
| 611490 | 5-10-5 MOE with mixed backbone chemistry | 53 | 55 | 44 | 666 |
| 611494 | 5-10-5 MOE with mixed backbone chemistry | 34 | 39 | 38 | 728 |
| 611495 | 5-10-5 MOE with mixed backbone chemistry | 33 | 19 | 38 | 729 |
| 611498 | 5-10-5 MOE with mixed backbone chemistry | 30 | 43 | 27 | 816 |
| 611499 | 5-10-5 MOE with mixed backbone chemistry | 45 | 56 | 40 | 822 |
| 611500 | 5-10-5 MOE with mixed backbone chemistry | 56 | 58 | 52 | 823 |

**Table 54**

| **Percent inhibition of human SOD-1 in the spinal cord regions of transgenic rats** | | | | | |
|---|---|---|---|---|---|
| ISIS No | Chemistry | Lumbar | Thoracic | Cervical | SEQ ID NO |
| 611457 | 5-10-5 MOE with mixed backbone chemistry | 56 | 46 | 43 | 54 |
| 611467 | 5-10-5 MOE with mixed backbone chemistry | 21 | 28 | 22 | 272 |
| 611488 | 5-10-5 MOE with mixed backbone chemistry | 14 | 23 | 4 | 593 |
| 612917 | Deoxy, MOE, and cEt with mixed backbone chemistry | 47 | 55 | 37 | 1173 |
| 612923 | Deoxy, MOE, and cEt with mixed backbone chemistry | 53 | 63 | 45 | 1164 |
| 612925 | Deoxy, MOE, and cEt with mixed backbone chemistry | 67 | 69 | 63 | 1348 |
| 612928 | Deoxy, MOE, and cEt with mixed backbone chemistry | 84 | 85 | 81 | 1351 |

**Table 55**

| **Percent inhibition of human SOD-1 in the spinal cord regions of transgenic rats** | | | | | |
|---|---|---|---|---|---|
| ISIS No | Chemistry | Lumbar | Thoracic | Cervical | SEQ ID NO |
| 612912 | Deoxy, MOE, and cEt with mixed backbone chemistry | 59 | 60 | 48 | 1146 |
| 612919 | Deoxy, MOE, and cEt with mixed backbone chemistry | 60 | 60 | 58 | 1146 |
| 612916 | Deoxy, MOE, and cEt with mixed backbone chemistry | 72 | 69 | 69 | 1170 |
| 612931 | Deoxy, MOE, and cEt with mixed backbone chemistry | 81 | 79 | 72 | 1173 |
| 612932 | Deoxy, MOE, and cEt with mixed backbone chemistry | 21 | 26 | 24 | 1175 |

**Table 56**

| **Percent inhibition of human SOD-1 in the spinal cord regions of transgenic rats** | | | | | |
|---|---|---|---|---|---|
| ISIS No | Chemistry | Lumbar | Thoracic | Cervical | SEQ ID NO |
| 612915 | Deoxy, MOE, and cEt with mixed backbone chemistry | 54 | 48 | 52 | 1164 |
| 612918 | Deoxy, MOE, and cEt with mixed backbone chemistry | 73 | 69 | 64 | 1175 |
| 612927 | Deoxy, MOE, and cEt with mixed backbone chemistry | 82 | 75 | 62 | 1350 |
| 612934 | Deoxy, MOE, and cEt with mixed backbone chemistry | 59 | 44 | 48 | 969 |
| 612935 | Deoxy, MOE, and cEt with mixed backbone chemistry | 64 | 54 | 62 | 1114 |
| 612940 | Deoxy, MOE, and cEt with mixed backbone chemistry | 11 | 26 | 17 | 1167 |
| 612941 | Deoxy, MOE, and cEt with mixed backbone chemistry | 81 | 75 | 71 | 1342 |
| 612942 | Deoxy, MOE, and cEt with mixed backbone chemistry | 40 | 42 | 41 | 1344 |
| 612943 | Deoxy, MOE, and cEt with mixed backbone chemistry | 61 | 54 | 51 | 1346 |
| 612944 | Deoxy, MOE, and cEt with mixed backbone chemistry | 59 | 52 | 51 | 1348 |

**Table 57**

| **Percent inhibition of human SOD-1 in the spinal cord regions of transgenic rats** | | | | | |
|---|---|---|---|---|---|
| ISIS No | Chemistry | Lumbar | Thoracic | Cervical | SEQ ID NO |
| 612924 | Deoxy, MOE, and cEt with mixed backbone chemistry | 42 | 64 | 53 | 1346 |
| 612947 | Deoxy, MOE, and cEt with mixed backbone chemistry | 68 | 75 | 74 | 1351 |
| 612948 | Deoxy, MOE, and cEt with mixed backbone chemistry | 80 | 90 | 87 | 1352 |
| 612949 | Deoxy, MOE, and cEt with mixed backbone chemistry | 73 | 82 | 85 | 1172 |

**Table 58**

| **Percent inhibition of human SOD-1 in the spinal cord regions of transgenic rats** | | | | |
|---|---|---|---|---|
| ISIS No | Chemistry | Lumbar | Cervical | SEQ ID NO |
| 654304 | 5-8-5 MOE with mixed backbone chemistry | 28 | 6 | 1429 |
| 654305 | 5-8-5 MOE with mixed backbone chemistry | 14 | 0 | 1430 |
| 654306 | 5-8-5 MOE with mixed backbone chemistry | 36 | 0 | 1432 |
| 654307 | 5-8-5 MOE with mixed backbone chemistry | 17 | 0 | 1432 |

**Table 59**

| **Percent inhibition of human SOD-1 in the spinal cord regions of transgenic rats** | | | | |
|---|---|---|---|---|
| ISIS No | Chemistry | Lumbar | Cervical | SEQ ID NO |
| 654334 | Deoxy, MOE, and cEt with mixed backbone chemistry | 39 | 19 | 1454 |
| 666854 | 5-9-5 MOE with mixed backbone chemistry | 52 | 39 | 1428 |
| 666855 | 5-9-5 MOE with mixed backbone chemistry | 37 | 17 | 1461 |
| 666857 | Deoxy, MOE, and cEt with mixed backbone chemistry | 59 | 39 | 1351 |
| 666858 | Deoxy, MOE, and cEt with mixed backbone chemistry | 38 | 22 | 1351 |
| 666859 | Deoxy, MOE, and cEt with mixed backbone chemistry | 79 | 64 | 1351 |
| 666864 | Deoxy, MOE, and cEt with mixed backbone chemistry | 50 | 40 | 1351 |
| 666865 | Deoxy, MOE, and cEt with mixed backbone chemistry | 73 | 44 | 1351 |
| 666866 | Deoxy, MOE, and cEt with mixed backbone chemistry | 67 | 56 | 1173 |
| 666908 | Deoxy, MOE, and cEt with mixed backbone chemistry | 38 | 13 | 1175 |
| 666923 | Deoxy, MOE, and cEt with mixed backbone chemistry | 45 | 26 | 1342 |

**Table 60**

| **Percent inhibition of human SOD-1 in the spinal cord regions of transgenic rats** | | | | |
|---|---|---|---|---|
| ISIS No | Chemistry | Lumbar | Cervical | SEQ ID NO |
| 654323 | Deoxy, MOE, and cEt with mixed backbone chemistry | 53 | 35 | 1433 |
| 666846 | 5-10-5 MOE with mixed backbone chemistry | 64 | 50 | 725 |
| 666849 | 5-10-5 MOE with mixed backbone chemistry | 63 | 55 | 725 |
| 666853 | 5-10-5 MOE with mixed backbone chemistry | 81 | 74 | 725 |
| 666861 | Deoxy, MOE, and cEt with mixed backbone chemistry | 55 | 47 | 1351 |
| 666867 | Deoxy, MOE, and cEt with mixed backbone chemistry | 59 | 48 | 1173 |
| 666869 | Deoxy, MOE, and cEt with mixed backbone chemistry | 82 | 81 | 1173 |
| 666870 | Deoxy, MOE, and cEt with mixed backbone chemistry | 76 | 68 | 1173 |
| 666919 | Deoxy, MOE, and cEt with mixed backbone chemistry | 76 | 68 | 1342 |
| 666921 | Deoxy, MOE, and cEt with mixed backbone chemistry | 71 | 65 | 1342 |
| 666922 | Deoxy, MOE, and cEt with mixed backbone chemistry | 67 | 62 | 1342 |

**Table 61**

| **Percent inhibition of human SOD-1 in the spinal cord regions of transgenic rats** | | | |
|---|---|---|---|
| ISIS No | Chemistry | Lumbar | SEQ ID NO |
| 684059 | Deoxy, MOE, and cEt with mixed backbone chemistry | 54 | 1348 |
| 684064 | Deoxy, MOE, and cEt with mixed backbone chemistry | 51 | 1348 |
| 684068 | 4-8-5 MOE with mixed backbone chemistry | 18 | 1348 |
| 684087 | 5-8-5 MOE with mixed backbone chemistry | 37 | 613 |
| 684088 | 5-8-5 MOE with mixed backbone chemistry | 31 | 1419 |
| 684095 | 5-10-5 MOE with mixed backbone chemistry | 34 | 21 |
| 684097 | 5-8-7 MOE with mixed backbone chemistry | 22 | 21 |
| 684101 | 6-8-6 MOE with mixed backbone chemistry | 22 | 47 |
| 684104 | 6-9-5 MOE with mixed backbone chemistry | 11 | 47 |

### Example 13: Dose-dependent inhibition of human SOD-1 with modified oligonucleotides in LLC-MK2 cells

Gapmers from the studies described above, including benchmark compound ISIS 333611, exhibiting significant *in vitro* inhibition of SOD-1 mRNA were selected and tested at various doses in LLC-MK2 cells. The cross-reactivity of the human modified oligonucleotides tested in this study with the rhesus monkey genomic sequence (the complement of GENBANK Accession No. NW_001114168.1 truncated from nucleotides 2258000 to 2271000, designated herein as SEQ ID NO: 3) is shown in the Table below.

**Table 62**

| **Cross-reactivity of antisense oligonucleotides targeting human SODlwith SEQ ID NO: 3** | | |
|---|---|---|
| ISIS No | Start Site of SEQ ID NO: 3 | Mismatches |
| 333611 | 1572 | 2 |
| 436839 | 1564 | 0 |
| 436854 | 9049 | 0 |
| 436867 | 10347 | 0 |
| 666853 | 10375 | 1 |
| 666859 | 10389 | 1 |
| 666919 | 10376 | 1 |
| 666921 | 10376 | 1 |

Cells were plated at a density of 20,000 cells per well and transfected using electroporation with 0.078 µM, 0.156 µM, 0.313 µM, 0.625 µM, 1.25 µM, 2.50 µM, 5.00 µM, and 10.000 µM concentrations of modified oligonucleotide, as specified in the Tables below. After a treatment period of approximately 16 hours, RNA was isolated from the cells and SOD-1 mRNA levels were measured by quantitative real-time PCR Primer probe set RTS3121 (forward sequence TGGAGATAATACACAAGGCTGTACCA, designated herein as SEQ ID NO: 17; reverse sequence CAACATGCCTCTCTTCATCCTTT, designated herein as SEQ ID NO: 18; probe sequence ATCCTCTATCCAGACAACACGGTGGGC, designated herein as SEQ ID NO: 19) was used to measure mRNA levels. SOD-1 mRNA levels were adjusted according to total RNA content, as measured by RIBOGREEN^{®}. Results are presented as percent inhibition of SOD-1, relative to untreated control cells. The half maximal inhibitory concentration (IC₅₀) of each oligonucleotide is also presented. As presented in the Table, several of the newly designed oligonucleotides were more potent than the benchmark, ISIS 336611.

**Table 63**

| **Dose-dependent inhibition of SOD-1 rhesus monkey mRNA** | | | | | | | | | |
|---|---|---|---|---|---|---|---|---|---|
| ISIS No | 0.078 µM | 0.156 µM | 0.313 µM | 0.625 µM | 1.25 µM | 2.50 µM | 5.00 µM | 10.00 µM | IC₅₀ (µM) |
| 333611 | 3 | 2 | 0 | 0 | 17 | 15 | 19 | 40 | >10 |
| 436839 | 0 | 0 | 5 | 0 | 20 | 22 | 37 | 61 | 7.1 |
| 436854 | 34 | 34 | 40 | 39 | 52 | 65 | 69 | 84 | 1.2 |
| 436867 | 3 | 0 | 11 | 18 | 34 | 49 | 70 | 87 | 2.2 |
| 666853 | 7 | 34 | 20 | 39 | 60 | 80 | 79 | 92 | 1.0 |
| 666859 | 0 | 9 | 20 | 18 | 15 | 25 | 30 | 44 | >10 |
| 666919 | 11 | 15 | 16 | 36 | 51 | 65 | 73 | 84 | 1.4 |
| 666921 | 0 | 13 | 28 | 37 | 50 | 52 | 62 | 74 | 1.8 |

### Example 14: Tolerability of SOD-1 modified oligonucleotides in a rat model

Gapmers from the studies described above, including benchmark compound ISIS 333611, which was previously disclosed in WO 2005/040180, were tested for tolerability in Sprague-Dawley rats.

The modified oligonucleotides were tested in a series of experiments that had similar conditions. Rats were injected intrathecally with3 mg of a single dose of ISIS oligonucleotide. A control group of rats was injected intrathecally with PBS. Acute tolerability was assessed 3 hours post-dose using a functional observational battery (FOB). This score is used to evaluate the acute tolerability of a compound with lower scores denoting better tolerated compounds. Control animals usually have a score of '0' or '1'. At 3 hours post injection, the rats are observed by placing each rat on the cage top and evaluating certain functions, assigning a number of '0' or '1' depending on whether the rat exhibits normal function in the region of interest (0) or does not (1) for each function, and then adding the total scores. Seven regions are assessed, including tail, hind paws, hind legs, hind end, front posture, fore paws, and head. The results of the scoring are presented in the Table below. As presented in the Table, several newly designed oligonucleotides demonstrated more acute tolerability compared to the benchmark, ISIS 333611.

**Table 64**

| **FOB scores in Sprague-Dawley rats** | | | |
|---|---|---|---|
| ISIS No | Target Start Site on SEQ ID NO: 1 | Chemistry | FOB score |
| 333611 | 167 | 5-10-5 MOE with phosphorothioate backbone | 4 |
| 684073 | 167 | Deoxy, MOE, and cEt with mixed backbone | 3 |
| 684081 | 167 | Deoxy, MOE, and cEt with mixed backbone | 1 |
| 684088 | 167 | 5-8-5 MOE with mixed backbone | 0 |
| 684093 | 167 | 5-9-5 MOE with mixed backbone | 0 |
| 684095 | 167 | 5-10-5 MOE with mixed backbone | 0 |
| 684096 | 167 | 6-8-6 MOE with mixed backbone | 0 |
| 684097 | 167 | 5-8-7 MOE with mixed backbone | 0 |
| 684098 | 167 | 7-8-5 MOE with mixed backbone | 0 |
| 684099 | 167 | 6-9-5 MOE with mixed backbone | 0 |
| 684074 | 168 | Deoxy, MOE, and cEt with mixed backbone | 0 |
| 684082 | 168 | Deoxy, MOE, and cEt with mixed backbone | 1 |
| 684089 | 168 | 5-8-5 MOE with mixed backbone | 0 |
| 684094 | 168 | 5-9-5 MOE with mixed backbone | 1 |
| 684075 | 169 | Deoxy, MOE, and cEt with mixed backbone | 3 |
| 684083 | 169 | Deoxy, MOE, and cEt with mixed backbone | 3 |
| 684090 | 169 | 5-8-5 MOE with mixed backbone | 2 |
| 684076 | 170 | Deoxy, MOE, and cEt with mixed backbone | 2 |
| 684084 | 170 | Deoxy, MOE, and cEt with mixed backbone | 4 |
| 611474 | 234 | 5-10-5 MOE with mixed backbone | 4 |
| 654301 | 234 | 5-8-5 MOE with mixed backbone | 3 |
| 654302 | 235 | 5-8-5 MOE with mixed backbone | 1 |
| 654303 | 236 | 5-8-5 MOE with mixed backbone | 0 |
| 684069 | 588 | Deoxy, MOE, and cEt with mixed backbone | 0 |
| 684077 | 588 | Deoxy, MOE, and cEt with mixed backbone | 1 |
| 684085 | 588 | 5-8-5 MOE with mixed backbone | 0 |
| 684091 | 588 | 5-9-5 MOE with mixed backbone | 0 |
| 684100 | 588 | 5-10-5 MOE with mixed backbone | 0 |
| 684101 | 588 | 6-8-6 MOE with mixed backbone | 0 |
| 684102 | 588 | 5-8-7 MOE with mixed backbone | 0 |
| 684103 | 588 | 7-8-5 MOE with mixed backbone | 0 |
| 684104 | 588 | 6-9-5 MOE with mixed backbone | 0 |
| 684070 | 589 | Deoxy, MOE, and cEt with mixed backbone | 0 |
| 684078 | 589 | Deoxy, MOE, and cEt with mixed backbone | 0 |
| 684086 | 589 | 5-8-5 MOE with mixed backbone | 0 |
| 684092 | 589 | 5-9-5 MOE with mixed backbone | 0 |
| 684071 | 590 | Deoxy, MOE, and cEt with mixed backbone | 0 |
| 684079 | 590 | Deoxy, MOE, and cEt with mixed backbone | 0 |
| 684087 | 590 | 5-8-5 MOE with mixed backbone | 0 |
| 684072 | 591 | Deoxy, MOE, and cEt with mixed backbone | 1 |
| 684080 | 591 | Deoxy, MOE, and cEt with mixed backbone | 1 |
| 654304 | 663 | 5-8-5 MOE with mixed backbone | 3 |
| 612916 | 664 | Deoxy, MOE, and cEt with mixed backbone | 0 |
| 654305 | 664 | 5-8-5 MOE with mixed backbone | 2 |
| 611492 | 665 | 5-10-5 MOE with mixed backbone | 0 |
| 654306 | 665 | 5-8-5 MOE with mixed backbone | 3 |
| 654323 | 665 | Deoxy, MOE, and cEt with mixed backbone | 0 |
| 654341 | 665 | Deoxy, MOE, and cEt with mixed backbone | 0 |
| 666846 | 665 | 5-10-5 MOE with mixed backbone | 0 |
| 666849 | 665 | 5-10-5 MOE with mixed backbone | 0 |
| 666851 | 665 | 5-10-5 MOE with mixed backbone | 1 |
| 666853 | 665 | 5-10-5 MOE with mixed backbone | 0 |
| 666854 | 665 | 5-9-5 MOE with mixed backbone | 1 |
| 611500 | 666 | 5-10-5 MOE with mixed backbone | 0 |
| 612941 | 666 | Deoxy, MOE, and cEt with mixed backbone | 3 |
| 654307 | 666 | 5-8-5 MOE with mixed backbone | 2 |
| 654342 | 666 | Deoxy, MOE, and cEt with mixed backbone | 2 |
| 666845 | 666 | 5-10-5 MOE with mixed backbone | 0 |
| 666848 | 666 | 5-10-5 MOE with mixed backbone | 1 |
| 666850 | 666 | 5-10-5 MOE with mixed backbone | 0 |
| 666852 | 666 | 5-10-5 MOE with mixed backbone | 1 |
| 666855 | 666 | 5-9-5 MOE with mixed backbone | 1 |
| 666917 | 666 | Deoxy, MOE, and cEt with mixed backbone | 3 |
| 666918 | 666 | Deoxy, MOE, and cEt with mixed backbone | 3 |
| 666919 | 666 | Deoxy, MOE, and cEt with mixed backbone | 2 |
| 666920 | 666 | Deoxy, MOE, and cEt with mixed backbone | 1 |
| 666921 | 666 | Deoxy, MOE, and cEt with mixed backbone | 2 |
| 666922 | 666 | Deoxy, MOE, and cEt with mixed backbone | 3 |
| 666923 | 666 | Deoxy, MOE, and cEt with mixed backbone | 2 |
| 666856 | 667 | 5-9-5 MOE with mixed backbone | 3 |
| 612925 | 676 | Deoxy, MOE, and cEt with mixed backbone | 4 |
| 684059 | 676 | Deoxy, MOE, and cEt with mixed backbone | 4 |
| 684060 | 676 | Deoxy, MOE, and cEt with mixed backbone | 3 |
| 684061 | 676 | Deoxy, MOE, and cEt with mixed backbone | 4 |
| 684062 | 676 | Deoxy, MOE, and cEt with mixed backbone | 4 |
| 684063 | 676 | Deoxy, MOE, and cEt with mixed backbone | 5 |
| 684064 | 676 | Deoxy, MOE, and cEt with mixed backbone | 4 |
| 684065 | 676 | Deoxy, MOE, and cEt with mixed backbone | 4 |
| 684066 | 676 | Deoxy, MOE, and cEt with mixed backbone | 4 |
| 684067 | 676 | Deoxy, MOE, and cEt with mixed backbone | 5 |
| 684068 | 676 | 4-8-5 MOE with mixed backbone | 4 |
| 612927 | 678 | Deoxy, MOE, and cEt with mixed backbone | 4 |
| 654309 | 678 | 5-8-5 MOE with mixed backbone | 4 |
| 612928 | 679 | Deoxy, MOE, and cEt with mixed backbone | 2 |
| 612947 | 679 | Deoxy, MOE, and cEt with mixed backbone | 7 |
| 654310 | 679 | 5-8-5 MOE with mixed backbone | 3 |
| 666857 | 679 | Deoxy, MOE, and cEt with mixed backbone | 1 |
| 666858 | 679 | Deoxy, MOE, and cEt with mixed backbone | 1 |
| 666859 | 679 | Deoxy, MOE, and cEt with mixed backbone | 1 |
| 666860 | 679 | Deoxy, MOE, and cEt with mixed backbone | 0 |
| 666861 | 679 | Deoxy, MOE, and cEt with mixed backbone | 5 |
| 666862 | 679 | Deoxy, MOE, and cEt with mixed backbone | 1 |
| 666863 | 679 | Deoxy, MOE, and cEt with mixed backbone | 4 |
| 666864 | 679 | Deoxy, MOE, and cEt with mixed backbone | 4 |
| 666865 | 679 | Deoxy, MOE, and cEt with mixed backbone | 5 |
| 611497 | 681 | 5-10-5 MOE with mixed backbone | 5 |
| 612948 | 681 | Deoxy, MOE, and cEt with mixed backbone | 3 |
| 666847 | 681 | 5-10-5 MOE with mixed backbone | 7 |
| 612949 | 683 | Deoxy, MOE, and cEt with mixed backbone | 4 |
| 612931 | 684 | Deoxy, MOE, and cEt with mixed backbone | 4 |
| 666866 | 684 | Deoxy, MOE, and cEt with mixed backbone | 6 |
| 666867 | 684 | Deoxy, MOE, and cEt with mixed backbone | 6 |
| 666868 | 684 | Deoxy, MOE, and cEt with mixed backbone | 6 |
| 666869 | 684 | Deoxy, MOE, and cEt with mixed backbone | 6 |
| 666870 | 684 | Deoxy, MOE, and cEt with mixed backbone | 6 |
| 666871 | 684 | Deoxy, MOE, and cEt with mixed backbone | 6 |
| 666872 | 684 | Deoxy, MOE, and cEt with mixed backbone | 6 |
| 666873 | 684 | Deoxy, MOE, and cEt with mixed backbone | 6 |
| 666874 | 684 | Deoxy, MOE, and cEt with mixed backbone | 5 |
| 612918 | 686 | Deoxy, MOE, and cEt with mixed backbone | 4 |
| 612932 | 686 | Deoxy, MOE, and cEt with mixed backbone | 2 |
| 666906 | 686 | Deoxy, MOE, and cEt with mixed backbone | 2 |
| 666907 | 686 | Deoxy, MOE, and cEt with mixed backbone | 3 |
| 666908 | 686 | Deoxy, MOE, and cEt with mixed backbone | 1 |
| 666909 | 686 | Deoxy, MOE, and cEt with mixed backbone | 0 |
| 666910 | 686 | Deoxy, MOE, and cEt with mixed backbone | 2 |
| 666911 | 686 | Deoxy, MOE, and cEt with mixed backbone | 0 |
| 666912 | 686 | Deoxy, MOE, and cEt with mixed backbone | 0 |
| 666913 | 686 | Deoxy, MOE, and cEt with mixed backbone | 0 |
| 666914 | 686 | Deoxy, MOE, and cEt with mixed backbone | 0 |
| 666915 | 686 | Deoxy, MOE, and cEt with mixed backbone | 1 |
| 666916 | 686 | Deoxy, MOE, and cEt with mixed backbone | 1 |
| 654318 | 687 | 5-8-5 MOE with mixed backbone | 1 |
| 654334 | 687 | Deoxy, MOE, and cEt with mixed backbone | 3 |

Tolerability was also assessed 8 weeks post-dose by measuring the levels of IBA1, a microglial marker, and GFAP, an astrocytic marker, in the lumbar spinal cord region. Both IBA1 and GFAP are markers of CNS inflammation (Frank, MG, Brain Behav. Immun. 2007, 21, 47-59), hence the higher the level of either marker, the less tolerable the antisense oligonucleotide is deemed to be in this rat model.

IBA1 mRNA levels were measured with primer probe set rAIF1_LTS00219 (forward sequence AGGAGAAAAACAAAGAACACCAGAA, designated herein as SEQ ID NO: 5; reverse sequence CAATTAGGGCAACTCAGAAATAGCT, designated herein as SEQ ID NO: 6; probe sequence CCAACTGGTCCCCCAGCCAAGA, designated herein as SEQ ID NO: 7). GFAP mRNA levels were measured with primer probe set mGFAP_LTS00370 (forward sequence GAAACCAGCCTGGACACCAA, designated herein as SEQ ID NO: 8; reverse sequence TCCACAGTCTTTACCACGATGTTC, designated herein as SEQ ID NO: 9; probe sequence TCCGTGTCAGAAGGCCACCTCAAGA, designated herein as SEQ ID NO: 10).

The results are presented in the Table below. As presented in the Table, several newly designed oligonucleotides were more tolerable compared to the benchmark, ISIS 333611.

**Table 65**

| **IBA1 and GFAP mRNA levels (% control) in the lumbar regions of Sprague-Dawley rats** | | |
|---|---|---|
| ISIS No. | IBA1 | GFAP |
| 333611 | 341 | 314 |
| 654301 | 149 | 137 |
| 654302 | 261 | 129 |
| 654303 | 110 | 80 |
| 654304 | 143 | 130 |
| 654305 | 185 | 158 |
| 654306 | 110 | 106 |
| 654307 | 152 | 144 |
| 654309 | 195 | 169 |
| 654310 | 119 | 141 |
| 654318 | 93 | 81 |
| 654323 | 125 | 113 |
| 654334 | 114 | 75 |
| 654341 | 209 | 224 |
| 654342 | 473 | 485 |
| 666845 | 389 | 416 |
| 666846 | 173 | 171 |
| 666847 | 271 | 297 |
| 666848 | 399 | 377 |
| 666849 | 140 | 150 |
| 666850 | 246 | 252 |
| 666851 | 246 | 199 |
| 666852 | 282 | 266 |
| 666853 | 168 | 147 |
| 666854 | 135 | 123 |
| 666855 | 238 | 221 |
| 666856 | 253 | 209 |
| 666857 | 242 | 182 |
| 666858 | 169 | 134 |
| 666859 | 185 | 162 |
| 666861 | 161 | 152 |
| 666862 | 254 | 285 |
| 666863 | 216 | 185 |
| 666864 | 174 | 154 |
| 666865 | 251 | 232 |
| 666866 | 281 | 135 |
| 666867 | 132 | 112 |
| 666868 | 199 | 211 |
| 666869 | 262 | 207 |
| 666870 | 201 | 189 |
| 666871 | 192 | 214 |
| 666872 | 441 | 136 |
| 666873 | 340 | 277 |
| 666874 | 204 | 199 |
| 666917 | 292 | 244 |
| 666919 | 115 | 85 |
| 666920 | 155 | 102 |
| 666921 | 108 | 82 |
| 666922 | 123 | 82 |
| 666923 | 118 | 93 |
| 684059 | 168 | 162 |
| 684060 | 158 | 141 |
| 684061 | 335 | 263 |
| 684062 | 218 | 265 |
| 684064 | 191 | 168 |
| 684065 | 245 | 304 |
| 684066 | 313 | 376 |
| 684067 | 171 | 151 |
| 684068 | 157 | 135 |
| 684085 | 459 | 586 |
| 684086 | 187 | 227 |
| 684087 | 215 | 263 |
| 684088 | 151 | 183 |
| 684089 | 507 | 667 |
| 684090 | 130 | 170 |
| 684091 | 350 | 426 |
| 684092 | 366 | 333 |
| 684093 | 412 | 264 |
| 684094 | 294 | 373 |
| 684095 | 213 | 215 |
| 684096 | 404 | 335 |
| 684097 | 217 | 206 |
| 684098 | 378 | 438 |
| 684099 | 534 | 473 |
| 684100 | 276 | 259 |
| 684101 | 153 | 125 |
| 684102 | 237 | 242 |
| 684103 | 588 | 416 |
| 684104 | 221 | 193 |

### Example 15: Dose dependent inhibition of human SOD-1 in a transgenic rat model

Gapmers from the studies described above, including benchmark compound ISIS 333611, were tested in an SOD-1 transgenic rat model (Taconic, Cat# 2148-F and 2148-M). These hemizygous rats express mutant human SOD-1 in the spinal cord, many brain regions, and peripheral organs.

Rats were injected intrathecally with 10, 30, 100, 300, 1000, or 3000 µg of a gapmer listed in the table below or with only PBS. Two weeks later, the animals were sacrificed. Inhibition of SOD-1 mRNA in the lumbar spinal cord, cervical spinal cord, rostral cortex, and caudal cortex was assessed by RT-PCR using primer probe set RTS3898, described in Example 1. The data is presented below as ED₅₀ values, and indicates that the oligonucleotides inhibited SOD1 mRNA in multiple CNS tissues more potently than Isis 333611. Indeed, ED₅₀ values for Isis No. 333611 could not even be calculated, as indicated by an entry of "n/a," because even the highest concentration tested (3000 µg) did not inhibit SOD-1 mRNA greater than 55-65%. "n.d." indicates that there is no data available for the indicated sample.

**Table 66**

| **Inhibition of human SOD1 in transgenic rats** | | | | | |
|---|---|---|---|---|---|
| Isis No. | ED₅₀ (µg) | | | | SEQ ID NO. |
| | Lumbar | Cervical | Rostral | Caudal | |
| 333611 | n/a | n/a | n.d. | n.d. | 21 |
| 666853 | 81.3 | 242.6 | 6434 | 931 | 725 |
| 666859 | 74.0 | 358.8 | 2360 | 1113 | 1351 |
| 666870 | 139.4 | 1111 | 5511 | 2105 | 1173 |
| 666919 | 104.1 | 613.5 | > 6000 | 2655 | 1342 |

### Example 16: Tolerability of SOD-1 modified oligonucleotides in rats

Gapmers from the studies described above, including benchmark compound ISIS 333611, were tested for tolerability in Sprague-Dawley rats. Groups of 4 to 6 rats were injected intrathecally with 1 mg or 3 mg of a single dose of an ISIS oligonucleotide. A control group of rats was injected intrathecally with PBS. Acute tolerability was assessed 3 hours post-dose, as described in Example 14. The results for the 1 mg dose are the averages for each group following one experiment. The results for the 3 mg dose are the averages for each group across two replicate experiments. The results of the study, presented in the table below, indicate that several newly designed oligonucleotides were more tolerable than the benchmark, ISIS 333611.

**Table 67**

| **FOB values** | | | | |
|---|---|---|---|---|
| Isis No. | 3 hour FOB | | 8 week FOB | |
| | 1 mg | 3 mg | 1 mg | 3 mg |
| 333611 | 3.0 | 4.9 | 0.0 | 1.2 |
| 666853 | 0.0 | 0.5 | 0.0 | 0.0 |
| 666859 | 0.0 | 2.1 | 0.0 | 0.3 |
| 666870 | 2.3 | 5.8 | 0.0 | 0.8 |
| 666919 | 1.3 | 3.5 | 0.0 | 0.1 |

### Example 17: Dose dependent inhibition of human SOD-1 in a transgenic mouse model

In order to confirm the results obtained in transgenic rats in another species, gapmers from the studies described above were tested in an SOD-1 transgenic mouse model that expresses the same G93A human mutant SOD1 gene that the transgenic rat expresses (see Examples 12 and 15).

Mice received an intracerebral ventricular bolus (ICVB) of 10, 30, 100, 300, or 700 µg of a gapmer listed in the table below, or PBS. Two weeks later, the animals were sacrificed. Inhibition of SOD-1 mRNA in the lumbar spinal cord and cortex was assessed by RT-PCR using primer probe set RTS3898, described in Example 1. The data is presented below as ED₅₀ values, and indicates that the oligonucleotides inhibited SOD1 mRNA more potently than Isis 333611 in both rats and mice.

**Table 68**

| **Inhibition of human SOD1 in transgenic mice** | | |
|---|---|---|
| Isis No. | Lumbar ED₅₀ (µg) | Cortex ED₅₀ (µg) |
| 333611 | 401 | 786 |
| 666853 | 136 | 188 |
| 666859 | 106 | 206 |
| 666870 | 148 | 409 |
| 666919 | 168 | 1211 |

### Example 18: Tolerability of SOD-1 modified oligonucleotides in mice

Gapmers from the studies described above, including benchmark compound ISIS 333611, were tested for tolerability in C57bl6 mice. Mice were injected stereotaxically into the cerebral ventricles with 700ug of a single dose of ISIS oligonucleotide. A control group of mice was injected into the cerebral ventricle with PBS. Acute tolerability was assessed at 3 hours post injection using a functional observation battery (FOB) different from that used for the rats. Each mouse was evaluated according to 7 different criteria. The 7 criteria are (1) the mouse was bright, alert, and responsive; (2) the mouse was standing or hunched without stimuli; (3) the mouse shows any movement without stimuli (4) the mouse demonstrates forward movement after its lifted; (5) the mouse demonstrates any movement after its lifted; (6) the mouse responds to a tail pinch; (7) regular breathing. For each of the 7 different criteria, each mouse was given a sub-score of 0 if it met the criteria or 1 if it did not. After all of the 7 criteria were evaluated, the sub-scores were summed for each mouse and then averaged for each group. For example, if a mouse was bright, alert, and responsive 3 hours after the 700 µg ICV dose, and met all other other criteria, it would get a summed score of 0. If another mouse was not bright, alert, and responsive 3 hours after the 700 µg ICV dose but met all other criteria, it would receive a score of 1. Saline treated mice generally receive a score of 0. A score at the top end of the range would be suggestive of acute toxicity.

Body weights were measured throughout the study and are reported below as percent change at 8 weeks relative to baseline. Long term tolerability was assessed 8 weeks post-dose by measuring the levels of IBA1 and GFAP, as described in Example 14. IBA1 and GFAP mRNA levels are reported relative to PBS treated animals. The results of the study, presented in the tables below, indicate that several newly designed oligonucleotides were more tolerable, in rats and mice, compared to the benchmark, ISIS 333611.

**Table 69**

| **FOB values and body weight change** | | |
|---|---|---|
| Isis No. | 3 hour FOB | Body weight (% change) |
| 333611 | 6.5 | 3.8 |
| 666853 | 1.25 | 8.0 |
| 666859 | 1.75 | 14.0 |
| 666870 | 4.75 | 7.3 |
| 666919 | 0.0 | 5.2 |

**Table 70**

| **Inflammation markers** | | | | |
|---|---|---|---|---|
| Isis No. | IBA1 (% PBS) | | GFAP (% PBS) | |
| | Lumbar | Cortex | Lumbar | Cortex |
| 333611 | 130.3 | 134.3 | 117.5 | 207.7 |
| 666853 | 102.8 | 109.3 | 103.3 | 103.7 |
| 666859 | 110.4 | 98.2 | 109.0 | 72.8 |
| 666870 | 158.8 | 117.8 | 106.7 | 128.6 |
| 666919 | 115.0 | 97.9 | 99.8 | 84.3 |

### Example 19: Dose dependent inhibition of monkey SOD-1 in cynomolgus monkey

Isis No. 666853 was tested in cynomolgus monkey. There is one mismatch between Isis No. 666853 and cynomolgus monkey SOD-1, and there are 17 contiguous bases in Isis No. 666853 that are 100% complementary to cynomolgus monkey SOD-1.

Groups of 6-10 male and female monkeys received an intrathecal lumbar bolus of PBS or 4, 12, or 35 mg of Isis No. 666853 on days 1, 14, 28, 56, and 84 of the study. Each group received the same dose on all five dosing days. On day 91, the animals were sacrificed. Inhibition of SOD-1 mRNA in the lumbar, thoracic, and cervical spinal cord and frontal cortex, motor cortex, hippocampus, pons, and cerebellum was assessed by RT-PCR using primer probe set RTS3898. The data is presented below as the average percent inhibition for each treatment group, relative to the PBS treated group. The results indicate that Isis No. 666853 inhibited SOD-1 mRNA in multiple target tissues in cynomolgus monkey.

Treatment with 666853 was well tolerated for the duration of the 13 week study and there were no clinical observations of adverse reactions in monkeys.

**Table 71**

| **Inhibition of SOD-1 mRNA in monkeys** | | | | | | | | |
|---|---|---|---|---|---|---|---|---|
| Amount of 666853 per dose (mg) | Inhibition (%) | | | | | | | |
| | Lumbar | Thoracic | Cervical | Frontal cortex | Motor cortex | Hippocampus | Pons | Cerebellum |
| 4 | 44.4 | 27.1 | 20.1 | 21.5 | 21.6 | 32.0 | 6.8 | 15.4 |
| 12 | 75.4 | 69.0 | 42.1 | 56.7 | 55.7 | 31.8 | 13.2 | 33.3 |
| 35 | 87.0 | 74.8 | 72.1 | 80.5 | 82.6 | 80.1 | 48.6 | 48.4 |

## Claims

1. An antisense compound or a pharmaceutically acceptable salt thereof, according to the following formula:
mCes Aeo Ges Geo Aes Tds Ads mCds Ads Tds Tds Tds mCds Tds Ads mCeo Aes Geo mCes Te (nucleobase sequence of SEQ ID NO: 725);
wherein,
A = an adenine,
mC = a 5-methylcytosine,
G = a guanine,
T = a thymine,
e = a 2'-O-methoxyethylribose modified sugar,
d = a 2'-deoxyribose sugar,
s = a phosphorothioate internucleoside linkage, and
o = a phosphodiester internucleoside linkage;
for use in therapy, wherein said use comprises the intrathecal administration of the antisense compound or the pharmaceutically acceptable salt thereof.

2. The antisense compound or pharmaceutically acceptable salt thereof as defined in claim 1, for use in treating or preventing a neurodegenerative disease, wherein said use comprises the intrathecal administration of the antisense compound or the pharmaceutically acceptable salt thereof.

3. The antisense compound or pharmaceutically acceptable salt thereof as defined in claim 1, for use in treating or preventing amyotrophic lateral sclerosis (ALS), wherein said use comprises the intrathecal administration of the antisense compound or the pharmaceutically acceptable salt thereof.

4. The antisense compound or pharmaceutically acceptable salt thereof for use of claim 3, wherein the ALS is familial ALS.

5. The antisense compound or pharmaceutically acceptable salt thereof for use of claim 3, wherein the ALS is sporadic ALS.

6. The antisense compound or pharmaceutically acceptable salt thereof for use of any one of claims 1-5, wherein the antisense compound or the pharmaceutically acceptable salt is to be administered to a human subject.

7. A pharmaceutical composition comprising, a pharmaceutically acceptable diluent or carrier, and a compound of the following structure: 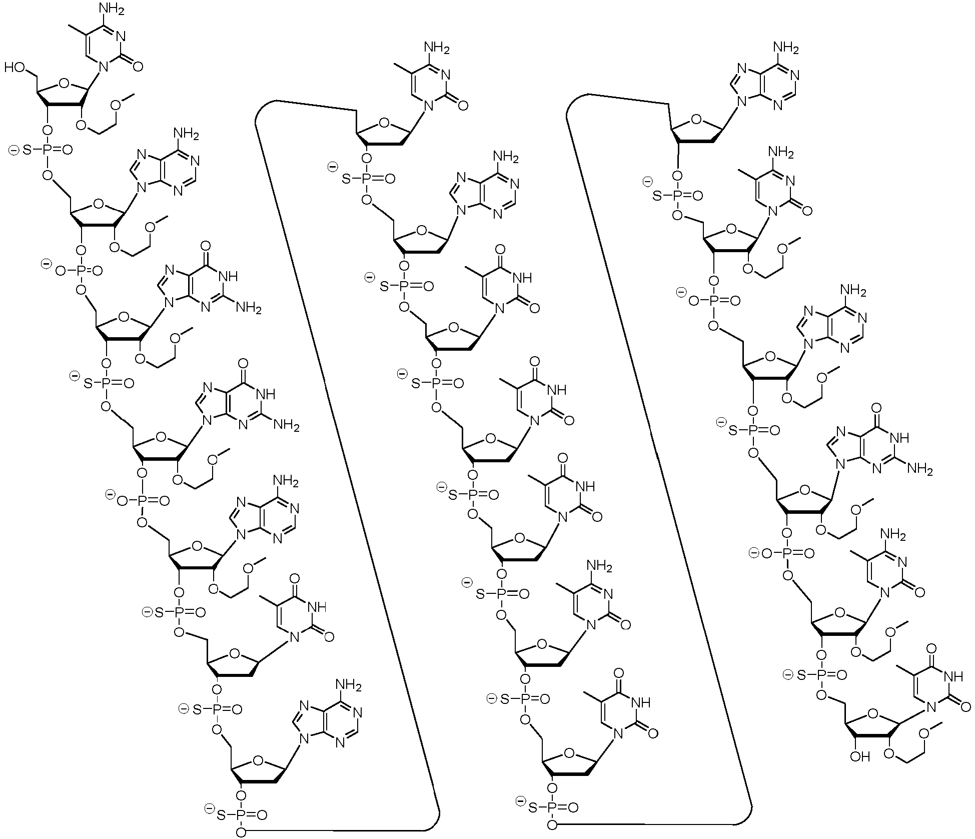 for use in therapy, wherein said use comprises the intrathecal administration of the pharmaceutical composition.

8. The pharmaceutical composition for use of claim 7, wherein said composition comprises a sterile aqueous solution.

9. The pharmaceutical composition as defined in claim 7 or claim 8, for use in treating or preventing a neurodegenerative disease, wherein said use comprises the intrathecal administration of the pharmaceutical composition.

10. The pharmaceutical composition as defined in claim 7 or claim 8, for use in treating or preventing amyotrophic lateral sclerosis (ALS), wherein said use comprises the intrathecal administration of the pharmaceutical composition.

11. The pharmaceutical composition for use of claim 10 wherein the ALS is familial ALS.

12. The pharmaceutical composition for use of claim 10, wherein the ALS is sporadic ALS.

13. The pharmaceutical composition for use of any one of claims 7-12 wherein the pharmaceutical composition is to be administered to a human subject.

## Patentansprüche

1. Antisense-Verbindung oder pharmazeutisch akzeptables Salz davon gemäß der folgenden Formel:
mCes Aeo Ges Geo Aes Tds Ads mCds Ads Tds Tds Tds mCds Tds Ads mCeo Aes Geo mCes Te (Nukleobasen-Sequenz von SEQ ID NO: 725);
wobei,
A = ein Adenin,
mC = ein 5-Methylcytosin,
G = ein Guanin,
T = ein Thymin,
e = ein 2'-O-Methoxyethylribose-modifizierter Zucker,
d = ein 2'-Desoxyribose-Zucker,
s = eine Phosphorothioat-Internukleosid-Bindung, und
o = eine Phosphodiester-Internukleosid-Bindung;
zur Verwendung bei der Therapie, wobei die Verwendung die intrathekale Verabreichung der Antisense-Verbindung oder des pharmazeutisch akzeptablen Salzes davon umfasst.

2. Antisense-Verbindung oder pharmazeutisch akzeptables Salz davon nach Anspruch 1 zur Verwendung beim Behandeln oder Vorbeugen einer neurodegenerativen Erkrankung, wobei die Verwendung die intrathekale Verabreichung der Antisense-Verbindung oder des pharmazeutisch akzeptablen Salzes davon umfasst.

3. Antisense-Verbindung oder pharmazeutisch akzeptables Salz davon nach Anspruch 1 zur Verwendung beim Behandeln oder Vorbeugen von amyotropher Lateralsklerose (ALS), wobei die Verwendung die intrathekale Verabreichung der Antisense-Verbindung oder des pharmazeutisch akzeptablen Salzes davon umfasst.

4. Antisense-Verbindung oder pharmazeutisch akzeptables Salz davon zur Verwendung nach Anspruch 3, wobei die ALS familiäre ALS ist.

5. Antisense-Verbindung oder pharmazeutisch akzeptables Salz davon zur Verwendung nach Anspruch 3, wobei die ALS sporadische ALS ist.

6. Antisense-Verbindung oder pharmazeutisch akzeptables Salz davon zur Verwendung nach einem der Ansprüche 1-5, wobei die Antisense-Verbindung oder das pharmazeutisch akzeptable Salz einem menschlichen Individuum zu verabreichen ist.

7. Pharmazeutische Zusammensetzung, umfassend einen pharmazeutisch akzeptablen Verdünner oder Träger und eine Verbindung der folgenden Struktur: 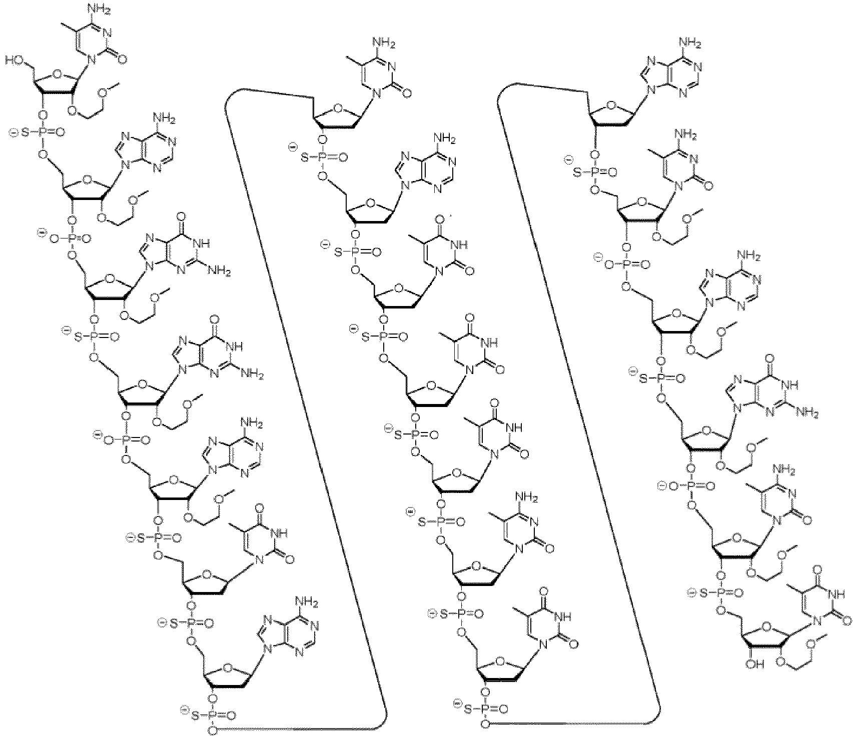 zur Verwendung bei der Therapie, wobei die Verwendung die intrathekale Verabreichung der pharmazeutischen Zusammensetzung umfasst.

8. Pharmazeutische Zusammensetzung zur Verwendung nach Anspruch 7, wobei die Zusammensetzung eine sterile wässrige Lösung umfasst.

9. Pharmazeutische Zusammensetzung nach Anspruch 7 oder Anspruch 8 zum Behandeln oder Vorbeugen einer neurodegenerativen Erkrankung, wobei die Verwendung die intrathekale Verabreichung der pharmazeutischen Zusammensetzung umfasst.

10. Pharmazeutische Zusammensetzung nach Anspruch 7 oder Anspruch 8 zum Behandeln oder Vorbeugen von amyotropher Lateralsklerose (ALS), wobei die Verwendung die intrathekale Verabreichung der pharmazeutischen Zusammensetzung umfasst.

11. Pharmazeutische Zusammensetzung zur Verwendung nach Anspruch 10, wobei die ALS familiäre ALS ist.

12. Pharmazeutische Zusammensetzung zur Verwendung nach Anspruch 10, wobei die ALS sporadische ALS ist.

13. Pharmazeutische Zusammensetzung zur Verwendung nach einem der Ansprüche 7-12, wobei die pharmazeutische Zusammensetzung einem menschlichen Individuum zu verabreichen ist.

## Revendications

1. Composé antisens ou un sel pharmaceutiquement acceptable de celui-ci, selon la formule suivante :
mCes Aeo Ges Geo Aes Tds Ads mCds Ads Tds Tds Tds mCds Tds Ads mCeo Aes Geo mCes Te (séquence de nucléobase de SEQ ID NO:725) ;
dans lequel,
A = une adénine,
mC = une 5-méthylcytosine,
G = une guanine,
T = une thymine,
e = un sucre modifié 2'-O-méthoxyéthylribose,
d = un sucre 2'-désoxyribose,
s = une liaison internucléosidique phosphorothioate, et
o = une liaison internucléosidique phosphodiester ;
pour une utilisation en thérapie, dans lequel ladite utilisation comprend l'administration intrathécale du composé antisens ou du sel pharmaceutiquement acceptable de celui-ci.

2. Composé antisens ou sel pharmaceutiquement acceptable de celui-ci selon la revendication 1, pour une utilisation dans le traitement ou la prévention d'une maladie neurodégénérative, dans lequel ladite utilisation comprend l'administration intrathécale du composé antisens ou du sel pharmaceutiquement acceptable de celui-ci.

3. Composé antisens ou sel pharmaceutiquement acceptable de celui-ci selon la revendication 1, pour une utilisation dans le traitement ou la prévention de la sclérose latérale amyotrophique (SLA), dans lequel ladite utilisation comprend l'administration intrathécale du composé antisens ou du sel pharmaceutiquement acceptable de celui-ci.

4. Composé antisens ou sel pharmaceutiquement acceptable de celui-ci pour une utilisation selon la revendication 3, dans lequel la SLA est la SLA familiale.

5. Composé antisens ou sel pharmaceutiquement acceptable de celui-ci pour une utilisation selon la revendication 3, dans lequel la SLA est la SLA sporadique.

6. Composé antisens ou sel pharmaceutiquement acceptable de celui-ci pour une utilisation selon l'une quelconque des revendications 1 à 5, dans lequel le composé antisens ou le sel pharmaceutiquement acceptable doit être administré à un sujet humain.

7. Composition pharmaceutique comprenant, un diluant ou un support pharmaceutiquement acceptable, et un composé ayant la structure suivante : 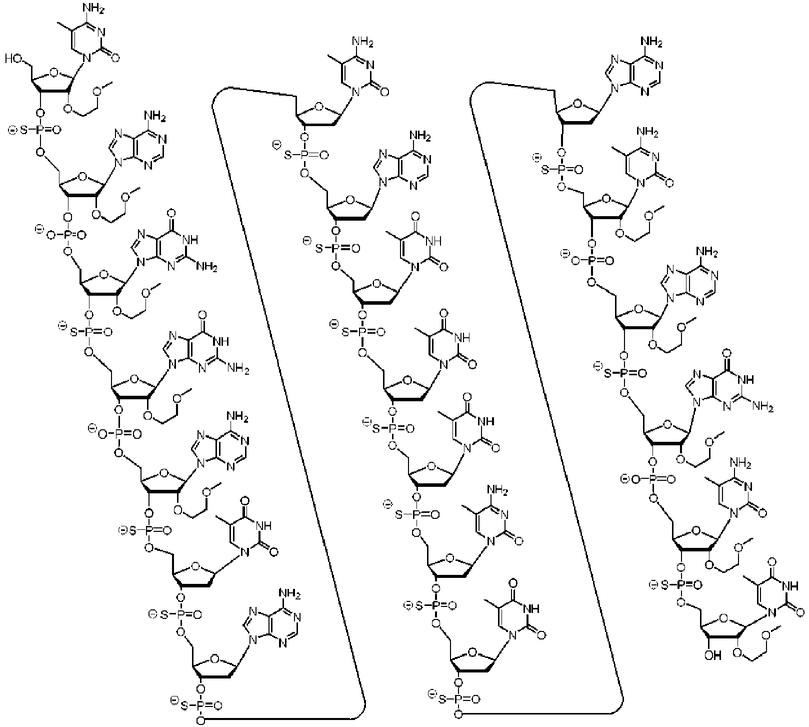 pour une utilisation en thérapie, dans laquelle ladite utilisation comprend l'administration intrathécale de la composition pharmaceutique.

8. Composition pharmaceutique pour une utilisation selon la revendication 7, dans laquelle ladite composition comprend une solution aqueuse stérile.

9. Composition pharmaceutique selon la revendication 7 ou la revendication 8, pour une utilisation dans le traitement ou la prévention d'une maladie neurodégénérative, dans laquelle ladite utilisation comprenant l'administration intrathécale de la composition pharmaceutique.

10. Composition pharmaceutique selon la revendication 7 ou la revendication 8, pour une utilisation dans le traitement ou la prévention de la sclérose latérale amyotrophique (SLA), dans laquelle ladite utilisation comprend l'administration intrathécale de la composition pharmaceutique.

11. Composition pharmaceutique pour une utilisation selon la revendication 10, dans laquelle la SLA est la SLA familiale.

12. Composition pharmaceutique pour une utilisation selon la revendication 10, dans laquelle la SLA est la SLA sporadique.

13. Composition pharmaceutique pour une utilisation selon l'une quelconque des revendications 7 à 12, dans laquelle la composition pharmaceutique doit être administrée à un sujet humain.


### SEQUENCE LISTING

<110> Isis Pharmaceuticals, Inc.
<120> COMPOSITIONS FOR MODULATING SOD-1 EXPRESSION
<130> BIOL0240WO
<150> 61/973,803 <151> 2014-04-01
<160> 1461
<170> PatentIn version 3.5
<210> 1
   <211> 981
   <212> DNA
   <213> Homo sapiens
<400> 1
   gtttggggcc agagtgggcg aggcgcggag gtctggccta taaagtagtc gcggagacgg        60
   ggtgctggtt tgcgtcgtag tctcctgcag cgtctggggt ttccgttgca gtcctcggaa       120
   ccaggacctc ggcgtggcct agcgagttat ggcgacgaag gccgtgtgcg tgctgaaggg       180
   cgacggccca gtgcagggca tcatcaattt cgagcagaag gaaagtaatg gaccagtgaa       240
   ggtgtgggga agcattaaag gactgactga aggcctgcat ggattccatg ttcatgagtt       300
   tggagataat acagcaggct gtaccagtgc aggtcctcac tttaatcctc tatccagaaa       360
   acacggtggg ccaaaggatg aagagaggca tgttggagac ttgggcaatg tgactgctga       420
   caaagatggt gtggccgatg tgtctattga agattctgtg atctcactct caggagacca       480
   ttgcatcatt ggccgcacac tggtggtcca tgaaaaagca gatgacttgg gcaaaggtgg       540
   aaatgaagaa agtacaaaga caggaaacgc tggaagtcgt ttggcttgtg gtgtaattgg       600
   gatcgcccaa taaacattcc cttggatgta gtctgaggcc ccttaactca tctgttatcc       660
   tgctagctgt agaaatgtat cctgataaac attaaacact gtaatcttaa aagtgtaatt       720
   gtgtgacttt ttcagagttg ctttaaagta cctgtagtga gaaactgatt tatgatcact       780
   tggaagattt gtatagtttt ataaaactca gttaaaatgt ctgtttcaat gacctgtatt       840
   ttgccagact taaatcacag atgggtatta aacttgtcag aatttctttg tcattcaagc       900
   ctgtgaataa aaaccctgta tggcacttat tatgaggcta ttaaaagaat ccaaattcaa       960
   actaaaaaaa aaaaaaaaaa a                                                 981
<210> 2
   <211> 11001
   <212> DNA
   <213> Homo sapiens
<400> 2
   cactgcactc cagcctgggt gacagagcga gaccctgtct caaaaatcaa acaaacaacc 60
   ccctcgcccc ggacaaaagt agtttgcact attttctcat ttcacaatat gtttttgaaa 120
   tatttccctt gaaaggtaag tcatatttat cattcctgtt gtatggaggc atcataaatt 180
   atttcaccat tctaccctcc ttgagtgttg tggcctttag gccagacaaa aacgcaggtg 240
   atgcctagaa gccaactagt tgccgtttgg ttatctgtag ggttgtggcc ttgccaaaca 300
   ggaaaaatat aaaaagaata ccgaattctg ccaaccaaat aagaaactct atactaagga 360
   ctaagaaaat tgcaggggaa gaaaaggtaa gtcccgggat tgaggtgtag cgactttcta 420
   taccctcaga aaactaaaaa acaagacaaa aaaatgaaaa ctacaaaagc atccatcttg 480
   gggcgtccca attgctgagt aacaaatgag acgctgtggc caaactcagt cataactaat 540
   gacatttcta gacaaagtga cttcagattt tcaaagcgta ccctgtttac atcattttgc 600
   caatttcgcg tactgcaacc ggcgggccac gcccccgtga aaagaaggtt gttttctcca 660
   catttcgggg ttctggacgt ttcccggctg cggggcgggg ggagtctccg gcgcacgcgg 720
   ccccttggcc ccgcccccag tcattcccgg ccactcgcga cccgaggctg ccgcaggggg 780
   cgggctgagc gcgtgcgagg cgattggttt ggggccagag tgggcgaggc gcggaggtct 840
   ggcctataaa gtagtcgcgg agacggggtg ctggtttgcg tcgtagtctc ctgcagcgtc 900
   tggggtttcc gttgcagtcc tcggaaccag gacctcggcg tggcctagcg agttatggcg 960
   acgaaggccg tgtgcgtgct gaagggcgac ggcccagtgc agggcatcat caatttcgag 1020
   cagaaggcaa gggctgggac ggaggcttgt ttgcgaggcc gctcccaccc gctcgtcccc 1080
   ccgcgcacct ttgctaggag cgggtcgccc gccaggcctc ggggccgccc tggtccagcg 1140
   cccggtcccg gcccgtgccg cccggtcggt gccttcgccc ccagcggtgc ggtgcccaag 1200
   tgctgagtca ccgggcgggc ccgggcgcgg ggcgtgggac cgaggccgcc gcggggctgg 1260
   gcctgcgcgt ggcgggagcg cggggaggga ttgccgcggg ccggggaggg gcgggggcgg 1320
   gcgtgctgcc ctctgtggtc cttgggccgc cgccgcgggt ctgtcgtggt gcctggagcg 1380
   gctgtgctcg tcccttgctt ggccgtgttc tcgttcctga gggtcccgcg gacaccgagt 1440
   ggcgcagtgc caggcccagc ccggggatgg cgactgcgcc tgggcccgcc tggtgtcttc 1500
   gcatccctct ccgctttccg gcttcagcgc tctaggtcag ggagtcttcg cttttgtaca 1560
   gctctaaggc taggaatggt ttttatattt ttaaaaggct ttggaaaaca aaaatacgca 1620
   acagagaccg tttgtgtgac actttgcagg gaagtttgct ggcctctgtt ctaggtcatg 1680
   attgggctgc aagggcagag aaggtagcct tgaacagagg tccttttcct cctcctaagc 1740
   tccgggagcc agaggtttaa ctgacccttt tggggatttt tgagggcagt gatcttaact 1800
   ttgggtgcac agttagctta tttgaagatc ttactaaaaa tacaccagag cccaacctcc 1860
   gaccaattac atcaaaacct gtcctagtgc agggtgagta ttgctgtttt ttgaaagttt 1920
   ccaaaagtga ttttgatgtg cacctacgat tgagaactgt cgtttgagga cagtgggtgg 1980
   agtttcgtat ttggaaatta gaagacctgg agtttccatt acaccgaatt ggcacttaat 2040
   aactgttgtc ggagcatttc ttaagccaca ttttcgtaaa gtggctttaa aattgctctg 2100
   ccagtaggca ggttgctaag atggtcagag acaaacttct gaacgactct tgtaaaatat 2160
   acagaaatat tttcagaact tttatcagta aaattacaaa acgtgttgca aggaaggtgc 2220
   ttgtgataac actgtcccca gaaccttagt gaagttacca actggtggaa aattttctct 2280
   tgcactcggc ttaaaaatca tgagggaata tttactatac gaatgagatt cagtctttaa 2340
   aggggtttac agaaacgtga gaggacagga acagttagtc tgtgtaaatg tctgaaatat 2400
   atgtgaggga gataatgagt ttagcctttt tctttaatag gtctccagat tttctggaaa 2460
   aggttctttg gcatttgact ccattttgct gtttcatttg tcagacttct ttttgtccct 2520
   ctttacttct ccccacataa ttcaccagta ctagtgtttt gtttttcaga ccaagtctcg 2580
   ctctgtcgcc caggctggag tgcagtggcg cgatctcagc tcactgcaac ctccgcctcc 2640
   caggttcaag caattctcct gcctcagcct cccgggtagc tgggactaca ggcgcgcgcc 2700
   gccacgcctg gctaattttt tatattttag tagagacggc gtttcaccat gttggccagg 2760
   atggtctcga tctgttgacg tcgtgatcca cccgcctcgg cctcccaaag tgctgggatt 2820
   acaggcgtga gccaccccgc ccggccacca gtgctattct taagacgcct ctgaggaatc 2880
   ccttctccct ggccattgag aatccatgca tgaacccagg ttttccacct tccctgagca 2940
   gcttgcatag ttccttcttt taagcgcctg acttcgtttt gtttggtgcc cgttgtacct 3000
   gagaatgagc cttggatagt ggagcattcc agctttccag atatgcagag ataatacatt 3060
   ggctatcagc tacttggctt ggcctattcc gtgtttaaaa tcttggactc tttgctagtt 3120
   tttacagatc agaatttttc acgtattaat ccagttttcc tagcttctct tgaagaattt 3180
   ttggagatct cttcatactg agccttcatt agcccaggac agtactgctg tagcagttca 3240
   tatatttttt cgcttcccag gcctgtgtta ttcacttaag ttcatagcct ggtccctgca 3300
   gggttgtacc cgagcacagc tacttagatg tcctgaatgt attaccggtt aaatggaggt 3360
   ttcaaagaac ctgctgtttt tggccctgtg ctcttgataa cagagtgttt gagggacaac 3420
   tttcacattt gagtttttcc aaaattaaag gttgtagaag agtcacagta tctattgtca 3480
   aaaagaaaag aatttaaaaa ggcagcaatt gccaggatac ttcatttgag caatgatatt 3540
   ttccagtgga aagtcacatc ttaagggtta atgcccctta actgttggcc gtatttgaaa 3600
   acaaaccaag ctaaaaacaa gagacactga catgttgtat gacggtgtgg tgtggatgtt 3660
   gtgtttattt tagtcctgag atctagttgt aacttccttg atttctgtat gtagccacgg 3720
   agcaccatta cctgtcacca ttacctgaat ggctatactg cttgctttca ttttggtaga 3780
   gtggaaaggt tacctaggtt tcagtgcttg aaaagatttc agaaagcagt agtacgtctg 3840
   gttagactag aatcagtcct ctcctggggg cagtggaata taatattttc tgactgctaa 3900
   ttaaaaatac ctgtgatagc cgggcgtggt ggcttacgcc tgtaatccca gcactttggg 3960
   aggccgagac gggtggatca cgaggtcagc agatggagac catcctggct aacacggtga 4020
   aaccccgtct ctactaaaaa tgcaaaaaaa ttagccgggt gtggtggtgg gcgcctgtag 4080
   tcccagctac tcaggaggct gaggcaggag aatggcatga acctgggagg cggagcttgc 4140
   agtgagccga gatcatgtca ctgcactcca gcctgggcga cagagcgaga ctcgtctcaa 4200
   aaaaaaaaag aaaaaaactt atgatggaca cttaaaaaca ctcactgagt ggggagtgga 4260
   gagcaggggt cccagggtag cctgttggac atttccaggg cgactttttc tttttttttt 4320
   tttaaagtca agtgagtatg ccatatggaa aagggtgtgc gtggagaaaa agcaaggggc 4380
   tccagagtgt aggatgagac atacaccttt tgggttaaaa aggctgaggc aggagaatgg 4440
   cgtgaacccg ggaggcggag cttgcagtga gctgagatca tgccactgca ctccagcctg 4500
   ggcgacagag cgagactctt gtctcaaaat aaaaaacgtt tacatgtaca tgtatattca 4560
   acatgtacaa atataaccta ttcaaaagta tttactacat aaataggtac ttacattacc 4620
   tatttactgt aatagtcaaa gcctatgaag tatctaacac tgatgtgtag gtactcactt 4680
   tgcttgccac tctattaggt gctttttatg ttatttaatc atgaagcctg gccacagggt 4740
   gcttgtgcat tgagtgtggg aacaagatta ccatctccct tttgaggaca caggcctaga 4800
   gcagttaagc agcttgctgg aggttcactg gctagaaagt ggtcagcctg ggatttggac 4860
   acagattttt ccactcccaa gtctggctgc tttttacttc actgtgaggg gtaaaggtaa 4920
   atcagctgtt ttctttgttc agaaactctc tccaactttg cacttttctt aaaggaaagt 4980
   aatggaccag tgaaggtgtg gggaagcatt aaaggactga ctgaaggcct gcatggattc 5040
   catgttcatg agtttggaga taatacagca ggtgggtgtt gtgctgtgct ggtgacccat 5100
   acttgttcac cctagttaga taaacagtag agtagcccct aaacgttaaa acccctcaac 5160
   ttgtttttgt ttttgagaaa gggtcttgct ctgtcgctca ggctggagtg cagtggcgct 5220
   gtgcgatcat ggctgacctt agccttgacc tcccaggctc cattgatcct catgccttgg 5280
   cccgtagctg ggactacagg tacacaccac cacgcctggc taatttttgt atttttttct 5340
   agaggtgggg tttcatcatg ttgcccaggc tggtcttgaa ctgctgggct caagtggtct 5400
   atcctcctcg acctcccaaa gtgctgggat tacatgtgtg agccactgtg cctgggaaaa 5460
   ccctcaactt ttcttttaaa aaagaggtca actttattgt atataagcac tgtgctaaaa 5520
   ttgcaggaac tgggaccata tcctgatttt tgtaataatg ccagcagagt acacacaaga 5580
   aaagtaactg cactagattg tgaagactgg ggtggacctg cttctgaagg tccagtgccc 5640
   tttgtcttaa gatttggtgt agtgtgtctt tagaaaccaa aaaaagagaa gaagatcaac 5700
   cttaagatta gccacaaaac tgggctttga tacctaggtg tggaaaagaa agggaaagag 5760
   ttgatgtttt gtcttacagc atcattgtag aagagggtgt ttttttgttt gtttgttttt 5820
   tgagacggag tcttactctg tggcccaggc tggagtgcag tggcgcgatc tcggctcact 5880
   gcaagctccg cctcccgggt tcatgccatt ctcctgcctc agccccctga gtagctggga 5940
   ctacaggtgc ccgccacccc gcctggctaa ttttttgtat ttttagtaga gacggggttt 6000
   cactgtgtta gccaagatgg tctctctcct gacctcgtga tccgcctgtc tcagcctccc 6060
   aaagtgctgg gattacaggc atgagccacc gcacccagcc agaagagggt gttttttaaa 6120
   gaaggcaaat aggaaataaa aacttgggct cttaactttt gtaatgatcc caggtgtttg 6180
   agctgggggt tgagggtggg tgcctcgagc aaaggggctg catttatttg cataatgcca 6240
   tgtaagagta gctctacacc ccaaacacag gcttcttagt gggaccaaag tatgatacaa 6300
   actgaagatg gaatgcagag gattattggt actttggaat atgcttaaaa aaaatttttt 6360
   taaagtattt ttaaaaaatc aggcaacccc tgaaccagag taggttcaga gaaactgcca 6420
   aattttattt tcttaatttg ggattggaag caagttaaca gaagtttatg agttaagttg 6480
   catttagtga tcttttgcca tatttgagta ataatctgat ttttttgttt atagatttct 6540
   tcttaaatta actttattca tcttgctaat ttagtttcaa atagtgattt gtaatgatca 6600
   gatttgatcc atttctgtaa ttgctgaaat tcccccgagt tgctttttgg ctttaccgcc 6660
   tctggtctgg gaggtgattg ctctgctgct tcctgtaact tgcctgcctt tctccctgtg 6720
   tgggactcct gcgggtgaga gcgtggctga agacagccgt gttatgaaag ggcctcctgt 6780
   gctgtcgagg ttgtgctctg tgaatgtcat cccctggtgc acagcagcac cttctacaca 6840
   ggatacagtt ggaatgccgc cccctcgagt tgtgtaaggc agcagccttg gcccttgcac 6900
   ataagatgct gttgaatatt ctgcctgcac caagtaaagg gcacagatag aactgcttgg 6960
   catatgttgc tggggagatg agttttttgt aaagtatact acgttcttaa gaatttggat 7020
   cataaccatg ggattttaat aatagaaaaa ctgttgaaga tcagtctggt cccttatttt 7080
   tacagtgaag aagccaaagc ccagagaagg gtgttaactt tacaagtgtc agacagtagt 7140
   tagaacttgg tggggttttt tttttttttt ttttgagatg gagtcttgct ctgttgccca 7200
   ggctggagtg cagtggtgcg atctcagctc actgcaacct ctgcctccca ggttcaagcg 7260
   attctcctgc ctcagcctac taagtagctg ggactatagg tgcgcaccac cacgcctagc 7320
   taatttttgt attttttcag tagagacagg gttttgctat gctggccagg ctggtctcaa 7380
   actcctgacc tcagatgatc cagccacctc agcttcccaa agtgctgggg ttccaggtgt 7440
   tagccaccat gcctggccat agacttgttt ctgttccctt ctcactgtgg ctgtaccaag 7500
   gtgttgctta tcccagaagt cgtgatgcag gtcagcactt tctccatggg aagttttagc 7560
   agtgtttctt tttagaatgt atttgggaac tttaattcat aatttagctt ttttttcttc 7620
   ttcttataaa taggctgtac cagtgcaggt cctcacttta atcctctatc cagaaaacac 7680
   ggtgggccaa aggatgaaga gaggtaacaa gatgcttaac tcttgtaata atggcgatag 7740
   ctttctggag ttcatatggt atactacttg taaatatgtg ctaagataat tccgtgtttc 7800
   ccccaccttt gcttttgaac ttgctgactc atctaaaccc ctgctcccaa atgctggaat 7860
   gcttttactt cctgggctta aaggaattga caaatgggga cacttaaaac gatttggttt 7920
   tgtagcattt attgaatata gaactaatac aagtgccaaa ggggaactaa tacaggaaat 7980
   gtcatgaaca gtactgtcaa ccactagcaa aatcaatcat cattgtgaaa cataggaagc 8040
   ttctgtagat aaaaaaaaaa attgatactg aaaactagtc gagactccat ttatatgtgt 8100
   atgttttctg aaagcctttc agaaaaatat taaatttaag gacaagattt ttatatcaga 8160
   ggccttggga catagctttg ttagctatgc cagtaattaa caggcataac tcagtaactg 8220
   agagtttacc ctttggtact tctgaaatca ggtgcagccc catctttctt cccagagcat 8280
   tagtgtgtag acgtgaagcc ttgtttgaag agctgtattt agaatgccta gctacttgtt 8340
   tgcaaatttg tgtctactca gtcaagtttt aatttagctc atgaactacc ttgatgttta 8400
   gtggcatcag ccctaatcca tctgatgctt tttcattatt aggcatgttg gagacttggg 8460
   caatgtgact gctgacaaag atggtgtggc cgatgtgtct attgaagatt ctgtgatctc 8520
   actctcagga gaccattgca tcattggccg cacactggtg gtaagttttc ataaaaggat 8580
   atgcataaaa cttcttctaa catacagtca tgtatctttt cactttgatt gttagtcgcg 8640
   gtttctaaag atccagataa actgtacttg cagttcaaat taggaaaagc aattttattg 8700
   gacaattacg gtgaaaatga attattttat ctaggtcagt taagaacact gttctgctaa 8760
   gatgcagtaa aaagcaggtt acatttgacc atattagatc tgagtttgga aaacagaagt 8820
   agtctttagt tttaaaatgg ccagattttc ttgccaggat tgggtttctc acttgttaaa 8880
   cagaacattt tgttaagttt aaaacctggg atggacttaa gtattcatgt tcattcatgt 8940
   tcattcagga ctgcaggtta tcatgacttg tttaacttgt gggaagctgt tgtcccaagt 9000
   tatcctgggg aactgcatct ggttcttgca aaacaccaag tagacaggct ctcttttacc 9060
   tccccttgag ggcattaaca ttcagtagtc acttccattc agttaaccct ttatttttat 9120
   ggtttttctt gagccatagt tgtaaagcag aaaaatcatt tataaaggtt tgttgaacaa 9180
   aattcaaaat actgttgctt aaagtattaa gattttttag gattatacct tacttatagg 9240
   cccgtcattc atttggcatg aaattttgag ttttattcac tttcactttc ctttttttcc 9300
   aaagcaatta aaaaaactgc caaagtaaga gtgactgcgg aactaaggtt actgtaactt 9360
   accatggagg attaagggta gcgtgtggtg gtctacaaca tagttatttg ggttttagta 9420
   tttcatttag acagcaacac ttacctaatg tttaaaggta atgtctttgc aacaccaaga 9480
   aaaagctttg agtagtagtt tctactttta aactactaaa tattagtata tctctctact 9540
   aggattaatg ttatttttct aatattatga ggttcttaaa catcttttgg gtattgttgg 9600
   gaggaggtag tgattacttg acagcccaaa gttatcttct taaaattttt tacaggtcca 9660
   tgaaaaagca gatgacttgg gcaaaggtgg aaatgaagaa agtacaaaga caggaaacgc 9720
   tggaagtcgt ttggcttgtg gtgtaattgg gatcgcccaa taaacattcc cttggatgta 9780
   gtctgaggcc ccttaactca tctgttatcc tgctagctgt agaaatgtat cctgataaac 9840
   attaaacact gtaatcttaa aagtgtaatt gtgtgacttt ttcagagttg ctttaaagta 9900
   cctgtagtga gaaactgatt tatgatcact tggaagattt gtatagtttt ataaaactca 9960
   gttaaaatgt ctgtttcaat gacctgtatt ttgccagact taaatcacag atgggtatta 10020
   aacttgtcag aatttctttg tcattcaagc ctgtgaataa aaaccctgta tggcacttat 10080
   tatgaggcta ttaaaagaat ccaaattcaa actaaattag ctctgatact tatttatata 10140
   aacagcttca gtggaacaga tttagtaata ctaacagtga tagcatttta ttttgaaagt 10200
   gttttgagac catcaaaatg catactttaa aacagcaggt cttttagcta aaactaacac 10260
   aactctgctt agacaaatag gctgtccttt gaaagcttta gggaaatgtt cctgcttagt 10320
   cattttagca ttttgattca taaagtacct cctcatttta aaaagacatt atgatgtaag 10380
   agagccattt gataactttt tagtgagctt tgaaaggcaa gttacagcct cagctagcta 10440
   gtaagattat ctacctgcca gaatggcaca aattctacat tcaagggtag acgctggcac 10500
   aacctactta cagattagcc ctttaaagca atctgtagca ttagaagatg gaaccaagga 10560
   aatgtttgac tgtgggttct ggctgttgag aaataattta cacaccgaat tagtgaaatg 10620
   agtcactttc tcttaatgta tttatgtacc tgagagaatg cttttcaatg ttaacctaac 10680
   tcaggtttga ctaaattatt caattggaaa ttgtagaata ttatttctga taaaccagaa 10740
   ataagtgaaa tgctgtttgt tcataaatat gtactttatc aaatgtagga gagatcattt 10800
   aggagaggaa aagctaaatt ggaagacaaa tctgtagtgt ttccaaagtt ttaaaattat 10860
   ggtaaacaac agtatgttca cagtaagtgg ttaaaacaac cattctttaa atctcagtag 10920
   agaattttta aaaagcagta tttaacacat ttccctaatg tagtttgttg cctatgtgga 10980
   ataactcaat tagagactca c 11001
<210> 3
   <211> 13001
   <212> DNA
   <213> Macaca mulata
<400> 3
<210> 4
<400> 4
   000
<210> 5
   <211> 25
   <212> DNA
   <213> Artificial sequence
<220>
   <223> Primer
<400> 5
   aggagaaaaa caaagaacac cagaa 25
<210> 6
   <211> 25
   <212> DNA
   <213> Artificial sequence
<220>
   <223> Primer
<400> 6
   caattagggc aactcagaaa tagct 25
<210> 7
   <211> 22
   <212> DNA
   <213> Artificial sequence
<220>
   <223> Probe
<400> 7
   ccaactggtc ccccagccaa ga 22
<210> 8
   <211> 20
   <212> DNA
   <213> Artificial sequence
<220>
   <223> Primer
<400> 8
   gaaaccagcc tggacaccaa 20
<210> 9
   <211> 24
   <212> DNA
   <213> Artificial sequence
<220>
   <223> Primer
<400> 9
   tccacagtct ttaccacgat gttc 24
<210> 10
   <211> 25
   <212> DNA
   <213> Artificial sequence
<220>
   <223> Probe
<400> 10
   tccgtgtcag aaggccacct caaga 25
<210> 11
   <211> 22
   <212> DNA
   <213> Artificial sequence
<220>
   <223> Primer
<400> 11
   ctctcaggag accattgcat ca 22
<210> 12
   <211> 27
   <212> DNA
   <213> Artificial sequence
<220>
   <223> Primer
<400> 12
   tcctgtcttt gtactttctt catttcc 27
<210> 13
   <211> 24
   <212> DNA
   <213> Artificial sequence
<220>
   <223> Probe
<400> 13
   ccgcacactg gtggtccatg aaaa 24
<210> 14
   <211> 20
   <212> DNA
   <213> Artificial sequence
<220>
   <223> Primer
<400> 14
   cgtggcctag cgagttatgg 20
<210> 15
   <211> 20
   <212> DNA
   <213> Artificial sequence
<220>
   <223> Primer
<400> 15
   gaaattgatg atgccctgca 20
<210> 16
   <211> 21
   <212> DNA
   <213> Artificial sequence
<220>
   <223> Probe
<400> 16
   acgaaggccg tgtgcgtgct g 21
<210> 17
   <211> 26
   <212> DNA
   <213> Artificial sequence
<220>
   <223> Primer
<400> 17
   tggagataat acacaaggct gtacca 26
<210> 18
   <211> 23
   <212> DNA
   <213> Artificial sequence
<220>
   <223> Primer
<400> 18
   caacatgcct ctcttcatcc ttt 23
<210> 19
   <211> 27
   <212> DNA
   <213> Artificial sequence
<220>
   <223> Probe
<400> 19
   atcctctatc cagacaacac ggtgggc 27
<210> 20
<400> 20
   000
<210> 21
   <211> 20
   <212> DNA
   <213> Artificial sequence
<220>
   <223> Synthetic oligonucleotide
<400> 21
   ccgtcgccct tcagcacgca 20
<210> 22
   <211> 20
   <212> DNA
   <213> Artificial sequence
<220>
   <223> Synthetic oligonucleotide
<400> 22
   cactggtcca ttactttcct 20
<210> 23
   <211> 20
   <212> DNA
   <213> Artificial sequence
<220>
   <223> Synthetic oligonucleotide
<400> 23
   acaccttcac tggtccatta 20
<210> 24
   <211> 20
   <212> DNA
   <213> Artificial sequence
<220>
   <223> Synthetic oligonucleotide
<400> 24
   ccacaccttc actggtccat 20
<210> 25
   <211> 20
   <212> DNA
   <213> Artificial sequence
<220>
   <223> Synthetic oligonucleotide
<400> 25
   caacatgcct ctcttcatcc 20
<210> 26
   <211> 20
   <212> DNA
   <213> Artificial sequence
<220>
   <223> Synthetic oligonucleotide
<400> 26
   ccaacatgcc tctcttcatc 20
<210> 27
   <211> 20
   <212> DNA
   <213> Artificial sequence
<220>
   <223> Synthetic oligonucleotide
<400> 27
   tccaacatgc ctctcttcat 20
<210> 28
   <211> 20
   <212> DNA
   <213> Artificial sequence
<220>
   <223> Synthetic oligonucleotide
<400> 28
   ctccaacatg cctctcttca 20
<210> 29
   <211> 20
   <212> DNA
   <213> Artificial sequence
<220>
   <223> Synthetic oligonucleotide
<400> 29
   tctccaacat gcctctcttc 20
<210> 30
   <211> 20
   <212> DNA
   <213> Artificial sequence
<220>
   <223> Synthetic oligonucleotide
<400> 30
   gtctccaaca tgcctctctt 20
<210> 31
   <211> 20
   <212> DNA
   <213> Artificial sequence
<220>
   <223> Synthetic oligonucleotide
<400> 31
   cacctttgcc caagtcatct 20
<210> 32
   <211> 20
   <212> DNA
   <213> Artificial sequence
<220>
   <223> Synthetic oligonucleotide
<400> 32
   ccacctttgc ccaagtcatc 20
<210> 33
   <211> 20
   <212> DNA
   <213> Artificial sequence
<220>
   <223> Synthetic oligonucleotide
<400> 33
   tccacctttg cccaagtcat 20
<210> 34
   <211> 20
   <212> DNA
   <213> Artificial sequence
<220>
   <223> Synthetic oligonucleotide
<400> 34
   ttccaccttt gcccaagtca 20
<210> 35
   <211> 20
   <212> DNA
   <213> Artificial sequence
<220>
   <223> Synthetic oligonucleotide
<400> 35
   tttccacctt tgcccaagtc 20
<210> 36
   <211> 20
   <212> DNA
   <213> Artificial sequence
<220>
   <223> Synthetic oligonucleotide
<400> 36
   atttccacct ttgcccaagt 20
<210> 37
   <211> 20
   <212> DNA
   <213> Artificial sequence
<220>
   <223> Synthetic oligonucleotide
<400> 37
   catttccacc tttgcccaag 20
<210> 38
   <211> 20
   <212> DNA
   <213> Artificial sequence
<220>
   <223> Synthetic oligonucleotide
<400> 38
   tcatttccac ctttgcccaa 20
<210> 39
   <211> 20
   <212> DNA
   <213> Artificial sequence
<220>
   <223> Synthetic oligonucleotide
<400> 39
   ttcatttcca cctttgccca 20
<210> 40
   <211> 20
   <212> DNA
   <213> Artificial sequence
<220>
   <223> Synthetic oligonucleotide
<400> 40
   cttcatttcc acctttgccc 20
<210> 41
   <211> 20
   <212> DNA
   <213> Artificial sequence
<220>
   <223> Synthetic oligonucleotide
<400> 41
   tcttcatttc cacctttgcc 20
<210> 42
   <211> 20
   <212> DNA
   <213> Artificial sequence
<220>
   <223> Synthetic oligonucleotide
<400> 42
   ttcttcattt ccacctttgc 20
<210> 43
   <211> 20
   <212> DNA
   <213> Artificial sequence
<220>
   <223> Synthetic oligonucleotide
<400> 43
   tttcttcatt tccacctttg 20
<210> 44
   <211> 20
   <212> DNA
   <213> Artificial sequence
<220>
   <223> Synthetic oligonucleotide
<400> 44
   ctttcttcat ttccaccttt 20
<210> 45
   <211> 20
   <212> DNA
   <213> Artificial sequence
<220>
   <223> Synthetic oligonucleotide
<400> 45
   actttcttca tttccacctt 20
<210> 46
   <211> 20
   <212> DNA
   <213> Artificial sequence
<220>
   <223> Synthetic oligonucleotide
<400> 46
   tactttcttc atttccacct 20
<210> 47
   <211> 20
   <212> DNA
   <213> Artificial sequence
<220>
   <223> Synthetic oligonucleotide
<400> 47
   ggcgatccca attacaccac 20
<210> 48
   <211> 20
   <212> DNA
   <213> Artificial sequence
<220>
   <223> Synthetic oligonucleotide
<400> 48
   ggaatgttta ttgggcgatc 20
<210> 49
   <211> 20
   <212> DNA
   <213> Artificial sequence
<220>
   <223> Synthetic oligonucleotide
<400> 49
   cctcagacta catccaaggg 20
<210> 50
   <211> 20
   <212> DNA
   <213> Artificial sequence
<220>
   <223> Synthetic oligonucleotide
<400> 50
   atacaaatct tccaagtgat 20
<210> 51
   <211> 20
   <212> DNA
   <213> Artificial sequence
<220>
   <223> Synthetic oligonucleotide
<400> 51
   tgagttttat aaaactatac 20
<210> 52
   <211> 20
   <212> DNA
   <213> Artificial sequence
<220>
   <223> Synthetic oligonucleotide
<400> 52
   tcattgaaac agacatttta 20
<210> 53
   <211> 20
   <212> DNA
   <213> Artificial sequence
<220>
   <223> Synthetic oligonucleotide
<400> 53
   atacaggtca ttgaaacaga 20
<210> 54
   <211> 20
   <212> DNA
   <213> Artificial sequence
<220>
   <223> Synthetic oligonucleotide
<400> 54
   gtcgcccttc agcacgcaca 20
<210> 55
   <211> 20
   <212> DNA
   <213> Artificial sequence
<220>
   <223> Synthetic oligonucleotide
<400> 55
   tttaataccc atctgtgatt 20
<210> 56
   <211> 20
   <212> DNA
   <213> Artificial sequence
<220>
   <223> Synthetic oligonucleotide
<400> 56
   agtttaatac ccatctgtga 20
<210> 57
   <211> 20
   <212> DNA
   <213> Artificial sequence
<220>
   <223> Synthetic oligonucleotide
<400> 57
   ggtttttatt cacaggcttg 20
<210> 58
   <211> 20
   <212> DNA
   <213> Artificial sequence
<220>
   <223> Synthetic oligonucleotide
<400> 58
   agggttttta ttcacaggct 20
<210> 59
   <211> 20
   <212> DNA
   <213> Artificial sequence
<220>
   <223> Synthetic oligonucleotide
<400> 59
   atacagggtt tttattcaca 20
<210> 60
   <211> 20
   <212> DNA
   <213> Artificial sequence
<220>
   <223> Synthetic oligonucleotide
<400> 60
   ccatacaggg tttttattca 20
<210> 61
   <211> 20
   <212> DNA
   <213> Artificial sequence
<220>
   <223> Synthetic oligonucleotide
<400> 61
   acgctgcagg agactacgac 20
<210> 62
   <211> 20
   <212> DNA
   <213> Artificial sequence
<220>
   <223> Synthetic oligonucleotide
<400> 62
   cgaggactgc aacggaaacc 20
<210> 63
   <211> 20
   <212> DNA
   <213> Artificial sequence
<220>
   <223> Synthetic oligonucleotide
<400> 63
   taggccacgc cgaggtcctg 20
<210> 64
   <211> 20
   <212> DNA
   <213> Artificial sequence
<220>
   <223> Synthetic oligonucleotide
<400> 64
   ttcagcacgc acacggcctt 20
<210> 65
   <211> 20
   <212> DNA
   <213> Artificial sequence
<220>
   <223> Synthetic oligonucleotide
<400> 65
   ccttcagcac gcacacggcc 20
<210> 66
   <211> 20
   <212> DNA
   <213> Artificial sequence
<220>
   <223> Synthetic oligonucleotide
<400> 66
   gcccttcagc acgcacacgg 20
<210> 67
   <211> 20
   <212> DNA
   <213> Artificial sequence
<220>
   <223> Synthetic oligonucleotide
<400> 67
   tcgcccttca gcacgcacac 20
<210> 68
   <211> 20
   <212> DNA
   <213> Artificial sequence
<220>
   <223> Synthetic oligonucleotide
<400> 68
   cgtcgccctt cagcacgcac 20
<210> 69
   <211> 20
   <212> DNA
   <213> Artificial sequence
<220>
   <223> Synthetic oligonucleotide
<400> 69
   gccctgcact gggccgtcgc 20
<210> 70
   <211> 20
   <212> DNA
   <213> Artificial sequence
<220>
   <223> Synthetic oligonucleotide
<400> 70
   aattgatgat gccctgcact 20
<210> 71
   <211> 20
   <212> DNA
   <213> Artificial sequence
<220>
   <223> Synthetic oligonucleotide
<400> 71
   agtcctttaa tgcttcccca 20
<210> 72
   <211> 20
   <212> DNA
   <213> Artificial sequence
<220>
   <223> Synthetic oligonucleotide
<400> 72
   aggccttcag tcagtccttt 20
<210> 73
   <211> 20
   <212> DNA
   <213> Artificial sequence
<220>
   <223> Synthetic oligonucleotide
<400> 73
   gctgtattat ctccaaactc 20
<210> 74
   <211> 20
   <212> DNA
   <213> Artificial sequence
<220>
   <223> Synthetic oligonucleotide
<400> 74
   tgcccaagtc tccaacatgc 20
<210> 75
   <211> 20
   <212> DNA
   <213> Artificial sequence
<220>
   <223> Synthetic oligonucleotide
<400> 75
   acacatcggc cacaccatct 20
<210> 76
   <211> 20
   <212> DNA
   <213> Artificial sequence
<220>
   <223> Synthetic oligonucleotide
<400> 76
   tctcctgaga gtgagatcac 20
<210> 77
   <211> 20
   <212> DNA
   <213> Artificial sequence
<220>
   <223> Synthetic oligonucleotide
<400> 77
   accaccagtg tgcggccaat 20
<210> 78
   <211> 20
   <212> DNA
   <213> Artificial sequence
<220>
   <223> Synthetic oligonucleotide
<400> 78
   tcatggacca ccagtgtgcg 20
<210> 79
   <211> 20
   <212> DNA
   <213> Artificial sequence
<220>
   <223> Synthetic oligonucleotide
<400> 79
   gtactttctt catttccacc 20
<210> 80
   <211> 20
   <212> DNA
   <213> Artificial sequence
<220>
   <223> Synthetic oligonucleotide
<400> 80
   ttgtactttc ttcatttcca 20
<210> 81
   <211> 20
   <212> DNA
   <213> Artificial sequence
<220>
   <223> Synthetic oligonucleotide
<400> 81
   ctttgtactt tcttcatttc 20
<210> 82
   <211> 20
   <212> DNA
   <213> Artificial sequence
<220>
   <223> Synthetic oligonucleotide
<400> 82
   gataacagat gagttaaggg 20
<210> 83
   <211> 20
   <212> DNA
   <213> Artificial sequence
<220>
   <223> Synthetic oligonucleotide
<400> 83
   cacaattaca cttttaagat 20
<210> 84
   <211> 20
   <212> DNA
   <213> Artificial sequence
<220>
   <223> Synthetic oligonucleotide
<400> 84
   aatcagtttc tcactacagg 20
<210> 85
   <211> 20
   <212> DNA
   <213> Artificial sequence
<220>
   <223> Synthetic oligonucleotide
<400> 85
   cataaatcag tttctcacta 20
<210> 86
   <211> 20
   <212> DNA
   <213> Artificial sequence
<220>
   <223> Synthetic oligonucleotide
<400> 86
   aactgagttt tataaaacta 20
<210> 87
   <211> 20
   <212> DNA
   <213> Artificial sequence
<220>
   <223> Synthetic oligonucleotide
<400> 87
   ccatctgtga tttaagtctg 20
<210> 88
   <211> 20
   <212> DNA
   <213> Artificial sequence
<220>
   <223> Synthetic oligonucleotide
<400> 88
   aagtgccata cagggttttt 20
<210> 89
   <211> 20
   <212> DNA
   <213> Artificial sequence
<220>
   <223> Synthetic oligonucleotide
<400> 89
   taataagtgc catacagggt 20
<210> 90
   <211> 20
   <212> DNA
   <213> Artificial sequence
<220>
   <223> Synthetic oligonucleotide
<400> 90
   ctcataataa gtgccataca 20
<210> 91
   <211> 20
   <212> DNA
   <213> Artificial sequence
<220>
   <223> Synthetic oligonucleotide
<400> 91
   gcctcataat aagtgccata 20
<210> 92
   <211> 20
   <212> DNA
   <213> Artificial sequence
<220>
   <223> Synthetic oligonucleotide
<400> 92
   ttttaatagc ctcataataa 20
<210> 93
   <211> 20
   <212> DNA
   <213> Artificial sequence
<220>
   <223> Synthetic oligonucleotide
<400> 93
   ggattctttt aatagcctca 20
<210> 94
   <211> 20
   <212> DNA
   <213> Artificial sequence
<220>
   <223> Synthetic oligonucleotide
<400> 94
   ctcactacag gtactttaaa 20
<210> 95
   <211> 20
   <212> DNA
   <213> Artificial sequence
<220>
   <223> Synthetic oligonucleotide
<400> 95
   aatcttccaa gtgatcataa 20
<210> 96
   <211> 20
   <212> DNA
   <213> Artificial sequence
<220>
   <223> Synthetic oligonucleotide
<400> 96
   attgaaacag acattttaac 20
<210> 97
   <211> 20
   <212> DNA
   <213> Artificial sequence
<220>
   <223> Synthetic oligonucleotide
<400> 97
   caaagaaatt ctgacaagtt 20
<210> 98
   <211> 20
   <212> DNA
   <213> Artificial sequence
<220>
   <223> Synthetic oligonucleotide
<400> 98
   acaggcttga atgacaaaga 20
<210> 99
   <211> 20
   <212> DNA
   <213> Artificial sequence
<220>
   <223> Synthetic oligonucleotide
<400> 99
   tcataataag tgccatacag 20
<210> 100
   <211> 20
   <212> DNA
   <213> Artificial sequence
<220>
   <223> Synthetic oligonucleotide
<400> 100
   caggccttca gtcagtcctt 20
<210> 101
   <211> 20
   <212> DNA
   <213> Artificial sequence
<220>
   <223> Synthetic oligonucleotide
<400> 101
   cacattgccc aagtctccaa 20
<210> 102
   <211> 20
   <212> DNA
   <213> Artificial sequence
<220>
   <223> Synthetic oligonucleotide
<400> 102
   tcggccacac catctttgtc 20
<210> 103
   <211> 20
   <212> DNA
   <213> Artificial sequence
<220>
   <223> Synthetic oligonucleotide
<400> 103
   catcggccac accatctttg 20
<210> 104
   <211> 20
   <212> DNA
   <213> Artificial sequence
<220>
   <223> Synthetic oligonucleotide
<400> 104
   tagacacatc ggccacacca 20
<210> 105
   <211> 20
   <212> DNA
   <213> Artificial sequence
<220>
   <223> Synthetic oligonucleotide
<400> 105
   ctcctgagag tgagatcaca 20
<210> 106
   <211> 20
   <212> DNA
   <213> Artificial sequence
<220>
   <223> Synthetic oligonucleotide
<400> 106
   catggaccac cagtgtgcgg 20
<210> 107
   <211> 20
   <212> DNA
   <213> Artificial sequence
<220>
   <223> Synthetic oligonucleotide
<400> 107
   taggccagac ctccgcgcct 20
<210> 108
   <211> 20
   <212> DNA
   <213> Artificial sequence
<220>
   <223> Synthetic oligonucleotide
<400> 108
   actttatagg ccagacctcc 20
<210> 109
   <211> 20
   <212> DNA
   <213> Artificial sequence
<220>
   <223> Synthetic oligonucleotide
<400> 109
   gacgcaaacc agcaccccgt 20
<210> 110
   <211> 20
   <212> DNA
   <213> Artificial sequence
<220>
   <223> Synthetic oligonucleotide
<400> 110
   ggttccgagg actgcaacgg 20
<210> 111
   <211> 20
   <212> DNA
   <213> Artificial sequence
<220>
   <223> Synthetic oligonucleotide
<400> 111
   tcctggttcc gaggactgca 20
<210> 112
   <211> 20
   <212> DNA
   <213> Artificial sequence
<220>
   <223> Synthetic oligonucleotide
<400> 112
   gaggtcctgg ttccgaggac 20
<210> 113
   <211> 20
   <212> DNA
   <213> Artificial sequence
<220>
   <223> Synthetic oligonucleotide
<400> 113
   gtcgccataa ctcgctaggc 20
<210> 114
   <211> 20
   <212> DNA
   <213> Artificial sequence
<220>
   <223> Synthetic oligonucleotide
<400> 114
   tcagcacgca cacggccttc 20
<210> 115
   <211> 20
   <212> DNA
   <213> Artificial sequence
<220>
   <223> Synthetic oligonucleotide
<400> 115
   cttcagcacg cacacggcct 20
<210> 116
   <211> 20
   <212> DNA
   <213> Artificial sequence
<220>
   <223> Synthetic oligonucleotide
<400> 116
   cccttcagca cgcacacggc 20
<210> 117
   <211> 20
   <212> DNA
   <213> Artificial sequence
<220>
   <223> Synthetic oligonucleotide
<400> 117
   cgcccttcag cacgcacacg 20
<210> 118
   <211> 20
   <212> DNA
   <213> Artificial sequence
<220>
   <223> Synthetic oligonucleotide
<400> 118
   cgcccactct ggccccaaac 20
<210> 119
   <211> 20
   <212> DNA
   <213> Artificial sequence
<220>
   <223> Synthetic oligonucleotide
<400> 119
   ccgcgactac tttataggcc 20
<210> 120
   <211> 20
   <212> DNA
   <213> Artificial sequence
<220>
   <223> Synthetic oligonucleotide
<400> 120
   ccttctgctc gaaattgatg 20
<210> 121
   <211> 20
   <212> DNA
   <213> Artificial sequence
<220>
   <223> Synthetic oligonucleotide
<400> 121
   tccttctgct cgaaattgat 20
<210> 122
   <211> 20
   <212> DNA
   <213> Artificial sequence
<220>
   <223> Synthetic oligonucleotide
<400> 122
   ttccttctgc tcgaaattga 20
<210> 123
   <211> 20
   <212> DNA
   <213> Artificial sequence
<220>
   <223> Synthetic oligonucleotide
<400> 123
   tttccttctg ctcgaaattg 20
<210> 124
   <211> 20
   <212> DNA
   <213> Artificial sequence
<220>
   <223> Synthetic oligonucleotide
<400> 124
   ctttccttct gctcgaaatt 20
<210> 125
   <211> 20
   <212> DNA
   <213> Artificial sequence
<220>
   <223> Synthetic oligonucleotide
<400> 125
   actttccttc tgctcgaaat 20
<210> 126
   <211> 20
   <212> DNA
   <213> Artificial sequence
<220>
   <223> Synthetic oligonucleotide
<400> 126
   tactttcctt ctgctcgaaa 20
<210> 127
   <211> 20
   <212> DNA
   <213> Artificial sequence
<220>
   <223> Synthetic oligonucleotide
<400> 127
   ttactttcct tctgctcgaa 20
<210> 128
   <211> 20
   <212> DNA
   <213> Artificial sequence
<220>
   <223> Synthetic oligonucleotide
<400> 128
   attactttcc ttctgctcga 20
<210> 129
   <211> 20
   <212> DNA
   <213> Artificial sequence
<220>
   <223> Synthetic oligonucleotide
<400> 129
   cattactttc cttctgctcg 20
<210> 130
   <211> 20
   <212> DNA
   <213> Artificial sequence
<220>
   <223> Synthetic oligonucleotide
<400> 130
   ccattacttt ccttctgctc 20
<210> 131
   <211> 20
   <212> DNA
   <213> Artificial sequence
<220>
   <223> Synthetic oligonucleotide
<400> 131
   tccattactt tccttctgct 20
<210> 132
   <211> 20
   <212> DNA
   <213> Artificial sequence
<220>
   <223> Synthetic oligonucleotide
<400> 132
   gtccattact ttccttctgc 20
<210> 133
   <211> 20
   <212> DNA
   <213> Artificial sequence
<220>
   <223> Synthetic oligonucleotide
<400> 133
   ggtccattac tttccttctg 20
<210> 134
   <211> 20
   <212> DNA
   <213> Artificial sequence
<220>
   <223> Synthetic oligonucleotide
<400> 134
   tggtccatta ctttccttct 20
<210> 135
   <211> 20
   <212> DNA
   <213> Artificial sequence
<220>
   <223> Synthetic oligonucleotide
<400> 135
   ctggtccatt actttccttc 20
<210> 136
   <211> 20
   <212> DNA
   <213> Artificial sequence
<220>
   <223> Synthetic oligonucleotide
<400> 136
   actggtccat tactttcctt 20
<210> 137
   <211> 20
   <212> DNA
   <213> Artificial sequence
<220>
   <223> Synthetic oligonucleotide
<400> 137
   tcactggtcc attactttcc 20
<210> 138
   <211> 20
   <212> DNA
   <213> Artificial sequence
<220>
   <223> Synthetic oligonucleotide
<400> 138
   ttcactggtc cattactttc 20
<210> 139
   <211> 20
   <212> DNA
   <213> Artificial sequence
<220>
   <223> Synthetic oligonucleotide
<400> 139
   cttcactggt ccattacttt 20
<210> 140
   <211> 20
   <212> DNA
   <213> Artificial sequence
<220>
   <223> Synthetic oligonucleotide
<400> 140
   ccttcactgg tccattactt 20
<210> 141
   <211> 20
   <212> DNA
   <213> Artificial sequence
<220>
   <223> Synthetic oligonucleotide
<400> 141
   accttcactg gtccattact 20
<210> 142
   <211> 20
   <212> DNA
   <213> Artificial sequence
<220>
   <223> Synthetic oligonucleotide
<400> 142
   caccttcact ggtccattac 20
<210> 143
   <211> 20
   <212> DNA
   <213> Artificial sequence
<220>
   <223> Synthetic oligonucleotide
<400> 143
   cacaccttca ctggtccatt 20
<210> 144
   <211> 20
   <212> DNA
   <213> Artificial sequence
<220>
   <223> Synthetic oligonucleotide
<400> 144
   cccacacctt cactggtcca 20
<210> 145
   <211> 20
   <212> DNA
   <213> Artificial sequence
<220>
   <223> Synthetic oligonucleotide
<400> 145
   ccccacacct tcactggtcc 20
<210> 146
   <211> 20
   <212> DNA
   <213> Artificial sequence
<220>
   <223> Synthetic oligonucleotide
<400> 146
   tccccacacc ttcactggtc 20
<210> 147
   <211> 20
   <212> DNA
   <213> Artificial sequence
<220>
   <223> Synthetic oligonucleotide
<400> 147
   ttccccacac cttcactggt 20
<210> 148
   <211> 20
   <212> DNA
   <213> Artificial sequence
<220>
   <223> Synthetic oligonucleotide
<400> 148
   cttccccaca ccttcactgg 20
<210> 149
   <211> 20
   <212> DNA
   <213> Artificial sequence
<220>
   <223> Synthetic oligonucleotide
<400> 149
   gcttccccac accttcactg 20
<210> 150
   <211> 20
   <212> DNA
   <213> Artificial sequence
<220>
   <223> Synthetic oligonucleotide
<400> 150
   tgcttcccca caccttcact 20
<210> 151
   <211> 20
   <212> DNA
   <213> Artificial sequence
<220>
   <223> Synthetic oligonucleotide
<400> 151
   atgcttcccc acaccttcac 20
<210> 152
   <211> 20
   <212> DNA
   <213> Artificial sequence
<220>
   <223> Synthetic oligonucleotide
<400> 152
   aatgcttccc cacaccttca 20
<210> 153
   <211> 20
   <212> DNA
   <213> Artificial sequence
<220>
   <223> Synthetic oligonucleotide
<400> 153
   taatgcttcc ccacaccttc 20
<210> 154
   <211> 20
   <212> DNA
   <213> Artificial sequence
<220>
   <223> Synthetic oligonucleotide
<400> 154
   tccatgcagg ccttcagtca 20
<210> 155
   <211> 20
   <212> DNA
   <213> Artificial sequence
<220>
   <223> Synthetic oligonucleotide
<400> 155
   atccatgcag gccttcagtc 20
<210> 156
   <211> 20
   <212> DNA
   <213> Artificial sequence
<220>
   <223> Synthetic oligonucleotide
<400> 156
   aatccatgca ggccttcagt 20
<210> 157
   <211> 20
   <212> DNA
   <213> Artificial sequence
<220>
   <223> Synthetic oligonucleotide
<400> 157
   gaatccatgc aggccttcag 20
<210> 158
   <211> 20
   <212> DNA
   <213> Artificial sequence
<220>
   <223> Synthetic oligonucleotide
<400> 158
   ggaatccatg caggccttca 20
<210> 159
   <211> 20
   <212> DNA
   <213> Artificial sequence
<220>
   <223> Synthetic oligonucleotide
<400> 159
   tggaatccat gcaggccttc 20
<210> 160
   <211> 20
   <212> DNA
   <213> Artificial sequence
<220>
   <223> Synthetic oligonucleotide
<400> 160
   atggaatcca tgcaggcctt 20
<210> 161
   <211> 20
   <212> DNA
   <213> Artificial sequence
<220>
   <223> Synthetic oligonucleotide
<400> 161
   catggaatcc atgcaggcct 20
<210> 162
   <211> 20
   <212> DNA
   <213> Artificial sequence
<220>
   <223> Synthetic oligonucleotide
<400> 162
   acatggaatc catgcaggcc 20
<210> 163
   <211> 20
   <212> DNA
   <213> Artificial sequence
<220>
   <223> Synthetic oligonucleotide
<400> 163
   aacatggaat ccatgcaggc 20
<210> 164
   <211> 20
   <212> DNA
   <213> Artificial sequence
<220>
   <223> Synthetic oligonucleotide
<400> 164
   gaacatggaa tccatgcagg 20
<210> 165
   <211> 20
   <212> DNA
   <213> Artificial sequence
<220>
   <223> Synthetic oligonucleotide
<400> 165
   tgaacatgga atccatgcag 20
<210> 166
   <211> 20
   <212> DNA
   <213> Artificial sequence
<220>
   <223> Synthetic oligonucleotide
<400> 166
   atgaacatgg aatccatgca 20
<210> 167
   <211> 20
   <212> DNA
   <213> Artificial sequence
<220>
   <223> Synthetic oligonucleotide
<400> 167
   gacctgcact ggtacagcct 20
<210> 168
   <211> 20
   <212> DNA
   <213> Artificial sequence
<220>
   <223> Synthetic oligonucleotide
<400> 168
   ggacctgcac tggtacagcc 20
<210> 169
   <211> 20
   <212> DNA
   <213> Artificial sequence
<220>
   <223> Synthetic oligonucleotide
<400> 169
   aggacctgca ctggtacagc 20
<210> 170
   <211> 20
   <212> DNA
   <213> Artificial sequence
<220>
   <223> Synthetic oligonucleotide
<400> 170
   gaggacctgc actggtacag 20
<210> 171
   <211> 20
   <212> DNA
   <213> Artificial sequence
<220>
   <223> Synthetic oligonucleotide
<400> 171
   tgaggacctg cactggtaca 20
<210> 172
   <211> 20
   <212> DNA
   <213> Artificial sequence
<220>
   <223> Synthetic oligonucleotide
<400> 172
   gtgaggacct gcactggtac 20
<210> 173
   <211> 20
   <212> DNA
   <213> Artificial sequence
<220>
   <223> Synthetic oligonucleotide
<400> 173
   agtgaggacc tgcactggta 20
<210> 174
   <211> 20
   <212> DNA
   <213> Artificial sequence
<220>
   <223> Synthetic oligonucleotide
<400> 174
   aagtgaggac ctgcactggt 20
<210> 175
   <211> 20
   <212> DNA
   <213> Artificial sequence
<220>
   <223> Synthetic oligonucleotide
<400> 175
   aaagtgagga cctgcactgg 20
<210> 176
   <211> 20
   <212> DNA
   <213> Artificial sequence
<220>
   <223> Synthetic oligonucleotide
<400> 176
   taaagtgagg acctgcactg 20
<210> 177
   <211> 20
   <212> DNA
   <213> Artificial sequence
<220>
   <223> Synthetic oligonucleotide
<400> 177
   ttaaagtgag gacctgcact 20
<210> 178
   <211> 20
   <212> DNA
   <213> Artificial sequence
<220>
   <223> Synthetic oligonucleotide
<400> 178
   attaaagtga ggacctgcac 20
<210> 179
   <211> 20
   <212> DNA
   <213> Artificial sequence
<220>
   <223> Synthetic oligonucleotide
<400> 179
   gattaaagtg aggacctgca 20
<210> 180
   <211> 20
   <212> DNA
   <213> Artificial sequence
<220>
   <223> Synthetic oligonucleotide
<400> 180
   ggattaaagt gaggacctgc 20
<210> 181
   <211> 20
   <212> DNA
   <213> Artificial sequence
<220>
   <223> Synthetic oligonucleotide
<400> 181
   aggattaaag tgaggacctg 20
<210> 182
   <211> 20
   <212> DNA
   <213> Artificial sequence
<220>
   <223> Synthetic oligonucleotide
<400> 182
   gaggattaaa gtgaggacct 20
<210> 183
   <211> 20
   <212> DNA
   <213> Artificial sequence
<220>
   <223> Synthetic oligonucleotide
<400> 183
   agaggattaa agtgaggacc 20
<210> 184
   <211> 20
   <212> DNA
   <213> Artificial sequence
<220>
   <223> Synthetic oligonucleotide
<400> 184
   tagaggatta aagtgaggac 20
<210> 185
   <211> 20
   <212> DNA
   <213> Artificial sequence
<220>
   <223> Synthetic oligonucleotide
<400> 185
   atagaggatt aaagtgagga 20
<210> 186
   <211> 20
   <212> DNA
   <213> Artificial sequence
<220>
   <223> Synthetic oligonucleotide
<400> 186
   gatagaggat taaagtgagg 20
<210> 187
   <211> 20
   <212> DNA
   <213> Artificial sequence
<220>
   <223> Synthetic oligonucleotide
<400> 187
   ggatagagga ttaaagtgag 20
<210> 188
   <211> 20
   <212> DNA
   <213> Artificial sequence
<220>
   <223> Synthetic oligonucleotide
<400> 188
   tggatagagg attaaagtga 20
<210> 189
   <211> 20
   <212> DNA
   <213> Artificial sequence
<220>
   <223> Synthetic oligonucleotide
<400> 189
   ctggatagag gattaaagtg 20
<210> 190
   <211> 20
   <212> DNA
   <213> Artificial sequence
<220>
   <223> Synthetic oligonucleotide
<400> 190
   tctggataga ggattaaagt 20
<210> 191
   <211> 20
   <212> DNA
   <213> Artificial sequence
<220>
   <223> Synthetic oligonucleotide
<400> 191
   atcctttggc ccaccgtgtt 20
<210> 192
   <211> 20
   <212> DNA
   <213> Artificial sequence
<220>
   <223> Synthetic oligonucleotide
<400> 192
   catcctttgg cccaccgtgt 20
<210> 193
   <211> 20
   <212> DNA
   <213> Artificial sequence
<220>
   <223> Synthetic oligonucleotide
<400> 193
   tcatcctttg gcccaccgtg 20
<210> 194
   <211> 20
   <212> DNA
   <213> Artificial sequence
<220>
   <223> Synthetic oligonucleotide
<400> 194
   ttcatccttt ggcccaccgt 20
<210> 195
   <211> 20
   <212> DNA
   <213> Artificial sequence
<220>
   <223> Synthetic oligonucleotide
<400> 195
   cttcatcctt tggcccaccg 20
<210> 196
   <211> 20
   <212> DNA
   <213> Artificial sequence
<220>
   <223> Synthetic oligonucleotide
<400> 196
   tcttcatcct ttggcccacc 20
<210> 197
   <211> 20
   <212> DNA
   <213> Artificial sequence
<220>
   <223> Synthetic oligonucleotide
<400> 197
   ctcttcatcc tttggcccac 20
<210> 198
   <211> 20
   <212> DNA
   <213> Artificial sequence
<220>
   <223> Synthetic oligonucleotide
<400> 198
   tctcttcatc ctttggccca 20
<210> 199
   <211> 20
   <212> DNA
   <213> Artificial sequence
<220>
   <223> Synthetic oligonucleotide
<400> 199
   ctctcttcat cctttggccc 20
<210> 200
   <211> 20
   <212> DNA
   <213> Artificial sequence
<220>
   <223> Synthetic oligonucleotide
<400> 200
   cctctcttca tcctttggcc 20
<210> 201
   <211> 20
   <212> DNA
   <213> Artificial sequence
<220>
   <223> Synthetic oligonucleotide
<400> 201
   gcctctcttc atcctttggc 20
<210> 202
   <211> 20
   <212> DNA
   <213> Artificial sequence
<220>
   <223> Synthetic oligonucleotide
<400> 202
   tgcctctctt catcctttgg 20
<210> 203
   <211> 20
   <212> DNA
   <213> Artificial sequence
<220>
   <223> Synthetic oligonucleotide
<400> 203
   atgcctctct tcatcctttg 20
<210> 204
   <211> 20
   <212> DNA
   <213> Artificial sequence
<220>
   <223> Synthetic oligonucleotide
<400> 204
   catgcctctc ttcatccttt 20
<210> 205
   <211> 20
   <212> DNA
   <213> Artificial sequence
<220>
   <223> Synthetic oligonucleotide
<400> 205
   acatgcctct cttcatcctt 20
<210> 206
   <211> 20
   <212> DNA
   <213> Artificial sequence
<220>
   <223> Synthetic oligonucleotide
<400> 206
   aacatgcctc tcttcatcct 20
<210> 207
   <211> 20
   <212> DNA
   <213> Artificial sequence
<220>
   <223> Synthetic oligonucleotide
<400> 207
   tgctttttca tggaccacca 20
<210> 208
   <211> 20
   <212> DNA
   <213> Artificial sequence
<220>
   <223> Synthetic oligonucleotide
<400> 208
   ctgctttttc atggaccacc 20
<210> 209
   <211> 20
   <212> DNA
   <213> Artificial sequence
<220>
   <223> Synthetic oligonucleotide
<400> 209
   tctgcttttt catggaccac 20
<210> 210
   <211> 20
   <212> DNA
   <213> Artificial sequence
<220>
   <223> Synthetic oligonucleotide
<400> 210
   atctgctttt tcatggacca 20
<210> 211
   <211> 20
   <212> DNA
   <213> Artificial sequence
<220>
   <223> Synthetic oligonucleotide
<400> 211
   catctgcttt ttcatggacc 20
<210> 212
   <211> 20
   <212> DNA
   <213> Artificial sequence
<220>
   <223> Synthetic oligonucleotide
<400> 212
   tcatctgctt tttcatggac 20
<210> 213
   <211> 20
   <212> DNA
   <213> Artificial sequence
<220>
   <223> Synthetic oligonucleotide
<400> 213
   gtcatctgct ttttcatgga 20
<210> 214
   <211> 20
   <212> DNA
   <213> Artificial sequence
<220>
   <223> Synthetic oligonucleotide
<400> 214
   agtcatctgc tttttcatgg 20
<210> 215
   <211> 20
   <212> DNA
   <213> Artificial sequence
<220>
   <223> Synthetic oligonucleotide
<400> 215
   aagtcatctg ctttttcatg 20
<210> 216
   <211> 20
   <212> DNA
   <213> Artificial sequence
<220>
   <223> Synthetic oligonucleotide
<400> 216
   caagtcatct gctttttcat 20
<210> 217
   <211> 20
   <212> DNA
   <213> Artificial sequence
<220>
   <223> Synthetic oligonucleotide
<400> 217
   ccaagtcatc tgctttttca 20
<210> 218
   <211> 20
   <212> DNA
   <213> Artificial sequence
<220>
   <223> Synthetic oligonucleotide
<400> 218
   cccaagtcat ctgctttttc 20
<210> 219
   <211> 20
   <212> DNA
   <213> Artificial sequence
<220>
   <223> Synthetic oligonucleotide
<400> 219
   gcccaagtca tctgcttttt 20
<210> 220
   <211> 20
   <212> DNA
   <213> Artificial sequence
<220>
   <223> Synthetic oligonucleotide
<400> 220
   tgcccaagtc atctgctttt 20
<210> 221
   <211> 20
   <212> DNA
   <213> Artificial sequence
<220>
   <223> Synthetic oligonucleotide
<400> 221
   ttgcccaagt catctgcttt 20
<210> 222
   <211> 20
   <212> DNA
   <213> Artificial sequence
<220>
   <223> Synthetic oligonucleotide
<400> 222
   tttgcccaag tcatctgctt 20
<210> 223
   <211> 20
   <212> DNA
   <213> Artificial sequence
<220>
   <223> Synthetic oligonucleotide
<400> 223
   ctttgcccaa gtcatctgct 20
<210> 224
   <211> 20
   <212> DNA
   <213> Artificial sequence
<220>
   <223> Synthetic oligonucleotide
<400> 224
   cctttgccca agtcatctgc 20
<210> 225
   <211> 20
   <212> DNA
   <213> Artificial sequence
<220>
   <223> Synthetic oligonucleotide
<400> 225
   acctttgccc aagtcatctg 20
<210> 226
   <211> 20
   <212> DNA
   <213> Artificial sequence
<220>
   <223> Synthetic oligonucleotide
<400> 226
   cccaattaca ccacaagcca 20
<210> 227
   <211> 20
   <212> DNA
   <213> Artificial sequence
<220>
   <223> Synthetic oligonucleotide
<400> 227
   tcccaattac accacaagcc 20
<210> 228
   <211> 20
   <212> DNA
   <213> Artificial sequence
<220>
   <223> Synthetic oligonucleotide
<400> 228
   atcccaatta caccacaagc 20
<210> 229
   <211> 20
   <212> DNA
   <213> Artificial sequence
<220>
   <223> Synthetic oligonucleotide
<400> 229
   gatcccaatt acaccacaag 20
<210> 230
   <211> 20
   <212> DNA
   <213> Artificial sequence
<220>
   <223> Synthetic oligonucleotide
<400> 230
   cgatcccaat tacaccacaa 20
<210> 231
   <211> 20
   <212> DNA
   <213> Artificial sequence
<220>
   <223> Synthetic oligonucleotide
<400> 231
   gcgatcccaa ttacaccaca 20
<210> 232
   <211> 20
   <212> DNA
   <213> Artificial sequence
<220>
   <223> Synthetic oligonucleotide
<400> 232
   gggcgatccc aattacacca 20
<210> 233
   <211> 20
   <212> DNA
   <213> Artificial sequence
<220>
   <223> Synthetic oligonucleotide
<400> 233
   tgggcgatcc caattacacc 20
<210> 234
   <211> 20
   <212> DNA
   <213> Artificial sequence
<220>
   <223> Synthetic oligonucleotide
<400> 234
   ttgggcgatc ccaattacac 20
<210> 235
   <211> 20
   <212> DNA
   <213> Artificial sequence
<220>
   <223> Synthetic oligonucleotide
<400> 235
   attgggcgat cccaattaca 20
<210> 236
   <211> 20
   <212> DNA
   <213> Artificial sequence
<220>
   <223> Synthetic oligonucleotide
<400> 236
   tattgggcga tcccaattac 20
<210> 237
   <211> 20
   <212> DNA
   <213> Artificial sequence
<220>
   <223> Synthetic oligonucleotide
<400> 237
   ttattgggcg atcccaatta 20
<210> 238
   <211> 20
   <212> DNA
   <213> Artificial sequence
<220>
   <223> Synthetic oligonucleotide
<400> 238
   tttattgggc gatcccaatt 20
<210> 239
   <211> 20
   <212> DNA
   <213> Artificial sequence
<220>
   <223> Synthetic oligonucleotide
<400> 239
   gtttattggg cgatcccaat 20
<210> 240
   <211> 20
   <212> DNA
   <213> Artificial sequence
<220>
   <223> Synthetic oligonucleotide
<400> 240
   tgtttattgg gcgatcccaa 20
<210> 241
   <211> 20
   <212> DNA
   <213> Artificial sequence
<220>
   <223> Synthetic oligonucleotide
<400> 241
   atgtttattg ggcgatccca 20
<210> 242
   <211> 20
   <212> DNA
   <213> Artificial sequence
<220>
   <223> Synthetic oligonucleotide
<400> 242
   aatgtttatt gggcgatccc 20
<210> 243
   <211> 20
   <212> DNA
   <213> Artificial sequence
<220>
   <223> Synthetic oligonucleotide
<400> 243
   gaatgtttat tgggcgatcc 20
<210> 244
   <211> 20
   <212> DNA
   <213> Artificial sequence
<220>
   <223> Synthetic oligonucleotide
<400> 244
   tccaagggaa tgtttattgg 20
<210> 245
   <211> 20
   <212> DNA
   <213> Artificial sequence
<220>
   <223> Synthetic oligonucleotide
<400> 245
   atccaaggga atgtttattg 20
<210> 246
   <211> 20
   <212> DNA
   <213> Artificial sequence
<220>
   <223> Synthetic oligonucleotide
<400> 246
   catccaaggg aatgtttatt 20
<210> 247
   <211> 20
   <212> DNA
   <213> Artificial sequence
<220>
   <223> Synthetic oligonucleotide
<400> 247
   acatccaagg gaatgtttat 20
<210> 248
   <211> 20
   <212> DNA
   <213> Artificial sequence
<220>
   <223> Synthetic oligonucleotide
<400> 248
   tacatccaag ggaatgttta 20
<210> 249
   <211> 20
   <212> DNA
   <213> Artificial sequence
<220>
   <223> Synthetic oligonucleotide
<400> 249
   ctacatccaa gggaatgttt 20
<210> 250
   <211> 20
   <212> DNA
   <213> Artificial sequence
<220>
   <223> Synthetic oligonucleotide
<400> 250
   actacatcca agggaatgtt 20
<210> 251
   <211> 20
   <212> DNA
   <213> Artificial sequence
<220>
   <223> Synthetic oligonucleotide
<400> 251
   gactacatcc aagggaatgt 20
<210> 252
   <211> 20
   <212> DNA
   <213> Artificial sequence
<220>
   <223> Synthetic oligonucleotide
<400> 252
   agactacatc caagggaatg 20
<210> 253
   <211> 20
   <212> DNA
   <213> Artificial sequence
<220>
   <223> Synthetic oligonucleotide
<400> 253
   cagactacat ccaagggaat 20
<210> 254
   <211> 20
   <212> DNA
   <213> Artificial sequence
<220>
   <223> Synthetic oligonucleotide
<400> 254
   tcagactaca tccaagggaa 20
<210> 255
   <211> 20
   <212> DNA
   <213> Artificial sequence
<220>
   <223> Synthetic oligonucleotide
<400> 255
   ctcagactac atccaaggga 20
<210> 256
   <211> 20
   <212> DNA
   <213> Artificial sequence
<220>
   <223> Synthetic oligonucleotide
<400> 256
   gcctcagact acatccaagg 20
<210> 257
   <211> 20
   <212> DNA
   <213> Artificial sequence
<220>
   <223> Synthetic oligonucleotide
<400> 257
   ggcctcagac tacatccaag 20
<210> 258
   <211> 20
   <212> DNA
   <213> Artificial sequence
<220>
   <223> Synthetic oligonucleotide
<400> 258
   gggcctcaga ctacatccaa 20
<210> 259
   <211> 20
   <212> DNA
   <213> Artificial sequence
<220>
   <223> Synthetic oligonucleotide
<400> 259
   caggataaca gatgagttaa 20
<210> 260
   <211> 20
   <212> DNA
   <213> Artificial sequence
<220>
   <223> Synthetic oligonucleotide
<400> 260
   gcaggataac agatgagtta 20
<210> 261
   <211> 20
   <212> DNA
   <213> Artificial sequence
<220>
   <223> Synthetic oligonucleotide
<400> 261
   agcaggataa cagatgagtt 20
<210> 262
   <211> 20
   <212> DNA
   <213> Artificial sequence
<220>
   <223> Synthetic oligonucleotide
<400> 262
   tagcaggata acagatgagt 20
<210> 263
   <211> 20
   <212> DNA
   <213> Artificial sequence
<220>
   <223> Synthetic oligonucleotide
<400> 263
   ctagcaggat aacagatgag 20
<210> 264
   <211> 20
   <212> DNA
   <213> Artificial sequence
<220>
   <223> Synthetic oligonucleotide
<400> 264
   gctagcagga taacagatga 20
<210> 265
   <211> 20
   <212> DNA
   <213> Artificial sequence
<220>
   <223> Synthetic oligonucleotide
<400> 265
   agctagcagg ataacagatg 20
<210> 266
   <211> 20
   <212> DNA
   <213> Artificial sequence
<220>
   <223> Synthetic oligonucleotide
<400> 266
   cagctagcag gataacagat 20
<210> 267
   <211> 20
   <212> DNA
   <213> Artificial sequence
<220>
   <223> Synthetic oligonucleotide
<400> 267
   acagctagca ggataacaga 20
<210> 268
   <211> 20
   <212> DNA
   <213> Artificial sequence
<220>
   <223> Synthetic oligonucleotide
<400> 268
   tacagctagc aggataacag 20
<210> 269
   <211> 20
   <212> DNA
   <213> Artificial sequence
<220>
   <223> Synthetic oligonucleotide
<400> 269
   ctacagctag caggataaca 20
<210> 270
   <211> 20
   <212> DNA
   <213> Artificial sequence
<220>
   <223> Synthetic oligonucleotide
<400> 270
   tctacagcta gcaggataac 20
<210> 271
   <211> 20
   <212> DNA
   <213> Artificial sequence
<220>
   <223> Synthetic oligonucleotide
<400> 271
   ttctacagct agcaggataa 20
<210> 272
   <211> 20
   <212> DNA
   <213> Artificial sequence
<220>
   <223> Synthetic oligonucleotide
<400> 272
   tttctacagc tagcaggata 20
<210> 273
   <211> 20
   <212> DNA
   <213> Artificial sequence
<220>
   <223> Synthetic oligonucleotide
<400> 273
   atttctacag ctagcaggat 20
<210> 274
   <211> 20
   <212> DNA
   <213> Artificial sequence
<220>
   <223> Synthetic oligonucleotide
<400> 274
   catttctaca gctagcagga 20
<210> 275
   <211> 20
   <212> DNA
   <213> Artificial sequence
<220>
   <223> Synthetic oligonucleotide
<400> 275
   acatttctac agctagcagg 20
<210> 276
   <211> 20
   <212> DNA
   <213> Artificial sequence
<220>
   <223> Synthetic oligonucleotide
<400> 276
   tacatttcta cagctagcag 20
<210> 277
   <211> 20
   <212> DNA
   <213> Artificial sequence
<220>
   <223> Synthetic oligonucleotide
<400> 277
   atacatttct acagctagca 20
<210> 278
   <211> 20
   <212> DNA
   <213> Artificial sequence
<220>
   <223> Synthetic oligonucleotide
<400> 278
   gatacatttc tacagctagc 20
<210> 279
   <211> 20
   <212> DNA
   <213> Artificial sequence
<220>
   <223> Synthetic oligonucleotide
<400> 279
   acagtgttta atgtttatca 20
<210> 280
   <211> 20
   <212> DNA
   <213> Artificial sequence
<220>
   <223> Synthetic oligonucleotide
<400> 280
   tacagtgttt aatgtttatc 20
<210> 281
   <211> 20
   <212> DNA
   <213> Artificial sequence
<220>
   <223> Synthetic oligonucleotide
<400> 281
   ttacagtgtt taatgtttat 20
<210> 282
   <211> 20
   <212> DNA
   <213> Artificial sequence
<220>
   <223> Synthetic oligonucleotide
<400> 282
   attacagtgt ttaatgttta 20
<210> 283
   <211> 20
   <212> DNA
   <213> Artificial sequence
<220>
   <223> Synthetic oligonucleotide
<400> 283
   gattacagtg tttaatgttt 20
<210> 284
   <211> 20
   <212> DNA
   <213> Artificial sequence
<220>
   <223> Synthetic oligonucleotide
<400> 284
   agattacagt gtttaatgtt 20
<210> 285
   <211> 20
   <212> DNA
   <213> Artificial sequence
<220>
   <223> Synthetic oligonucleotide
<400> 285
   aagattacag tgtttaatgt 20
<210> 286
   <211> 20
   <212> DNA
   <213> Artificial sequence
<220>
   <223> Synthetic oligonucleotide
<400> 286
   taagattaca gtgtttaatg 20
<210> 287
   <211> 20
   <212> DNA
   <213> Artificial sequence
<220>
   <223> Synthetic oligonucleotide
<400> 287
   ttaagattac agtgtttaat 20
<210> 288
   <211> 20
   <212> DNA
   <213> Artificial sequence
<220>
   <223> Synthetic oligonucleotide
<400> 288
   caaatcttcc aagtgatcat 20
<210> 289
   <211> 20
   <212> DNA
   <213> Artificial sequence
<220>
   <223> Synthetic oligonucleotide
<400> 289
   acaaatcttc caagtgatca 20
<210> 290
   <211> 20
   <212> DNA
   <213> Artificial sequence
<220>
   <223> Synthetic oligonucleotide
<400> 290
   tacaaatctt ccaagtgatc 20
<210> 291
   <211> 20
   <212> DNA
   <213> Artificial sequence
<220>
   <223> Synthetic oligonucleotide
<400> 291
   tatacaaatc ttccaagtga 20
<210> 292
   <211> 20
   <212> DNA
   <213> Artificial sequence
<220>
   <223> Synthetic oligonucleotide
<400> 292
   ctatacaaat cttccaagtg 20
<210> 293
   <211> 20
   <212> DNA
   <213> Artificial sequence
<220>
   <223> Synthetic oligonucleotide
<400> 293
   actatacaaa tcttccaagt 20
<210> 294
   <211> 20
   <212> DNA
   <213> Artificial sequence
<220>
   <223> Synthetic oligonucleotide
<400> 294
   aactatacaa atcttccaag 20
<210> 295
   <211> 20
   <212> DNA
   <213> Artificial sequence
<220>
   <223> Synthetic oligonucleotide
<400> 295
   aaactataca aatcttccaa 20
<210> 296
   <211> 20
   <212> DNA
   <213> Artificial sequence
<220>
   <223> Synthetic oligonucleotide
<400> 296
   aaaactatac aaatcttcca 20
<210> 297
   <211> 20
   <212> DNA
   <213> Artificial sequence
<220>
   <223> Synthetic oligonucleotide
<400> 297
   taaaactata caaatcttcc 20
<210> 298
   <211> 20
   <212> DNA
   <213> Artificial sequence
<220>
   <223> Synthetic oligonucleotide
<400> 298
   ataaaactat acaaatcttc 20
<210> 299
   <211> 20
   <212> DNA
   <213> Artificial sequence
<220>
   <223> Synthetic oligonucleotide
<400> 299
   tataaaacta tacaaatctt 20
<210> 300
   <211> 20
   <212> DNA
   <213> Artificial sequence
<220>
   <223> Synthetic oligonucleotide
<400> 300
   ttataaaact atacaaatct 20
<210> 301
   <211> 20
   <212> DNA
   <213> Artificial sequence
<220>
   <223> Synthetic oligonucleotide
<400> 301
   tttataaaac tatacaaatc 20
<210> 302
   <211> 20
   <212> DNA
   <213> Artificial sequence
<220>
   <223> Synthetic oligonucleotide
<400> 302
   ttttataaaa ctatacaaat 20
<210> 303
   <211> 20
   <212> DNA
   <213> Artificial sequence
<220>
   <223> Synthetic oligonucleotide
<400> 303
   gttttataaa actatacaaa 20
<210> 304
   <211> 20
   <212> DNA
   <213> Artificial sequence
<220>
   <223> Synthetic oligonucleotide
<400> 304
   agttttataa aactatacaa 20
<210> 305
   <211> 20
   <212> DNA
   <213> Artificial sequence
<220>
   <223> Synthetic oligonucleotide
<400> 305
   gagttttata aaactataca 20
<210> 306
   <211> 20
   <212> DNA
   <213> Artificial sequence
<220>
   <223> Synthetic oligonucleotide
<400> 306
   cattgaaaca gacattttaa 20
<210> 307
   <211> 20
   <212> DNA
   <213> Artificial sequence
<220>
   <223> Synthetic oligonucleotide
<400> 307
   gtcattgaaa cagacatttt 20
<210> 308
   <211> 20
   <212> DNA
   <213> Artificial sequence
<220>
   <223> Synthetic oligonucleotide
<400> 308
   ggtcattgaa acagacattt 20
<210> 309
   <211> 20
   <212> DNA
   <213> Artificial sequence
<220>
   <223> Synthetic oligonucleotide
<400> 309
   aggtcattga aacagacatt 20
<210> 310
   <211> 20
   <212> DNA
   <213> Artificial sequence
<220>
   <223> Synthetic oligonucleotide
<400> 310
   caggtcattg aaacagacat 20
<210> 311
   <211> 20
   <212> DNA
   <213> Artificial sequence
<220>
   <223> Synthetic oligonucleotide
<400> 311
   acaggtcatt gaaacagaca 20
<210> 312
   <211> 20
   <212> DNA
   <213> Artificial sequence
<220>
   <223> Synthetic oligonucleotide
<400> 312
   tacaggtcat tgaaacagac 20
<210> 313
   <211> 20
   <212> DNA
   <213> Artificial sequence
<220>
   <223> Synthetic oligonucleotide
<400> 313
   aatacaggtc attgaaacag 20
<210> 314
   <211> 20
   <212> DNA
   <213> Artificial sequence
<220>
   <223> Synthetic oligonucleotide
<400> 314
   aaatacaggt cattgaaaca 20
<210> 315
   <211> 20
   <212> DNA
   <213> Artificial sequence
<220>
   <223> Synthetic oligonucleotide
<400> 315
   aaaatacagg tcattgaaac 20
<210> 316
   <211> 20
   <212> DNA
   <213> Artificial sequence
<220>
   <223> Synthetic oligonucleotide
<400> 316
   caaaatacag gtcattgaaa 20
<210> 317
   <211> 20
   <212> DNA
   <213> Artificial sequence
<220>
   <223> Synthetic oligonucleotide
<400> 317
   ccttgccttc tgctcgaaat 20
<210> 318
   <211> 20
   <212> DNA
   <213> Artificial sequence
<220>
   <223> Synthetic oligonucleotide
<400> 318
   aataaagttg acctcttttt 20
<210> 319
   <211> 20
   <212> DNA
   <213> Artificial sequence
<220>
   <223> Synthetic oligonucleotide
<400> 319
   ctctgatata aaaatcttgt 20
<210> 320
   <211> 20
   <212> DNA
   <213> Artificial sequence
<220>
   <223> Synthetic oligonucleotide
<400> 320
   gccccgcggc ggcctcggtc 20
<210> 321
   <211> 20
   <212> DNA
   <213> Artificial sequence
<220>
   <223> Synthetic oligonucleotide
<400> 321
   gctatcgcca ttattacaag 20
<210> 322
   <211> 20
   <212> DNA
   <213> Artificial sequence
<220>
   <223> Synthetic oligonucleotide
<400> 322
   ctcaaatgtg aaagttgtcc 20
<210> 323
   <211> 20
   <212> DNA
   <213> Artificial sequence
<220>
   <223> Synthetic oligonucleotide
<400> 323
   gttctatatt caataaatgc 20
<210> 324
   <211> 20
   <212> DNA
   <213> Artificial sequence
<220>
   <223> Synthetic oligonucleotide
<400> 324
   aattaaagtt cccaaataca 20
<210> 325
   <211> 20
   <212> DNA
   <213> Artificial sequence
<220>
   <223> Synthetic oligonucleotide
<400> 325
   gatcattaca aaagttaaga 20
<210> 326
   <211> 20
   <212> DNA
   <213> Artificial sequence
<220>
   <223> Synthetic oligonucleotide
<400> 326
   ccttctctgc ccttgcagcc 20
<210> 327
   <211> 20
   <212> DNA
   <213> Artificial sequence
<220>
   <223> Synthetic oligonucleotide
<400> 327
   acccaaataa ctatgttgta 20
<210> 328
   <211> 20
   <212> DNA
   <213> Artificial sequence
<220>
   <223> Synthetic oligonucleotide
<400> 328
   ccaggtttta aacttaacaa 20
<210> 329
   <211> 20
   <212> DNA
   <213> Artificial sequence
<220>
   <223> Synthetic oligonucleotide
<400> 329
   atctcaggac taaaataaac 20
<210> 330
   <211> 20
   <212> DNA
   <213> Artificial sequence
<220>
   <223> Synthetic oligonucleotide
<400> 330
   aaataactat gttgtagacc 20
<210> 331
   <211> 20
   <212> DNA
   <213> Artificial sequence
<220>
   <223> Synthetic oligonucleotide
<400> 331
   aagaaccttt tccagaaaat 20
<210> 332
   <211> 20
   <212> DNA
   <213> Artificial sequence
<220>
   <223> Synthetic oligonucleotide
<400> 332
   ggaacagaaa caagtctatg 20
<210> 333
   <211> 20
   <212> DNA
   <213> Artificial sequence
<220>
   <223> Synthetic oligonucleotide
<400> 333
   agaaagctat cgccattatt 20
<210> 334
   <211> 20
   <212> DNA
   <213> Artificial sequence
<220>
   <223> Synthetic oligonucleotide
<400> 334
   ttcccaaata cattctaaaa 20
<210> 335
   <211> 20
   <212> DNA
   <213> Artificial sequence
<220>
   <223> Synthetic oligonucleotide
<400> 335
   aactgctcta ggcctgtgtc 20
<210> 336
   <211> 20
   <212> DNA
   <213> Artificial sequence
<220>
   <223> Synthetic oligonucleotide
<400> 336
   aaatggatca aatctgatca 20
<210> 337
   <211> 20
   <212> DNA
   <213> Artificial sequence
<220>
   <223> Synthetic oligonucleotide
<400> 337
   gtaggtgcac atcaaaatca 20
<210> 338
   <211> 20
   <212> DNA
   <213> Artificial sequence
<220>
   <223> Synthetic oligonucleotide
<400> 338
   tctgatataa aaatcttgtc 20
<210> 339
   <211> 20
   <212> DNA
   <213> Artificial sequence
<220>
   <223> Synthetic oligonucleotide
<400> 339
   accatatgaa ctccagaaag 20
<210> 340
   <211> 20
   <212> DNA
   <213> Artificial sequence
<220>
   <223> Synthetic oligonucleotide
<400> 340
   aacatcaagg tagttcatga 20
<210> 341
   <211> 20
   <212> DNA
   <213> Artificial sequence
<220>
   <223> Synthetic oligonucleotide
<400> 341
   gcaattacag aaatggatca 20
<210> 342
   <211> 20
   <212> DNA
   <213> Artificial sequence
<220>
   <223> Synthetic oligonucleotide
<400> 342
   ttttaagcat attccaaagt 20
<210> 343
   <211> 20
   <212> DNA
   <213> Artificial sequence
<220>
   <223> Synthetic oligonucleotide
<400> 343
   tcaaccccca gctcaaacac 20
<210> 344
   <211> 20
   <212> DNA
   <213> Artificial sequence
<220>
   <223> Synthetic oligonucleotide
<400> 344
   agaaaaataa cattaatcct 20
<210> 345
   <211> 20
   <212> DNA
   <213> Artificial sequence
<220>
   <223> Synthetic oligonucleotide
<400> 345
   aagattttaa acacggaata 20
<210> 346
   <211> 20
   <212> DNA
   <213> Artificial sequence
<220>
   <223> Synthetic oligonucleotide
<400> 346
   agcagtcaca ttgcccaagt 20
<210> 347
   <211> 20
   <212> DNA
   <213> Artificial sequence
<220>
   <223> Synthetic oligonucleotide
<400> 347
   cagtgtttaa tgtttatcag 20
<210> 348
   <211> 20
   <212> DNA
   <213> Artificial sequence
<220>
   <223> Synthetic oligonucleotide
<400> 348
   gcaaaataca ggtcattgaa 20
<210> 349
   <211> 20
   <212> DNA
   <213> Artificial sequence
<220>
   <223> Synthetic oligonucleotide
<400> 349
   ggcaaaatac aggtcattga 20
<210> 350
   <211> 20
   <212> DNA
   <213> Artificial sequence
<220>
   <223> Synthetic oligonucleotide
<400> 350
   tggcaaaata caggtcattg 20
<210> 351
   <211> 20
   <212> DNA
   <213> Artificial sequence
<220>
   <223> Synthetic oligonucleotide
<400> 351
   ctggcaaaat acaggtcatt 20
<210> 352
   <211> 20
   <212> DNA
   <213> Artificial sequence
<220>
   <223> Synthetic oligonucleotide
<400> 352
   tctggcaaaa tacaggtcat 20
<210> 353
   <211> 20
   <212> DNA
   <213> Artificial sequence
<220>
   <223> Synthetic oligonucleotide
<400> 353
   gtctggcaaa atacaggtca 20
<210> 354
   <211> 20
   <212> DNA
   <213> Artificial sequence
<220>
   <223> Synthetic oligonucleotide
<400> 354
   agtctggcaa aatacaggtc 20
<210> 355
   <211> 20
   <212> DNA
   <213> Artificial sequence
<220>
   <223> Synthetic oligonucleotide
<400> 355
   aagtctggca aaatacaggt 20
<210> 356
   <211> 20
   <212> DNA
   <213> Artificial sequence
<220>
   <223> Synthetic oligonucleotide
<400> 356
   taagtctggc aaaatacagg 20
<210> 357
   <211> 20
   <212> DNA
   <213> Artificial sequence
<220>
   <223> Synthetic oligonucleotide
<400> 357
   ttaagtctgg caaaatacag 20
<210> 358
   <211> 20
   <212> DNA
   <213> Artificial sequence
<220>
   <223> Synthetic oligonucleotide
<400> 358
   gtttaatacc catctgtgat 20
<210> 359
   <211> 20
   <212> DNA
   <213> Artificial sequence
<220>
   <223> Synthetic oligonucleotide
<400> 359
   aagtttaata cccatctgtg 20
<210> 360
   <211> 20
   <212> DNA
   <213> Artificial sequence
<220>
   <223> Synthetic oligonucleotide
<400> 360
   caagtttaat acccatctgt 20
<210> 361
   <211> 20
   <212> DNA
   <213> Artificial sequence
<220>
   <223> Synthetic oligonucleotide
<400> 361
   acaagtttaa tacccatctg 20
<210> 362
   <211> 20
   <212> DNA
   <213> Artificial sequence
<220>
   <223> Synthetic oligonucleotide
<400> 362
   gacaagttta atacccatct 20
<210> 363
   <211> 20
   <212> DNA
   <213> Artificial sequence
<220>
   <223> Synthetic oligonucleotide
<400> 363
   tgacaagttt aatacccatc 20
<210> 364
   <211> 20
   <212> DNA
   <213> Artificial sequence
<220>
   <223> Synthetic oligonucleotide
<400> 364
   ctgacaagtt taatacccat 20
<210> 365
   <211> 20
   <212> DNA
   <213> Artificial sequence
<220>
   <223> Synthetic oligonucleotide
<400> 365
   tctgacaagt ttaataccca 20
<210> 366
   <211> 20
   <212> DNA
   <213> Artificial sequence
<220>
   <223> Synthetic oligonucleotide
<400> 366
   ttctgacaag tttaataccc 20
<210> 367
   <211> 20
   <212> DNA
   <213> Artificial sequence
<220>
   <223> Synthetic oligonucleotide
<400> 367
   attctgacaa gtttaatacc 20
<210> 368
   <211> 20
   <212> DNA
   <213> Artificial sequence
<220>
   <223> Synthetic oligonucleotide
<400> 368
   aattctgaca agtttaatac 20
<210> 369
   <211> 20
   <212> DNA
   <213> Artificial sequence
<220>
   <223> Synthetic oligonucleotide
<400> 369
   aaattctgac aagtttaata 20
<210> 370
   <211> 20
   <212> DNA
   <213> Artificial sequence
<220>
   <223> Synthetic oligonucleotide
<400> 370
   gaaattctga caagtttaat 20
<210> 371
   <211> 20
   <212> DNA
   <213> Artificial sequence
<220>
   <223> Synthetic oligonucleotide
<400> 371
   agaaattctg acaagtttaa 20
<210> 372
   <211> 20
   <212> DNA
   <213> Artificial sequence
<220>
   <223> Synthetic oligonucleotide
<400> 372
   aagaaattct gacaagttta 20
<210> 373
   <211> 20
   <212> DNA
   <213> Artificial sequence
<220>
   <223> Synthetic oligonucleotide
<400> 373
   ttattcacag gcttgaatga 20
<210> 374
   <211> 20
   <212> DNA
   <213> Artificial sequence
<220>
   <223> Synthetic oligonucleotide
<400> 374
   tttattcaca ggcttgaatg 20
<210> 375
   <211> 20
   <212> DNA
   <213> Artificial sequence
<220>
   <223> Synthetic oligonucleotide
<400> 375
   ttttattcac aggcttgaat 20
<210> 376
   <211> 20
   <212> DNA
   <213> Artificial sequence
<220>
   <223> Synthetic oligonucleotide
<400> 376
   tttttattca caggcttgaa 20
<210> 377
   <211> 20
   <212> DNA
   <213> Artificial sequence
<220>
   <223> Synthetic oligonucleotide
<400> 377
   gtttttattc acaggcttga 20
<210> 378
   <211> 20
   <212> DNA
   <213> Artificial sequence
<220>
   <223> Synthetic oligonucleotide
<400> 378
   gggtttttat tcacaggctt 20
<210> 379
   <211> 20
   <212> DNA
   <213> Artificial sequence
<220>
   <223> Synthetic oligonucleotide
<400> 379
   cagggttttt attcacaggc 20
<210> 380
   <211> 20
   <212> DNA
   <213> Artificial sequence
<220>
   <223> Synthetic oligonucleotide
<400> 380
   acagggtttt tattcacagg 20
<210> 381
   <211> 20
   <212> DNA
   <213> Artificial sequence
<220>
   <223> Synthetic oligonucleotide
<400> 381
   tacagggttt ttattcacag 20
<210> 382
   <211> 20
   <212> DNA
   <213> Artificial sequence
<220>
   <223> Synthetic oligonucleotide
<400> 382
   catacagggt ttttattcac 20
<210> 383
   <211> 20
   <212> DNA
   <213> Artificial sequence
<220>
   <223> Synthetic oligonucleotide
<400> 383
   gccatacagg gtttttattc 20
<210> 384
   <211> 20
   <212> DNA
   <213> Artificial sequence
<220>
   <223> Synthetic oligonucleotide
<400> 384
   tgccatacag ggtttttatt 20
<210> 385
   <211> 20
   <212> DNA
   <213> Artificial sequence
<220>
   <223> Synthetic oligonucleotide
<400> 385
   gtgccataca gggtttttat 20
<210> 386
   <211> 20
   <212> DNA
   <213> Artificial sequence
<220>
   <223> Synthetic oligonucleotide
<400> 386
   agtgccatac agggttttta 20
<210> 387
   <211> 20
   <212> DNA
   <213> Artificial sequence
<220>
   <223> Synthetic oligonucleotide
<400> 387
   tggaaaaact caaatgtgaa 20
<210> 388
   <211> 20
   <212> DNA
   <213> Artificial sequence
<220>
   <223> Synthetic oligonucleotide
<400> 388
   tttccctttc ttttccacac 20
<210> 389
   <211> 20
   <212> DNA
   <213> Artificial sequence
<220>
   <223> Synthetic oligonucleotide
<400> 389
   tctttccctt tcttttccac 20
<210> 390
   <211> 20
   <212> DNA
   <213> Artificial sequence
<220>
   <223> Synthetic oligonucleotide
<400> 390
   taccttctct gcccttgcag 20
<210> 391
   <211> 20
   <212> DNA
   <213> Artificial sequence
<220>
   <223> Synthetic oligonucleotide
<400> 391
   gcaagggcca aggctgctgc 20
<210> 392
   <211> 20
   <212> DNA
   <213> Artificial sequence
<220>
   <223> Synthetic oligonucleotide
<400> 392
   aaagctaaat tatgaattaa 20
<210> 393
   <211> 20
   <212> DNA
   <213> Artificial sequence
<220>
   <223> Synthetic oligonucleotide
<400> 393
   ctaatgaagg ctcagtatga 20
<210> 394
   <211> 20
   <212> DNA
   <213> Artificial sequence
<220>
   <223> Synthetic oligonucleotide
<400> 394
   ggagtcaaat gccaaagaac 20
<210> 395
   <211> 20
   <212> DNA
   <213> Artificial sequence
<220>
   <223> Synthetic oligonucleotide
<400> 395
   tgaattaaag ttcccaaata 20
<210> 396
   <211> 20
   <212> DNA
   <213> Artificial sequence
<220>
   <223> Synthetic oligonucleotide
<400> 396
   acttggtgca ggcagaatat 20
<210> 397
   <211> 20
   <212> DNA
   <213> Artificial sequence
<220>
   <223> Synthetic oligonucleotide
<400> 397
   cctctgatat aaaaatcttg 20
<210> 398
   <211> 20
   <212> DNA
   <213> Artificial sequence
<220>
   <223> Synthetic oligonucleotide
<400> 398
   aaagttggag agagtttctg 20
<210> 399
   <211> 20
   <212> DNA
   <213> Artificial sequence
<220>
   <223> Synthetic oligonucleotide
<400> 399
   tctctgccct tgcagcccaa 20
<210> 400
   <211> 20
   <212> DNA
   <213> Artificial sequence
<220>
   <223> Synthetic oligonucleotide
<400> 400
   ttacttggtg caggcagaat 20
<210> 401
   <211> 20
   <212> DNA
   <213> Artificial sequence
<220>
   <223> Synthetic oligonucleotide
<400> 401
   aatggagtca aatgccaaag 20
<210> 402
   <211> 20
   <212> DNA
   <213> Artificial sequence
<220>
   <223> Synthetic oligonucleotide
<400> 402
   tatgaattaa agttcccaaa 20
<210> 403
   <211> 20
   <212> DNA
   <213> Artificial sequence
<220>
   <223> Synthetic oligonucleotide
<400> 403
   agttctatat tcaataaatg 20
<210> 404
   <211> 20
   <212> DNA
   <213> Artificial sequence
<220>
   <223> Synthetic oligonucleotide
<400> 404
   tacaagtagt ataccatatg 20
<210> 405
   <211> 20
   <212> DNA
   <213> Artificial sequence
<220>
   <223> Synthetic oligonucleotide
<400> 405
   tagccttaga gctgtacaaa 20
<210> 406
   <211> 20
   <212> DNA
   <213> Artificial sequence
<220>
   <223> Synthetic oligonucleotide
<400> 406
   gtccccattt gtcaattcct 20
<210> 407
   <211> 20
   <212> DNA
   <213> Artificial sequence
<220>
   <223> Synthetic oligonucleotide
<400> 407
   aacctgccta ctggcagagc 20
<210> 408
   <211> 20
   <212> DNA
   <213> Artificial sequence
<220>
   <223> Synthetic oligonucleotide
<400> 408
   cttgttccca cactcaatgc 20
<210> 409
   <211> 20
   <212> DNA
   <213> Artificial sequence
<220>
   <223> Synthetic oligonucleotide
<400> 409
   acaagtcatg ataacctgca 20
<210> 410
   <211> 20
   <212> DNA
   <213> Artificial sequence
<220>
   <223> Synthetic oligonucleotide
<400> 410
   tgttttccaa actcagatct 20
<210> 411
   <211> 20
   <212> DNA
   <213> Artificial sequence
<220>
   <223> Synthetic oligonucleotide
<400> 411
   agaacctcat aatattagaa 20
<210> 412
   <211> 20
   <212> DNA
   <213> Artificial sequence
<220>
   <223> Synthetic oligonucleotide
<400> 412
   ggttttaaac ttaacaaaat 20
<210> 413
   <211> 20
   <212> DNA
   <213> Artificial sequence
<220>
   <223> Synthetic oligonucleotide
<400> 413
   ctctggtgta tttttagtaa 20
<210> 414
   <211> 20
   <212> DNA
   <213> Artificial sequence
<220>
   <223> Synthetic oligonucleotide
<400> 414
   tatctctgca tatctggaaa 20
<210> 415
   <211> 20
   <212> DNA
   <213> Artificial sequence
<220>
   <223> Synthetic oligonucleotide
<400> 415
   cagccttttt aacccaaaag 20
<210> 416
   <211> 20
   <212> DNA
   <213> Artificial sequence
<220>
   <223> Synthetic oligonucleotide
<400> 416
   tggaatgctc cactatccaa 20
<210> 417
   <211> 20
   <212> DNA
   <213> Artificial sequence
<220>
   <223> Synthetic oligonucleotide
<400> 417
   cgttcagaag tttgtctctg 20
<210> 418
   <211> 20
   <212> DNA
   <213> Artificial sequence
<220>
   <223> Synthetic oligonucleotide
<400> 418
   ctgctcaggg aaggtggaaa 20
<210> 419
   <211> 20
   <212> DNA
   <213> Artificial sequence
<220>
   <223> Synthetic oligonucleotide
<400> 419
   tcaagagaag ctaggaaaac 20
<210> 420
   <211> 20
   <212> DNA
   <213> Artificial sequence
<220>
   <223> Synthetic oligonucleotide
<400> 420
   tccctttctt ttccacacct 20
<210> 421
   <211> 20
   <212> DNA
   <213> Artificial sequence
<220>
   <223> Synthetic oligonucleotide
<400> 421
   ttgttcccac actcaatgca 20
<210> 422
   <211> 20
   <212> DNA
   <213> Artificial sequence
<220>
   <223> Synthetic oligonucleotide
<400> 422
   tcaccagcac agcacaacac 20
<210> 423
   <211> 20
   <212> DNA
   <213> Artificial sequence
<220>
   <223> Synthetic oligonucleotide
<400> 423
   cctgggatca ttacaaaagt 20
<210> 424
   <211> 20
   <212> DNA
   <213> Artificial sequence
<220>
   <223> Synthetic oligonucleotide
<400> 424
   agtagtatac catatgaact 20
<210> 425
   <211> 20
   <212> DNA
   <213> Artificial sequence
<220>
   <223> Synthetic oligonucleotide
<400> 425
   tctaatatgg tcaaatgtaa 20
<210> 426
   <211> 20
   <212> DNA
   <213> Artificial sequence
<220>
   <223> Synthetic oligonucleotide
<400> 426
   ggttgggctc tggtgtattt 20
<210> 427
   <211> 20
   <212> DNA
   <213> Artificial sequence
<220>
   <223> Synthetic oligonucleotide
<400> 427
   tgccctttac ttggtgcagg 20
<210> 428
   <211> 20
   <212> DNA
   <213> Artificial sequence
<220>
   <223> Synthetic oligonucleotide
<400> 428
   agagagtttc tgaacaaaga 20
<210> 429
   <211> 20
   <212> DNA
   <213> Artificial sequence
<220>
   <223> Synthetic oligonucleotide
<400> 429
   gaatttcagc aattacagaa 20
<210> 430
   <211> 20
   <212> DNA
   <213> Artificial sequence
<220>
   <223> Synthetic oligonucleotide
<400> 430
   acaagttaaa caagtcatga 20
<210> 431
   <211> 20
   <212> DNA
   <213> Artificial sequence
<220>
   <223> Synthetic oligonucleotide
<400> 431
   tgtgcccttt acttggtgca 20
<210> 432
   <211> 20
   <212> DNA
   <213> Artificial sequence
<220>
   <223> Synthetic oligonucleotide
<400> 432
   ttaggaggag gaaaaggacc 20
<210> 433
   <211> 20
   <212> DNA
   <213> Artificial sequence
<220>
   <223> Synthetic oligonucleotide
<400> 433
   actggcagag caattttaaa 20
<210> 434
   <211> 20
   <212> DNA
   <213> Artificial sequence
<220>
   <223> Synthetic oligonucleotide
<400> 434
   agtcaaatgc caaagaacct 20
<210> 435
   <211> 20
   <212> DNA
   <213> Artificial sequence
<220>
   <223> Synthetic oligonucleotide
<400> 435
   aagcatcaga tggattaggg 20
<210> 436
   <211> 20
   <212> DNA
   <213> Artificial sequence
<220>
   <223> Synthetic oligonucleotide
<400> 436
   gtccgcggga ccctcaggaa 20
<210> 437
   <211> 20
   <212> DNA
   <213> Artificial sequence
<220>
   <223> Synthetic oligonucleotide
<400> 437
   caattacaga aatggatcaa 20
<210> 438
   <211> 20
   <212> DNA
   <213> Artificial sequence
<220>
   <223> Synthetic oligonucleotide
<400> 438
   gctgtcaagt aatcactacc 20
<210> 439
   <211> 20
   <212> DNA
   <213> Artificial sequence
<220>
   <223> Synthetic oligonucleotide
<400> 439
   agtgcaaagt tggagagagt 20
<210> 440
   <211> 20
   <212> DNA
   <213> Artificial sequence
<220>
   <223> Synthetic oligonucleotide
<400> 440
   acttgcttcc aatcccaaat 20
<210> 441
   <211> 20
   <212> DNA
   <213> Artificial sequence
<220>
   <223> Synthetic oligonucleotide
<400> 441
   aactcaaatg tgaaagttgt 20
<210> 442
   <211> 20
   <212> DNA
   <213> Artificial sequence
<220>
   <223> Synthetic oligonucleotide
<400> 442
   ttttagtaag atcttcaaat 20
<210> 443
   <211> 20
   <212> DNA
   <213> Artificial sequence
<220>
   <223> Synthetic oligonucleotide
<400> 443
   atttcagcaa ttacagaaat 20
<210> 444
   <211> 20
   <212> DNA
   <213> Artificial sequence
<220>
   <223> Synthetic oligonucleotide
<400> 444
   ttaagtgtcc ccatttgtca 20
<210> 445
   <211> 20
   <212> DNA
   <213> Artificial sequence
<220>
   <223> Synthetic oligonucleotide
<400> 445
   ttagcaacct gcctactggc 20
<210> 446
   <211> 20
   <212> DNA
   <213> Artificial sequence
<220>
   <223> Synthetic oligonucleotide
<400> 446
   tattacaaga gttaagcatc 20
<210> 447
   <211> 20
   <212> DNA
   <213> Artificial sequence
<220>
   <223> Synthetic oligonucleotide
<400> 447
   atgttgaata tacatgtaca 20
<210> 448
   <211> 20
   <212> DNA
   <213> Artificial sequence
<220>
   <223> Synthetic oligonucleotide
<400> 448
   tttgtctctg accatcttag 20
<210> 449
   <211> 20
   <212> DNA
   <213> Artificial sequence
<220>
   <223> Synthetic oligonucleotide
<400> 449
   ttttccacca gttggtaact 20
<210> 450
   <211> 20
   <212> DNA
   <213> Artificial sequence
<220>
   <223> Synthetic oligonucleotide
<400> 450
   caacagcttc ccacaagtta 20
<210> 451
   <211> 20
   <212> DNA
   <213> Artificial sequence
<220>
   <223> Synthetic oligonucleotide
<400> 451
   caaatgtgaa agttgtccct 20
<210> 452
   <211> 20
   <212> DNA
   <213> Artificial sequence
<220>
   <223> Synthetic oligonucleotide
<400> 452
   gctaccttct ctgcccttgc 20
<210> 453
   <211> 20
   <212> DNA
   <213> Artificial sequence
<220>
   <223> Synthetic oligonucleotide
<400> 453
   tcttagcaga acagtgttct 20
<210> 454
   <211> 20
   <212> DNA
   <213> Artificial sequence
<220>
   <223> Synthetic oligonucleotide
<400> 454
   atacattcta aaaagaaaca 20
<210> 455
   <211> 20
   <212> DNA
   <213> Artificial sequence
<220>
   <223> Synthetic oligonucleotide
<400> 455
   gcacatattt acaagtagta 20
<210> 456
   <211> 20
   <212> DNA
   <213> Artificial sequence
<220>
   <223> Synthetic oligonucleotide
<400> 456
   gggtcaccag cacagcacaa 20
<210> 457
   <211> 20
   <212> DNA
   <213> Artificial sequence
<220>
   <223> Synthetic oligonucleotide
<400> 457
   gtgcaagggc caaggctgct 20
<210> 458
   <211> 20
   <212> DNA
   <213> Artificial sequence
<220>
   <223> Synthetic oligonucleotide
<400> 458
   acctgggttc atgcatggat 20
<210> 459
   <211> 20
   <212> DNA
   <213> Artificial sequence
<220>
   <223> Synthetic oligonucleotide
<400> 459
   atcactattt gaaactaaat 20
<210> 460
   <211> 20
   <212> DNA
   <213> Artificial sequence
<220>
   <223> Synthetic oligonucleotide
<400> 460
   atacaataaa gttgacctct 20
<210> 461
   <211> 20
   <212> DNA
   <213> Artificial sequence
<220>
   <223> Synthetic oligonucleotide
<400> 461
   ttttaaactt aacaaaatgt 20
<210> 462
   <211> 20
   <212> DNA
   <213> Artificial sequence
<220>
   <223> Synthetic oligonucleotide
<400> 462
   ctccccgcgc tcccgccacg 20
<210> 463
   <211> 20
   <212> DNA
   <213> Artificial sequence
<220>
   <223> Synthetic oligonucleotide
<400> 463
   gaaggctcag tatgaagaga 20
<210> 464
   <211> 20
   <212> DNA
   <213> Artificial sequence
<220>
   <223> Synthetic oligonucleotide
<400> 464
   agaaaacagc tgatttacct 20
<210> 465
   <211> 20
   <212> DNA
   <213> Artificial sequence
<220>
   <223> Synthetic oligonucleotide
<400> 465
   ccacaagtta aacaagtcat 20
<210> 466
   <211> 20
   <212> DNA
   <213> Artificial sequence
<220>
   <223> Synthetic oligonucleotide
<400> 466
   caaatttgca aacaagtagc 20
<210> 467
   <211> 20
   <212> DNA
   <213> Artificial sequence
<220>
   <223> Synthetic oligonucleotide
<400> 467
   cctaatttga actgcaagta 20
<210> 468
   <211> 20
   <212> DNA
   <213> Artificial sequence
<220>
   <223> Synthetic oligonucleotide
<400> 468
   aaaaaactca tctccccagc 20
<210> 469
   <211> 20
   <212> DNA
   <213> Artificial sequence
<220>
   <223> Synthetic oligonucleotide
<400> 469
   aggctcagta tgaagagatc 20
<210> 470
   <211> 20
   <212> DNA
   <213> Artificial sequence
<220>
   <223> Synthetic oligonucleotide
<400> 470
   tgttatcaag agcacagggc 20
<210> 471
   <211> 20
   <212> DNA
   <213> Artificial sequence
<220>
   <223> Synthetic oligonucleotide
<400> 471
   cctcaaaagg gagatggtaa 20
<210> 472
   <211> 20
   <212> DNA
   <213> Artificial sequence
<220>
   <223> Synthetic oligonucleotide
<400> 472
   agtatgggtc accagcacag 20
<210> 473
   <211> 20
   <212> DNA
   <213> Artificial sequence
<220>
   <223> Synthetic oligonucleotide
<400> 473
   tcacaatcta gtgcagttac 20
<210> 474
   <211> 20
   <212> DNA
   <213> Artificial sequence
<220>
   <223> Synthetic oligonucleotide
<400> 474
   caagtgagaa acccaatcct 20
<210> 475
   <211> 20
   <212> DNA
   <213> Artificial sequence
<220>
   <223> Synthetic oligonucleotide
<400> 475
   agaaaatctg gccattttaa 20
<210> 476
   <211> 20
   <212> DNA
   <213> Artificial sequence
<220>
   <223> Synthetic oligonucleotide
<400> 476
   acaggtaatg gtgctccgtg 20
<210> 477
   <211> 20
   <212> DNA
   <213> Artificial sequence
<220>
   <223> Synthetic oligonucleotide
<400> 477
   tgaaaggctt tcagaaaaca 20
<210> 478
   <211> 20
   <212> DNA
   <213> Artificial sequence
<220>
   <223> Synthetic oligonucleotide
<400> 478
   caggcaagtt acaggaagca 20
<210> 479
   <211> 20
   <212> DNA
   <213> Artificial sequence
<220>
   <223> Synthetic oligonucleotide
<400> 479
   cagcaagctg cttaactgct 20
<210> 480
   <211> 20
   <212> DNA
   <213> Artificial sequence
<220>
   <223> Synthetic oligonucleotide
<400> 480
   tgttgcaaag acattacctt 20
<210> 481
   <211> 20
   <212> DNA
   <213> Artificial sequence
<220>
   <223> Synthetic oligonucleotide
<400> 481
   gaaactaaat tagcaagatg 20
<210> 482
   <211> 20
   <212> DNA
   <213> Artificial sequence
<220>
   <223> Synthetic oligonucleotide
<400> 482
   tcaagagcac agggccaaaa 20
<210> 483
   <211> 20
   <212> DNA
   <213> Artificial sequence
<220>
   <223> Synthetic oligonucleotide
<400> 483
   aggaggagga aaaggacctc 20
<210> 484
   <211> 20
   <212> DNA
   <213> Artificial sequence
<220>
   <223> Synthetic oligonucleotide
<400> 484
   cctcagcctt tttaacccaa 20
<210> 485
   <211> 20
   <212> DNA
   <213> Artificial sequence
<220>
   <223> Synthetic oligonucleotide
<400> 485
   ctatgttgta gaccaccaca 20
<210> 486
   <211> 20
   <212> DNA
   <213> Artificial sequence
<220>
   <223> Synthetic oligonucleotide
<400> 486
   ctccgtggct acatacagaa 20
<210> 487
   <211> 20
   <212> DNA
   <213> Artificial sequence
<220>
   <223> Synthetic oligonucleotide
<400> 487
   tttatctgga tctttagaaa 20
<210> 488
   <211> 20
   <212> DNA
   <213> Artificial sequence
<220>
   <223> Synthetic oligonucleotide
<400> 488
   aaaaaaagga aagtgaaagt 20
<210> 489
   <211> 20
   <212> DNA
   <213> Artificial sequence
<220>
   <223> Synthetic oligonucleotide
<400> 489
   ggttcatgca tggattctca 20
<210> 490
   <211> 20
   <212> DNA
   <213> Artificial sequence
<220>
   <223> Synthetic oligonucleotide
<400> 490
   ctgcaaagtg tcacacaaac 20
<210> 491
   <211> 20
   <212> DNA
   <213> Artificial sequence
<220>
   <223> Synthetic oligonucleotide
<400> 491
   ttcagaagta ccaaagggta 20
<210> 492
   <211> 20
   <212> DNA
   <213> Artificial sequence
<220>
   <223> Synthetic oligonucleotide
<400> 492
   taaaagcatt ccagcatttg 20
<210> 493
   <211> 20
   <212> DNA
   <213> Artificial sequence
<220>
   <223> Synthetic oligonucleotide
<400> 493
   tagtatacca tatgaactcc 20
<210> 494
   <211> 20
   <212> DNA
   <213> Artificial sequence
<220>
   <223> Synthetic oligonucleotide
<400> 494
   tgcatatctg gaaagctgga 20
<210> 495
   <211> 20
   <212> DNA
   <213> Artificial sequence
<220>
   <223> Synthetic oligonucleotide
<400> 495
   cttaactgct ctaggcctgt 20
<210> 496
   <211> 20
   <212> DNA
   <213> Artificial sequence
<220>
   <223> Synthetic oligonucleotide
<400> 496
   aggcaccgac cgggcggcac 20
<210> 497
   <211> 20
   <212> DNA
   <213> Artificial sequence
<220>
   <223> Synthetic oligonucleotide
<400> 497
   tgcaaagttg gagagagttt 20
<210> 498
   <211> 20
   <212> DNA
   <213> Artificial sequence
<220>
   <223> Synthetic oligonucleotide
<400> 498
   tcctcaaaag ggagatggta 20
<210> 499
   <211> 20
   <212> DNA
   <213> Artificial sequence
<220>
   <223> Synthetic oligonucleotide
<400> 499
   agtataccat atgaactcca 20
<210> 500
   <211> 20
   <212> DNA
   <213> Artificial sequence
<220>
   <223> Synthetic oligonucleotide
<400> 500
   tatttgtaca tgttgaatat 20
<210> 501
   <211> 20
   <212> DNA
   <213> Artificial sequence
<220>
   <223> Synthetic oligonucleotide
<400> 501
   acccaaaagg tgtatgtctc 20
<210> 502
   <211> 20
   <212> DNA
   <213> Artificial sequence
<220>
   <223> Synthetic oligonucleotide
<400> 502
   ctttggaaaa aaaggaaagt 20
<210> 503
   <211> 20
   <212> DNA
   <213> Artificial sequence
<220>
   <223> Synthetic oligonucleotide
<400> 503
   gggagaaagg caggcaagtt 20
<210> 504
   <211> 20
   <212> DNA
   <213> Artificial sequence
<220>
   <223> Synthetic oligonucleotide
<400> 504
   ttaagcccag gaagtaaaag 20
<210> 505
   <211> 20
   <212> DNA
   <213> Artificial sequence
<220>
   <223> Synthetic oligonucleotide
<400> 505
   agacattacc tttaaacatt 20
<210> 506
   <211> 20
   <212> DNA
   <213> Artificial sequence
<220>
   <223> Synthetic oligonucleotide
<400> 506
   gtggcttaag aaatgctccg 20
<210> 507
   <211> 20
   <212> DNA
   <213> Artificial sequence
<220>
   <223> Synthetic oligonucleotide
<400> 507
   gtgagaaggg aacagaaaca 20
<210> 508
   <211> 20
   <212> DNA
   <213> Artificial sequence
<220>
   <223> Synthetic oligonucleotide
<400> 508
   aaaagcatca gatggattag 20
<210> 509
   <211> 20
   <212> DNA
   <213> Artificial sequence
<220>
   <223> Synthetic oligonucleotide
<400> 509
   ttccaccagt tggtaacttc 20
<210> 510
   <211> 20
   <212> DNA
   <213> Artificial sequence
<220>
   <223> Synthetic oligonucleotide
<400> 510
   tttttagtaa gatcttcaaa 20
<210> 511
   <211> 20
   <212> DNA
   <213> Artificial sequence
<220>
   <223> Synthetic oligonucleotide
<400> 511
   atctgtgtcc aaatcccagg 20
<210> 512
   <211> 20
   <212> DNA
   <213> Artificial sequence
<220>
   <223> Synthetic oligonucleotide
<400> 512
   taagatcttc aaataagcta 20
<210> 513
   <211> 20
   <212> DNA
   <213> Artificial sequence
<220>
   <223> Synthetic oligonucleotide
<400> 513
   atcaactctt tccctttctt 20
<210> 514
   <211> 20
   <212> DNA
   <213> Artificial sequence
<220>
   <223> Synthetic oligonucleotide
<400> 514
   tgtgtcctca aaagggagat 20
<210> 515
   <211> 20
   <212> DNA
   <213> Artificial sequence
<220>
   <223> Synthetic oligonucleotide
<400> 515
   tacctcctcc caacaatacc 20
<210> 516
   <211> 20
   <212> DNA
   <213> Artificial sequence
<220>
   <223> Synthetic oligonucleotide
<400> 516
   ttctgcttta caactatggc 20
<210> 517
   <211> 20
   <212> DNA
   <213> Artificial sequence
<220>
   <223> Synthetic oligonucleotide
<400> 517
   gtacatgttg aatatacatg 20
<210> 518
   <211> 20
   <212> DNA
   <213> Artificial sequence
<220>
   <223> Synthetic oligonucleotide
<400> 518
   tttgtggcta atcttaaggt 20
<210> 519
   <211> 20
   <212> DNA
   <213> Artificial sequence
<220>
   <223> Synthetic oligonucleotide
<400> 519
   tcctgcctca gcctttttaa 20
<210> 520
   <211> 20
   <212> DNA
   <213> Artificial sequence
<220>
   <223> Synthetic oligonucleotide
<400> 520
   cggtgtccgc gggaccctca 20
<210> 521
   <211> 20
   <212> DNA
   <213> Artificial sequence
<220>
   <223> Synthetic oligonucleotide
<400> 521
   gaaatggatc aaatctgatc 20
<210> 522
   <211> 20
   <212> DNA
   <213> Artificial sequence
<220>
   <223> Synthetic oligonucleotide
<400> 522
   ggtagttcat gagctaaatt 20
<210> 523
   <211> 20
   <212> DNA
   <213> Artificial sequence
<220>
   <223> Synthetic oligonucleotide
<400> 523
   aatggagtct cgactagttt 20
<210> 524
   <211> 20
   <212> DNA
   <213> Artificial sequence
<220>
   <223> Synthetic oligonucleotide
<400> 524
   caagtatggg tcaccagcac 20
<210> 525
   <211> 20
   <212> DNA
   <213> Artificial sequence
<220>
   <223> Synthetic oligonucleotide
<400> 525
   ggtgtccgcg ggaccctcag 20
<210> 526
   <211> 20
   <212> DNA
   <213> Artificial sequence
<220>
   <223> Synthetic oligonucleotide
<400> 526
   cgccacgcgc aggcccagcc 20
<210> 527
   <211> 20
   <212> DNA
   <213> Artificial sequence
<220>
   <223> Synthetic oligonucleotide
<400> 527
   tctaggcctg tgtcctcaaa 20
<210> 528
   <211> 20
   <212> DNA
   <213> Artificial sequence
<220>
   <223> Synthetic oligonucleotide
<400> 528
   actgtcctgg gctaatgaag 20
<210> 529
   <211> 20
   <212> DNA
   <213> Artificial sequence
<220>
   <223> Synthetic oligonucleotide
<400> 529
   aagcatcttg ttacctctct 20
<210> 530
   <211> 20
   <212> DNA
   <213> Artificial sequence
<220>
   <223> Synthetic oligonucleotide
<400> 530
   gcccaggaag taaaagcatt 20
<210> 531
   <211> 20
   <212> DNA
   <213> Artificial sequence
<220>
   <223> Synthetic oligonucleotide
<400> 531
   gtaagatctt caaataagct 20
<210> 532
   <211> 20
   <212> DNA
   <213> Artificial sequence
<220>
   <223> Synthetic oligonucleotide
<400> 532
   aaagggagat ggtaatcttg 20
<210> 533
   <211> 20
   <212> DNA
   <213> Artificial sequence
<220>
   <223> Synthetic oligonucleotide
<400> 533
   gccaaggctg ctgccttaca 20
<210> 534
   <211> 20
   <212> DNA
   <213> Artificial sequence
<220>
   <223> Synthetic oligonucleotide
<400> 534
   cagactaact gttcctgtcc 20
<210> 535
   <211> 20
   <212> DNA
   <213> Artificial sequence
<220>
   <223> Synthetic oligonucleotide
<400> 535
   tttgtcaatt cctttaagcc 20
<210> 536
   <211> 20
   <212> DNA
   <213> Artificial sequence
<220>
   <223> Synthetic oligonucleotide
<400> 536
   actacctcct cccaacaata 20
<210> 537
   <211> 20
   <212> DNA
   <213> Artificial sequence
<220>
   <223> Synthetic oligonucleotide
<400> 537
   tacctctctt catcctttgg 20
<210> 538
   <211> 20
   <212> DNA
   <213> Artificial sequence
<220>
   <223> Synthetic oligonucleotide
<400> 538
   actgctctag gcctgtgtcc 20
<210> 539
   <211> 20
   <212> DNA
   <213> Artificial sequence
<220>
   <223> Synthetic oligonucleotide
<400> 539
   cctcctccca acaataccca 20
<210> 540
   <211> 20
   <212> DNA
   <213> Artificial sequence
<220>
   <223> Synthetic oligonucleotide
<400> 540
   ggcaggcaag ttacaggaag 20
<210> 541
   <211> 20
   <212> DNA
   <213> Artificial sequence
<220>
   <223> Synthetic oligonucleotide
<400> 541
   tcgcccactc tggccccaaa 20
<210> 542
   <211> 20
   <212> DNA
   <213> Artificial sequence
<220>
   <223> Synthetic oligonucleotide
<400> 542
   cctcgcccac tctggcccca 20
<210> 543
   <211> 20
   <212> DNA
   <213> Artificial sequence
<220>
   <223> Synthetic oligonucleotide
<400> 543
   cgcctcgccc actctggccc 20
<210> 544
   <211> 20
   <212> DNA
   <213> Artificial sequence
<220>
   <223> Synthetic oligonucleotide
<400> 544
   cgcgcctcgc ccactctggc 20
<210> 545
   <211> 20
   <212> DNA
   <213> Artificial sequence
<220>
   <223> Synthetic oligonucleotide
<400> 545
   tccgcgcctc gcccactctg 20
<210> 546
   <211> 20
   <212> DNA
   <213> Artificial sequence
<220>
   <223> Synthetic oligonucleotide
<400> 546
   cctccgcgcc tcgcccactc 20
<210> 547
   <211> 20
   <212> DNA
   <213> Artificial sequence
<220>
   <223> Synthetic oligonucleotide
<400> 547
   gacctccgcg cctcgcccac 20
<210> 548
   <211> 20
   <212> DNA
   <213> Artificial sequence
<220>
   <223> Synthetic oligonucleotide
<400> 548
   cagacctccg cgcctcgccc 20
<210> 549
   <211> 20
   <212> DNA
   <213> Artificial sequence
<220>
   <223> Synthetic oligonucleotide
<400> 549
   gccagacctc cgcgcctcgc 20
<210> 550
   <211> 20
   <212> DNA
   <213> Artificial sequence
<220>
   <223> Synthetic oligonucleotide
<400> 550
   aggccagacc tccgcgcctc 20
<210> 551
   <211> 20
   <212> DNA
   <213> Artificial sequence
<220>
   <223> Synthetic oligonucleotide
<400> 551
   ataggccaga cctccgcgcc 20
<210> 552
   <211> 20
   <212> DNA
   <213> Artificial sequence
<220>
   <223> Synthetic oligonucleotide
<400> 552
   ttataggcca gacctccgcg 20
<210> 553
   <211> 20
   <212> DNA
   <213> Artificial sequence
<220>
   <223> Synthetic oligonucleotide
<400> 553
   ctttataggc cagacctccg 20
<210> 554
   <211> 20
   <212> DNA
   <213> Artificial sequence
<220>
   <223> Synthetic oligonucleotide
<400> 554
   tactttatag gccagacctc 20
<210> 555
   <211> 20
   <212> DNA
   <213> Artificial sequence
<220>
   <223> Synthetic oligonucleotide
<400> 555
   actactttat aggccagacc 20
<210> 556
   <211> 20
   <212> DNA
   <213> Artificial sequence
<220>
   <223> Synthetic oligonucleotide
<400> 556
   cgactacttt ataggccaga 20
<210> 557
   <211> 20
   <212> DNA
   <213> Artificial sequence
<220>
   <223> Synthetic oligonucleotide
<400> 557
   cgcgactact ttataggcca 20
<210> 558
   <211> 20
   <212> DNA
   <213> Artificial sequence
<220>
   <223> Synthetic oligonucleotide
<400> 558
   tccgcgacta ctttataggc 20
<210> 559
   <211> 20
   <212> DNA
   <213> Artificial sequence
<220>
   <223> Synthetic oligonucleotide
<400> 559
   tctccgcgac tactttatag 20
<210> 560
   <211> 20
   <212> DNA
   <213> Artificial sequence
<220>
   <223> Synthetic oligonucleotide
<400> 560
   cgtctccgcg actactttat 20
<210> 561
   <211> 20
   <212> DNA
   <213> Artificial sequence
<220>
   <223> Synthetic oligonucleotide
<400> 561
   cccgtctccg cgactacttt 20
<210> 562
   <211> 20
   <212> DNA
   <213> Artificial sequence
<220>
   <223> Synthetic oligonucleotide
<400> 562
   accccgtctc cgcgactact 20
<210> 563
   <211> 20
   <212> DNA
   <213> Artificial sequence
<220>
   <223> Synthetic oligonucleotide
<400> 563
   gcaccccgtc tccgcgacta 20
<210> 564
   <211> 20
   <212> DNA
   <213> Artificial sequence
<220>
   <223> Synthetic oligonucleotide
<400> 564
   cagcaccccg tctccgcgac 20
<210> 565
   <211> 20
   <212> DNA
   <213> Artificial sequence
<220>
   <223> Synthetic oligonucleotide
<400> 565
   accagcaccc cgtctccgcg 20
<210> 566
   <211> 20
   <212> DNA
   <213> Artificial sequence
<220>
   <223> Synthetic oligonucleotide
<400> 566
   aaaccagcac cccgtctccg 20
<210> 567
   <211> 20
   <212> DNA
   <213> Artificial sequence
<220>
   <223> Synthetic oligonucleotide
<400> 567
   gcaaaccagc accccgtctc 20
<210> 568
   <211> 20
   <212> DNA
   <213> Artificial sequence
<220>
   <223> Synthetic oligonucleotide
<400> 568
   acgcaaacca gcaccccgtc 20
<210> 569
   <211> 20
   <212> DNA
   <213> Artificial sequence
<220>
   <223> Synthetic oligonucleotide
<400> 569
   cgacgcaaac cagcaccccg 20
<210> 570
   <211> 20
   <212> DNA
   <213> Artificial sequence
<220>
   <223> Synthetic oligonucleotide
<400> 570
   tacgacgcaa accagcaccc 20
<210> 571
   <211> 20
   <212> DNA
   <213> Artificial sequence
<220>
   <223> Synthetic oligonucleotide
<400> 571
   actacgacgc aaaccagcac 20
<210> 572
   <211> 20
   <212> DNA
   <213> Artificial sequence
<220>
   <223> Synthetic oligonucleotide
<400> 572
   agactacgac gcaaaccagc 20
<210> 573
   <211> 20
   <212> DNA
   <213> Artificial sequence
<220>
   <223> Synthetic oligonucleotide
<400> 573
   ggagactacg acgcaaacca 20
<210> 574
   <211> 20
   <212> DNA
   <213> Artificial sequence
<220>
   <223> Synthetic oligonucleotide
<400> 574
   caggagacta cgacgcaaac 20
<210> 575
   <211> 20
   <212> DNA
   <213> Artificial sequence
<220>
   <223> Synthetic oligonucleotide
<400> 575
   tgcaggagac tacgacgcaa 20
<210> 576
   <211> 20
   <212> DNA
   <213> Artificial sequence
<220>
   <223> Synthetic oligonucleotide
<400> 576
   gctgcaggag actacgacgc 20
<210> 577
   <211> 20
   <212> DNA
   <213> Artificial sequence
<220>
   <223> Synthetic oligonucleotide
<400> 577
   gcaacggaaa ccccagacgc 20
<210> 578
   <211> 20
   <212> DNA
   <213> Artificial sequence
<220>
   <223> Synthetic oligonucleotide
<400> 578
   ctgcaacgga aaccccagac 20
<210> 579
   <211> 20
   <212> DNA
   <213> Artificial sequence
<220>
   <223> Synthetic oligonucleotide
<400> 579
   gactgcaacg gaaaccccag 20
<210> 580
   <211> 20
   <212> DNA
   <213> Artificial sequence
<220>
   <223> Synthetic oligonucleotide
<400> 580
   ggactgcaac ggaaacccca 20
<210> 581
   <211> 20
   <212> DNA
   <213> Artificial sequence
<220>
   <223> Synthetic oligonucleotide
<400> 581
   aggactgcaa cggaaacccc 20
<210> 582
   <211> 20
   <212> DNA
   <213> Artificial sequence
<220>
   <223> Synthetic oligonucleotide
<400> 582
   tccgaggact gcaacggaaa 20
<210> 583
   <211> 20
   <212> DNA
   <213> Artificial sequence
<220>
   <223> Synthetic oligonucleotide
<400> 583
   gttccgagga ctgcaacgga 20
<210> 584
   <211> 20
   <212> DNA
   <213> Artificial sequence
<220>
   <223> Synthetic oligonucleotide
<400> 584
   tggttccgag gactgcaacg 20
<210> 585
   <211> 20
   <212> DNA
   <213> Artificial sequence
<220>
   <223> Synthetic oligonucleotide
<400> 585
   cctggttccg aggactgcaa 20
<210> 586
   <211> 20
   <212> DNA
   <213> Artificial sequence
<220>
   <223> Synthetic oligonucleotide
<400> 586
   gtcctggttc cgaggactgc 20
<210> 587
   <211> 20
   <212> DNA
   <213> Artificial sequence
<220>
   <223> Synthetic oligonucleotide
<400> 587
   aggtcctggt tccgaggact 20
<210> 588
   <211> 20
   <212> DNA
   <213> Artificial sequence
<220>
   <223> Synthetic oligonucleotide
<400> 588
   cgaggtcctg gttccgagga 20
<210> 589
   <211> 20
   <212> DNA
   <213> Artificial sequence
<220>
   <223> Synthetic oligonucleotide
<400> 589
   gccgaggtcc tggttccgag 20
<210> 590
   <211> 20
   <212> DNA
   <213> Artificial sequence
<220>
   <223> Synthetic oligonucleotide
<400> 590
   acgccgaggt cctggttccg 20
<210> 591
   <211> 20
   <212> DNA
   <213> Artificial sequence
<220>
   <223> Synthetic oligonucleotide
<400> 591
   ccacgccgag gtcctggttc 20
<210> 592
   <211> 20
   <212> DNA
   <213> Artificial sequence
<220>
   <223> Synthetic oligonucleotide
<400> 592
   ggccacgccg aggtcctggt 20
<210> 593
   <211> 20
   <212> DNA
   <213> Artificial sequence
<220>
   <223> Synthetic oligonucleotide
<400> 593
   gctaggccac gccgaggtcc 20
<210> 594
   <211> 20
   <212> DNA
   <213> Artificial sequence
<220>
   <223> Synthetic oligonucleotide
<400> 594
   tcgctaggcc acgccgaggt 20
<210> 595
   <211> 20
   <212> DNA
   <213> Artificial sequence
<220>
   <223> Synthetic oligonucleotide
<400> 595
   actcgctagg ccacgccgag 20
<210> 596
   <211> 20
   <212> DNA
   <213> Artificial sequence
<220>
   <223> Synthetic oligonucleotide
<400> 596
   taactcgcta ggccacgccg 20
<210> 597
   <211> 20
   <212> DNA
   <213> Artificial sequence
<220>
   <223> Synthetic oligonucleotide
<400> 597
   cataactcgc taggccacgc 20
<210> 598
   <211> 20
   <212> DNA
   <213> Artificial sequence
<220>
   <223> Synthetic oligonucleotide
<400> 598
   gccataactc gctaggccac 20
<210> 599
   <211> 20
   <212> DNA
   <213> Artificial sequence
<220>
   <223> Synthetic oligonucleotide
<400> 599
   tcgccataac tcgctaggcc 20
<210> 600
   <211> 20
   <212> DNA
   <213> Artificial sequence
<220>
   <223> Synthetic oligonucleotide
<400> 600
   cgtcgccata actcgctagg 20
<210> 601
   <211> 20
   <212> DNA
   <213> Artificial sequence
<220>
   <223> Synthetic oligonucleotide
<400> 601
   cagcacgcac acggccttcg 20
<210> 602
   <211> 20
   <212> DNA
   <213> Artificial sequence
<220>
   <223> Synthetic oligonucleotide
<400> 602
   gccgtcgccc ttcagcacgc 20
<210> 603
   <211> 20
   <212> DNA
   <213> Artificial sequence
<220>
   <223> Synthetic oligonucleotide
<400> 603
   ggccgtcgcc cttcagcacg 20
<210> 604
   <211> 20
   <212> DNA
   <213> Artificial sequence
<220>
   <223> Synthetic oligonucleotide
<400> 604
   gggccgtcgc ccttcagcac 20
<210> 605
   <211> 20
   <212> DNA
   <213> Artificial sequence
<220>
   <223> Synthetic oligonucleotide
<400> 605
   ctgggccgtc gcccttcagc 20
<210> 606
   <211> 20
   <212> DNA
   <213> Artificial sequence
<220>
   <223> Synthetic oligonucleotide
<400> 606
   cactgggccg tcgcccttca 20
<210> 607
   <211> 20
   <212> DNA
   <213> Artificial sequence
<220>
   <223> Synthetic oligonucleotide
<400> 607
   tgcactgggc cgtcgccctt 20
<210> 608
   <211> 20
   <212> DNA
   <213> Artificial sequence
<220>
   <223> Synthetic oligonucleotide
<400> 608
   cctgcactgg gccgtcgccc 20
<210> 609
   <211> 20
   <212> DNA
   <213> Artificial sequence
<220>
   <223> Synthetic oligonucleotide
<400> 609
   atgccctgca ctgggccgtc 20
<210> 610
   <211> 20
   <212> DNA
   <213> Artificial sequence
<220>
   <223> Synthetic oligonucleotide
<400> 610
   tgatgccctg cactgggccg 20
<210> 611
   <211> 20
   <212> DNA
   <213> Artificial sequence
<220>
   <223> Synthetic oligonucleotide
<400> 611
   gatgatgccc tgcactgggc 20
<210> 612
   <211> 20
   <212> DNA
   <213> Artificial sequence
<220>
   <223> Synthetic oligonucleotide
<400> 612
   ttgatgatgc cctgcactgg 20
<210> 613
   <211> 18
   <212> DNA
   <213> Artificial sequence
<220>
   <223> Synthetic oligonucleotide
<400> 613
   ggcgatccca attacacc 18
<210> 614
   <211> 20
   <212> DNA
   <213> Artificial sequence
<220>
   <223> Synthetic oligonucleotide
<400> 614
   tcgaaattga tgatgccctg 20
<210> 615
   <211> 20
   <212> DNA
   <213> Artificial sequence
<220>
   <223> Synthetic oligonucleotide
<400> 615
   gctcgaaatt gatgatgccc 20
<210> 616
   <211> 20
   <212> DNA
   <213> Artificial sequence
<220>
   <223> Synthetic oligonucleotide
<400> 616
   ctgctcgaaa ttgatgatgc 20
<210> 617
   <211> 20
   <212> DNA
   <213> Artificial sequence
<220>
   <223> Synthetic oligonucleotide
<400> 617
   ttctgctcga aattgatgat 20
<210> 618
   <211> 20
   <212> DNA
   <213> Artificial sequence
<220>
   <223> Synthetic oligonucleotide
<400> 618
   ttaatgcttc cccacacctt 20
<210> 619
   <211> 20
   <212> DNA
   <213> Artificial sequence
<220>
   <223> Synthetic oligonucleotide
<400> 619
   ctttaatgct tccccacacc 20
<210> 620
   <211> 20
   <212> DNA
   <213> Artificial sequence
<220>
   <223> Synthetic oligonucleotide
<400> 620
   tcctttaatg cttccccaca 20
<210> 621
   <211> 20
   <212> DNA
   <213> Artificial sequence
<220>
   <223> Synthetic oligonucleotide
<400> 621
   tcagtccttt aatgcttccc 20
<210> 622
   <211> 20
   <212> DNA
   <213> Artificial sequence
<220>
   <223> Synthetic oligonucleotide
<400> 622
   agtcagtcct ttaatgcttc 20
<210> 623
   <211> 20
   <212> DNA
   <213> Artificial sequence
<220>
   <223> Synthetic oligonucleotide
<400> 623
   tcagtcagtc ctttaatgct 20
<210> 624
   <211> 20
   <212> DNA
   <213> Artificial sequence
<220>
   <223> Synthetic oligonucleotide
<400> 624
   cttcagtcag tcctttaatg 20
<210> 625
   <211> 20
   <212> DNA
   <213> Artificial sequence
<220>
   <223> Synthetic oligonucleotide
<400> 625
   gccttcagtc agtcctttaa 20
<210> 626
   <211> 20
   <212> DNA
   <213> Artificial sequence
<220>
   <223> Synthetic oligonucleotide
<400> 626
   gcaggccttc agtcagtcct 20
<210> 627
   <211> 20
   <212> DNA
   <213> Artificial sequence
<220>
   <223> Synthetic oligonucleotide
<400> 627
   atgcaggcct tcagtcagtc 20
<210> 628
   <211> 20
   <212> DNA
   <213> Artificial sequence
<220>
   <223> Synthetic oligonucleotide
<400> 628
   ccatgcaggc cttcagtcag 20
<210> 629
   <211> 20
   <212> DNA
   <213> Artificial sequence
<220>
   <223> Synthetic oligonucleotide
<400> 629
   catgaacatg gaatccatgc 20
<210> 630
   <211> 20
   <212> DNA
   <213> Artificial sequence
<220>
   <223> Synthetic oligonucleotide
<400> 630
   ctcatgaaca tggaatccat 20
<210> 631
   <211> 20
   <212> DNA
   <213> Artificial sequence
<220>
   <223> Synthetic oligonucleotide
<400> 631
   aactcatgaa catggaatcc 20
<210> 632
   <211> 20
   <212> DNA
   <213> Artificial sequence
<220>
   <223> Synthetic oligonucleotide
<400> 632
   ccaaactcat gaacatggaa 20
<210> 633
   <211> 20
   <212> DNA
   <213> Artificial sequence
<220>
   <223> Synthetic oligonucleotide
<400> 633
   ctccaaactc atgaacatgg 20
<210> 634
   <211> 20
   <212> DNA
   <213> Artificial sequence
<220>
   <223> Synthetic oligonucleotide
<400> 634
   atctccaaac tcatgaacat 20
<210> 635
   <211> 20
   <212> DNA
   <213> Artificial sequence
<220>
   <223> Synthetic oligonucleotide
<400> 635
   ttatctccaa actcatgaac 20
<210> 636
   <211> 20
   <212> DNA
   <213> Artificial sequence
<220>
   <223> Synthetic oligonucleotide
<400> 636
   tattatctcc aaactcatga 20
<210> 637
   <211> 20
   <212> DNA
   <213> Artificial sequence
<220>
   <223> Synthetic oligonucleotide
<400> 637
   tgtattatct ccaaactcat 20
<210> 638
   <211> 20
   <212> DNA
   <213> Artificial sequence
<220>
   <223> Synthetic oligonucleotide
<400> 638
   ctgctgtatt atctccaaac 20
<210> 639
   <211> 20
   <212> DNA
   <213> Artificial sequence
<220>
   <223> Synthetic oligonucleotide
<400> 639
   gcctgctgta ttatctccaa 20
<210> 640
   <211> 20
   <212> DNA
   <213> Artificial sequence
<220>
   <223> Synthetic oligonucleotide
<400> 640
   cagcctgctg tattatctcc 20
<210> 641
   <211> 20
   <212> DNA
   <213> Artificial sequence
<220>
   <223> Synthetic oligonucleotide
<400> 641
   tacagcctgc tgtattatct 20
<210> 642
   <211> 20
   <212> DNA
   <213> Artificial sequence
<220>
   <223> Synthetic oligonucleotide
<400> 642
   ggtacagcct gctgtattat 20
<210> 643
   <211> 20
   <212> DNA
   <213> Artificial sequence
<220>
   <223> Synthetic oligonucleotide
<400> 643
   ctggtacagc ctgctgtatt 20
<210> 644
   <211> 20
   <212> DNA
   <213> Artificial sequence
<220>
   <223> Synthetic oligonucleotide
<400> 644
   cactggtaca gcctgctgta 20
<210> 645
   <211> 20
   <212> DNA
   <213> Artificial sequence
<220>
   <223> Synthetic oligonucleotide
<400> 645
   tgcactggta cagcctgctg 20
<210> 646
   <211> 20
   <212> DNA
   <213> Artificial sequence
<220>
   <223> Synthetic oligonucleotide
<400> 646
   cctgcactgg tacagcctgc 20
<210> 647
   <211> 20
   <212> DNA
   <213> Artificial sequence
<220>
   <223> Synthetic oligonucleotide
<400> 647
   ttctggatag aggattaaag 20
<210> 648
   <211> 20
   <212> DNA
   <213> Artificial sequence
<220>
   <223> Synthetic oligonucleotide
<400> 648
   ttttctggat agaggattaa 20
<210> 649
   <211> 20
   <212> DNA
   <213> Artificial sequence
<220>
   <223> Synthetic oligonucleotide
<400> 649
   tgttttctgg atagaggatt 20
<210> 650
   <211> 20
   <212> DNA
   <213> Artificial sequence
<220>
   <223> Synthetic oligonucleotide
<400> 650
   cgtgttttct ggatagagga 20
<210> 651
   <211> 20
   <212> DNA
   <213> Artificial sequence
<220>
   <223> Synthetic oligonucleotide
<400> 651
   accgtgtttt ctggatagag 20
<210> 652
   <211> 20
   <212> DNA
   <213> Artificial sequence
<220>
   <223> Synthetic oligonucleotide
<400> 652
   ccaccgtgtt ttctggatag 20
<210> 653
   <211> 20
   <212> DNA
   <213> Artificial sequence
<220>
   <223> Synthetic oligonucleotide
<400> 653
   gcccaccgtg ttttctggat 20
<210> 654
   <211> 20
   <212> DNA
   <213> Artificial sequence
<220>
   <223> Synthetic oligonucleotide
<400> 654
   tggcccaccg tgttttctgg 20
<210> 655
   <211> 20
   <212> DNA
   <213> Artificial sequence
<220>
   <223> Synthetic oligonucleotide
<400> 655
   tttggcccac cgtgttttct 20
<210> 656
   <211> 20
   <212> DNA
   <213> Artificial sequence
<220>
   <223> Synthetic oligonucleotide
<400> 656
   cctttggccc accgtgtttt 20
<210> 657
   <211> 20
   <212> DNA
   <213> Artificial sequence
<220>
   <223> Synthetic oligonucleotide
<400> 657
   agtctccaac atgcctctct 20
<210> 658
   <211> 20
   <212> DNA
   <213> Artificial sequence
<220>
   <223> Synthetic oligonucleotide
<400> 658
   caagtctcca acatgcctct 20
<210> 659
   <211> 20
   <212> DNA
   <213> Artificial sequence
<220>
   <223> Synthetic oligonucleotide
<400> 659
   cccaagtctc caacatgcct 20
<210> 660
   <211> 20
   <212> DNA
   <213> Artificial sequence
<220>
   <223> Synthetic oligonucleotide
<400> 660
   attgcccaag tctccaacat 20
<210> 661
   <211> 20
   <212> DNA
   <213> Artificial sequence
<220>
   <223> Synthetic oligonucleotide
<400> 661
   acattgccca agtctccaac 20
<210> 662
   <211> 20
   <212> DNA
   <213> Artificial sequence
<220>
   <223> Synthetic oligonucleotide
<400> 662
   tcacattgcc caagtctcca 20
<210> 663
   <211> 20
   <212> DNA
   <213> Artificial sequence
<220>
   <223> Synthetic oligonucleotide
<400> 663
   agtcacattg cccaagtctc 20
<210> 664
   <211> 20
   <212> DNA
   <213> Artificial sequence
<220>
   <223> Synthetic oligonucleotide
<400> 664
   gcagtcacat tgcccaagtc 20
<210> 665
   <211> 20
   <212> DNA
   <213> Artificial sequence
<220>
   <223> Synthetic oligonucleotide
<400> 665
   cagcagtcac attgcccaag 20
<210> 666
   <211> 20
   <212> DNA
   <213> Artificial sequence
<220>
   <223> Synthetic oligonucleotide
<400> 666
   gtcagcagtc acattgccca 20
<210> 667
   <211> 20
   <212> DNA
   <213> Artificial sequence
<220>
   <223> Synthetic oligonucleotide
<400> 667
   ttgtcagcag tcacattgcc 20
<210> 668
   <211> 20
   <212> DNA
   <213> Artificial sequence
<220>
   <223> Synthetic oligonucleotide
<400> 668
   ctttgtcagc agtcacattg 20
<210> 669
   <211> 20
   <212> DNA
   <213> Artificial sequence
<220>
   <223> Synthetic oligonucleotide
<400> 669
   atctttgtca gcagtcacat 20
<210> 670
   <211> 20
   <212> DNA
   <213> Artificial sequence
<220>
   <223> Synthetic oligonucleotide
<400> 670
   ccatctttgt cagcagtcac 20
<210> 671
   <211> 20
   <212> DNA
   <213> Artificial sequence
<220>
   <223> Synthetic oligonucleotide
<400> 671
   caccatcttt gtcagcagtc 20
<210> 672
   <211> 20
   <212> DNA
   <213> Artificial sequence
<220>
   <223> Synthetic oligonucleotide
<400> 672
   cacaccatct ttgtcagcag 20
<210> 673
   <211> 20
   <212> DNA
   <213> Artificial sequence
<220>
   <223> Synthetic oligonucleotide
<400> 673
   gccacaccat ctttgtcagc 20
<210> 674
   <211> 20
   <212> DNA
   <213> Artificial sequence
<220>
   <223> Synthetic oligonucleotide
<400> 674
   cggccacacc atctttgtca 20
<210> 675
   <211> 20
   <212> DNA
   <213> Artificial sequence
<220>
   <223> Synthetic oligonucleotide
<400> 675
   atcggccaca ccatctttgt 20
<210> 676
   <211> 20
   <212> DNA
   <213> Artificial sequence
<220>
   <223> Synthetic oligonucleotide
<400> 676
   acatcggcca caccatcttt 20
<210> 677
   <211> 20
   <212> DNA
   <213> Artificial sequence
<220>
   <223> Synthetic oligonucleotide
<400> 677
   agacacatcg gccacaccat 20
<210> 678
   <211> 20
   <212> DNA
   <213> Artificial sequence
<220>
   <223> Synthetic oligonucleotide
<400> 678
   atagacacat cggccacacc 20
<210> 679
   <211> 20
   <212> DNA
   <213> Artificial sequence
<220>
   <223> Synthetic oligonucleotide
<400> 679
   caatagacac atcggccaca 20
<210> 680
   <211> 20
   <212> DNA
   <213> Artificial sequence
<220>
   <223> Synthetic oligonucleotide
<400> 680
   ttcaatagac acatcggcca 20
<210> 681
   <211> 20
   <212> DNA
   <213> Artificial sequence
<220>
   <223> Synthetic oligonucleotide
<400> 681
   tcttcaatag acacatcggc 20
<210> 682
   <211> 20
   <212> DNA
   <213> Artificial sequence
<220>
   <223> Synthetic oligonucleotide
<400> 682
   aatcttcaat agacacatcg 20
<210> 683
   <211> 20
   <212> DNA
   <213> Artificial sequence
<220>
   <223> Synthetic oligonucleotide
<400> 683
   agaatcttca atagacacat 20
<210> 684
   <211> 20
   <212> DNA
   <213> Artificial sequence
<220>
   <223> Synthetic oligonucleotide
<400> 684
   acagaatctt caatagacac 20
<210> 685
   <211> 20
   <212> DNA
   <213> Artificial sequence
<220>
   <223> Synthetic oligonucleotide
<400> 685
   tcacagaatc ttcaatagac 20
<210> 686
   <211> 20
   <212> DNA
   <213> Artificial sequence
<220>
   <223> Synthetic oligonucleotide
<400> 686
   gatcacagaa tcttcaatag 20
<210> 687
   <211> 20
   <212> DNA
   <213> Artificial sequence
<220>
   <223> Synthetic oligonucleotide
<400> 687
   gagatcacag aatcttcaat 20
<210> 688
   <211> 20
   <212> DNA
   <213> Artificial sequence
<220>
   <223> Synthetic oligonucleotide
<400> 688
   gtgagatcac agaatcttca 20
<210> 689
   <211> 20
   <212> DNA
   <213> Artificial sequence
<220>
   <223> Synthetic oligonucleotide
<400> 689
   gagtgagatc acagaatctt 20
<210> 690
   <211> 20
   <212> DNA
   <213> Artificial sequence
<220>
   <223> Synthetic oligonucleotide
<400> 690
   gagagtgaga tcacagaatc 20
<210> 691
   <211> 20
   <212> DNA
   <213> Artificial sequence
<220>
   <223> Synthetic oligonucleotide
<400> 691
   ctgagagtga gatcacagaa 20
<210> 692
   <211> 20
   <212> DNA
   <213> Artificial sequence
<220>
   <223> Synthetic oligonucleotide
<400> 692
   tcctgagagt gagatcacag 20
<210> 693
   <211> 20
   <212> DNA
   <213> Artificial sequence
<220>
   <223> Synthetic oligonucleotide
<400> 693
   ggtctcctga gagtgagatc 20
<210> 694
   <211> 20
   <212> DNA
   <213> Artificial sequence
<220>
   <223> Synthetic oligonucleotide
<400> 694
   atggtctcct gagagtgaga 20
<210> 695
   <211> 20
   <212> DNA
   <213> Artificial sequence
<220>
   <223> Synthetic oligonucleotide
<400> 695
   caatggtctc ctgagagtga 20
<210> 696
   <211> 20
   <212> DNA
   <213> Artificial sequence
<220>
   <223> Synthetic oligonucleotide
<400> 696
   tgcaatggtc tcctgagagt 20
<210> 697
   <211> 20
   <212> DNA
   <213> Artificial sequence
<220>
   <223> Synthetic oligonucleotide
<400> 697
   gatgcaatgg tctcctgaga 20
<210> 698
   <211> 20
   <212> DNA
   <213> Artificial sequence
<220>
   <223> Synthetic oligonucleotide
<400> 698
   atgatgcaat ggtctcctga 20
<210> 699
   <211> 20
   <212> DNA
   <213> Artificial sequence
<220>
   <223> Synthetic oligonucleotide
<400> 699
   caatgatgca atggtctcct 20
<210> 700
   <211> 20
   <212> DNA
   <213> Artificial sequence
<220>
   <223> Synthetic oligonucleotide
<400> 700
   gccaatgatg caatggtctc 20
<210> 701
   <211> 20
   <212> DNA
   <213> Artificial sequence
<220>
   <223> Synthetic oligonucleotide
<400> 701
   cggccaatga tgcaatggtc 20
<210> 702
   <211> 20
   <212> DNA
   <213> Artificial sequence
<220>
   <223> Synthetic oligonucleotide
<400> 702
   tgcggccaat gatgcaatgg 20
<210> 703
   <211> 20
   <212> DNA
   <213> Artificial sequence
<220>
   <223> Synthetic oligonucleotide
<400> 703
   tgtgcggcca atgatgcaat 20
<210> 704
   <211> 20
   <212> DNA
   <213> Artificial sequence
<220>
   <223> Synthetic oligonucleotide
<400> 704
   agtgtgcggc caatgatgca 20
<210> 705
   <211> 20
   <212> DNA
   <213> Artificial sequence
<220>
   <223> Synthetic oligonucleotide
<400> 705
   ccagtgtgcg gccaatgatg 20
<210> 706
   <211> 20
   <212> DNA
   <213> Artificial sequence
<220>
   <223> Synthetic oligonucleotide
<400> 706
   caccagtgtg cggccaatga 20
<210> 707
   <211> 20
   <212> DNA
   <213> Artificial sequence
<220>
   <223> Synthetic oligonucleotide
<400> 707
   ggaccaccag tgtgcggcca 20
<210> 708
   <211> 20
   <212> DNA
   <213> Artificial sequence
<220>
   <223> Synthetic oligonucleotide
<400> 708
   atggaccacc agtgtgcggc 20
<210> 709
   <211> 20
   <212> DNA
   <213> Artificial sequence
<220>
   <223> Synthetic oligonucleotide
<400> 709
   tttcatggac caccagtgtg 20
<210> 710
   <211> 20
   <212> DNA
   <213> Artificial sequence
<220>
   <223> Synthetic oligonucleotide
<400> 710
   tttttcatgg accaccagtg 20
<210> 711
   <211> 20
   <212> DNA
   <213> Artificial sequence
<220>
   <223> Synthetic oligonucleotide
<400> 711
   gctttttcat ggaccaccag 20
<210> 712
   <211> 20
   <212> DNA
   <213> Artificial sequence
<220>
   <223> Synthetic oligonucleotide
<400> 712
   tgtctttgta ctttcttcat 20
<210> 713
   <211> 20
   <212> DNA
   <213> Artificial sequence
<220>
   <223> Synthetic oligonucleotide
<400> 713
   cctgtctttg tactttcttc 20
<210> 714
   <211> 20
   <212> DNA
   <213> Artificial sequence
<220>
   <223> Synthetic oligonucleotide
<400> 714
   ttcctgtctt tgtactttct 20
<210> 715
   <211> 20
   <212> DNA
   <213> Artificial sequence
<220>
   <223> Synthetic oligonucleotide
<400> 715
   gtttcctgtc tttgtacttt 20
<210> 716
   <211> 20
   <212> DNA
   <213> Artificial sequence
<220>
   <223> Synthetic oligonucleotide
<400> 716
   aagccaaacg acttccagcg 20
<210> 717
   <211> 20
   <212> DNA
   <213> Artificial sequence
<220>
   <223> Synthetic oligonucleotide
<400> 717
   acaagccaaa cgacttccag 20
<210> 718
   <211> 20
   <212> DNA
   <213> Artificial sequence
<220>
   <223> Synthetic oligonucleotide
<400> 718
   ccacaagcca aacgacttcc 20
<210> 719
   <211> 20
   <212> DNA
   <213> Artificial sequence
<220>
   <223> Synthetic oligonucleotide
<400> 719
   caccacaagc caaacgactt 20
<210> 720
   <211> 20
   <212> DNA
   <213> Artificial sequence
<220>
   <223> Synthetic oligonucleotide
<400> 720
   tacaccacaa gccaaacgac 20
<210> 721
   <211> 20
   <212> DNA
   <213> Artificial sequence
<220>
   <223> Synthetic oligonucleotide
<400> 721
   attacaccac aagccaaacg 20
<210> 722
   <211> 20
   <212> DNA
   <213> Artificial sequence
<220>
   <223> Synthetic oligonucleotide
<400> 722
   caattacacc acaagccaaa 20
<210> 723
   <211> 20
   <212> DNA
   <213> Artificial sequence
<220>
   <223> Synthetic oligonucleotide
<400> 723
   aggataacag atgagttaag 20
<210> 724
   <211> 20
   <212> DNA
   <213> Artificial sequence
<220>
   <223> Synthetic oligonucleotide
<400> 724
   ggatacattt ctacagctag 20
<210> 725
   <211> 20
   <212> DNA
   <213> Artificial sequence
<220>
   <223> Synthetic oligonucleotide
<400> 725
   caggatacat ttctacagct 20
<210> 726
   <211> 20
   <212> DNA
   <213> Artificial sequence
<220>
   <223> Synthetic oligonucleotide
<400> 726
   atcaggatac atttctacag 20
<210> 727
   <211> 20
   <212> DNA
   <213> Artificial sequence
<220>
   <223> Synthetic oligonucleotide
<400> 727
   ttatcaggat acatttctac 20
<210> 728
   <211> 20
   <212> DNA
   <213> Artificial sequence
<220>
   <223> Synthetic oligonucleotide
<400> 728
   gtttatcagg atacatttct 20
<210> 729
   <211> 20
   <212> DNA
   <213> Artificial sequence
<220>
   <223> Synthetic oligonucleotide
<400> 729
   atgtttatca ggatacattt 20
<210> 730
   <211> 20
   <212> DNA
   <213> Artificial sequence
<220>
   <223> Synthetic oligonucleotide
<400> 730
   taatgtttat caggatacat 20
<210> 731
   <211> 20
   <212> DNA
   <213> Artificial sequence
<220>
   <223> Synthetic oligonucleotide
<400> 731
   tttaatgttt atcaggatac 20
<210> 732
   <211> 20
   <212> DNA
   <213> Artificial sequence
<220>
   <223> Synthetic oligonucleotide
<400> 732
   tgtttaatgt ttatcaggat 20
<210> 733
   <211> 20
   <212> DNA
   <213> Artificial sequence
<220>
   <223> Synthetic oligonucleotide
<400> 733
   agtgtttaat gtttatcagg 20
<210> 734
   <211> 20
   <212> DNA
   <213> Artificial sequence
<220>
   <223> Synthetic oligonucleotide
<400> 734
   tttaagatta cagtgtttaa 20
<210> 735
   <211> 20
   <212> DNA
   <213> Artificial sequence
<220>
   <223> Synthetic oligonucleotide
<400> 735
   cttttaagat tacagtgttt 20
<210> 736
   <211> 20
   <212> DNA
   <213> Artificial sequence
<220>
   <223> Synthetic oligonucleotide
<400> 736
   cacttttaag attacagtgt 20
<210> 737
   <211> 20
   <212> DNA
   <213> Artificial sequence
<220>
   <223> Synthetic oligonucleotide
<400> 737
   tacactttta agattacagt 20
<210> 738
   <211> 20
   <212> DNA
   <213> Artificial sequence
<220>
   <223> Synthetic oligonucleotide
<400> 738
   attacacttt taagattaca 20
<210> 739
   <211> 20
   <212> DNA
   <213> Artificial sequence
<220>
   <223> Synthetic oligonucleotide
<400> 739
   caattacact tttaagatta 20
<210> 740
   <211> 20
   <212> DNA
   <213> Artificial sequence
<220>
   <223> Synthetic oligonucleotide
<400> 740
   cacacaatta cacttttaag 20
<210> 741
   <211> 20
   <212> DNA
   <213> Artificial sequence
<220>
   <223> Synthetic oligonucleotide
<400> 741
   gtcacacaat tacactttta 20
<210> 742
   <211> 20
   <212> DNA
   <213> Artificial sequence
<220>
   <223> Synthetic oligonucleotide
<400> 742
   aagtcacaca attacacttt 20
<210> 743
   <211> 20
   <212> DNA
   <213> Artificial sequence
<220>
   <223> Synthetic oligonucleotide
<400> 743
   aaaagtcaca caattacact 20
<210> 744
   <211> 20
   <212> DNA
   <213> Artificial sequence
<220>
   <223> Synthetic oligonucleotide
<400> 744
   gaaaaagtca cacaattaca 20
<210> 745
   <211> 20
   <212> DNA
   <213> Artificial sequence
<220>
   <223> Synthetic oligonucleotide
<400> 745
   ctgaaaaagt cacacaatta 20
<210> 746
   <211> 20
   <212> DNA
   <213> Artificial sequence
<220>
   <223> Synthetic oligonucleotide
<400> 746
   ctctgaaaaa gtcacacaat 20
<210> 747
   <211> 20
   <212> DNA
   <213> Artificial sequence
<220>
   <223> Synthetic oligonucleotide
<400> 747
   aactctgaaa aagtcacaca 20
<210> 748
   <211> 20
   <212> DNA
   <213> Artificial sequence
<220>
   <223> Synthetic oligonucleotide
<400> 748
   gcaactctga aaaagtcaca 20
<210> 749
   <211> 20
   <212> DNA
   <213> Artificial sequence
<220>
   <223> Synthetic oligonucleotide
<400> 749
   aagcaactct gaaaaagtca 20
<210> 750
   <211> 20
   <212> DNA
   <213> Artificial sequence
<220>
   <223> Synthetic oligonucleotide
<400> 750
   ttaaagcaac tctgaaaaag 20
<210> 751
   <211> 20
   <212> DNA
   <213> Artificial sequence
<220>
   <223> Synthetic oligonucleotide
<400> 751
   ctttaaagca actctgaaaa 20
<210> 752
   <211> 20
   <212> DNA
   <213> Artificial sequence
<220>
   <223> Synthetic oligonucleotide
<400> 752
   tactttaaag caactctgaa 20
<210> 753
   <211> 20
   <212> DNA
   <213> Artificial sequence
<220>
   <223> Synthetic oligonucleotide
<400> 753
   ggtactttaa agcaactctg 20
<210> 754
   <211> 20
   <212> DNA
   <213> Artificial sequence
<220>
   <223> Synthetic oligonucleotide
<400> 754
   caggtacttt aaagcaactc 20
<210> 755
   <211> 20
   <212> DNA
   <213> Artificial sequence
<220>
   <223> Synthetic oligonucleotide
<400> 755
   tacaggtact ttaaagcaac 20
<210> 756
   <211> 20
   <212> DNA
   <213> Artificial sequence
<220>
   <223> Synthetic oligonucleotide
<400> 756
   actacaggta ctttaaagca 20
<210> 757
   <211> 20
   <212> DNA
   <213> Artificial sequence
<220>
   <223> Synthetic oligonucleotide
<400> 757
   tcactacagg tactttaaag 20
<210> 758
   <211> 20
   <212> DNA
   <213> Artificial sequence
<220>
   <223> Synthetic oligonucleotide
<400> 758
   tctcactaca ggtactttaa 20
<210> 759
   <211> 20
   <212> DNA
   <213> Artificial sequence
<220>
   <223> Synthetic oligonucleotide
<400> 759
   tttctcacta caggtacttt 20
<210> 760
   <211> 20
   <212> DNA
   <213> Artificial sequence
<220>
   <223> Synthetic oligonucleotide
<400> 760
   agtttctcac tacaggtact 20
<210> 761
   <211> 20
   <212> DNA
   <213> Artificial sequence
<220>
   <223> Synthetic oligonucleotide
<400> 761
   tcagtttctc actacaggta 20
<210> 762
   <211> 20
   <212> DNA
   <213> Artificial sequence
<220>
   <223> Synthetic oligonucleotide
<400> 762
   taaatcagtt tctcactaca 20
<210> 763
   <211> 20
   <212> DNA
   <213> Artificial sequence
<220>
   <223> Synthetic oligonucleotide
<400> 763
   atcataaatc agtttctcac 20
<210> 764
   <211> 20
   <212> DNA
   <213> Artificial sequence
<220>
   <223> Synthetic oligonucleotide
<400> 764
   tgatcataaa tcagtttctc 20
<210> 765
   <211> 20
   <212> DNA
   <213> Artificial sequence
<220>
   <223> Synthetic oligonucleotide
<400> 765
   agtgatcata aatcagtttc 20
<210> 766
   <211> 20
   <212> DNA
   <213> Artificial sequence
<220>
   <223> Synthetic oligonucleotide
<400> 766
   caagtgatca taaatcagtt 20
<210> 767
   <211> 20
   <212> DNA
   <213> Artificial sequence
<220>
   <223> Synthetic oligonucleotide
<400> 767
   tccaagtgat cataaatcag 20
<210> 768
   <211> 20
   <212> DNA
   <213> Artificial sequence
<220>
   <223> Synthetic oligonucleotide
<400> 768
   cttccaagtg atcataaatc 20
<210> 769
   <211> 20
   <212> DNA
   <213> Artificial sequence
<220>
   <223> Synthetic oligonucleotide
<400> 769
   atcttccaag tgatcataaa 20
<210> 770
   <211> 20
   <212> DNA
   <213> Artificial sequence
<220>
   <223> Synthetic oligonucleotide
<400> 770
   aaatcttcca agtgatcata 20
<210> 771
   <211> 20
   <212> DNA
   <213> Artificial sequence
<220>
   <223> Synthetic oligonucleotide
<400> 771
   ctgagtttta taaaactata 20
<210> 772
   <211> 20
   <212> DNA
   <213> Artificial sequence
<220>
   <223> Synthetic oligonucleotide
<400> 772
   ttaactgagt tttataaaac 20
<210> 773
   <211> 20
   <212> DNA
   <213> Artificial sequence
<220>
   <223> Synthetic oligonucleotide
<400> 773
   ttttaactga gttttataaa 20
<210> 774
   <211> 20
   <212> DNA
   <213> Artificial sequence
<220>
   <223> Synthetic oligonucleotide
<400> 774
   cattttaact gagttttata 20
<210> 775
   <211> 20
   <212> DNA
   <213> Artificial sequence
<220>
   <223> Synthetic oligonucleotide
<400> 775
   gacattttaa ctgagtttta 20
<210> 776
   <211> 20
   <212> DNA
   <213> Artificial sequence
<220>
   <223> Synthetic oligonucleotide
<400> 776
   cagacatttt aactgagttt 20
<210> 777
   <211> 20
   <212> DNA
   <213> Artificial sequence
<220>
   <223> Synthetic oligonucleotide
<400> 777
   aacagacatt ttaactgagt 20
<210> 778
   <211> 20
   <212> DNA
   <213> Artificial sequence
<220>
   <223> Synthetic oligonucleotide
<400> 778
   gaaacagaca ttttaactga 20
<210> 779
   <211> 20
   <212> DNA
   <213> Artificial sequence
<220>
   <223> Synthetic oligonucleotide
<400> 779
   ttgaaacaga cattttaact 20
<210> 780
   <211> 20
   <212> DNA
   <213> Artificial sequence
<220>
   <223> Synthetic oligonucleotide
<400> 780
   tttaagtctg gcaaaataca 20
<210> 781
   <211> 20
   <212> DNA
   <213> Artificial sequence
<220>
   <223> Synthetic oligonucleotide
<400> 781
   gatttaagtc tggcaaaata 20
<210> 782
   <211> 20
   <212> DNA
   <213> Artificial sequence
<220>
   <223> Synthetic oligonucleotide
<400> 782
   gtgatttaag tctggcaaaa 20
<210> 783
   <211> 20
   <212> DNA
   <213> Artificial sequence
<220>
   <223> Synthetic oligonucleotide
<400> 783
   ctgtgattta agtctggcaa 20
<210> 784
   <211> 20
   <212> DNA
   <213> Artificial sequence
<220>
   <223> Synthetic oligonucleotide
<400> 784
   atctgtgatt taagtctggc 20
<210> 785
   <211> 20
   <212> DNA
   <213> Artificial sequence
<220>
   <223> Synthetic oligonucleotide
<400> 785
   acccatctgt gatttaagtc 20
<210> 786
   <211> 20
   <212> DNA
   <213> Artificial sequence
<220>
   <223> Synthetic oligonucleotide
<400> 786
   atacccatct gtgatttaag 20
<210> 787
   <211> 20
   <212> DNA
   <213> Artificial sequence
<220>
   <223> Synthetic oligonucleotide
<400> 787
   taatacccat ctgtgattta 20
<210> 788
   <211> 20
   <212> DNA
   <213> Artificial sequence
<220>
   <223> Synthetic oligonucleotide
<400> 788
   aaagaaattc tgacaagttt 20
<210> 789
   <211> 20
   <212> DNA
   <213> Artificial sequence
<220>
   <223> Synthetic oligonucleotide
<400> 789
   acaaagaaat tctgacaagt 20
<210> 790
   <211> 20
   <212> DNA
   <213> Artificial sequence
<220>
   <223> Synthetic oligonucleotide
<400> 790
   tgacaaagaa attctgacaa 20
<210> 791
   <211> 20
   <212> DNA
   <213> Artificial sequence
<220>
   <223> Synthetic oligonucleotide
<400> 791
   aatgacaaag aaattctgac 20
<210> 792
   <211> 20
   <212> DNA
   <213> Artificial sequence
<220>
   <223> Synthetic oligonucleotide
<400> 792
   tgaatgacaa agaaattctg 20
<210> 793
   <211> 20
   <212> DNA
   <213> Artificial sequence
<220>
   <223> Synthetic oligonucleotide
<400> 793
   cttgaatgac aaagaaattc 20
<210> 794
   <211> 20
   <212> DNA
   <213> Artificial sequence
<220>
   <223> Synthetic oligonucleotide
<400> 794
   ggcttgaatg acaaagaaat 20
<210> 795
   <211> 20
   <212> DNA
   <213> Artificial sequence
<220>
   <223> Synthetic oligonucleotide
<400> 795
   caggcttgaa tgacaaagaa 20
<210> 796
   <211> 20
   <212> DNA
   <213> Artificial sequence
<220>
   <223> Synthetic oligonucleotide
<400> 796
   cacaggcttg aatgacaaag 20
<210> 797
   <211> 20
   <212> DNA
   <213> Artificial sequence
<220>
   <223> Synthetic oligonucleotide
<400> 797
   ttcacaggct tgaatgacaa 20
<210> 798
   <211> 20
   <212> DNA
   <213> Artificial sequence
<220>
   <223> Synthetic oligonucleotide
<400> 798
   tattcacagg cttgaatgac 20
<210> 799
   <211> 20
   <212> DNA
   <213> Artificial sequence
<220>
   <223> Synthetic oligonucleotide
<400> 799
   ataagtgcca tacagggttt 20
<210> 800
   <211> 20
   <212> DNA
   <213> Artificial sequence
<220>
   <223> Synthetic oligonucleotide
<400> 800
   cataataagt gccatacagg 20
<210> 801
   <211> 20
   <212> DNA
   <213> Artificial sequence
<220>
   <223> Synthetic oligonucleotide
<400> 801
   tagcctcata ataagtgcca 20
<210> 802
   <211> 20
   <212> DNA
   <213> Artificial sequence
<220>
   <223> Synthetic oligonucleotide
<400> 802
   aatagcctca taataagtgc 20
<210> 803
   <211> 20
   <212> DNA
   <213> Artificial sequence
<220>
   <223> Synthetic oligonucleotide
<400> 803
   ttaatagcct cataataagt 20
<210> 804
   <211> 20
   <212> DNA
   <213> Artificial sequence
<220>
   <223> Synthetic oligonucleotide
<400> 804
   tcttttaata gcctcataat 20
<210> 805
   <211> 20
   <212> DNA
   <213> Artificial sequence
<220>
   <223> Synthetic oligonucleotide
<400> 805
   attcttttaa tagcctcata 20
<210> 806
   <211> 20
   <212> DNA
   <213> Artificial sequence
<220>
   <223> Synthetic oligonucleotide
<400> 806
   ttggattctt ttaatagcct 20
<210> 807
   <211> 20
   <212> DNA
   <213> Artificial sequence
<220>
   <223> Synthetic oligonucleotide
<400> 807
   atttggattc ttttaatagc 20
<210> 808
   <211> 20
   <212> DNA
   <213> Artificial sequence
<220>
   <223> Synthetic oligonucleotide
<400> 808
   gaatttggat tcttttaata 20
<210> 809
   <211> 20
   <212> DNA
   <213> Artificial sequence
<220>
   <223> Synthetic oligonucleotide
<400> 809
   ttgaatttgg attcttttaa 20
<210> 810
   <211> 20
   <212> DNA
   <213> Artificial sequence
<220>
   <223> Synthetic oligonucleotide
<400> 810
   gtttgaattt ggattctttt 20
<210> 811
   <211> 20
   <212> DNA
   <213> Artificial sequence
<220>
   <223> Synthetic oligonucleotide
<400> 811
   tagtttgaat ttggattctt 20
<210> 812
   <211> 20
   <212> DNA
   <213> Artificial sequence
<220>
   <223> Synthetic oligonucleotide
<400> 812
   tttagtttga atttggattc 20
<210> 813
   <211> 20
   <212> DNA
   <213> Artificial sequence
<220>
   <223> Synthetic oligonucleotide
<400> 813
   tttttagttt gaatttggat 20
<210> 814
   <211> 20
   <212> DNA
   <213> Artificial sequence
<220>
   <223> Synthetic oligonucleotide
<400> 814
   tttttttagt ttgaatttgg 20
<210> 815
   <211> 20
   <212> DNA
   <213> Artificial sequence
<220>
   <223> Synthetic oligonucleotide
<400> 815
   cgtttcctgt ctttgtactt 20
<210> 816
   <211> 20
   <212> DNA
   <213> Artificial sequence
<220>
   <223> Synthetic oligonucleotide
<400> 816
   caagccaaac gacttccagc 20
<210> 817
   <211> 20
   <212> DNA
   <213> Artificial sequence
<220>
   <223> Synthetic oligonucleotide
<400> 817
   cacaagccaa acgacttcca 20
<210> 818
   <211> 20
   <212> DNA
   <213> Artificial sequence
<220>
   <223> Synthetic oligonucleotide
<400> 818
   accacaagcc aaacgacttc 20
<210> 819
   <211> 20
   <212> DNA
   <213> Artificial sequence
<220>
   <223> Synthetic oligonucleotide
<400> 819
   acaccacaag ccaaacgact 20
<210> 820
   <211> 20
   <212> DNA
   <213> Artificial sequence
<220>
   <223> Synthetic oligonucleotide
<400> 820
   ttacaccaca agccaaacga 20
<210> 821
   <211> 20
   <212> DNA
   <213> Artificial sequence
<220>
   <223> Synthetic oligonucleotide
<400> 821
   ggataacaga tgagttaagg 20
<210> 822
   <211> 20
   <212> DNA
   <213> Artificial sequence
<220>
   <223> Synthetic oligonucleotide
<400> 822
   aggatacatt tctacagcta 20
<210> 823
   <211> 20
   <212> DNA
   <213> Artificial sequence
<220>
   <223> Synthetic oligonucleotide
<400> 823
   tcaggataca tttctacagc 20
<210> 824
   <211> 20
   <212> DNA
   <213> Artificial sequence
<220>
   <223> Synthetic oligonucleotide
<400> 824
   tatcaggata catttctaca 20
<210> 825
   <211> 20
   <212> DNA
   <213> Artificial sequence
<220>
   <223> Synthetic oligonucleotide
<400> 825
   tgtttatcag gatacatttc 20
<210> 826
   <211> 20
   <212> DNA
   <213> Artificial sequence
<220>
   <223> Synthetic oligonucleotide
<400> 826
   aatgtttatc aggatacatt 20
<210> 827
   <211> 20
   <212> DNA
   <213> Artificial sequence
<220>
   <223> Synthetic oligonucleotide
<400> 827
   ttaatgttta tcaggataca 20
<210> 828
   <211> 20
   <212> DNA
   <213> Artificial sequence
<220>
   <223> Synthetic oligonucleotide
<400> 828
   gtttaatgtt tatcaggata 20
<210> 829
   <211> 20
   <212> DNA
   <213> Artificial sequence
<220>
   <223> Synthetic oligonucleotide
<400> 829
   gtgtttaatg tttatcagga 20
<210> 830
   <211> 20
   <212> DNA
   <213> Artificial sequence
<220>
   <223> Synthetic oligonucleotide
<400> 830
   ttttaagatt acagtgttta 20
<210> 831
   <211> 20
   <212> DNA
   <213> Artificial sequence
<220>
   <223> Synthetic oligonucleotide
<400> 831
   acttttaaga ttacagtgtt 20
<210> 832
   <211> 20
   <212> DNA
   <213> Artificial sequence
<220>
   <223> Synthetic oligonucleotide
<400> 832
   acacttttaa gattacagtg 20
<210> 833
   <211> 20
   <212> DNA
   <213> Artificial sequence
<220>
   <223> Synthetic oligonucleotide
<400> 833
   ttacactttt aagattacag 20
<210> 834
   <211> 20
   <212> DNA
   <213> Artificial sequence
<220>
   <223> Synthetic oligonucleotide
<400> 834
   aattacactt ttaagattac 20
<210> 835
   <211> 20
   <212> DNA
   <213> Artificial sequence
<220>
   <223> Synthetic oligonucleotide
<400> 835
   acacaattac acttttaaga 20
<210> 836
   <211> 20
   <212> DNA
   <213> Artificial sequence
<220>
   <223> Synthetic oligonucleotide
<400> 836
   tcacacaatt acacttttaa 20
<210> 837
   <211> 20
   <212> DNA
   <213> Artificial sequence
<220>
   <223> Synthetic oligonucleotide
<400> 837
   aaagtcacac aattacactt 20
<210> 838
   <211> 20
   <212> DNA
   <213> Artificial sequence
<220>
   <223> Synthetic oligonucleotide
<400> 838
   tgaaaaagtc acacaattac 20
<210> 839
   <211> 20
   <212> DNA
   <213> Artificial sequence
<220>
   <223> Synthetic oligonucleotide
<400> 839
   tctgaaaaag tcacacaatt 20
<210> 840
   <211> 20
   <212> DNA
   <213> Artificial sequence
<220>
   <223> Synthetic oligonucleotide
<400> 840
   actctgaaaa agtcacacaa 20
<210> 841
   <211> 20
   <212> DNA
   <213> Artificial sequence
<220>
   <223> Synthetic oligonucleotide
<400> 841
   agcaactctg aaaaagtcac 20
<210> 842
   <211> 20
   <212> DNA
   <213> Artificial sequence
<220>
   <223> Synthetic oligonucleotide
<400> 842
   actttaaagc aactctgaaa 20
<210> 843
   <211> 20
   <212> DNA
   <213> Artificial sequence
<220>
   <223> Synthetic oligonucleotide
<400> 843
   gtactttaaa gcaactctga 20
<210> 844
   <211> 20
   <212> DNA
   <213> Artificial sequence
<220>
   <223> Synthetic oligonucleotide
<400> 844
   aggtacttta aagcaactct 20
<210> 845
   <211> 20
   <212> DNA
   <213> Artificial sequence
<220>
   <223> Synthetic oligonucleotide
<400> 845
   cactacaggt actttaaagc 20
<210> 846
   <211> 20
   <212> DNA
   <213> Artificial sequence
<220>
   <223> Synthetic oligonucleotide
<400> 846
   ttctcactac aggtacttta 20
<210> 847
   <211> 20
   <212> DNA
   <213> Artificial sequence
<220>
   <223> Synthetic oligonucleotide
<400> 847
   gtttctcact acaggtactt 20
<210> 848
   <211> 20
   <212> DNA
   <213> Artificial sequence
<220>
   <223> Synthetic oligonucleotide
<400> 848
   cagtttctca ctacaggtac 20
<210> 849
   <211> 20
   <212> DNA
   <213> Artificial sequence
<220>
   <223> Synthetic oligonucleotide
<400> 849
   atcagtttct cactacaggt 20
<210> 850
   <211> 20
   <212> DNA
   <213> Artificial sequence
<220>
   <223> Synthetic oligonucleotide
<400> 850
   aaatcagttt ctcactacag 20
<210> 851
   <211> 20
   <212> DNA
   <213> Artificial sequence
<220>
   <223> Synthetic oligonucleotide
<400> 851
   tcataaatca gtttctcact 20
<210> 852
   <211> 20
   <212> DNA
   <213> Artificial sequence
<220>
   <223> Synthetic oligonucleotide
<400> 852
   gtgatcataa atcagtttct 20
<210> 853
   <211> 20
   <212> DNA
   <213> Artificial sequence
<220>
   <223> Synthetic oligonucleotide
<400> 853
   aagtgatcat aaatcagttt 20
<210> 854
   <211> 20
   <212> DNA
   <213> Artificial sequence
<220>
   <223> Synthetic oligonucleotide
<400> 854
   ccaagtgatc ataaatcagt 20
<210> 855
   <211> 20
   <212> DNA
   <213> Artificial sequence
<220>
   <223> Synthetic oligonucleotide
<400> 855
   ttccaagtga tcataaatca 20
<210> 856
   <211> 20
   <212> DNA
   <213> Artificial sequence
<220>
   <223> Synthetic oligonucleotide
<400> 856
   tcttccaagt gatcataaat 20
<210> 857
   <211> 20
   <212> DNA
   <213> Artificial sequence
<220>
   <223> Synthetic oligonucleotide
<400> 857
   taactgagtt ttataaaact 20
<210> 858
   <211> 20
   <212> DNA
   <213> Artificial sequence
<220>
   <223> Synthetic oligonucleotide
<400> 858
   aaacagacat tttaactgag 20
<210> 859
   <211> 20
   <212> DNA
   <213> Artificial sequence
<220>
   <223> Synthetic oligonucleotide
<400> 859
   tgaaacagac attttaactg 20
<210> 860
   <211> 20
   <212> DNA
   <213> Artificial sequence
<220>
   <223> Synthetic oligonucleotide
<400> 860
   atttaagtct ggcaaaatac 20
<210> 861
   <211> 20
   <212> DNA
   <213> Artificial sequence
<220>
   <223> Synthetic oligonucleotide
<400> 861
   tgtgatttaa gtctggcaaa 20
<210> 862
   <211> 20
   <212> DNA
   <213> Artificial sequence
<220>
   <223> Synthetic oligonucleotide
<400> 862
   tctgtgattt aagtctggca 20
<210> 863
   <211> 20
   <212> DNA
   <213> Artificial sequence
<220>
   <223> Synthetic oligonucleotide
<400> 863
   catctgtgat ttaagtctgg 20
<210> 864
   <211> 20
   <212> DNA
   <213> Artificial sequence
<220>
   <223> Synthetic oligonucleotide
<400> 864
   cccatctgtg atttaagtct 20
<210> 865
   <211> 20
   <212> DNA
   <213> Artificial sequence
<220>
   <223> Synthetic oligonucleotide
<400> 865
   tacccatctg tgatttaagt 20
<210> 866
   <211> 20
   <212> DNA
   <213> Artificial sequence
<220>
   <223> Synthetic oligonucleotide
<400> 866
   aatacccatc tgtgatttaa 20
<210> 867
   <211> 20
   <212> DNA
   <213> Artificial sequence
<220>
   <223> Synthetic oligonucleotide
<400> 867
   ttaataccca tctgtgattt 20
<210> 868
   <211> 20
   <212> DNA
   <213> Artificial sequence
<220>
   <223> Synthetic oligonucleotide
<400> 868
   gacaaagaaa ttctgacaag 20
<210> 869
   <211> 20
   <212> DNA
   <213> Artificial sequence
<220>
   <223> Synthetic oligonucleotide
<400> 869
   gaatgacaaa gaaattctga 20
<210> 870
   <211> 20
   <212> DNA
   <213> Artificial sequence
<220>
   <223> Synthetic oligonucleotide
<400> 870
   aggcttgaat gacaaagaaa 20
<210> 871
   <211> 20
   <212> DNA
   <213> Artificial sequence
<220>
   <223> Synthetic oligonucleotide
<400> 871
   taagtgccat acagggtttt 20
<210> 872
   <211> 20
   <212> DNA
   <213> Artificial sequence
<220>
   <223> Synthetic oligonucleotide
<400> 872
   ataataagtg ccatacaggg 20
<210> 873
   <211> 20
   <212> DNA
   <213> Artificial sequence
<220>
   <223> Synthetic oligonucleotide
<400> 873
   cctcataata agtgccatac 20
<210> 874
   <211> 20
   <212> DNA
   <213> Artificial sequence
<220>
   <223> Synthetic oligonucleotide
<400> 874
   agcctcataa taagtgccat 20
<210> 875
   <211> 20
   <212> DNA
   <213> Artificial sequence
<220>
   <223> Synthetic oligonucleotide
<400> 875
   atagcctcat aataagtgcc 20
<210> 876
   <211> 20
   <212> DNA
   <213> Artificial sequence
<220>
   <223> Synthetic oligonucleotide
<400> 876
   cttttaatag cctcataata 20
<210> 877
   <211> 20
   <212> DNA
   <213> Artificial sequence
<220>
   <223> Synthetic oligonucleotide
<400> 877
   ttcttttaat agcctcataa 20
<210> 878
   <211> 20
   <212> DNA
   <213> Artificial sequence
<220>
   <223> Synthetic oligonucleotide
<400> 878
   gattctttta atagcctcat 20
<210> 879
   <211> 20
   <212> DNA
   <213> Artificial sequence
<220>
   <223> Synthetic oligonucleotide
<400> 879
   tggattcttt taatagcctc 20
<210> 880
   <211> 20
   <212> DNA
   <213> Artificial sequence
<220>
   <223> Synthetic oligonucleotide
<400> 880
   tttggattct tttaatagcc 20
<210> 881
   <211> 20
   <212> DNA
   <213> Artificial sequence
<220>
   <223> Synthetic oligonucleotide
<400> 881
   aatttggatt cttttaatag 20
<210> 882
   <211> 20
   <212> DNA
   <213> Artificial sequence
<220>
   <223> Synthetic oligonucleotide
<400> 882
   tgaatttgga ttcttttaat 20
<210> 883
   <211> 20
   <212> DNA
   <213> Artificial sequence
<220>
   <223> Synthetic oligonucleotide
<400> 883
   ttagtttgaa tttggattct 20
<210> 884
   <211> 20
   <212> DNA
   <213> Artificial sequence
<220>
   <223> Synthetic oligonucleotide
<400> 884
   ttttagtttg aatttggatt 20
<210> 885
   <211> 20
   <212> DNA
   <213> Artificial sequence
<220>
   <223> Synthetic oligonucleotide
<400> 885
   ttttttagtt tgaatttgga 20
<210> 886
   <211> 20
   <212> DNA
   <213> Artificial sequence
<220>
   <223> Synthetic oligonucleotide
<400> 886
   cagtccttta atgcttcccc 20
<210> 887
   <211> 20
   <212> DNA
   <213> Artificial sequence
<220>
   <223> Synthetic oligonucleotide
<400> 887
   gtcagtcctt taatgcttcc 20
<210> 888
   <211> 20
   <212> DNA
   <213> Artificial sequence
<220>
   <223> Synthetic oligonucleotide
<400> 888
   cagtcagtcc tttaatgctt 20
<210> 889
   <211> 20
   <212> DNA
   <213> Artificial sequence
<220>
   <223> Synthetic oligonucleotide
<400> 889
   ttcagtcagt cctttaatgc 20
<210> 890
   <211> 20
   <212> DNA
   <213> Artificial sequence
<220>
   <223> Synthetic oligonucleotide
<400> 890
   ggccttcagt cagtccttta 20
<210> 891
   <211> 20
   <212> DNA
   <213> Artificial sequence
<220>
   <223> Synthetic oligonucleotide
<400> 891
   tgcaggcctt cagtcagtcc 20
<210> 892
   <211> 20
   <212> DNA
   <213> Artificial sequence
<220>
   <223> Synthetic oligonucleotide
<400> 892
   catgcaggcc ttcagtcagt 20
<210> 893
   <211> 20
   <212> DNA
   <213> Artificial sequence
<220>
   <223> Synthetic oligonucleotide
<400> 893
   tcatgaacat ggaatccatg 20
<210> 894
   <211> 20
   <212> DNA
   <213> Artificial sequence
<220>
   <223> Synthetic oligonucleotide
<400> 894
   actcatgaac atggaatcca 20
<210> 895
   <211> 20
   <212> DNA
   <213> Artificial sequence
<220>
   <223> Synthetic oligonucleotide
<400> 895
   ctgtattatc tccaaactca 20
<210> 896
   <211> 20
   <212> DNA
   <213> Artificial sequence
<220>
   <223> Synthetic oligonucleotide
<400> 896
   actggtacag cctgctgtat 20
<210> 897
   <211> 20
   <212> DNA
   <213> Artificial sequence
<220>
   <223> Synthetic oligonucleotide
<400> 897
   gcactggtac agcctgctgt 20
<210> 898
   <211> 20
   <212> DNA
   <213> Artificial sequence
<220>
   <223> Synthetic oligonucleotide
<400> 898
   ctgcactggt acagcctgct 20
<210> 899
   <211> 20
   <212> DNA
   <213> Artificial sequence
<220>
   <223> Synthetic oligonucleotide
<400> 899
   acctgcactg gtacagcctg 20
<210> 900
   <211> 20
   <212> DNA
   <213> Artificial sequence
<220>
   <223> Synthetic oligonucleotide
<400> 900
   tttctggata gaggattaaa 20
<210> 901
   <211> 20
   <212> DNA
   <213> Artificial sequence
<220>
   <223> Synthetic oligonucleotide
<400> 901
   gttttctgga tagaggatta 20
<210> 902
   <211> 20
   <212> DNA
   <213> Artificial sequence
<220>
   <223> Synthetic oligonucleotide
<400> 902
   ccgtgttttc tggatagagg 20
<210> 903
   <211> 20
   <212> DNA
   <213> Artificial sequence
<220>
   <223> Synthetic oligonucleotide
<400> 903
   caccgtgttt tctggataga 20
<210> 904
   <211> 20
   <212> DNA
   <213> Artificial sequence
<220>
   <223> Synthetic oligonucleotide
<400> 904
   cccaccgtgt tttctggata 20
<210> 905
   <211> 20
   <212> DNA
   <213> Artificial sequence
<220>
   <223> Synthetic oligonucleotide
<400> 905
   ggcccaccgt gttttctgga 20
<210> 906
   <211> 20
   <212> DNA
   <213> Artificial sequence
<220>
   <223> Synthetic oligonucleotide
<400> 906
   ttggcccacc gtgttttctg 20
<210> 907
   <211> 20
   <212> DNA
   <213> Artificial sequence
<220>
   <223> Synthetic oligonucleotide
<400> 907
   ctttggccca ccgtgttttc 20
<210> 908
   <211> 20
   <212> DNA
   <213> Artificial sequence
<220>
   <223> Synthetic oligonucleotide
<400> 908
   tcctttggcc caccgtgttt 20
<210> 909
   <211> 20
   <212> DNA
   <213> Artificial sequence
<220>
   <223> Synthetic oligonucleotide
<400> 909
   aagtctccaa catgcctctc 20
<210> 910
   <211> 20
   <212> DNA
   <213> Artificial sequence
<220>
   <223> Synthetic oligonucleotide
<400> 910
   gcccaagtct ccaacatgcc 20
<210> 911
   <211> 20
   <212> DNA
   <213> Artificial sequence
<220>
   <223> Synthetic oligonucleotide
<400> 911
   ttgcccaagt ctccaacatg 20
<210> 912
   <211> 20
   <212> DNA
   <213> Artificial sequence
<220>
   <223> Synthetic oligonucleotide
<400> 912
   cattgcccaa gtctccaaca 20
<210> 913
   <211> 20
   <212> DNA
   <213> Artificial sequence
<220>
   <223> Synthetic oligonucleotide
<400> 913
   cagtcacatt gcccaagtct 20
<210> 914
   <211> 20
   <212> DNA
   <213> Artificial sequence
<220>
   <223> Synthetic oligonucleotide
<400> 914
   tcagcagtca cattgcccaa 20
<210> 915
   <211> 20
   <212> DNA
   <213> Artificial sequence
<220>
   <223> Synthetic oligonucleotide
<400> 915
   tgtcagcagt cacattgccc 20
<210> 916
   <211> 20
   <212> DNA
   <213> Artificial sequence
<220>
   <223> Synthetic oligonucleotide
<400> 916
   tttgtcagca gtcacattgc 20
<210> 917
   <211> 20
   <212> DNA
   <213> Artificial sequence
<220>
   <223> Synthetic oligonucleotide
<400> 917
   tctttgtcag cagtcacatt 20
<210> 918
   <211> 20
   <212> DNA
   <213> Artificial sequence
<220>
   <223> Synthetic oligonucleotide
<400> 918
   catctttgtc agcagtcaca 20
<210> 919
   <211> 20
   <212> DNA
   <213> Artificial sequence
<220>
   <223> Synthetic oligonucleotide
<400> 919
   accatctttg tcagcagtca 20
<210> 920
   <211> 20
   <212> DNA
   <213> Artificial sequence
<220>
   <223> Synthetic oligonucleotide
<400> 920
   ccacaccatc tttgtcagca 20
<210> 921
   <211> 20
   <212> DNA
   <213> Artificial sequence
<220>
   <223> Synthetic oligonucleotide
<400> 921
   ggccacacca tctttgtcag 20
<210> 922
   <211> 20
   <212> DNA
   <213> Artificial sequence
<220>
   <223> Synthetic oligonucleotide
<400> 922
   cacatcggcc acaccatctt 20
<210> 923
   <211> 20
   <212> DNA
   <213> Artificial sequence
<220>
   <223> Synthetic oligonucleotide
<400> 923
   gacacatcgg ccacaccatc 20
<210> 924
   <211> 20
   <212> DNA
   <213> Artificial sequence
<220>
   <223> Synthetic oligonucleotide
<400> 924
   aatagacaca tcggccacac 20
<210> 925
   <211> 20
   <212> DNA
   <213> Artificial sequence
<220>
   <223> Synthetic oligonucleotide
<400> 925
   tcaatagaca catcggccac 20
<210> 926
   <211> 20
   <212> DNA
   <213> Artificial sequence
<220>
   <223> Synthetic oligonucleotide
<400> 926
   cttcaataga cacatcggcc 20
<210> 927
   <211> 20
   <212> DNA
   <213> Artificial sequence
<220>
   <223> Synthetic oligonucleotide
<400> 927
   atcttcaata gacacatcgg 20
<210> 928
   <211> 20
   <212> DNA
   <213> Artificial sequence
<220>
   <223> Synthetic oligonucleotide
<400> 928
   gaatcttcaa tagacacatc 20
<210> 929
   <211> 20
   <212> DNA
   <213> Artificial sequence
<220>
   <223> Synthetic oligonucleotide
<400> 929
   cagaatcttc aatagacaca 20
<210> 930
   <211> 20
   <212> DNA
   <213> Artificial sequence
<220>
   <223> Synthetic oligonucleotide
<400> 930
   cacagaatct tcaatagaca 20
<210> 931
   <211> 20
   <212> DNA
   <213> Artificial sequence
<220>
   <223> Synthetic oligonucleotide
<400> 931
   atcacagaat cttcaataga 20
<210> 932
   <211> 20
   <212> DNA
   <213> Artificial sequence
<220>
   <223> Synthetic oligonucleotide
<400> 932
   agatcacaga atcttcaata 20
<210> 933
   <211> 20
   <212> DNA
   <213> Artificial sequence
<220>
   <223> Synthetic oligonucleotide
<400> 933
   tgagatcaca gaatcttcaa 20
<210> 934
   <211> 20
   <212> DNA
   <213> Artificial sequence
<220>
   <223> Synthetic oligonucleotide
<400> 934
   agtgagatca cagaatcttc 20
<210> 935
   <211> 20
   <212> DNA
   <213> Artificial sequence
<220>
   <223> Synthetic oligonucleotide
<400> 935
   tgagagtgag atcacagaat 20
<210> 936
   <211> 20
   <212> DNA
   <213> Artificial sequence
<220>
   <223> Synthetic oligonucleotide
<400> 936
   gtctcctgag agtgagatca 20
<210> 937
   <211> 20
   <212> DNA
   <213> Artificial sequence
<220>
   <223> Synthetic oligonucleotide
<400> 937
   tggtctcctg agagtgagat 20
<210> 938
   <211> 20
   <212> DNA
   <213> Artificial sequence
<220>
   <223> Synthetic oligonucleotide
<400> 938
   aatggtctcc tgagagtgag 20
<210> 939
   <211> 20
   <212> DNA
   <213> Artificial sequence
<220>
   <223> Synthetic oligonucleotide
<400> 939
   gcaatggtct cctgagagtg 20
<210> 940
   <211> 20
   <212> DNA
   <213> Artificial sequence
<220>
   <223> Synthetic oligonucleotide
<400> 940
   atgcaatggt ctcctgagag 20
<210> 941
   <211> 20
   <212> DNA
   <213> Artificial sequence
<220>
   <223> Synthetic oligonucleotide
<400> 941
   tgatgcaatg gtctcctgag 20
<210> 942
   <211> 20
   <212> DNA
   <213> Artificial sequence
<220>
   <223> Synthetic oligonucleotide
<400> 942
   aatgatgcaa tggtctcctg 20
<210> 943
   <211> 20
   <212> DNA
   <213> Artificial sequence
<220>
   <223> Synthetic oligonucleotide
<400> 943
   ccaatgatgc aatggtctcc 20
<210> 944
   <211> 20
   <212> DNA
   <213> Artificial sequence
<220>
   <223> Synthetic oligonucleotide
<400> 944
   ggccaatgat gcaatggtct 20
<210> 945
   <211> 20
   <212> DNA
   <213> Artificial sequence
<220>
   <223> Synthetic oligonucleotide
<400> 945
   gcggccaatg atgcaatggt 20
<210> 946
   <211> 20
   <212> DNA
   <213> Artificial sequence
<220>
   <223> Synthetic oligonucleotide
<400> 946
   gtgcggccaa tgatgcaatg 20
<210> 947
   <211> 20
   <212> DNA
   <213> Artificial sequence
<220>
   <223> Synthetic oligonucleotide
<400> 947
   gtgtgcggcc aatgatgcaa 20
<210> 948
   <211> 20
   <212> DNA
   <213> Artificial sequence
<220>
   <223> Synthetic oligonucleotide
<400> 948
   cagtgtgcgg ccaatgatgc 20
<210> 949
   <211> 20
   <212> DNA
   <213> Artificial sequence
<220>
   <223> Synthetic oligonucleotide
<400> 949
   accagtgtgc ggccaatgat 20
<210> 950
   <211> 20
   <212> DNA
   <213> Artificial sequence
<220>
   <223> Synthetic oligonucleotide
<400> 950
   ccaccagtgt gcggccaatg 20
<210> 951
   <211> 20
   <212> DNA
   <213> Artificial sequence
<220>
   <223> Synthetic oligonucleotide
<400> 951
   gaccaccagt gtgcggccaa 20
<210> 952
   <211> 20
   <212> DNA
   <213> Artificial sequence
<220>
   <223> Synthetic oligonucleotide
<400> 952
   tggaccacca gtgtgcggcc 20
<210> 953
   <211> 20
   <212> DNA
   <213> Artificial sequence
<220>
   <223> Synthetic oligonucleotide
<400> 953
   ttcatggacc accagtgtgc 20
<210> 954
   <211> 20
   <212> DNA
   <213> Artificial sequence
<220>
   <223> Synthetic oligonucleotide
<400> 954
   ttttcatgga ccaccagtgt 20
<210> 955
   <211> 20
   <212> DNA
   <213> Artificial sequence
<220>
   <223> Synthetic oligonucleotide
<400> 955
   ctttttcatg gaccaccagt 20
<210> 956
   <211> 17
   <212> DNA
   <213> Artificial sequence
<220>
   <223> Synthetic oligonucleotide
<400> 956
   ccactctggc cccaaac 17
<210> 957
   <211> 17
   <212> DNA
   <213> Artificial sequence
<220>
   <223> Synthetic oligonucleotide
<400> 957
   cccactctgg ccccaaa 17
<210> 958
   <211> 17
   <212> DNA
   <213> Artificial sequence
<220>
   <223> Synthetic oligonucleotide
<400> 958
   gcccactctg gccccaa 17
<210> 959
   <211> 17
   <212> DNA
   <213> Artificial sequence
<220>
   <223> Synthetic oligonucleotide
<400> 959
   cgcccactct ggcccca 17
<210> 960
   <211> 17
   <212> DNA
   <213> Artificial sequence
<220>
   <223> Synthetic oligonucleotide
<400> 960
   cgactacttt ataggcc 17
<210> 961
   <211> 17
   <212> DNA
   <213> Artificial sequence
<220>
   <223> Synthetic oligonucleotide
<400> 961
   gcgactactt tataggc 17
<210> 962
   <211> 17
   <212> DNA
   <213> Artificial sequence
<220>
   <223> Synthetic oligonucleotide
<400> 962
   cgcgactact ttatagg 17
<210> 963
   <211> 17
   <212> DNA
   <213> Artificial sequence
<220>
   <223> Synthetic oligonucleotide
<400> 963
   ccgcgactac tttatag 17
<210> 964
   <211> 17
   <212> DNA
   <213> Artificial sequence
<220>
   <223> Synthetic oligonucleotide
<400> 964
   cgctgcagga gactacg 17
<210> 965
   <211> 17
   <212> DNA
   <213> Artificial sequence
<220>
   <223> Synthetic oligonucleotide
<400> 965
   acgctgcagg agactac 17
<210> 966
   <211> 17
   <212> DNA
   <213> Artificial sequence
<220>
   <223> Synthetic oligonucleotide
<400> 966
   tcgcccttca gcacgca 17
<210> 967
   <211> 17
   <212> DNA
   <213> Artificial sequence
<220>
   <223> Synthetic oligonucleotide
<400> 967
   gtcgcccttc agcacgc 17
<210> 968
   <211> 17
   <212> DNA
   <213> Artificial sequence
<220>
   <223> Synthetic oligonucleotide
<400> 968
   cgtcgccctt cagcacg 17
<210> 969
   <211> 17
   <212> DNA
   <213> Artificial sequence
<220>
   <223> Synthetic oligonucleotide
<400> 969
   ccgtcgccct tcagcac 17
<210> 970
   <211> 17
   <212> DNA
   <213> Artificial sequence
<220>
   <223> Synthetic oligonucleotide
<400> 970
   gccgtcgccc ttcagca 17
<210> 971
   <211> 17
   <212> DNA
   <213> Artificial sequence
<220>
   <223> Synthetic oligonucleotide
<400> 971
   tctgctcgaa attgatg 17
<210> 972
   <211> 17
   <212> DNA
   <213> Artificial sequence
<220>
   <223> Synthetic oligonucleotide
<400> 972
   ttctgctcga aattgat 17
<210> 973
   <211> 17
   <212> DNA
   <213> Artificial sequence
<220>
   <223> Synthetic oligonucleotide
<400> 973
   cttctgctcg aaattga 17
<210> 974
   <211> 17
   <212> DNA
   <213> Artificial sequence
<220>
   <223> Synthetic oligonucleotide
<400> 974
   ccttctgctc gaaattg 17
<210> 975
   <211> 17
   <212> DNA
   <213> Artificial sequence
<220>
   <223> Synthetic oligonucleotide
<400> 975
   tccttctgct cgaaatt 17
<210> 976
   <211> 17
   <212> DNA
   <213> Artificial sequence
<220>
   <223> Synthetic oligonucleotide
<400> 976
   ttccttctgc tcgaaat 17
<210> 977
   <211> 17
   <212> DNA
   <213> Artificial sequence
<220>
   <223> Synthetic oligonucleotide
<400> 977
   tttccttctg ctcgaaa 17
<210> 978
   <211> 17
   <212> DNA
   <213> Artificial sequence
<220>
   <223> Synthetic oligonucleotide
<400> 978
   ctttccttct gctcgaa 17
<210> 979
   <211> 17
   <212> DNA
   <213> Artificial sequence
<220>
   <223> Synthetic oligonucleotide
<400> 979
   actttccttc tgctcga 17
<210> 980
   <211> 17
   <212> DNA
   <213> Artificial sequence
<220>
   <223> Synthetic oligonucleotide
<400> 980
   tactttcctt ctgctcg 17
<210> 981
   <211> 17
   <212> DNA
   <213> Artificial sequence
<220>
   <223> Synthetic oligonucleotide
<400> 981
   ttactttcct tctgctc 17
<210> 982
   <211> 17
   <212> DNA
   <213> Artificial sequence
<220>
   <223> Synthetic oligonucleotide
<400> 982
   attactttcc ttctgct 17
<210> 983
   <211> 17
   <212> DNA
   <213> Artificial sequence
<220>
   <223> Synthetic oligonucleotide
<400> 983
   cattactttc cttctgc 17
<210> 984
   <211> 17
   <212> DNA
   <213> Artificial sequence
<220>
   <223> Synthetic oligonucleotide
<400> 984
   ccattacttt ccttctg 17
<210> 985
   <211> 17
   <212> DNA
   <213> Artificial sequence
<220>
   <223> Synthetic oligonucleotide
<400> 985
   tccattactt tccttct 17
<210> 986
   <211> 17
   <212> DNA
   <213> Artificial sequence
<220>
   <223> Synthetic oligonucleotide
<400> 986
   gtccattact ttccttc 17
<210> 987
   <211> 17
   <212> DNA
   <213> Artificial sequence
<220>
   <223> Synthetic oligonucleotide
<400> 987
   ggtccattac tttcctt 17
<210> 988
   <211> 17
   <212> DNA
   <213> Artificial sequence
<220>
   <223> Synthetic oligonucleotide
<400> 988
   tggtccatta ctttcct 17
<210> 989
   <211> 17
   <212> DNA
   <213> Artificial sequence
<220>
   <223> Synthetic oligonucleotide
<400> 989
   ctggtccatt actttcc 17
<210> 990
   <211> 17
   <212> DNA
   <213> Artificial sequence
<220>
   <223> Synthetic oligonucleotide
<400> 990
   actggtccat tactttc 17
<210> 991
   <211> 17
   <212> DNA
   <213> Artificial sequence
<220>
   <223> Synthetic oligonucleotide
<400> 991
   cactggtcca ttacttt 17
<210> 992
   <211> 17
   <212> DNA
   <213> Artificial sequence
<220>
   <223> Synthetic oligonucleotide
<400> 992
   tcactggtcc attactt 17
<210> 993
   <211> 17
   <212> DNA
   <213> Artificial sequence
<220>
   <223> Synthetic oligonucleotide
<400> 993
   ttcactggtc cattact 17
<210> 994
   <211> 17
   <212> DNA
   <213> Artificial sequence
<220>
   <223> Synthetic oligonucleotide
<400> 994
   cttcactggt ccattac 17
<210> 995
   <211> 17
   <212> DNA
   <213> Artificial sequence
<220>
   <223> Synthetic oligonucleotide
<400> 995
   ccttcactgg tccatta 17
<210> 996
   <211> 17
   <212> DNA
   <213> Artificial sequence
<220>
   <223> Synthetic oligonucleotide
<400> 996
   accttcactg gtccatt 17
<210> 997
   <211> 17
   <212> DNA
   <213> Artificial sequence
<220>
   <223> Synthetic oligonucleotide
<400> 997
   caccttcact ggtccat 17
<210> 998
   <211> 17
   <212> DNA
   <213> Artificial sequence
<220>
   <223> Synthetic oligonucleotide
<400> 998
   acaccttcac tggtcca 17
<210> 999
   <211> 17
   <212> DNA
   <213> Artificial sequence
<220>
   <223> Synthetic oligonucleotide
<400> 999
   cacaccttca ctggtcc 17
<210> 1000
   <211> 17
   <212> DNA
   <213> Artificial sequence
<220>
   <223> Synthetic oligonucleotide
<400> 1000
   ccacaccttc actggtc 17
<210> 1001
   <211> 17
   <212> DNA
   <213> Artificial sequence
<220>
   <223> Synthetic oligonucleotide
<400> 1001
   cccacacctt cactggt 17
<210> 1002
   <211> 17
   <212> DNA
   <213> Artificial sequence
<220>
   <223> Synthetic oligonucleotide
<400> 1002
   ccccacacct tcactgg 17
<210> 1003
   <211> 17
   <212> DNA
   <213> Artificial sequence
<220>
   <223> Synthetic oligonucleotide
<400> 1003
   ttccccacac cttcact 17
<210> 1004
   <211> 17
   <212> DNA
   <213> Artificial sequence
<220>
   <223> Synthetic oligonucleotide
<400> 1004
   cttccccaca ccttcac 17
<210> 1005
   <211> 17
   <212> DNA
   <213> Artificial sequence
<220>
   <223> Synthetic oligonucleotide
<400> 1005
   gcttccccac accttca 17
<210> 1006
   <211> 17
   <212> DNA
   <213> Artificial sequence
<220>
   <223> Synthetic oligonucleotide
<400> 1006
   tgcttcccca caccttc 17
<210> 1007
   <211> 17
   <212> DNA
   <213> Artificial sequence
<220>
   <223> Synthetic oligonucleotide
<400> 1007
   atgcttcccc acacctt 17
<210> 1008
   <211> 17
   <212> DNA
   <213> Artificial sequence
<220>
   <223> Synthetic oligonucleotide
<400> 1008
   aatgcttccc cacacct 17
<210> 1009
   <211> 17
   <212> DNA
   <213> Artificial sequence
<220>
   <223> Synthetic oligonucleotide
<400> 1009
   taatgcttcc ccacacc 17
<210> 1010
   <211> 17
   <212> DNA
   <213> Artificial sequence
<220>
   <223> Synthetic oligonucleotide
<400> 1010
   atgcaggcct tcagtca 17
<210> 1011
   <211> 17
   <212> DNA
   <213> Artificial sequence
<220>
   <223> Synthetic oligonucleotide
<400> 1011
   catgcaggcc ttcagtc 17
<210> 1012
   <211> 17
   <212> DNA
   <213> Artificial sequence
<220>
   <223> Synthetic oligonucleotide
<400> 1012
   ccatgcaggc cttcagt 17
<210> 1013
   <211> 17
   <212> DNA
   <213> Artificial sequence
<220>
   <223> Synthetic oligonucleotide
<400> 1013
   tccatgcagg ccttcag 17
<210> 1014
   <211> 17
   <212> DNA
   <213> Artificial sequence
<220>
   <223> Synthetic oligonucleotide
<400> 1014
   atccatgcag gccttca 17
<210> 1015
   <211> 17
   <212> DNA
   <213> Artificial sequence
<220>
   <223> Synthetic oligonucleotide
<400> 1015
   aatccatgca ggccttc 17
<210> 1016
   <211> 17
   <212> DNA
   <213> Artificial sequence
<220>
   <223> Synthetic oligonucleotide
<400> 1016
   gaatccatgc aggcctt 17
<210> 1017
   <211> 17
   <212> DNA
   <213> Artificial sequence
<220>
   <223> Synthetic oligonucleotide
<400> 1017
   ggaatccatg caggcct 17
<210> 1018
   <211> 17
   <212> DNA
   <213> Artificial sequence
<220>
   <223> Synthetic oligonucleotide
<400> 1018
   tggaatccat gcaggcc 17
<210> 1019
   <211> 17
   <212> DNA
   <213> Artificial sequence
<220>
   <223> Synthetic oligonucleotide
<400> 1019
   atggaatcca tgcaggc 17
<210> 1020
   <211> 17
   <212> DNA
   <213> Artificial sequence
<220>
   <223> Synthetic oligonucleotide
<400> 1020
   catggaatcc atgcagg 17
<210> 1021
   <211> 17
   <212> DNA
   <213> Artificial sequence
<220>
   <223> Synthetic oligonucleotide
<400> 1021
   acatggaatc catgcag 17
<210> 1022
   <211> 17
   <212> DNA
   <213> Artificial sequence
<220>
   <223> Synthetic oligonucleotide
<400> 1022
   aacatggaat ccatgca 17
<210> 1023
   <211> 17
   <212> DNA
   <213> Artificial sequence
<220>
   <223> Synthetic oligonucleotide
<400> 1023
   gaacatggaa tccatgc 17
<210> 1024
   <211> 17
   <212> DNA
   <213> Artificial sequence
<220>
   <223> Synthetic oligonucleotide
<400> 1024
   tgaacatgga atccatg 17
<210> 1025
   <211> 17
   <212> DNA
   <213> Artificial sequence
<220>
   <223> Synthetic oligonucleotide
<400> 1025
   atgaacatgg aatccat 17
<210> 1026
   <211> 17
   <212> DNA
   <213> Artificial sequence
<220>
   <223> Synthetic oligonucleotide
<400> 1026
   ctgcactggt acagcct 17
<210> 1027
   <211> 17
   <212> DNA
   <213> Artificial sequence
<220>
   <223> Synthetic oligonucleotide
<400> 1027
   cctgcactgg tacagcc 17
<210> 1028
   <211> 17
   <212> DNA
   <213> Artificial sequence
<220>
   <223> Synthetic oligonucleotide
<400> 1028
   acctgcactg gtacagc 17
<210> 1029
   <211> 17
   <212> DNA
   <213> Artificial sequence
<220>
   <223> Synthetic oligonucleotide
<400> 1029
   gacctgcact ggtacag 17
<210> 1030
   <211> 17
   <212> DNA
   <213> Artificial sequence
<220>
   <223> Synthetic oligonucleotide
<400> 1030
   ggacctgcac tggtaca 17
<210> 1031
   <211> 17
   <212> DNA
   <213> Artificial sequence
<220>
   <223> Synthetic oligonucleotide
<400> 1031
   aggacctgca ctggtac 17
<210> 1032
   <211> 17
   <212> DNA
   <213> Artificial sequence
<220>
   <223> Synthetic oligonucleotide
<400> 1032
   gaggacctgc actggta 17
<210> 1033
   <211> 17
   <212> DNA
   <213> Artificial sequence
<220>
   <223> Synthetic oligonucleotide
<400> 1033
   tgaggacctg cactggt 17
<210> 1034
   <211> 17
   <212> DNA
   <213> Artificial sequence
<220>
   <223> Synthetic oligonucleotide
<400> 1034
   gtgaggacct gcactgg 17
<210> 1035
   <211> 17
   <212> DNA
   <213> Artificial sequence
<220>
   <223> Synthetic oligonucleotide
<400> 1035
   agtgaggacc tgcactg 17
<210> 1036
   <211> 17
   <212> DNA
   <213> Artificial sequence
<220>
   <223> Synthetic oligonucleotide
<400> 1036
   aagtgaggac ctgcact 17
<210> 1037
   <211> 17
   <212> DNA
   <213> Artificial sequence
<220>
   <223> Synthetic oligonucleotide
<400> 1037
   aaagtgagga cctgcac 17
<210> 1038
   <211> 17
   <212> DNA
   <213> Artificial sequence
<220>
   <223> Synthetic oligonucleotide
<400> 1038
   taaagtgagg acctgca 17
<210> 1039
   <211> 17
   <212> DNA
   <213> Artificial sequence
<220>
   <223> Synthetic oligonucleotide
<400> 1039
   ttaaagtgag gacctgc 17
<210> 1040
   <211> 17
   <212> DNA
   <213> Artificial sequence
<220>
   <223> Synthetic oligonucleotide
<400> 1040
   attaaagtga ggacctg 17
<210> 1041
   <211> 17
   <212> DNA
   <213> Artificial sequence
<220>
   <223> Synthetic oligonucleotide
<400> 1041
   gattaaagtg aggacct 17
<210> 1042
   <211> 17
   <212> DNA
   <213> Artificial sequence
<220>
   <223> Synthetic oligonucleotide
<400> 1042
   ggattaaagt gaggacc 17
<210> 1043
   <211> 17
   <212> DNA
   <213> Artificial sequence
<220>
   <223> Synthetic oligonucleotide
<400> 1043
   aggattaaag tgaggac 17
<210> 1044
   <211> 17
   <212> DNA
   <213> Artificial sequence
<220>
   <223> Synthetic oligonucleotide
<400> 1044
   gaggattaaa gtgagga 17
<210> 1045
   <211> 17
   <212> DNA
   <213> Artificial sequence
<220>
   <223> Synthetic oligonucleotide
<400> 1045
   agaggattaa agtgagg 17
<210> 1046
   <211> 17
   <212> DNA
   <213> Artificial sequence
<220>
   <223> Synthetic oligonucleotide
<400> 1046
   tagaggatta aagtgag 17
<210> 1047
   <211> 17
   <212> DNA
   <213> Artificial sequence
<220>
   <223> Synthetic oligonucleotide
<400> 1047
   atagaggatt aaagtga 17
<210> 1048
   <211> 17
   <212> DNA
   <213> Artificial sequence
<220>
   <223> Synthetic oligonucleotide
<400> 1048
   gatagaggat taaagtg 17
<210> 1049
   <211> 17
   <212> DNA
   <213> Artificial sequence
<220>
   <223> Synthetic oligonucleotide
<400> 1049
   ggatagagga ttaaagt 17
<210> 1050
   <211> 17
   <212> DNA
   <213> Artificial sequence
<220>
   <223> Synthetic oligonucleotide
<400> 1050
   tggatagagg attaaag 17
<210> 1051
   <211> 17
   <212> DNA
   <213> Artificial sequence
<220>
   <223> Synthetic oligonucleotide
<400> 1051
   ctggatagag gattaaa 17
<210> 1052
   <211> 17
   <212> DNA
   <213> Artificial sequence
<220>
   <223> Synthetic oligonucleotide
<400> 1052
   tctggataga ggattaa 17
<210> 1053
   <211> 17
   <212> DNA
   <213> Artificial sequence
<220>
   <223> Synthetic oligonucleotide
<400> 1053
   ctttggccca ccgtgtt 17
<210> 1054
   <211> 17
   <212> DNA
   <213> Artificial sequence
<220>
   <223> Synthetic oligonucleotide
<400> 1054
   cctttggccc accgtgt 17
<210> 1055
   <211> 17
   <212> DNA
   <213> Artificial sequence
<220>
   <223> Synthetic oligonucleotide
<400> 1055
   tcctttggcc caccgtg 17
<210> 1056
   <211> 17
   <212> DNA
   <213> Artificial sequence
<220>
   <223> Synthetic oligonucleotide
<400> 1056
   atcctttggc ccaccgt 17
<210> 1057
   <211> 17
   <212> DNA
   <213> Artificial sequence
<220>
   <223> Synthetic oligonucleotide
<400> 1057
   catcctttgg cccaccg 17
<210> 1058
   <211> 17
   <212> DNA
   <213> Artificial sequence
<220>
   <223> Synthetic oligonucleotide
<400> 1058
   tcatcctttg gcccacc 17
<210> 1059
   <211> 17
   <212> DNA
   <213> Artificial sequence
<220>
   <223> Synthetic oligonucleotide
<400> 1059
   ttcatccttt ggcccac 17
<210> 1060
   <211> 17
   <212> DNA
   <213> Artificial sequence
<220>
   <223> Synthetic oligonucleotide
<400> 1060
   cttcatcctt tggccca 17
<210> 1061
   <211> 17
   <212> DNA
   <213> Artificial sequence
<220>
   <223> Synthetic oligonucleotide
<400> 1061
   tcttcatcct ttggccc 17
<210> 1062
   <211> 17
   <212> DNA
   <213> Artificial sequence
<220>
   <223> Synthetic oligonucleotide
<400> 1062
   ctcttcatcc tttggcc 17
<210> 1063
   <211> 17
   <212> DNA
   <213> Artificial sequence
<220>
   <223> Synthetic oligonucleotide
<400> 1063
   tctcttcatc ctttggc 17
<210> 1064
   <211> 17
   <212> DNA
   <213> Artificial sequence
<220>
   <223> Synthetic oligonucleotide
<400> 1064
   ctctcttcat cctttgg 17
<210> 1065
   <211> 17
   <212> DNA
   <213> Artificial sequence
<220>
   <223> Synthetic oligonucleotide
<400> 1065
   tgcctctctt catcctt 17
<210> 1066
   <211> 17
   <212> DNA
   <213> Artificial sequence
<220>
   <223> Synthetic oligonucleotide
<400> 1066
   atgcctctct tcatcct 17
<210> 1067
   <211> 17
   <212> DNA
   <213> Artificial sequence
<220>
   <223> Synthetic oligonucleotide
<400> 1067
   catgcctctc ttcatcc 17
<210> 1068
   <211> 17
   <212> DNA
   <213> Artificial sequence
<220>
   <223> Synthetic oligonucleotide
<400> 1068
   acatgcctct cttcatc 17
<210> 1069
   <211> 17
   <212> DNA
   <213> Artificial sequence
<220>
   <223> Synthetic oligonucleotide
<400> 1069
   aacatgcctc tcttcat 17
<210> 1070
   <211> 17
   <212> DNA
   <213> Artificial sequence
<220>
   <223> Synthetic oligonucleotide
<400> 1070
   caacatgcct ctcttca 17
<210> 1071
   <211> 17
   <212> DNA
   <213> Artificial sequence
<220>
   <223> Synthetic oligonucleotide
<400> 1071
   ccaacatgcc tctcttc 17
<210> 1072
   <211> 17
   <212> DNA
   <213> Artificial sequence
<220>
   <223> Synthetic oligonucleotide
<400> 1072
   tccaacatgc ctctctt 17
<210> 1073
   <211> 17
   <212> DNA
   <213> Artificial sequence
<220>
   <223> Synthetic oligonucleotide
<400> 1073
   ctccaacatg cctctct 17
<210> 1074
   <211> 17
   <212> DNA
   <213> Artificial sequence
<220>
   <223> Synthetic oligonucleotide
<400> 1074
   tctccaacat gcctctc 17
<210> 1075
   <211> 17
   <212> DNA
   <213> Artificial sequence
<220>
   <223> Synthetic oligonucleotide
<400> 1075
   gtctccaaca tgcctct 17
<210> 1076
   <211> 17
   <212> DNA
   <213> Artificial sequence
<220>
   <223> Synthetic oligonucleotide
<400> 1076
   agcagtcaca ttgccca 17
<210> 1077
   <211> 17
   <212> DNA
   <213> Artificial sequence
<220>
   <223> Synthetic oligonucleotide
<400> 1077
   cagcagtcac attgccc 17
<210> 1078
   <211> 17
   <212> DNA
   <213> Artificial sequence
<220>
   <223> Synthetic oligonucleotide
<400> 1078
   agacacatcg gccacac 17
<210> 1079
   <211> 17
   <212> DNA
   <213> Artificial sequence
<220>
   <223> Synthetic oligonucleotide
<400> 1079
   cttcaataga cacatcg 17
<210> 1080
   <211> 17
   <212> DNA
   <213> Artificial sequence
<220>
   <223> Synthetic oligonucleotide
<400> 1080
   gagatcacag aatcttc 17
<210> 1081
   <211> 17
   <212> DNA
   <213> Artificial sequence
<220>
   <223> Synthetic oligonucleotide
<400> 1081
   tttttcatgg accacca 17
<210> 1082
   <211> 17
   <212> DNA
   <213> Artificial sequence
<220>
   <223> Synthetic oligonucleotide
<400> 1082
   ctttttcatg gaccacc 17
<210> 1083
   <211> 17
   <212> DNA
   <213> Artificial sequence
<220>
   <223> Synthetic oligonucleotide
<400> 1083
   gctttttcat ggaccac 17
<210> 1084
   <211> 17
   <212> DNA
   <213> Artificial sequence
<220>
   <223> Synthetic oligonucleotide
<400> 1084
   tgctttttca tggacca 17
<210> 1085
   <211> 17
   <212> DNA
   <213> Artificial sequence
<220>
   <223> Synthetic oligonucleotide
<400> 1085
   ctgctttttc atggacc 17
<210> 1086
   <211> 17
   <212> DNA
   <213> Artificial sequence
<220>
   <223> Synthetic oligonucleotide
<400> 1086
   tctgcttttt catggac 17
<210> 1087
   <211> 17
   <212> DNA
   <213> Artificial sequence
<220>
   <223> Synthetic oligonucleotide
<400> 1087
   atctgctttt tcatgga 17
<210> 1088
   <211> 17
   <212> DNA
   <213> Artificial sequence
<220>
   <223> Synthetic oligonucleotide
<400> 1088
   catctgcttt ttcatgg 17
<210> 1089
   <211> 17
   <212> DNA
   <213> Artificial sequence
<220>
   <223> Synthetic oligonucleotide
<400> 1089
   tcatctgctt tttcatg 17
<210> 1090
   <211> 17
   <212> DNA
   <213> Artificial sequence
<220>
   <223> Synthetic oligonucleotide
<400> 1090
   gtcatctgct ttttcat 17
<210> 1091
   <211> 17
   <212> DNA
   <213> Artificial sequence
<220>
   <223> Synthetic oligonucleotide
<400> 1091
   agtcatctgc tttttca 17
<210> 1092
   <211> 17
   <212> DNA
   <213> Artificial sequence
<220>
   <223> Synthetic oligonucleotide
<400> 1092
   aagtcatctg ctttttc 17
<210> 1093
   <211> 17
   <212> DNA
   <213> Artificial sequence
<220>
   <223> Synthetic oligonucleotide
<400> 1093
   caagtcatct gcttttt 17
<210> 1094
   <211> 17
   <212> DNA
   <213> Artificial sequence
<220>
   <223> Synthetic oligonucleotide
<400> 1094
   ccaagtcatc tgctttt 17
<210> 1095
   <211> 17
   <212> DNA
   <213> Artificial sequence
<220>
   <223> Synthetic oligonucleotide
<400> 1095
   cccaagtcat ctgcttt 17
<210> 1096
   <211> 17
   <212> DNA
   <213> Artificial sequence
<220>
   <223> Synthetic oligonucleotide
<400> 1096
   gcccaagtca tctgctt 17
<210> 1097
   <211> 17
   <212> DNA
   <213> Artificial sequence
<220>
   <223> Synthetic oligonucleotide
<400> 1097
   tgcccaagtc atctgct 17
<210> 1098
   <211> 17
   <212> DNA
   <213> Artificial sequence
<220>
   <223> Synthetic oligonucleotide
<400> 1098
   ttgcccaagt catctgc 17
<210> 1099
   <211> 17
   <212> DNA
   <213> Artificial sequence
<220>
   <223> Synthetic oligonucleotide
<400> 1099
   ccacctttgc ccaagtc 17
<210> 1100
   <211> 17
   <212> DNA
   <213> Artificial sequence
<220>
   <223> Synthetic oligonucleotide
<400> 1100
   tccacctttg cccaagt 17
<210> 1101
   <211> 17
   <212> DNA
   <213> Artificial sequence
<220>
   <223> Synthetic oligonucleotide
<400> 1101
   ttccaccttt gcccaag 17
<210> 1102
   <211> 17
   <212> DNA
   <213> Artificial sequence
<220>
   <223> Synthetic oligonucleotide
<400> 1102
   tttccacctt tgcccaa 17
<210> 1103
   <211> 17
   <212> DNA
   <213> Artificial sequence
<220>
   <223> Synthetic oligonucleotide
<400> 1103
   atttccacct ttgccca 17
<210> 1104
   <211> 17
   <212> DNA
   <213> Artificial sequence
<220>
   <223> Synthetic oligonucleotide
<400> 1104
   catttccacc tttgccc 17
<210> 1105
   <211> 17
   <212> DNA
   <213> Artificial sequence
<220>
   <223> Synthetic oligonucleotide
<400> 1105
   tcatttccac ctttgcc 17
<210> 1106
   <211> 17
   <212> DNA
   <213> Artificial sequence
<220>
   <223> Synthetic oligonucleotide
<400> 1106
   ttcatttcca cctttgc 17
<210> 1107
   <211> 17
   <212> DNA
   <213> Artificial sequence
<220>
   <223> Synthetic oligonucleotide
<400> 1107
   tcttcatttc caccttt 17
<210> 1108
   <211> 17
   <212> DNA
   <213> Artificial sequence
<220>
   <223> Synthetic oligonucleotide
<400> 1108
   ctttcttcat ttccacc 17
<210> 1109
   <211> 17
   <212> DNA
   <213> Artificial sequence
<220>
   <223> Synthetic oligonucleotide
<400> 1109
   actttcttca tttccac 17
<210> 1110
   <211> 17
   <212> DNA
   <213> Artificial sequence
<220>
   <223> Synthetic oligonucleotide
<400> 1110
   tactttcttc atttcca 17
<210> 1111
   <211> 17
   <212> DNA
   <213> Artificial sequence
<220>
   <223> Synthetic oligonucleotide
<400> 1111
   aattacacca caagcca 17
<210> 1112
   <211> 17
   <212> DNA
   <213> Artificial sequence
<220>
   <223> Synthetic oligonucleotide
<400> 1112
   caattacacc acaagcc 17
<210> 1113
   <211> 17
   <212> DNA
   <213> Artificial sequence
<220>
   <223> Synthetic oligonucleotide
<400> 1113
   ccaattacac cacaagc 17
<210> 1114
   <211> 17
   <212> DNA
   <213> Artificial sequence
<220>
   <223> Synthetic oligonucleotide
<400> 1114
   cccaattaca ccacaag 17
<210> 1115
   <211> 17
   <212> DNA
   <213> Artificial sequence
<220>
   <223> Synthetic oligonucleotide
<400> 1115
   gatcccaatt acaccac 17
<210> 1116
   <211> 17
   <212> DNA
   <213> Artificial sequence
<220>
   <223> Synthetic oligonucleotide
<400> 1116
   cgatcccaat tacacca 17
<210> 1117
   <211> 17
   <212> DNA
   <213> Artificial sequence
<220>
   <223> Synthetic oligonucleotide
<400> 1117
   gcgatcccaa ttacacc 17
<210> 1118
   <211> 17
   <212> DNA
   <213> Artificial sequence
<220>
   <223> Synthetic oligonucleotide
<400> 1118
   ggcgatccca attacac 17
<210> 1119
   <211> 17
   <212> DNA
   <213> Artificial sequence
<220>
   <223> Synthetic oligonucleotide
<400> 1119
   gggcgatccc aattaca 17
<210> 1120
   <211> 17
   <212> DNA
   <213> Artificial sequence
<220>
   <223> Synthetic oligonucleotide
<400> 1120
   tgggcgatcc caattac 17
<210> 1121
   <211> 17
   <212> DNA
   <213> Artificial sequence
<220>
   <223> Synthetic oligonucleotide
<400> 1121
   ttgggcgatc ccaatta 17
<210> 1122
   <211> 17
   <212> DNA
   <213> Artificial sequence
<220>
   <223> Synthetic oligonucleotide
<400> 1122
   attgggcgat cccaatt 17
<210> 1123
   <211> 17
   <212> DNA
   <213> Artificial sequence
<220>
   <223> Synthetic oligonucleotide
<400> 1123
   tattgggcga tcccaat 17
<210> 1124
   <211> 17
   <212> DNA
   <213> Artificial sequence
<220>
   <223> Synthetic oligonucleotide
<400> 1124
   ttattgggcg atcccaa 17
<210> 1125
   <211> 17
   <212> DNA
   <213> Artificial sequence
<220>
   <223> Synthetic oligonucleotide
<400> 1125
   tttattgggc gatccca 17
<210> 1126
   <211> 17
   <212> DNA
   <213> Artificial sequence
<220>
   <223> Synthetic oligonucleotide
<400> 1126
   gtttattggg cgatccc 17
<210> 1127
   <211> 17
   <212> DNA
   <213> Artificial sequence
<220>
   <223> Synthetic oligonucleotide
<400> 1127
   tgtttattgg gcgatcc 17
<210> 1128
   <211> 17
   <212> DNA
   <213> Artificial sequence
<220>
   <223> Synthetic oligonucleotide
<400> 1128
   atgtttattg ggcgatc 17
<210> 1129
   <211> 17
   <212> DNA
   <213> Artificial sequence
<220>
   <223> Synthetic oligonucleotide
<400> 1129
   aatgtttatt gggcgat 17
<210> 1130
   <211> 17
   <212> DNA
   <213> Artificial sequence
<220>
   <223> Synthetic oligonucleotide
<400> 1130
   gaatgtttat tgggcga 17
<210> 1131
   <211> 17
   <212> DNA
   <213> Artificial sequence
<220>
   <223> Synthetic oligonucleotide
<400> 1131
   ggaatgttta ttgggcg 17
<210> 1132
   <211> 17
   <212> DNA
   <213> Artificial sequence
<220>
   <223> Synthetic oligonucleotide
<400> 1132
   aagggaatgt ttattgg 17
<210> 1133
   <211> 17
   <212> DNA
   <213> Artificial sequence
<220>
   <223> Synthetic oligonucleotide
<400> 1133
   caagggaatg tttattg 17
<210> 1134
   <211> 17
   <212> DNA
   <213> Artificial sequence
<220>
   <223> Synthetic oligonucleotide
<400> 1134
   ccaagggaat gtttatt 17
<210> 1135
   <211> 17
   <212> DNA
   <213> Artificial sequence
<220>
   <223> Synthetic oligonucleotide
<400> 1135
   tccaagggaa tgtttat 17
<210> 1136
   <211> 17
   <212> DNA
   <213> Artificial sequence
<220>
   <223> Synthetic oligonucleotide
<400> 1136
   atccaaggga atgttta 17
<210> 1137
   <211> 17
   <212> DNA
   <213> Artificial sequence
<220>
   <223> Synthetic oligonucleotide
<400> 1137
   catccaaggg aatgttt 17
<210> 1138
   <211> 17
   <212> DNA
   <213> Artificial sequence
<220>
   <223> Synthetic oligonucleotide
<400> 1138
   acatccaagg gaatgtt 17
<210> 1139
   <211> 17
   <212> DNA
   <213> Artificial sequence
<220>
   <223> Synthetic oligonucleotide
<400> 1139
   tacatccaag ggaatgt 17
<210> 1140
   <211> 17
   <212> DNA
   <213> Artificial sequence
<220>
   <223> Synthetic oligonucleotide
<400> 1140
   ctacatccaa gggaatg 17
<210> 1141
   <211> 17
   <212> DNA
   <213> Artificial sequence
<220>
   <223> Synthetic oligonucleotide
<400> 1141
   actacatcca agggaat 17
<210> 1142
   <211> 17
   <212> DNA
   <213> Artificial sequence
<220>
   <223> Synthetic oligonucleotide
<400> 1142
   gactacatcc aagggaa 17
<210> 1143
   <211> 17
   <212> DNA
   <213> Artificial sequence
<220>
   <223> Synthetic oligonucleotide
<400> 1143
   agactacatc caaggga 17
<210> 1144
   <211> 17
   <212> DNA
   <213> Artificial sequence
<220>
   <223> Synthetic oligonucleotide
<400> 1144
   cagactacat ccaaggg 17
<210> 1145
   <211> 17
   <212> DNA
   <213> Artificial sequence
<220>
   <223> Synthetic oligonucleotide
<400> 1145
   tcagactaca tccaagg 17
<210> 1146
   <211> 17
   <212> DNA
   <213> Artificial sequence
<220>
   <223> Synthetic oligonucleotide
<400> 1146
   ctcagactac atccaag 17
<210> 1147
   <211> 17
   <212> DNA
   <213> Artificial sequence
<220>
   <223> Synthetic oligonucleotide
<400> 1147
   cctcagacta catccaa 17
<210> 1148
   <211> 17
   <212> DNA
   <213> Artificial sequence
<220>
   <223> Synthetic oligonucleotide
<400> 1148
   gcctcagact acatcca 17
<210> 1149
   <211> 17
   <212> DNA
   <213> Artificial sequence
<220>
   <223> Synthetic oligonucleotide
<400> 1149
   ggcctcagac tacatcc 17
<210> 1150
   <211> 17
   <212> DNA
   <213> Artificial sequence
<220>
   <223> Synthetic oligonucleotide
<400> 1150
   gggcctcaga ctacatc 17
<210> 1151
   <211> 17
   <212> DNA
   <213> Artificial sequence
<220>
   <223> Synthetic oligonucleotide
<400> 1151
   gataacagat gagttaa 17
<210> 1152
   <211> 17
   <212> DNA
   <213> Artificial sequence
<220>
   <223> Synthetic oligonucleotide
<400> 1152
   ggataacaga tgagtta 17
<210> 1153
   <211> 17
   <212> DNA
   <213> Artificial sequence
<220>
   <223> Synthetic oligonucleotide
<400> 1153
   aggataacag atgagtt 17
<210> 1154
   <211> 17
   <212> DNA
   <213> Artificial sequence
<220>
   <223> Synthetic oligonucleotide
<400> 1154
   caggataaca gatgagt 17
<210> 1155
   <211> 17
   <212> DNA
   <213> Artificial sequence
<220>
   <223> Synthetic oligonucleotide
<400> 1155
   gcaggataac agatgag 17
<210> 1156
   <211> 17
   <212> DNA
   <213> Artificial sequence
<220>
   <223> Synthetic oligonucleotide
<400> 1156
   agcaggataa cagatga 17
<210> 1157
   <211> 17
   <212> DNA
   <213> Artificial sequence
<220>
   <223> Synthetic oligonucleotide
<400> 1157
   tagcaggata acagatg 17
<210> 1158
   <211> 17
   <212> DNA
   <213> Artificial sequence
<220>
   <223> Synthetic oligonucleotide
<400> 1158
   ctagcaggat aacagat 17
<210> 1159
   <211> 17
   <212> DNA
   <213> Artificial sequence
<220>
   <223> Synthetic oligonucleotide
<400> 1159
   gctagcagga taacaga 17
<210> 1160
   <211> 17
   <212> DNA
   <213> Artificial sequence
<220>
   <223> Synthetic oligonucleotide
<400> 1160
   agctagcagg ataacag 17
<210> 1161
   <211> 17
   <212> DNA
   <213> Artificial sequence
<220>
   <223> Synthetic oligonucleotide
<400> 1161
   cagctagcag gataaca 17
<210> 1162
   <211> 17
   <212> DNA
   <213> Artificial sequence
<220>
   <223> Synthetic oligonucleotide
<400> 1162
   acagctagca ggataac 17
<210> 1163
   <211> 17
   <212> DNA
   <213> Artificial sequence
<220>
   <223> Synthetic oligonucleotide
<400> 1163
   tacagctagc aggataa 17
<210> 1164
   <211> 17
   <212> DNA
   <213> Artificial sequence
<220>
   <223> Synthetic oligonucleotide
<400> 1164
   ctacagctag caggata 17
<210> 1165
   <211> 17
   <212> DNA
   <213> Artificial sequence
<220>
   <223> Synthetic oligonucleotide
<400> 1165
   tctacagcta gcaggat 17
<210> 1166
   <211> 17
   <212> DNA
   <213> Artificial sequence
<220>
   <223> Synthetic oligonucleotide
<400> 1166
   ttctacagct agcagga 17
<210> 1167
   <211> 17
   <212> DNA
   <213> Artificial sequence
<220>
   <223> Synthetic oligonucleotide
<400> 1167
   tttctacagc tagcagg 17
<210> 1168
   <211> 17
   <212> DNA
   <213> Artificial sequence
<220>
   <223> Synthetic oligonucleotide
<400> 1168
   atttctacag ctagcag 17
<210> 1169
   <211> 17
   <212> DNA
   <213> Artificial sequence
<220>
   <223> Synthetic oligonucleotide
<400> 1169
   catttctaca gctagca 17
<210> 1170
   <211> 17
   <212> DNA
   <213> Artificial sequence
<220>
   <223> Synthetic oligonucleotide
<400> 1170
   atacatttct acagcta 17
<210> 1171
   <211> 17
   <212> DNA
   <213> Artificial sequence
<220>
   <223> Synthetic oligonucleotide
<400> 1171
   gatacatttc tacagct 17
<210> 1172
   <211> 17
   <212> DNA
   <213> Artificial sequence
<220>
   <223> Synthetic oligonucleotide
<400> 1172
   gtgtttaatg tttatca 17
<210> 1173
   <211> 17
   <212> DNA
   <213> Artificial sequence
<220>
   <223> Synthetic oligonucleotide
<400> 1173
   agtgtttaat gtttatc 17
<210> 1174
   <211> 17
   <212> DNA
   <213> Artificial sequence
<220>
   <223> Synthetic oligonucleotide
<400> 1174
   cagtgtttaa tgtttat 17
<210> 1175
   <211> 17
   <212> DNA
   <213> Artificial sequence
<220>
   <223> Synthetic oligonucleotide
<400> 1175
   acagtgttta atgttta 17
<210> 1176
   <211> 17
   <212> DNA
   <213> Artificial sequence
<220>
   <223> Synthetic oligonucleotide
<400> 1176
   tacagtgttt aatgttt 17
<210> 1177
   <211> 17
   <212> DNA
   <213> Artificial sequence
<220>
   <223> Synthetic oligonucleotide
<400> 1177
   ttacagtgtt taatgtt 17
<210> 1178
   <211> 17
   <212> DNA
   <213> Artificial sequence
<220>
   <223> Synthetic oligonucleotide
<400> 1178
   attacagtgt ttaatgt 17
<210> 1179
   <211> 17
   <212> DNA
   <213> Artificial sequence
<220>
   <223> Synthetic oligonucleotide
<400> 1179
   gattacagtg tttaatg 17
<210> 1180
   <211> 17
   <212> DNA
   <213> Artificial sequence
<220>
   <223> Synthetic oligonucleotide
<400> 1180
   agattacagt gtttaat 17
<210> 1181
   <211> 17
   <212> DNA
   <213> Artificial sequence
<220>
   <223> Synthetic oligonucleotide
<400> 1181
   aagattacag tgtttaa 17
<210> 1182
   <211> 17
   <212> DNA
   <213> Artificial sequence
<220>
   <223> Synthetic oligonucleotide
<400> 1182
   taagattaca gtgttta 17
<210> 1183
   <211> 17
   <212> DNA
   <213> Artificial sequence
<220>
   <223> Synthetic oligonucleotide
<400> 1183
   ttaagattac agtgttt 17
<210> 1184
   <211> 17
   <212> DNA
   <213> Artificial sequence
<220>
   <223> Synthetic oligonucleotide
<400> 1184
   atcttccaag tgatcat 17
<210> 1185
   <211> 17
   <212> DNA
   <213> Artificial sequence
<220>
   <223> Synthetic oligonucleotide
<400> 1185
   aatcttccaa gtgatca 17
<210> 1186
   <211> 17
   <212> DNA
   <213> Artificial sequence
<220>
   <223> Synthetic oligonucleotide
<400> 1186
   aaatcttcca agtgatc 17
<210> 1187
   <211> 17
   <212> DNA
   <213> Artificial sequence
<220>
   <223> Synthetic oligonucleotide
<400> 1187
   caaatcttcc aagtgat 17
<210> 1188
   <211> 17
   <212> DNA
   <213> Artificial sequence
<220>
   <223> Synthetic oligonucleotide
<400> 1188
   tacaaatctt ccaagtg 17
<210> 1189
   <211> 17
   <212> DNA
   <213> Artificial sequence
<220>
   <223> Synthetic oligonucleotide
<400> 1189
   atacaaatct tccaagt 17
<210> 1190
   <211> 17
   <212> DNA
   <213> Artificial sequence
<220>
   <223> Synthetic oligonucleotide
<400> 1190
   tatacaaatc ttccaag 17
<210> 1191
   <211> 17
   <212> DNA
   <213> Artificial sequence
<220>
   <223> Synthetic oligonucleotide
<400> 1191
   ctatacaaat cttccaa 17
<210> 1192
   <211> 17
   <212> DNA
   <213> Artificial sequence
<220>
   <223> Synthetic oligonucleotide
<400> 1192
   actatacaaa tcttcca 17
<210> 1193
   <211> 17
   <212> DNA
   <213> Artificial sequence
<220>
   <223> Synthetic oligonucleotide
<400> 1193
   aactatacaa atcttcc 17
<210> 1194
   <211> 17
   <212> DNA
   <213> Artificial sequence
<220>
   <223> Synthetic oligonucleotide
<400> 1194
   aaactataca aatcttc 17
<210> 1195
   <211> 17
   <212> DNA
   <213> Artificial sequence
<220>
   <223> Synthetic oligonucleotide
<400> 1195
   ataaaactat acaaatc 17
<210> 1196
   <211> 17
   <212> DNA
   <213> Artificial sequence
<220>
   <223> Synthetic oligonucleotide
<400> 1196
   gttttataaa actatac 17
<210> 1197
   <211> 17
   <212> DNA
   <213> Artificial sequence
<220>
   <223> Synthetic oligonucleotide
<400> 1197
   gagttttata aaactat 17
<210> 1198
   <211> 17
   <212> DNA
   <213> Artificial sequence
<220>
   <223> Synthetic oligonucleotide
<400> 1198
   attgaaacag acatttt 17
<210> 1199
   <211> 17
   <212> DNA
   <213> Artificial sequence
<220>
   <223> Synthetic oligonucleotide
<400> 1199
   cattgaaaca gacattt 17
<210> 1200
   <211> 17
   <212> DNA
   <213> Artificial sequence
<220>
   <223> Synthetic oligonucleotide
<400> 1200
   tcattgaaac agacatt 17
<210> 1201
   <211> 17
   <212> DNA
   <213> Artificial sequence
<220>
   <223> Synthetic oligonucleotide
<400> 1201
   gtcattgaaa cagacat 17
<210> 1202
   <211> 17
   <212> DNA
   <213> Artificial sequence
<220>
   <223> Synthetic oligonucleotide
<400> 1202
   ggtcattgaa acagaca 17
<210> 1203
   <211> 17
   <212> DNA
   <213> Artificial sequence
<220>
   <223> Synthetic oligonucleotide
<400> 1203
   aggtcattga aacagac 17
<210> 1204
   <211> 17
   <212> DNA
   <213> Artificial sequence
<220>
   <223> Synthetic oligonucleotide
<400> 1204
   caggtcattg aaacaga 17
<210> 1205
   <211> 17
   <212> DNA
   <213> Artificial sequence
<220>
   <223> Synthetic oligonucleotide
<400> 1205
   acaggtcatt gaaacag 17
<210> 1206
   <211> 17
   <212> DNA
   <213> Artificial sequence
<220>
   <223> Synthetic oligonucleotide
<400> 1206
   tacaggtcat tgaaaca 17
<210> 1207
   <211> 17
   <212> DNA
   <213> Artificial sequence
<220>
   <223> Synthetic oligonucleotide
<400> 1207
   atacaggtca ttgaaac 17
<210> 1208
   <211> 17
   <212> DNA
   <213> Artificial sequence
<220>
   <223> Synthetic oligonucleotide
<400> 1208
   aatacaggtc attgaaa 17
<210> 1209
   <211> 17
   <212> DNA
   <213> Artificial sequence
<220>
   <223> Synthetic oligonucleotide
<400> 1209
   aaatacaggt cattgaa 17
<210> 1210
   <211> 17
   <212> DNA
   <213> Artificial sequence
<220>
   <223> Synthetic oligonucleotide
<400> 1210
   aaaatacagg tcattga 17
<210> 1211
   <211> 17
   <212> DNA
   <213> Artificial sequence
<220>
   <223> Synthetic oligonucleotide
<400> 1211
   caaaatacag gtcattg 17
<210> 1212
   <211> 17
   <212> DNA
   <213> Artificial sequence
<220>
   <223> Synthetic oligonucleotide
<400> 1212
   gcaaaataca ggtcatt 17
<210> 1213
   <211> 17
   <212> DNA
   <213> Artificial sequence
<220>
   <223> Synthetic oligonucleotide
<400> 1213
   ggcaaaatac aggtcat 17
<210> 1214
   <211> 17
   <212> DNA
   <213> Artificial sequence
<220>
   <223> Synthetic oligonucleotide
<400> 1214
   tggcaaaata caggtca 17
<210> 1215
   <211> 17
   <212> DNA
   <213> Artificial sequence
<220>
   <223> Synthetic oligonucleotide
<400> 1215
   ctggcaaaat acaggtc 17
<210> 1216
   <211> 17
   <212> DNA
   <213> Artificial sequence
<220>
   <223> Synthetic oligonucleotide
<400> 1216
   tctggcaaaa tacaggt 17
<210> 1217
   <211> 17
   <212> DNA
   <213> Artificial sequence
<220>
   <223> Synthetic oligonucleotide
<400> 1217
   gtctggcaaa atacagg 17
<210> 1218
   <211> 17
   <212> DNA
   <213> Artificial sequence
<220>
   <223> Synthetic oligonucleotide
<400> 1218
   agtctggcaa aatacag 17
<210> 1219
   <211> 17
   <212> DNA
   <213> Artificial sequence
<220>
   <223> Synthetic oligonucleotide
<400> 1219
   aagtctggca aaataca 17
<210> 1220
   <211> 17
   <212> DNA
   <213> Artificial sequence
<220>
   <223> Synthetic oligonucleotide
<400> 1220
   taagtctggc aaaatac 17
<210> 1221
   <211> 17
   <212> DNA
   <213> Artificial sequence
<220>
   <223> Synthetic oligonucleotide
<400> 1221
   ttaagtctgg caaaata 17
<210> 1222
   <211> 17
   <212> DNA
   <213> Artificial sequence
<220>
   <223> Synthetic oligonucleotide
<400> 1222
   aatacccatc tgtgatt 17
<210> 1223
   <211> 17
   <212> DNA
   <213> Artificial sequence
<220>
   <223> Synthetic oligonucleotide
<400> 1223
   taatacccat ctgtgat 17
<210> 1224
   <211> 17
   <212> DNA
   <213> Artificial sequence
<220>
   <223> Synthetic oligonucleotide
<400> 1224
   ttaataccca tctgtga 17
<210> 1225
   <211> 17
   <212> DNA
   <213> Artificial sequence
<220>
   <223> Synthetic oligonucleotide
<400> 1225
   tttaataccc atctgtg 17
<210> 1226
   <211> 17
   <212> DNA
   <213> Artificial sequence
<220>
   <223> Synthetic oligonucleotide
<400> 1226
   gtttaatacc catctgt 17
<210> 1227
   <211> 17
   <212> DNA
   <213> Artificial sequence
<220>
   <223> Synthetic oligonucleotide
<400> 1227
   agtttaatac ccatctg 17
<210> 1228
   <211> 17
   <212> DNA
   <213> Artificial sequence
<220>
   <223> Synthetic oligonucleotide
<400> 1228
   aagtttaata cccatct 17
<210> 1229
   <211> 17
   <212> DNA
   <213> Artificial sequence
<220>
   <223> Synthetic oligonucleotide
<400> 1229
   caagtttaat acccatc 17
<210> 1230
   <211> 17
   <212> DNA
   <213> Artificial sequence
<220>
   <223> Synthetic oligonucleotide
<400> 1230
   acaagtttaa tacccat 17
<210> 1231
   <211> 17
   <212> DNA
   <213> Artificial sequence
<220>
   <223> Synthetic oligonucleotide
<400> 1231
   gacaagttta ataccca 17
<210> 1232
   <211> 17
   <212> DNA
   <213> Artificial sequence
<220>
   <223> Synthetic oligonucleotide
<400> 1232
   tgacaagttt aataccc 17
<210> 1233
   <211> 17
   <212> DNA
   <213> Artificial sequence
<220>
   <223> Synthetic oligonucleotide
<400> 1233
   ctgacaagtt taatacc 17
<210> 1234
   <211> 17
   <212> DNA
   <213> Artificial sequence
<220>
   <223> Synthetic oligonucleotide
<400> 1234
   tctgacaagt ttaatac 17
<210> 1235
   <211> 17
   <212> DNA
   <213> Artificial sequence
<220>
   <223> Synthetic oligonucleotide
<400> 1235
   ttctgacaag tttaata 17
<210> 1236
   <211> 17
   <212> DNA
   <213> Artificial sequence
<220>
   <223> Synthetic oligonucleotide
<400> 1236
   attctgacaa gtttaat 17
<210> 1237
   <211> 17
   <212> DNA
   <213> Artificial sequence
<220>
   <223> Synthetic oligonucleotide
<400> 1237
   aattctgaca agtttaa 17
<210> 1238
   <211> 17
   <212> DNA
   <213> Artificial sequence
<220>
   <223> Synthetic oligonucleotide
<400> 1238
   aaattctgac aagttta 17
<210> 1239
   <211> 17
   <212> DNA
   <213> Artificial sequence
<220>
   <223> Synthetic oligonucleotide
<400> 1239
   gaaattctga caagttt 17
<210> 1240
   <211> 17
   <212> DNA
   <213> Artificial sequence
<220>
   <223> Synthetic oligonucleotide
<400> 1240
   agaaattctg acaagtt 17
<210> 1241
   <211> 17
   <212> DNA
   <213> Artificial sequence
<220>
   <223> Synthetic oligonucleotide
<400> 1241
   aagaaattct gacaagt 17
<210> 1242
   <211> 17
   <212> DNA
   <213> Artificial sequence
<220>
   <223> Synthetic oligonucleotide
<400> 1242
   ttcacaggct tgaatga 17
<210> 1243
   <211> 17
   <212> DNA
   <213> Artificial sequence
<220>
   <223> Synthetic oligonucleotide
<400> 1243
   attcacaggc ttgaatg 17
<210> 1244
   <211> 17
   <212> DNA
   <213> Artificial sequence
<220>
   <223> Synthetic oligonucleotide
<400> 1244
   tattcacagg cttgaat 17
<210> 1245
   <211> 17
   <212> DNA
   <213> Artificial sequence
<220>
   <223> Synthetic oligonucleotide
<400> 1245
   ttattcacag gcttgaa 17
<210> 1246
   <211> 17
   <212> DNA
   <213> Artificial sequence
<220>
   <223> Synthetic oligonucleotide
<400> 1246
   tttattcaca ggcttga 17
<210> 1247
   <211> 17
   <212> DNA
   <213> Artificial sequence
<220>
   <223> Synthetic oligonucleotide
<400> 1247
   ttttattcac aggcttg 17
<210> 1248
   <211> 17
   <212> DNA
   <213> Artificial sequence
<220>
   <223> Synthetic oligonucleotide
<400> 1248
   tttttattca caggctt 17
<210> 1249
   <211> 17
   <212> DNA
   <213> Artificial sequence
<220>
   <223> Synthetic oligonucleotide
<400> 1249
   gtttttattc acaggct 17
<210> 1250
   <211> 17
   <212> DNA
   <213> Artificial sequence
<220>
   <223> Synthetic oligonucleotide
<400> 1250
   ggtttttatt cacaggc 17
<210> 1251
   <211> 17
   <212> DNA
   <213> Artificial sequence
<220>
   <223> Synthetic oligonucleotide
<400> 1251
   gggtttttat tcacagg 17
<210> 1252
   <211> 17
   <212> DNA
   <213> Artificial sequence
<220>
   <223> Synthetic oligonucleotide
<400> 1252
   agggttttta ttcacag 17
<210> 1253
   <211> 17
   <212> DNA
   <213> Artificial sequence
<220>
   <223> Synthetic oligonucleotide
<400> 1253
   cagggttttt attcaca 17
<210> 1254
   <211> 17
   <212> DNA
   <213> Artificial sequence
<220>
   <223> Synthetic oligonucleotide
<400> 1254
   acagggtttt tattcac 17
<210> 1255
   <211> 17
   <212> DNA
   <213> Artificial sequence
<220>
   <223> Synthetic oligonucleotide
<400> 1255
   tacagggttt ttattca 17
<210> 1256
   <211> 17
   <212> DNA
   <213> Artificial sequence
<220>
   <223> Synthetic oligonucleotide
<400> 1256
   atacagggtt tttattc 17
<210> 1257
   <211> 17
   <212> DNA
   <213> Artificial sequence
<220>
   <223> Synthetic oligonucleotide
<400> 1257
   catacagggt ttttatt 17
<210> 1258
   <211> 17
   <212> DNA
   <213> Artificial sequence
<220>
   <223> Synthetic oligonucleotide
<400> 1258
   ccatacaggg tttttat 17
<210> 1259
   <211> 17
   <212> DNA
   <213> Artificial sequence
<220>
   <223> Synthetic oligonucleotide
<400> 1259
   gccatacagg gttttta 17
<210> 1260
   <211> 17
   <212> DNA
   <213> Artificial sequence
<220>
   <223> Synthetic oligonucleotide
<400> 1260
   tgccatacag ggttttt 17
<210> 1261
   <211> 17
   <212> DNA
   <213> Artificial sequence
<220>
   <223> Synthetic oligonucleotide
<400> 1261
   gtgccataca gggtttt 17
<210> 1262
   <211> 17
   <212> DNA
   <213> Artificial sequence
<220>
   <223> Synthetic oligonucleotide
<400> 1262
   agtgccatac agggttt 17
<210> 1263
   <211> 17
   <212> DNA
   <213> Artificial sequence
<220>
   <223> Synthetic oligonucleotide
<400> 1263
   ttggattctt ttaatag 17
<210> 1264
   <211> 17
   <212> DNA
   <213> Artificial sequence
<220>
   <223> Synthetic oligonucleotide
<400> 1264
   ttttagtttg aatttgg 17
<210> 1265
   <211> 20
   <212> DNA
   <213> Artificial sequence
<220>
   <223> Synthetic oligonucleotide
<400> 1265
   ctcgcccact ctggccccaa 20
<210> 1266
   <211> 20
   <212> DNA
   <213> Artificial sequence
<220>
   <223> Synthetic oligonucleotide
<400> 1266
   gcctcgccca ctctggcccc 20
<210> 1267
   <211> 20
   <212> DNA
   <213> Artificial sequence
<220>
   <223> Synthetic oligonucleotide
<400> 1267
   gcgcctcgcc cactctggcc 20
<210> 1268
   <211> 20
   <212> DNA
   <213> Artificial sequence
<220>
   <223> Synthetic oligonucleotide
<400> 1268
   ccgcgcctcg cccactctgg 20
<210> 1269
   <211> 20
   <212> DNA
   <213> Artificial sequence
<220>
   <223> Synthetic oligonucleotide
<400> 1269
   ctccgcgcct cgcccactct 20
<210> 1270
   <211> 20
   <212> DNA
   <213> Artificial sequence
<220>
   <223> Synthetic oligonucleotide
<400> 1270
   acctccgcgc ctcgcccact 20
<210> 1271
   <211> 20
   <212> DNA
   <213> Artificial sequence
<220>
   <223> Synthetic oligonucleotide
<400> 1271
   agacctccgc gcctcgccca 20
<210> 1272
   <211> 20
   <212> DNA
   <213> Artificial sequence
<220>
   <223> Synthetic oligonucleotide
<400> 1272
   ccagacctcc gcgcctcgcc 20
<210> 1273
   <211> 20
   <212> DNA
   <213> Artificial sequence
<220>
   <223> Synthetic oligonucleotide
<400> 1273
   ggccagacct ccgcgcctcg 20
<210> 1274
   <211> 20
   <212> DNA
   <213> Artificial sequence
<220>
   <223> Synthetic oligonucleotide
<400> 1274
   tataggccag acctccgcgc 20
<210> 1275
   <211> 20
   <212> DNA
   <213> Artificial sequence
<220>
   <223> Synthetic oligonucleotide
<400> 1275
   tttataggcc agacctccgc 20
<210> 1276
   <211> 20
   <212> DNA
   <213> Artificial sequence
<220>
   <223> Synthetic oligonucleotide
<400> 1276
   gactacttta taggccagac 20
<210> 1277
   <211> 20
   <212> DNA
   <213> Artificial sequence
<220>
   <223> Synthetic oligonucleotide
<400> 1277
   gcgactactt tataggccag 20
<210> 1278
   <211> 20
   <212> DNA
   <213> Artificial sequence
<220>
   <223> Synthetic oligonucleotide
<400> 1278
   ctccgcgact actttatagg 20
<210> 1279
   <211> 20
   <212> DNA
   <213> Artificial sequence
<220>
   <223> Synthetic oligonucleotide
<400> 1279
   gtctccgcga ctactttata 20
<210> 1280
   <211> 20
   <212> DNA
   <213> Artificial sequence
<220>
   <223> Synthetic oligonucleotide
<400> 1280
   ccgtctccgc gactacttta 20
<210> 1281
   <211> 20
   <212> DNA
   <213> Artificial sequence
<220>
   <223> Synthetic oligonucleotide
<400> 1281
   ccccgtctcc gcgactactt 20
<210> 1282
   <211> 20
   <212> DNA
   <213> Artificial sequence
<220>
   <223> Synthetic oligonucleotide
<400> 1282
   caccccgtct ccgcgactac 20
<210> 1283
   <211> 20
   <212> DNA
   <213> Artificial sequence
<220>
   <223> Synthetic oligonucleotide
<400> 1283
   agcaccccgt ctccgcgact 20
<210> 1284
   <211> 20
   <212> DNA
   <213> Artificial sequence
<220>
   <223> Synthetic oligonucleotide
<400> 1284
   ccagcacccc gtctccgcga 20
<210> 1285
   <211> 20
   <212> DNA
   <213> Artificial sequence
<220>
   <223> Synthetic oligonucleotide
<400> 1285
   aaccagcacc ccgtctccgc 20
<210> 1286
   <211> 20
   <212> DNA
   <213> Artificial sequence
<220>
   <223> Synthetic oligonucleotide
<400> 1286
   caaaccagca ccccgtctcc 20
<210> 1287
   <211> 20
   <212> DNA
   <213> Artificial sequence
<220>
   <223> Synthetic oligonucleotide
<400> 1287
   cgcaaaccag caccccgtct 20
<210> 1288
   <211> 20
   <212> DNA
   <213> Artificial sequence
<220>
   <223> Synthetic oligonucleotide
<400> 1288
   acgacgcaaa ccagcacccc 20
<210> 1289
   <211> 20
   <212> DNA
   <213> Artificial sequence
<220>
   <223> Synthetic oligonucleotide
<400> 1289
   ctacgacgca aaccagcacc 20
<210> 1290
   <211> 20
   <212> DNA
   <213> Artificial sequence
<220>
   <223> Synthetic oligonucleotide
<400> 1290
   gactacgacg caaaccagca 20
<210> 1291
   <211> 20
   <212> DNA
   <213> Artificial sequence
<220>
   <223> Synthetic oligonucleotide
<400> 1291
   gagactacga cgcaaaccag 20
<210> 1292
   <211> 20
   <212> DNA
   <213> Artificial sequence
<220>
   <223> Synthetic oligonucleotide
<400> 1292
   aggagactac gacgcaaacc 20
<210> 1293
   <211> 20
   <212> DNA
   <213> Artificial sequence
<220>
   <223> Synthetic oligonucleotide
<400> 1293
   gcaggagact acgacgcaaa 20
<210> 1294
   <211> 20
   <212> DNA
   <213> Artificial sequence
<220>
   <223> Synthetic oligonucleotide
<400> 1294
   ctgcaggaga ctacgacgca 20
<210> 1295
   <211> 20
   <212> DNA
   <213> Artificial sequence
<220>
   <223> Synthetic oligonucleotide
<400> 1295
   cgctgcagga gactacgacg 20
<210> 1296
   <211> 20
   <212> DNA
   <213> Artificial sequence
<220>
   <223> Synthetic oligonucleotide
<400> 1296
   tgcaacggaa accccagacg 20
<210> 1297
   <211> 20
   <212> DNA
   <213> Artificial sequence
<220>
   <223> Synthetic oligonucleotide
<400> 1297
   actgcaacgg aaaccccaga 20
<210> 1298
   <211> 20
   <212> DNA
   <213> Artificial sequence
<220>
   <223> Synthetic oligonucleotide
<400> 1298
   gaggactgca acggaaaccc 20
<210> 1299
   <211> 20
   <212> DNA
   <213> Artificial sequence
<220>
   <223> Synthetic oligonucleotide
<400> 1299
   ccgaggactg caacggaaac 20
<210> 1300
   <211> 20
   <212> DNA
   <213> Artificial sequence
<220>
   <223> Synthetic oligonucleotide
<400> 1300
   ttccgaggac tgcaacggaa 20
<210> 1301
   <211> 20
   <212> DNA
   <213> Artificial sequence
<220>
   <223> Synthetic oligonucleotide
<400> 1301
   ctggttccga ggactgcaac 20
<210> 1302
   <211> 20
   <212> DNA
   <213> Artificial sequence
<220>
   <223> Synthetic oligonucleotide
<400> 1302
   ggtcctggtt ccgaggactg 20
<210> 1303
   <211> 20
   <212> DNA
   <213> Artificial sequence
<220>
   <223> Synthetic oligonucleotide
<400> 1303
   ccgaggtcct ggttccgagg 20
<210> 1304
   <211> 20
   <212> DNA
   <213> Artificial sequence
<220>
   <223> Synthetic oligonucleotide
<400> 1304
   cgccgaggtc ctggttccga 20
<210> 1305
   <211> 20
   <212> DNA
   <213> Artificial sequence
<220>
   <223> Synthetic oligonucleotide
<400> 1305
   cacgccgagg tcctggttcc 20
<210> 1306
   <211> 20
   <212> DNA
   <213> Artificial sequence
<220>
   <223> Synthetic oligonucleotide
<400> 1306
   gccacgccga ggtcctggtt 20
<210> 1307
   <211> 20
   <212> DNA
   <213> Artificial sequence
<220>
   <223> Synthetic oligonucleotide
<400> 1307
   ctaggccacg ccgaggtcct 20
<210> 1308
   <211> 20
   <212> DNA
   <213> Artificial sequence
<220>
   <223> Synthetic oligonucleotide
<400> 1308
   cgctaggcca cgccgaggtc 20
<210> 1309
   <211> 20
   <212> DNA
   <213> Artificial sequence
<220>
   <223> Synthetic oligonucleotide
<400> 1309
   ctcgctaggc cacgccgagg 20
<210> 1310
   <211> 20
   <212> DNA
   <213> Artificial sequence
<220>
   <223> Synthetic oligonucleotide
<400> 1310
   aactcgctag gccacgccga 20
<210> 1311
   <211> 20
   <212> DNA
   <213> Artificial sequence
<220>
   <223> Synthetic oligonucleotide
<400> 1311
   ataactcgct aggccacgcc 20
<210> 1312
   <211> 20
   <212> DNA
   <213> Artificial sequence
<220>
   <223> Synthetic oligonucleotide
<400> 1312
   ccataactcg ctaggccacg 20
<210> 1313
   <211> 20
   <212> DNA
   <213> Artificial sequence
<220>
   <223> Synthetic oligonucleotide
<400> 1313
   cgccataact cgctaggcca 20
<210> 1314
   <211> 20
   <212> DNA
   <213> Artificial sequence
<220>
   <223> Synthetic oligonucleotide
<400> 1314
   tgggccgtcg cccttcagca 20
<210> 1315
   <211> 20
   <212> DNA
   <213> Artificial sequence
<220>
   <223> Synthetic oligonucleotide
<400> 1315
   actgggccgt cgcccttcag 20
<210> 1316
   <211> 20
   <212> DNA
   <213> Artificial sequence
<220>
   <223> Synthetic oligonucleotide
<400> 1316
   gcactgggcc gtcgcccttc 20
<210> 1317
   <211> 20
   <212> DNA
   <213> Artificial sequence
<220>
   <223> Synthetic oligonucleotide
<400> 1317
   ctgcactggg ccgtcgccct 20
<210> 1318
   <211> 20
   <212> DNA
   <213> Artificial sequence
<220>
   <223> Synthetic oligonucleotide
<400> 1318
   tgccctgcac tgggccgtcg 20
<210> 1319
   <211> 20
   <212> DNA
   <213> Artificial sequence
<220>
   <223> Synthetic oligonucleotide
<400> 1319
   gatgccctgc actgggccgt 20
<210> 1320
   <211> 20
   <212> DNA
   <213> Artificial sequence
<220>
   <223> Synthetic oligonucleotide
<400> 1320
   atgatgccct gcactgggcc 20
<210> 1321
   <211> 20
   <212> DNA
   <213> Artificial sequence
<220>
   <223> Synthetic oligonucleotide
<400> 1321
   attgatgatg ccctgcactg 20
<210> 1322
   <211> 20
   <212> DNA
   <213> Artificial sequence
<220>
   <223> Synthetic oligonucleotide
<400> 1322
   aaattgatga tgccctgcac 20
<210> 1323
   <211> 20
   <212> DNA
   <213> Artificial sequence
<220>
   <223> Synthetic oligonucleotide
<400> 1323
   cgaaattgat gatgccctgc 20
<210> 1324
   <211> 20
   <212> DNA
   <213> Artificial sequence
<220>
   <223> Synthetic oligonucleotide
<400> 1324
   ctcgaaattg atgatgccct 20
<210> 1325
   <211> 20
   <212> DNA
   <213> Artificial sequence
<220>
   <223> Synthetic oligonucleotide
<400> 1325
   tgctcgaaat tgatgatgcc 20
<210> 1326
   <211> 20
   <212> DNA
   <213> Artificial sequence
<220>
   <223> Synthetic oligonucleotide
<400> 1326
   tctgctcgaa attgatgatg 20
<210> 1327
   <211> 20
   <212> DNA
   <213> Artificial sequence
<220>
   <223> Synthetic oligonucleotide
<400> 1327
   cttctgctcg aaattgatga 20
<210> 1328
   <211> 20
   <212> DNA
   <213> Artificial sequence
<220>
   <223> Synthetic oligonucleotide
<400> 1328
   tttaatgctt ccccacacct 20
<210> 1329
   <211> 20
   <212> DNA
   <213> Artificial sequence
<220>
   <223> Synthetic oligonucleotide
<400> 1329
   cctttaatgc ttccccacac 20
<210> 1330
   <211> 20
   <212> DNA
   <213> Artificial sequence
<220>
   <223> Synthetic oligonucleotide
<400> 1330
   gtcctttaat gcttccccac 20
<210> 1331
   <211> 17
   <212> DNA
   <213> Artificial sequence
<220>
   <223> Synthetic oligonucleotide
<400> 1331
   cccttcagca cgcacac 17
<210> 1332
   <211> 17
   <212> DNA
   <213> Artificial sequence
<220>
   <223> Synthetic oligonucleotide
<400> 1332
   gcccttcagc acgcaca 17
<210> 1333
   <211> 17
   <212> DNA
   <213> Artificial sequence
<220>
   <223> Synthetic oligonucleotide
<400> 1333
   cgcccttcag cacgcac 17
<210> 1334
   <211> 17
   <212> DNA
   <213> Artificial sequence
<220>
   <223> Synthetic oligonucleotide
<400> 1334
   caccacaagc caaacga 17
<210> 1335
   <211> 17
   <212> DNA
   <213> Artificial sequence
<220>
   <223> Synthetic oligonucleotide
<400> 1335
   acaccacaag ccaaacg 17
<210> 1336
   <211> 17
   <212> DNA
   <213> Artificial sequence
<220>
   <223> Synthetic oligonucleotide
<400> 1336
   tacaccacaa gccaaac 17
<210> 1337
   <211> 17
   <212> DNA
   <213> Artificial sequence
<220>
   <223> Synthetic oligonucleotide
<400> 1337
   ttacaccaca agccaaa 17
<210> 1338
   <211> 17
   <212> DNA
   <213> Artificial sequence
<220>
   <223> Synthetic oligonucleotide
<400> 1338
   attacaccac aagccaa 17
<210> 1339
   <211> 17
   <212> DNA
   <213> Artificial sequence
<220>
   <223> Synthetic oligonucleotide
<400> 1339
   aacagatgag ttaaggg 17
<210> 1340
   <211> 17
   <212> DNA
   <213> Artificial sequence
<220>
   <223> Synthetic oligonucleotide
<400> 1340
   taacagatga gttaagg 17
<210> 1341
   <211> 17
   <212> DNA
   <213> Artificial sequence
<220>
   <223> Synthetic oligonucleotide
<400> 1341
   ataacagatg agttaag 17
<210> 1342
   <211> 17
   <212> DNA
   <213> Artificial sequence
<220>
   <223> Synthetic oligonucleotide
<400> 1342
   ggatacattt ctacagc 17
<210> 1343
   <211> 17
   <212> DNA
   <213> Artificial sequence
<220>
   <223> Synthetic oligonucleotide
<400> 1343
   aggatacatt tctacag 17
<210> 1344
   <211> 17
   <212> DNA
   <213> Artificial sequence
<220>
   <223> Synthetic oligonucleotide
<400> 1344
   caggatacat ttctaca 17
<210> 1345
   <211> 17
   <212> DNA
   <213> Artificial sequence
<220>
   <223> Synthetic oligonucleotide
<400> 1345
   ttatcaggat acatttc 17
<210> 1346
   <211> 17
   <212> DNA
   <213> Artificial sequence
<220>
   <223> Synthetic oligonucleotide
<400> 1346
   gtttatcagg atacatt 17
<210> 1347
   <211> 17
   <212> DNA
   <213> Artificial sequence
<220>
   <223> Synthetic oligonucleotide
<400> 1347
   tgtttatcag gatacat 17
<210> 1348
   <211> 17
   <212> DNA
   <213> Artificial sequence
<220>
   <223> Synthetic oligonucleotide
<400> 1348
   atgtttatca ggataca 17
<210> 1349
   <211> 17
   <212> DNA
   <213> Artificial sequence
<220>
   <223> Synthetic oligonucleotide
<400> 1349
   aatgtttatc aggatac 17
<210> 1350
   <211> 17
   <212> DNA
   <213> Artificial sequence
<220>
   <223> Synthetic oligonucleotide
<400> 1350
   taatgtttat caggata 17
<210> 1351
   <211> 17
   <212> DNA
   <213> Artificial sequence
<220>
   <223> Synthetic oligonucleotide
<400> 1351
   ttaatgttta tcaggat 17
<210> 1352
   <211> 17
   <212> DNA
   <213> Artificial sequence
<220>
   <223> Synthetic oligonucleotide
<400> 1352
   gtttaatgtt tatcagg 17
<210> 1353
   <211> 17
   <212> DNA
   <213> Artificial sequence
<220>
   <223> Synthetic oligonucleotide
<400> 1353
   cttttaagat tacagtg 17
<210> 1354
   <211> 17
   <212> DNA
   <213> Artificial sequence
<220>
   <223> Synthetic oligonucleotide
<400> 1354
   cacttttaag attacag 17
<210> 1355
   <211> 17
   <212> DNA
   <213> Artificial sequence
<220>
   <223> Synthetic oligonucleotide
<400> 1355
   tcacacaatt acacttt 17
<210> 1356
   <211> 17
   <212> DNA
   <213> Artificial sequence
<220>
   <223> Synthetic oligonucleotide
<400> 1356
   gtcacacaat tacactt 17
<210> 1357
   <211> 17
   <212> DNA
   <213> Artificial sequence
<220>
   <223> Synthetic oligonucleotide
<400> 1357
   aagtcacaca attacac 17
<210> 1358
   <211> 17
   <212> DNA
   <213> Artificial sequence
<220>
   <223> Synthetic oligonucleotide
<400> 1358
   aggtacttta aagcaac 17
<210> 1359
   <211> 17
   <212> DNA
   <213> Artificial sequence
<220>
   <223> Synthetic oligonucleotide
<400> 1359
   acaggtactt taaagca 17
<210> 1360
   <211> 17
   <212> DNA
   <213> Artificial sequence
<220>
   <223> Synthetic oligonucleotide
<400> 1360
   tacaggtact ttaaagc 17
<210> 1361
   <211> 17
   <212> DNA
   <213> Artificial sequence
<220>
   <223> Synthetic oligonucleotide
<400> 1361
   ctacaggtac tttaaag 17
<210> 1362
   <211> 17
   <212> DNA
   <213> Artificial sequence
<220>
   <223> Synthetic oligonucleotide
<400> 1362
   cactacaggt actttaa 17
<210> 1363
   <211> 17
   <212> DNA
   <213> Artificial sequence
<220>
   <223> Synthetic oligonucleotide
<400> 1363
   tcactacagg tacttta 17
<210> 1364
   <211> 17
   <212> DNA
   <213> Artificial sequence
<220>
   <223> Synthetic oligonucleotide
<400> 1364
   ctcactacag gtacttt 17
<210> 1365
   <211> 17
   <212> DNA
   <213> Artificial sequence
<220>
   <223> Synthetic oligonucleotide
<400> 1365
   tctcactaca ggtactt 17
<210> 1366
   <211> 17
   <212> DNA
   <213> Artificial sequence
<220>
   <223> Synthetic oligonucleotide
<400> 1366
   ttctcactac aggtact 17
<210> 1367
   <211> 17
   <212> DNA
   <213> Artificial sequence
<220>
   <223> Synthetic oligonucleotide
<400> 1367
   tttctcacta caggtac 17
<210> 1368
   <211> 17
   <212> DNA
   <213> Artificial sequence
<220>
   <223> Synthetic oligonucleotide
<400> 1368
   gtttctcact acaggta 17
<210> 1369
   <211> 17
   <212> DNA
   <213> Artificial sequence
<220>
   <223> Synthetic oligonucleotide
<400> 1369
   agtttctcac tacaggt 17
<210> 1370
   <211> 17
   <212> DNA
   <213> Artificial sequence
<220>
   <223> Synthetic oligonucleotide
<400> 1370
   cagtttctca ctacagg 17
<210> 1371
   <211> 17
   <212> DNA
   <213> Artificial sequence
<220>
   <223> Synthetic oligonucleotide
<400> 1371
   tcagtttctc actacag 17
<210> 1372
   <211> 17
   <212> DNA
   <213> Artificial sequence
<220>
   <223> Synthetic oligonucleotide
<400> 1372
   atcagtttct cactaca 17
<210> 1373
   <211> 17
   <212> DNA
   <213> Artificial sequence
<220>
   <223> Synthetic oligonucleotide
<400> 1373
   aatcagtttc tcactac 17
<210> 1374
   <211> 17
   <212> DNA
   <213> Artificial sequence
<220>
   <223> Synthetic oligonucleotide
<400> 1374
   gatcataaat cagtttc 17
<210> 1375
   <211> 17
   <212> DNA
   <213> Artificial sequence
<220>
   <223> Synthetic oligonucleotide
<400> 1375
   tgatcataaa tcagttt 17
<210> 1376
   <211> 17
   <212> DNA
   <213> Artificial sequence
<220>
   <223> Synthetic oligonucleotide
<400> 1376
   gtgatcataa atcagtt 17
<210> 1377
   <211> 17
   <212> DNA
   <213> Artificial sequence
<220>
   <223> Synthetic oligonucleotide
<400> 1377
   agtgatcata aatcagt 17
<210> 1378
   <211> 17
   <212> DNA
   <213> Artificial sequence
<220>
   <223> Synthetic oligonucleotide
<400> 1378
   caagtgatca taaatca 17
<210> 1379
   <211> 17
   <212> DNA
   <213> Artificial sequence
<220>
   <223> Synthetic oligonucleotide
<400> 1379
   ccaagtgatc ataaatc 17
<210> 1380
   <211> 17
   <212> DNA
   <213> Artificial sequence
<220>
   <223> Synthetic oligonucleotide
<400> 1380
   tccaagtgat cataaat 17
<210> 1381
   <211> 17
   <212> DNA
   <213> Artificial sequence
<220>
   <223> Synthetic oligonucleotide
<400> 1381
   cttccaagtg atcataa 17
<210> 1382
   <211> 17
   <212> DNA
   <213> Artificial sequence
<220>
   <223> Synthetic oligonucleotide
<400> 1382
   tcttccaagt gatcata 17
<210> 1383
   <211> 17
   <212> DNA
   <213> Artificial sequence
<220>
   <223> Synthetic oligonucleotide
<400> 1383
   agacatttta actgagt 17
<210> 1384
   <211> 17
   <212> DNA
   <213> Artificial sequence
<220>
   <223> Synthetic oligonucleotide
<400> 1384
   gatttaagtc tggcaaa 17
<210> 1385
   <211> 17
   <212> DNA
   <213> Artificial sequence
<220>
   <223> Synthetic oligonucleotide
<400> 1385
   tgatttaagt ctggcaa 17
<210> 1386
   <211> 17
   <212> DNA
   <213> Artificial sequence
<220>
   <223> Synthetic oligonucleotide
<400> 1386
   gtgatttaag tctggca 17
<210> 1387
   <211> 17
   <212> DNA
   <213> Artificial sequence
<220>
   <223> Synthetic oligonucleotide
<400> 1387
   tgtgatttaa gtctggc 17
<210> 1388
   <211> 17
   <212> DNA
   <213> Artificial sequence
<220>
   <223> Synthetic oligonucleotide
<400> 1388
   ctgtgattta agtctgg 17
<210> 1389
   <211> 17
   <212> DNA
   <213> Artificial sequence
<220>
   <223> Synthetic oligonucleotide
<400> 1389
   tctgtgattt aagtctg 17
<210> 1390
   <211> 17
   <212> DNA
   <213> Artificial sequence
<220>
   <223> Synthetic oligonucleotide
<400> 1390
   atctgtgatt taagtct 17
<210> 1391
   <211> 17
   <212> DNA
   <213> Artificial sequence
<220>
   <223> Synthetic oligonucleotide
<400> 1391
   catctgtgat ttaagtc 17
<210> 1392
   <211> 17
   <212> DNA
   <213> Artificial sequence
<220>
   <223> Synthetic oligonucleotide
<400> 1392
   ccatctgtga tttaagt 17
<210> 1393
   <211> 17
   <212> DNA
   <213> Artificial sequence
<220>
   <223> Synthetic oligonucleotide
<400> 1393
   cccatctgtg atttaag 17
<210> 1394
   <211> 17
   <212> DNA
   <213> Artificial sequence
<220>
   <223> Synthetic oligonucleotide
<400> 1394
   acccatctgt gatttaa 17
<210> 1395
   <211> 17
   <212> DNA
   <213> Artificial sequence
<220>
   <223> Synthetic oligonucleotide
<400> 1395
   tacccatctg tgattta 17
<210> 1396
   <211> 17
   <212> DNA
   <213> Artificial sequence
<220>
   <223> Synthetic oligonucleotide
<400> 1396
   gcttgaatga caaagaa 17
<210> 1397
   <211> 17
   <212> DNA
   <213> Artificial sequence
<220>
   <223> Synthetic oligonucleotide
<400> 1397
   ggcttgaatg acaaaga 17
<210> 1398
   <211> 17
   <212> DNA
   <213> Artificial sequence
<220>
   <223> Synthetic oligonucleotide
<400> 1398
   cacaggcttg aatgaca 17
<210> 1399
   <211> 17
   <212> DNA
   <213> Artificial sequence
<220>
   <223> Synthetic oligonucleotide
<400> 1399
   tcacaggctt gaatgac 17
<210> 1400
   <211> 17
   <212> DNA
   <213> Artificial sequence
<220>
   <223> Synthetic oligonucleotide
<400> 1400
   aagtgccata cagggtt 17
<210> 1401
   <211> 17
   <212> DNA
   <213> Artificial sequence
<220>
   <223> Synthetic oligonucleotide
<400> 1401
   taagtgccat acagggt 17
<210> 1402
   <211> 17
   <212> DNA
   <213> Artificial sequence
<220>
   <223> Synthetic oligonucleotide
<400> 1402
   ataagtgcca tacaggg 17
<210> 1403
   <211> 17
   <212> DNA
   <213> Artificial sequence
<220>
   <223> Synthetic oligonucleotide
<400> 1403
   aataagtgcc atacagg 17
<210> 1404
   <211> 17
   <212> DNA
   <213> Artificial sequence
<220>
   <223> Synthetic oligonucleotide
<400> 1404
   taataagtgc catacag 17
<210> 1405
   <211> 17
   <212> DNA
   <213> Artificial sequence
<220>
   <223> Synthetic oligonucleotide
<400> 1405
   ataataagtg ccataca 17
<210> 1406
   <211> 17
   <212> DNA
   <213> Artificial sequence
<220>
   <223> Synthetic oligonucleotide
<400> 1406
   cataataagt gccatac 17
<210> 1407
   <211> 17
   <212> DNA
   <213> Artificial sequence
<220>
   <223> Synthetic oligonucleotide
<400> 1407
   tcataataag tgccata 17
<210> 1408
   <211> 17
   <212> DNA
   <213> Artificial sequence
<220>
   <223> Synthetic oligonucleotide
<400> 1408
   ctcataataa gtgccat 17
<210> 1409
   <211> 17
   <212> DNA
   <213> Artificial sequence
<220>
   <223> Synthetic oligonucleotide
<400> 1409
   cctcataata agtgcca 17
<210> 1410
   <211> 17
   <212> DNA
   <213> Artificial sequence
<220>
   <223> Synthetic oligonucleotide
<400> 1410
   gcctcataat aagtgcc 17
<210> 1411
   <211> 17
   <212> DNA
   <213> Artificial sequence
<220>
   <223> Synthetic oligonucleotide
<400> 1411
   agcctcataa taagtgc 17
<210> 1412
   <211> 17
   <212> DNA
   <213> Artificial sequence
<220>
   <223> Synthetic oligonucleotide
<400> 1412
   tagcctcata ataagtg 17
<210> 1413
   <211> 17
   <212> DNA
   <213> Artificial sequence
<220>
   <223> Synthetic oligonucleotide
<400> 1413
   atagcctcat aataagt 17
<210> 1414
   <211> 17
   <212> DNA
   <213> Artificial sequence
<220>
   <223> Synthetic oligonucleotide
<400> 1414
   aatagcctca taataag 17
<210> 1415
   <211> 17
   <212> DNA
   <213> Artificial sequence
<220>
   <223> Synthetic oligonucleotide
<400> 1415
   cttttaatag cctcata 17
<210> 1416
   <211> 17
   <212> DNA
   <213> Artificial sequence
<220>
   <223> Synthetic oligonucleotide
<400> 1416
   tcttttaata gcctcat 17
<210> 1417
   <211> 17
   <212> DNA
   <213> Artificial sequence
<220>
   <223> Synthetic oligonucleotide
<400> 1417
   ggattctttt aatagcc 17
<210> 1418
   <211> 17
   <212> DNA
   <213> Artificial sequence
<220>
   <223> Synthetic oligonucleotide
<400> 1418
   ttagtttgaa tttggat 17
<210> 1419
   <211> 18
   <212> DNA
   <213> Artificial sequence
<220>
   <223> Synthetic oligonucleotide
<400> 1419
   gtcgcccttc agcacgca 18
<210> 1420
   <211> 16
   <212> DNA
   <213> Artificial sequence
<220>
   <223> Synthetic oligonucleotide
<400> 1420
   cccacacctt cactgg 16
<210> 1421
   <211> 18
   <212> DNA
   <213> Artificial sequence
<220>
   <223> Synthetic oligonucleotide
<400> 1421
   ttccccacac cttcactg 18
<210> 1422
   <211> 16
   <212> DNA
   <213> Artificial sequence
<220>
   <223> Synthetic oligonucleotide
<400> 1422
   ccccacacct tcactg 16
<210> 1423
   <211> 18
   <212> DNA
   <213> Artificial sequence
<220>
   <223> Synthetic oligonucleotide
<400> 1423
   cttccccaca ccttcact 18
<210> 1424
   <211> 16
   <212> DNA
   <213> Artificial sequence
<220>
   <223> Synthetic oligonucleotide
<400> 1424
   tccccacacc ttcact 16
<210> 1425
   <211> 18
   <212> DNA
   <213> Artificial sequence
<220>
   <223> Synthetic oligonucleotide
<400> 1425
   gcttccccac accttcac 18
<210> 1426
   <211> 16
   <212> DNA
   <213> Artificial sequence
<220>
   <223> Synthetic oligonucleotide
<400> 1426
   ttccccacac cttcac 16
<210> 1427
   <211> 16
   <212> DNA
   <213> Artificial sequence
<220>
   <223> Synthetic oligonucleotide
<400> 1427
   cttccccaca ccttca 16
<210> 1428
   <211> 19
   <212> DNA
   <213> Artificial sequence
<220>
   <223> Synthetic oligonucleotide
<400> 1428
   aggatacatt tctacagct 19
<210> 1429
   <211> 18
   <212> DNA
   <213> Artificial sequence
<220>
   <223> Synthetic oligonucleotide
<400> 1429
   atacatttct acagctag 18
<210> 1430
   <211> 18
   <212> DNA
   <213> Artificial sequence
<220>
   <223> Synthetic oligonucleotide
<400> 1430
   gatacatttc tacagcta 18
<210> 1431
   <211> 16
   <212> DNA
   <213> Artificial sequence
<220>
   <223> Synthetic oligonucleotide
<400> 1431
   tacatttcta cagcta 16
<210> 1432
   <211> 18
   <212> DNA
   <213> Artificial sequence
<220>
   <223> Synthetic oligonucleotide
<400> 1432
   ggatacattt ctacagct 18
<210> 1433
   <211> 16
   <212> DNA
   <213> Artificial sequence
<220>
   <223> Synthetic oligonucleotide
<400> 1433
   atacatttct acagct 16
<210> 1434
   <211> 18
   <212> DNA
   <213> Artificial sequence
<220>
   <223> Synthetic oligonucleotide
<400> 1434
   aggatacatt tctacagc 18
<210> 1435
   <211> 16
   <212> DNA
   <213> Artificial sequence
<220>
   <223> Synthetic oligonucleotide
<400> 1435
   gatacatttc tacagc 16
<210> 1436
   <211> 18
   <212> DNA
   <213> Artificial sequence
<220>
   <223> Synthetic oligonucleotide
<400> 1436
   caggatacat ttctacag 18
<210> 1437
   <211> 16
   <212> DNA
   <213> Artificial sequence
<220>
   <223> Synthetic oligonucleotide
<400> 1437
   atgtttatca ggatac 16
<210> 1438
   <211> 18
   <212> DNA
   <213> Artificial sequence
<220>
   <223> Synthetic oligonucleotide
<400> 1438
   ttaatgttta tcaggata 18
<210> 1439
   <211> 18
   <212> DNA
   <213> Artificial sequence
<220>
   <223> Synthetic oligonucleotide
<400> 1439
   tttaatgttt atcaggat 18
<210> 1440
   <211> 18
   <212> DNA
   <213> Artificial sequence
<220>
   <223> Synthetic oligonucleotide
<400> 1440
   gtttaatgtt tatcagga 18
<210> 1441
   <211> 16
   <212> DNA
   <213> Artificial sequence
<220>
   <223> Synthetic oligonucleotide
<400> 1441
   ttaatgttta tcagga 16
<210> 1442
   <211> 18
   <212> DNA
   <213> Artificial sequence
<220>
   <223> Synthetic oligonucleotide
<400> 1442
   tgtttaatgt ttatcagg 18
<210> 1443
   <211> 16
   <212> DNA
   <213> Artificial sequence
<220>
   <223> Synthetic oligonucleotide
<400> 1443
   tttaatgttt atcagg 16
<210> 1444
   <211> 18
   <212> DNA
   <213> Artificial sequence
<220>
   <223> Synthetic oligonucleotide
<400> 1444
   gtgtttaatg tttatcag 18
<210> 1445
   <211> 16
   <212> DNA
   <213> Artificial sequence
<220>
   <223> Synthetic oligonucleotide
<400> 1445
   gtttaatgtt tatcag 16
<210> 1446
   <211> 18
   <212> DNA
   <213> Artificial sequence
<220>
   <223> Synthetic oligonucleotide
<400> 1446
   agtgtttaat gtttatca 18
<210> 1447
   <211> 16
   <212> DNA
   <213> Artificial sequence
<220>
   <223> Synthetic oligonucleotide
<400> 1447
   tgtttaatgt ttatca 16
<210> 1448
   <211> 18
   <212> DNA
   <213> Artificial sequence
<220>
   <223> Synthetic oligonucleotide
<400> 1448
   cagtgtttaa tgtttatc 18
<210> 1449
   <211> 16
   <212> DNA
   <213> Artificial sequence
<220>
   <223> Synthetic oligonucleotide
<400> 1449
   gtgtttaatg tttatc 16
<210> 1450
   <211> 18
   <212> DNA
   <213> Artificial sequence
<220>
   <223> Synthetic oligonucleotide
<400> 1450
   acagtgttta atgtttat 18
<210> 1451
   <211> 18
   <212> DNA
   <213> Artificial sequence
<220>
   <223> Synthetic oligonucleotide
<400> 1451
   tacagtgttt aatgttta 18
<210> 1452
   <211> 16
   <212> DNA
   <213> Artificial sequence
<220>
   <223> Synthetic oligonucleotide
<400> 1452
   cagtgtttaa tgttta 16
<210> 1453
   <211> 18
   <212> DNA
   <213> Artificial sequence
<220>
   <223> Synthetic oligonucleotide
<400> 1453
   ttacagtgtt taatgttt 18
<210> 1454
   <211> 16
   <212> DNA
   <213> Artificial sequence
<220>
   <223> Synthetic oligonucleotide
<400> 1454
   acagtgttta atgttt 16
<210> 1455
   <211> 16
   <212> DNA
   <213> Artificial sequence
<220>
   <223> Synthetic oligonucleotide
<400> 1455
   ggatacattt ctacag 16
<210> 1456
   <211> 16
   <212> DNA
   <213> Artificial sequence
<220>
   <223> Synthetic oligonucleotide
<400> 1456
   taatgtttat caggat 16
<210> 1457
   <211> 16
   <212> DNA
   <213> Artificial sequence
<220>
   <223> Synthetic oligonucleotide
<400> 1457
   agtgtttaat gtttat 16
<210> 1458
   <211> 16
   <212> DNA
   <213> Artificial sequence
<220>
   <223> Synthetic oligonucleotide
<400> 1458
   catttctaca gctagc 16
<210> 1459
   <211> 16
   <212> DNA
   <213> Artificial sequence
<220>
   <223> Synthetic oligonucleotide
<400> 1459
   acatttctac agctag 16
<210> 1460
   <211> 16
   <212> DNA
   <213> Artificial sequence
<220>
   <223> Synthetic oligonucleotide
<400> 1460
   aatgtttatc aggata 16
<210> 1461
   <211> 19
   <212> DNA
   <213> Artificial sequence
<220>
   <223> Synthetic oligonucleotide
<400> 1461
   caggatacat ttctacagc 19